(19) Europäisches Patentamt
European Patent Office
Office européen des brevets

(11) EP 4 015 529 A1

(12) EUROPEAN PATENT APPLICATION

(43) Date of publication:
**22.06.2022 Bulletin 2022/25**

(21) Application number: **20215815.0**

(22) Date of filing: **18.12.2020**

(51) International Patent Classification (IPC):
***C07K 14/72*** *(2006.01)*

(52) Cooperative Patent Classification (CPC):
**C07K 14/4705; C07K 14/70571**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Vrije Universiteit Brussel**
**1050 Brussel (BE)**

(72) Inventors:
- **BALLET, Steven**
  **2222 Itegem (BE)**
- **MARTIN, Charlotte**
  **1050 Brussel (BE)**
- **MANNES, Morgane**
  **1150 Brussel (BE)**

(74) Representative: **De Clercq & Partners**
**Edgard Gevaertdreef 10a**
**9830 Sint-Martens-Latem (BE)**

# (54) G PROTEIN PEPTIDOMIMETICS

(57) The present invention relates to G protein peptidomimetics capable of stabilizing a GPCR in an active conformational state. The G protein peptidomimetics are derived from the G protein epitope interacting with the GPCR, in particular from the C-terminus of the $\alpha_5$ helix comprising the amino acid sequence FNDCRDIIQRMHLRQYELL (SEQ ID NO:117). The invention further provides complexes of the G protein peptidomimetics and a GPCR, fusion polypeptides of a GPCR and the G protein peptidomimetics and compositions comprising the same. Further disclosed herein are uses of the G protein peptidomimetics, complexes, fusion polypeptides and compositions for determining the structure of the GPCR conformer and for screening for compounds capable of specifically binding to the GPCR conformer.

EP 4 015 529 A1

## Description

### TECHNICAL FIELD

The application generally relates to structural biology of G protein coupled receptors. In particular, the present invention relates G protein peptidomimetics capable of stabilizing a GPCR in a functional conformational state, in particular an active conformational state. Further disclosed herein are uses of the G protein peptidomimetics for determining the structure of the GPCR conformer and for screening for compounds capable of specifically binding to the GPCR conformer.

### BACKGROUND

G protein-coupled receptors (GPCRs) are membrane proteins that play a central role in intra- and intercellular communication throughout the human body. Binding of an agonist to the receptor's extracellular orthosteric pocket ultimately results in a conformational change at its intracellular surface that allows downstream signalling (e.g. consequent to G protein coupling) and ultimately results in a biological phenotype.

So far, the pharmaceutical industry has more effectively exploited GPCRs than any other target class. About 30-40% of all marketed drugs target GPCRs. However, the vast majority (about 80%) of this large class of targets still remains unexploited.

To date, most GPCR drug discovery programs have relied on cell-based assay systems combined with high-throughput screening of large compound libraries. While this approach has been successful for many GPCRs, more recent targets of interest, which include peptide receptors, are proving less tractable to traditional lead-generation methods, especially to identify agonists or inverse agonists.

Given the success that fragment screening and structure-based drug discovery methods have had for soluble targets such as kinases and proteases, it would be of great benefit to apply these to GPCRs. However, biophysical assays and structural techniques are extremely challenging for GPCRs due to the following main challenges faced with GPCRs:

- GPCRs are highly unstable when extracted from the cell membrane, making them particularly difficult to isolate and purify for screening via biophysical methods.
- the difficulty to stabilize the GPCR in the appropriate conformation (e.g. active state) to identify weak binders with the desired pharmacology (i.e. agonists).

While there has been great progress in the structure determination of GPCRs in the inactive state (bound to antagonists), it is still a major challenge to determine the structures of receptors in their active signalling state, which can be defined structurally as the conformation bound to a heterotrimeric G protein or pharmacologically as the high-affinity agonist bound state. Strategies that have been developed to determine the active state structures of GPCRs so far include:

- The active structure of opsin stabilized at low pH (Park et al. 2008 Nature 477:549) and bound to a C-terminal fragment of the G protein transducin (Scheerer et al. 2008 Nature 455:497). Unfortunately, this methodology has not been successful for any other GPCR.
- The active state structure of the $\beta_2$ adrenergic receptor bound to a single-chain domain camelid antibody (also known in the art as Nanobody™ or Confobody™) that mimics the pharmacological effects of a heterotrimeric G protein (Rasmussen et al. 2011 Nature 469:175). This strategy has been applied successfully to a number of other GPCRs (Kruse et al. 2013 Nature 504:101; Sounier et al. 2015 Nature 524:375).
- The $\beta_2$AR with the entire heterotrimeric G protein (Rasmussen et al. 2011 Nature 477:175) in presence of a single chain camelid antibody able to stabilize the complex by interacting with the G protein sub-units. Another GPCR structure was recently published using this technology, solved by cryoEM (Liang et al. 2017 Nature 546:118).
- The structure of the adenosine A2A receptor (A2AR) coupled to a mini-G protein (Carpenter et al. 2016 Protein Engineering Design and Selection 29:583), in particular a mini-Gs protein, was determined (Carpenter et al. 2016 Nature 536:104). Mini-Gs attempts to recapitulate all the receptor pharmacology upon coupling, but so far the obtained active conformation is between an inactive conformation and a full active conformation as shown with the Nanobody/Confobody technology.
- $\beta_2$AR specific peptides were identified and derived from the third loop (CDR3) of Nb80 (Rasmussen et al. 2011 Nature 469:175), a well-known allosteric modulator of the $\beta_2$AR, but were unsuccessful to generate a functional (i.e. active state-stabilizing) $\beta_2$AR G protein peptidomimetic based on this epitope (Martin et al. 2017 Chemistry-A European Journal 23:9632).
- Boyhus et al. (2018 RSC Advances 8:2219) described the concept of Gs protein peptidomimetics as allosteric

modulators of the $\beta_2$AR. The authors designed peptidomimetics to mimic the 15 residue-long C-terminal $\alpha$-helix of the Gs protein, stabilized by peptide stapling. However, no Gs mimetics able to function as the Gs were described. In other words, they were not successful on stabilizing the active state GPCR.

Accordingly, there remains a need in the art for further tools capable of stabilizing a GPCR in a desired conformation, in particular an active conformation, which are preferably easy and cheap to generate and which are preferably universal tools (i.e. able to bind to a large number of GPCRs).

**SUMMARY OF THE INVENTION**

The present invention is based, at least in part, on the finding that peptides derived from the $\alpha_5$ helix wherein at least one of the C-terminal residues is substituted by an alanine analogue as defined herein below and/or comprising stabilizing staples, increase agonist affinity to the GPCR. These peptides bind the GPCR and show an increased agonist affinity to the receptor, similarly to the affinity shifts shown for e.g. Nb80, allowing to perform fragment-based screening for drug discovery. Advantageously, the G protein peptidomimetics as described herein can be developed fast, their synthesis is cheap and allows easy modifications. Moreover, they were shown to stabilize another GPCR, namely the dopamine (D1) receptor, in an active state and can thus be generic stabilising tools.

In particular, the invention relates to a G protein peptidomimetic or salt thereof comprising the sequence of formula (VIII):

$RDX_8IQRX_7HLX_6X_5X_4X_3X_2X_1$ (SEQ ID NO:8) (VIII)

wherein $X_1$ is selected from the group consisting of: leucine (L), L-$NH_2$, isoleucine (I), and $X_{1a}$; wherein $X_{1a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein $X_2$ is selected from the group consisting of: leucine (L), isoleucine (I), , tyrosine (Y), histidine (H), and $X_{2a}$, wherein $X_{2a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein $X_3$ is selected from the group consisting of: glutamic acid (E), glutamine (Q), homoglutamic acid (hGlu), aspartic acid (D), alanine (A), and $X_{3a}$, wherein $X_{3a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein $X_4$ is selected from the group consisting of: tyrosine (Y), 2-naphthalanine (2-Nal), 1-naphthalanine (1-Nal), and $X_{4a}$, wherein $X_{4a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl,

$C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein the sequence $X_4X_3X_2X_1$ (SEQ ID NO:18) is selected from the group consisting of $X_4X_3X_{2a}X_1$ (SEQ ID NO:133), $X_4X_3X_2X_{1a}$ (SEQ ID NO:134), $X_{4a}X_3X_2X_1$ (SEQ ID NO:135) and $X_4X_{3a}X_2X_1$ (SEQ ID NO:136), preferably wherein the sequence $X_4X_3X_2X_1$ (SEQ ID NO:18) is selected from the group consisting of: YE$X_{2a}$L (SEQ ID NO:19), YEL$X_{1a}$ (SEQ ID NO:20), $X_{4a}$ELL (SEQ ID NO:21) and Y$X_{3a}$LL (SEQ ID NO:22),

wherein the sequence $X_4X_3X_2X_1$ (SEQ ID NO:18) is not YELL (SEQ ID NO:23),

wherein $X_5$ is selected from the group consisting of: glutamine (Q), cysteine (C), olefinic amino acids, amino acids containing a carboxylic acid group side chain, amino acids containing an amine side-chain, amino acid containing an alkynyl side-chain, and amino acids containing an azidated side-chain,

wherein $X_6$ is selected from the group consisting of: arginine (R), C, olefinic amino acids, amino acids containing a carboxylic acid group side chain, amino acids containing an amine side-chain, amino acid containing an alkynyl side-chain, and amino acids containing an azidated side-chain,

wherein $X_7$ is selected from the group consisting of: methionine (M), C, olefinic amino acids, amino acids containing a carboxylic acid group side chain, amino acids containing an amine side-chain, amino acid containing an alkynyl side-chain, and amino acids containing an azidated side-chain, and

wherein $X_8$ is selected from the group consisting of: isoleucine (I), C, olefinic amino acids, amino acids containing a carboxylic acid group side chain, amino acids containing an amine side-chain, amino acid containing an alkynyl side-chain, and amino acids containing an azidated side-chain,

and optionally wherein said peptidomimetic comprises at least one covalent tether, said covalent tether being formed from the reaction of an amino acid containing an amine side-chain with an amino acid containing a carboxylic acid group side-chain, or from the reaction between two olefinic amino acids, or from the reaction of an amino acid containing an azidated side-chain with an amino acid containing an alkynyl side-chain, or from the reaction between two amino acids each containing a thiol group side-chain, or from the reaction of an olefinic amino acid with an amino acid containing a thiol group side-chain, preferably said covalent tether is formed from the reaction of an amino acid containing an azidated side-chain with an amino acid containing an alkynyl side-chain.

## BRIEF DESCRIPTION OF THE FIGURES

The teaching of the application is illustrated by the following Figures which are to be considered as illustrative only and do not in any way limit the scope of the claims.

Figures 1-15 represent graphs plotting the results of GPCR-radioligand binding assay (RLA) with a human β2AR radioligand binding, in the presence of peptidomimetics according to embodiments of the invention, using 3H-dihydroalprenolol (DHA) as the radioligand and increasing concentrations of isoproterenol (agonist) as cold competitor.

Figures 16a-g represents the circular dichroism spectra of the peptidomimetics according to embodiments of the invention.

Figure 17 represents a graph plotting the results of single point radioligand displacement assay in parallel on the basal state ($\beta_2$AR) and on the structurally constrained receptor ($\beta_2$AR + compound 51). Fragments from the Maybridge Ro3 library were screened by incubation with the radioactive labelled antagonist [$^3$H]-DHA in the presence and absence of the peptidomimetic. Agonist-prone fragments (fb) were selected based on the comparison of the residual binding on the basal and active state.

Figure 18 represents a graph plotting the results of single point radioligand displacement assay in parallel on the basal state (D1R) and on the structurally constrained receptor (D1R + compound 51). Fragments from the Maybridge Ro3 library were screened by incubation with the radioactive labelled antagonist [3H]-SCH23390 in the presence

and absence of the peptidomimetic. Agonist-prone fragments (fd) were selected based on the comparison of the residual binding on the basal and active state.

Figure 19 represents a graph plotting the results of GPCR-radioligand binding assay (RLA) with a dopamine 1 receptor (D1R) binding, in the presence of peptidomimetics according to embodiments of the invention, using [$^3$H]-SCH 23390 as radiolabelled antagonist, and increasing concentrations of A-77636 (agonist).

Figure 20 represents a graph of a theoretical radioligand displacement assay of a $G_s$ peptidomimetic at $\beta_2$AR, using [$^3$H]-dihydroalprenolol ([$^3$H]-DHA) as radioligand and isoproterenol as agonist.

Figure 21 is a schematic illustration of an interaction map of a peptide representing the $\alpha$5 helix with $\beta_2$AR.

Interacting residues of the receptor and the peptide representing the $\alpha$5 helix. The different contacts are represented by full lines (hydrogen bonds) or dotted lines (hydrophobic contacts) between the receptor and peptide residues, while the non-interacting amino acids in the peptide are shown in bold. The interaction scheme is based on the coordinates from the X-ray crystal structure of the $\beta_2$AR-Gs complex, PDB: 3SN6.

**DETAILED DESCRIPTION OF THE INVENTION**

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. Where reference is made to embodiments as comprising certain elements or steps, this encompasses also embodiments which consist essentially of the recited elements or steps.

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

As used herein, the term "and/or" when used in a list of two or more items, means that any one of the listed items can be employed by itself or any combination of two or more of the listed items can be employed. For example, if a list is described as comprising group A, B, and/or C, the list can comprise A alone; B alone; C alone; A and B in combination; A and C in combination, B and C in combination; or A, B, and C in combination.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, aspects, structures or characteristics may be combined in any suitable manner, as would be apparent to one of ordinary skill in the art from this disclosure, in one or more embodiments. Also embodiments described for an aspect of the invention may be used for another aspect of the invention and can be combined.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects.

In each of the following definitions, the number of carbon atoms represents the maximum number of carbon atoms generally optimally present in the moiety, group, substituent or linker; it is understood that where otherwise indicated in the present application, the number of carbon atoms represents the optimal maximum number of carbon atoms for that particular moiety, group, substituent or linker.

Whenever the term "substituted" is used herein, it is meant to indicate that one or more hydrogen atoms on the atom indicated in the expression using "substituted" is replaced with a selection from the indicated group, provided that the indicated atom's normal valence is not exceeded, and that the substitution results in a chemically stable compound, i.e. a compound that is sufficiently robust to survive isolation from a reaction mixture.

The term "halo" or "halogen" as a group or part of a group is generic for fluoro, chloro, bromo, iodo.

The term "oxo" as used herein means the =O group.

The term "amino" as used herein means the $-NH_2$ group.

The term "azido" refers to the -N=N=N group.

The term "azidated" refers to a compound comprising an azido group.

The term "alkyl", as a group or part of a group, refers to normal, secondary, or tertiary, linear, branched or straight hydrocarbon with no site of unsaturation of formula CnH2n+1 wherein n is preferably a number ranging from 1 to 6. Thus, for example, "$C_{1-6}$alkyl" includes all linear or branched alkyl groups with between 1 and 6 carbon atoms, and thus includes methyl, ethyl, 1-propyl (n-propyl), 2-propyl (iPr), 1-butyl, 2-methyl-1-propyl (i-Bu), 2-butyl (s-Bu), 2-dimethyl-2-propyl (t-Bu), 1-pentyl (n-pentyl), 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl. For example, "$C_{1-5}$alkyl" includes all linear or branched alkyl groups with between 1 and 5 carbon atoms, and thus includes methyl, ethyl, n-propyl, i-propyl, butyl and its isomers (e.g. n-butyl, i-butyl and t-butyl); pentyl and its isomers. For example, "$C_{1-4}$alkyl" includes all linear or branched alkyl groups with between 1 and 4 carbon atoms, and thus includes methyl, ethyl, n-propyl, i-propyl, butyl and its isomers (e.g. *n*-butyl, *i*-butyl and *t*-butyl). For example, "$C_{1-3}$alkyl" includes all linear or branched alkyl groups with between 1 and 3 carbon atoms, and thus includes methyl, ethyl, n-propyl, i-propyl.

The term "halo$C_{1-6}$alkyl" as a group or part of a group, refers to a $C_{1-6}$alkyl group having the meaning as defined above wherein one or more hydrogen atoms are each replaced with one or more halogen as defined herein. Non-limiting examples of such halo$C_{1-6}$alkyl groups include chloromethyl, 1-bromoethyl, fluoromethyl, difluoromethyl, trifluoromethyl, 1,1,1-trifluoroethyl and the like.

The term "$C_{1-8}$alkoxy", as a group or part of a group, refers to a group having the formula $-OR^a$ wherein $R^a$ is $C_{1-6}$alkyl as defined herein above. Non-limiting examples of suitable $C_{1-6}$alkoxy include methoxy, ethoxy, propoxy, isopropoxy, butoxy, isobutoxy, sec-butoxy, tert-butoxy, pentyloxy and hexyloxy.

The term "$C_{2-6}$alkenyl" as a group or part of a group, refers to an unsaturated hydrocarbyl group, which may be linear, or branched, comprising one or more carbon-carbon double bonds, and comprising from 2 to 6 carbon atoms. Examples of $C_{2-6}$alkenyl groups are ethenyl, 2-propenyl, 2-butenyl, 3-butenyl, 2-pentenyl and its isomers, 2-hexenyl and its isomers, 2,4-pentadienyl, and the like.

The term "alkynyl" or as used herein refers to $C_{2-6}$ normal, secondary, tertiary, linear, branched or straight hydrocarbon with at least one site (usually 1 to 3, preferably 1) of unsaturation, namely a carbon-carbon, sp triple bond. Examples include, but are not limited to: ethynyl ($-C{\equiv}CH$), 3-ethyl-cyclohept-1-ynylene, and 1-propynyl (propargyl, $-CH_2C{\equiv}CH$).

The term "$C_{3-12}$cycloalkyl", as a group or part of a group, refers to a cyclic alkyl group, that is a monovalent, saturated, hydrocarbyl group having 1 or more cyclic structure, and comprising from 3 to 12 carbon atoms, preferably from 5 to 6 carbon atoms. Cycloalkyl includes all saturated hydrocarbon groups containing one or more rings, including monocyclic or bicyclic groups. The further rings of multi-ring cycloalkyls may be either fused, bridged and/or joined through one or more spiro atoms. Examples of $C_{3-12}$cycloalkyl include by are not limited to such instance cyclopropyl, cyclobutyl, cyclopentyl, cyclopropylethylene, methylcyclopropylene, cyclohexyl, cycloheptyl, cyclooctyl, cyclooctylmethylene, norbornyl, fenchyl, trimethyltricycloheptyl, decalinyl, adamantyl and the like. Examples of $C_{3-6}$cycloalkyl groups include but are not limited to cyclopropyl, cyclobutyl, cyclopentyl, and cyclohexyl.

The term "cycloalkenyl" as used herein refers to a non-aromatic hydrocarbon group having from 5 to 12 carbon atoms with at least one site (usually 1 to 3, preferably 1) of unsaturation, namely a carbon-carbon, sp2 double bond and consisting of or comprising a $C_{5-10}$ monocyclic or $C_{7-12}$ polycyclic hydrocarbon. Examples include, but are not limited to: cyclopentenyl ($-C_5H_7$), cyclopentenylpropylene, methylcyclohexenylene and cyclohexenyl ($-C_6H_9$). The double bond may be in the cis or trans configuration. In particular embodiments, the term cycloalkenyl refers to $C_{5-12}$cycloalkenyl (cyclic $C_{5-12}$ hydrocarbons), yet more in particular to $C_{6-12}$cycloalkenyl (cyclic $C_{6-12}$ hydrocarbons), still more in particular to $C_{6-10}$cycloalkenyl (cyclic $C_{6-10}$ hydrocarbons) as further defined herein above with at least one site of unsaturation, namely a carbon-carbon, sp2 double bond.

The term "$C_{6-12}$aryl", as a group or part of a group, refers to a polyunsaturated, aromatic hydrocarbyl group having a single ring (i.e. phenyl) or multiple aromatic rings fused together (e.g. naphthyl), or linked covalently, typically comprising 6 to 12 carbon atoms; wherein at least one ring is aromatic, preferably comprising 6 to 10 carbon atoms, wherein at least one ring is aromatic. The aromatic ring may optionally include one to two additional rings (either cycloalkyl, heterocyclyl or heteroaryl) fused thereto. Examples of suitable aryl include $C_{6-10}$aryl, more preferably $C_{6-8}$aryl. Non-limiting examples of $C_{6-12}$aryl comprise phenyl, biphenylyl, biphenylenyl, or 1-or 2-naphthalenyl; 5- or 6-tetralinyl, 1-, 2-, 3-, 4-, 5-, 6-, 7- or 8-azulenyl, 4-, 5-, 6 or 7-indenyl, 4- or 5-indanyl, 5-, 6-, 7- or 8-tetrahydronaphthyl, 1,2,3,4-tetrahydronaphthyl, and 1,4-dihydronaphthyl; 1-, 2-, 3-, 4- or 5-pyrenyl.

The term "heteroaryl" refers but is not limited to an aromatic ring system of 5 to 12 atoms including at least one N, O, S, or P, containing 1 or 2 rings which can be fused together or linked covalently, each ring typically containing 5 to 6 atoms; at least one of said ring is aromatic, where the N and S heteroatoms may optionally be oxidized and the N

heteroatoms may optionally be quaternized, and wherein at least one carbon atom of said heteroaryl can be oxidized to form at least one C=O. Such rings may be fused to an aryl, cycloalkyl, heteroaryl or heterocyclyl ring. Non-limiting examples of such heteroaryl, include: triazol-2-yl, pyridinyl, 1H-pyrazol-5-yl, pyrrolyl, furanyl, thiophenyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyrimidyl, pyrazinyl, pyridazinyl, oxazinyl, dioxinyl, thiazinyl, triazinyl, imidazo[2,1-b][1,3]thiazolyl, thieno[3,2-b]furanyl, thieno[3,2-b]thiophenyl, thieno[2,3-d][1,3]thiazolyl, thieno[2,3-d]imidazolyl, tetrazolo[1,5-a]pyridinyl, indolyl, indolizinyl, isoindolyl, benzofuranyl, isobenzofuranyl, benzothiophenyl, isobenzothiophenyl, indazolyl, benzimidazolyl, 1,3-benzoxazolyl, 1,2-benzisoxazolyl, 2,1-benzisoxazolyl, 1,3-benzothiazolyl, 1,2-benzoisothiazolyl, 2,1-benzoisothiazolyl, benzotriazolyl, 1,2,3-benzoxadiazolyl, 2,1,3-benzoxadiazolyl, 1,2,3-benzothiadiazolyl, 2,1,3-benzothiadiazolyl, benzo[d]oxazol-2(3H)-one, 2,3-dihydro-benzofuranyl, thienopyridinyl, purinyl, imidazo[1,2-a]pyridinyl, 6-oxo-pyridazin-1(6H)-yl, 2-oxopyridin-1(2H)-yl, 6-oxo-pyridazin-1(6H)-yl, 2-oxopyridin-1(2H)-yl, 1,3-benzodioxolyl, quinolinyl, isoquinolinyl, cinnolinyl, quinazolinyl, quinoxalinyl; preferably said heteroaryl group is selected from the group comprising pyridyl, 1,3-benzodioxolyl, benzo[d]oxazol-2(3H)-one, 2,3-dihydro-benzofuranyl, pyrazinyl, pyrazolyl, pyrrolyl, isoxazolyl, thiophenyl, imidazolyl, benzimidazolyl, pyrimidinyl, s-triazinyl, oxazolyl, isothiazolyl, furyl, thienyl, triazolyl and thiazolyl.

The term "heterocycloalkyl" as used herein refer to non-aromatic, fully saturated ring system of 3 to 12 atoms, comprising at least two ring forming carbon atoms and at least one ring forming heteroatom such as at least one N, O, or S, (for example, 3 to 7 member monocyclic, 7 to 11 member bicyclic, or comprising a total of 3 to 10 ring atoms) wherein at least one ring is a heterocycloalkyl and wherein said ring may be fused to an aryl, cycloalkyl, heteroaryl or heterocycloalkyl ring. Each ring of the heterocyclyl may have 1, 2, 3 or 4 heteroatoms selected from N, O and/or S, where the N and S heteroatoms may optionally be oxidized and the N heteroatoms may optionally be quaternized; and wherein at least one carbon atom of heterocyclyl can be oxidized to form at least one C=O. The heterocyclic may be attached at any heteroatom or carbon atom of the ring or ring system, where valence allows. The rings of multi-ring heterocyclyls may be fused, bridged and/or joined through one or more spiro atoms. Suitable heterocycloalkyl groups include oxetanyl, azetidinyl, tetrahydrofuranyl, dioxolanyl, pyrrolidinyl, oxazolidinyl, thiazolidinyl, isothiazolidinyl, imidazolidinyl, tetrahydropyranyl, tetrahydrothiopyranyl, piperidinyl, piperazinyl, morpholinyl, thiomorpholinyl, azepanyl, oxazepanyl, diazepanyl, thiadiazepanyl and azocanyl.

The term "mono- or di-$C_{1-6}$alkylamino", as a group or part of a group, refers to a group of formula $-N(R^a)(R^b)$ wherein $R^b$ is hydrogen, or $C_{1-6}$alkyl, $R^a$ is $C_{1-6}$alkyl. Thus, alkylamino include mono-alkyl amino group (e.g. mono-alkylamino group such as methylamino and ethylamino), and di-alkylamino group (e.g. di-alkylamino group such as dimethylamino and diethylamino). Non-limiting examples of suitable mono- or di-alkylamino groups include *n*-propylamino, isopropylamino, *n*-butylamino, *i*-butylamino, sec-butylamino, *t*-butylamino, pentylamino, *n*-hexylamino, di-*n*-propylamino, di-*i*-propylamino, ethylmethylamino, methyl-*n*-propylamino, methyl-*i*-propylamino, *n*-butylmethylamino, *i*-butylmethylamino, *t*-butylmethylamino, ethyl-*n*-propylamino, ethyl-*i*-propylamino, *n*-butylethylamino, i-butylethylamino, *t*-butylethylamino, di-*n*-butylamino, di-*i*-butylamino, methylpentylamino, methylhexylamino, ethylpentylamino, ethylhexylamino, propylpentylamino, propylhexylamino, and the like.

The term "Pra" as used herein refers to moiety of formula

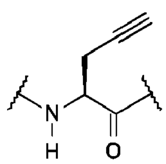

.

The term "Azk" as used herein refers to moiety of formula

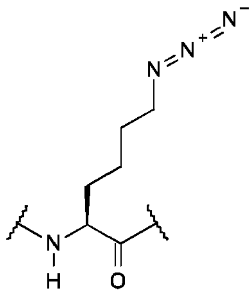

.

The term "[Pra-$Y^a$-Azk]" refers to a moiety of formula

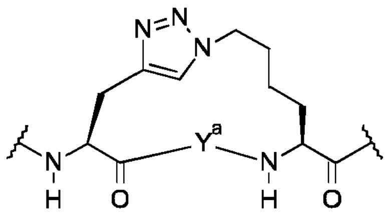

,

wherein $Y^a$ represents one or more amino acids.

The term "S5" as used herein refers to moiety of formula

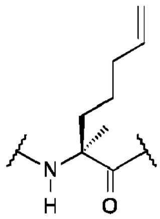

.

The term "R5" as used herein refers to moiety of formula

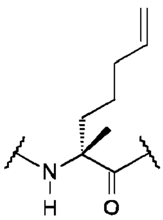

.

The term "R8" as used herein refers to moiety of formula

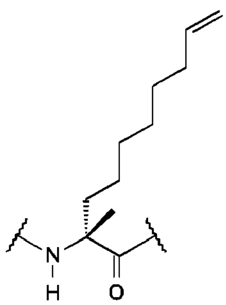

.

The term "[$S^5$-$Y^a$-$S^5$]" refers to a moiety of formula

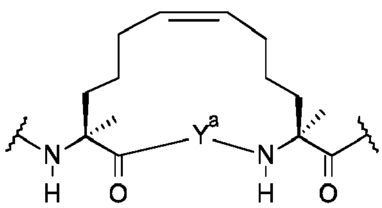

,

wherein $Y^a$ represents one or more amino acids.

The term "[$R^5$-$Y^a$-$S^5$]" refers to a moiety of formula

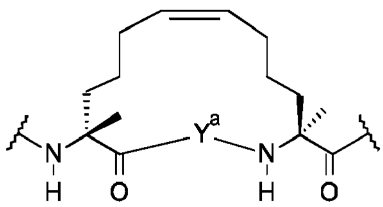

,

wherein $Y^a$ represents one or more amino acids.

The term "hGlu" as used herein refers to homoglutamic acid moiety of formula

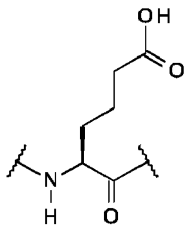

.

The term "Cha" as used herein refers to cyclohexylalanine moiety of formula

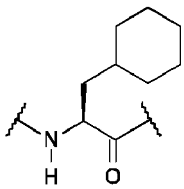

.

The term "1-Nal" as used herein refers to 1-naphthalanine moiety of formula

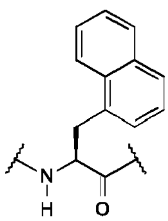

.

As used herein, the terms "1-naphthalanine", "1-naphthylalanine" and "1-naphthyl-L-alanine" are synonymous and used interchangeably.

The term "2-Nal" as used herein refers to 2-naphthalanine moiety of formula

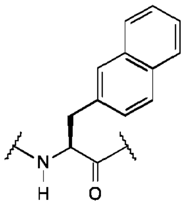

.

As used herein, the terms "2-naphthalanine", "2-naphthylalanine" and "2-naphthyl-L-alanine" are synonymous and used interchangeably.

Whenever used in the present invention the term "G protein peptidomimetics" or a similar term is meant to include the compounds of the general formula disclosed therein and any subgroup thereof, including all polymorphs and crystal habits thereof, and isomers thereof (including optical, geometric and tautomeric isomers) as hereinafter defined.

As used herein and unless otherwise stated, the term "stereoisomer" refers to all possible different isomeric as well as conformational forms which the "peptidomimetics" herein may possess, in particular all possible stereochemically and conformationally isomeric forms, all diastereomers, enantiomers and/or conformers of the basic molecular structure. Some compounds of the present invention may exist in different tautomeric forms, all of the latter being included within the scope of the present invention.

All documents cited in the present specification are hereby incorporated by reference in their entirety.

Preferred aspects, statements (features) and embodiments of this invention are set herein below. Each aspects, statements and embodiments of the invention so defined may be combined with any other aspects, statement and/or embodiments unless clearly indicated to the contrary. In particular, any feature indicated as being preferred or advantageous may be combined with any other feature or features or statements or aspects indicated as being preferred or advantageous.

The present invention is in particular captured by any one or any combination of one or more of the below numbered aspects and embodiments with any other aspects, statement and/or embodiments:

Aspect 1. A G protein peptidomimetic or a salt thereof having an amino acid sequence that is derived from the amino acid sequence NARRIFNDCRDIIQRMHLRQYELL (SEQ ID NO:132) or from a fragment of SEQ ID NO:132, preferably from the amino acid sequence FNDCRDIIQRMHLRQYELL (SEQ ID NO:117) or from a fragment of SEQ ID NO:117.

Aspect 2. The G protein peptidomimetic or salt thereof according to aspect 1 comprising or consisting of the structure shown in formula (I):

$$HLX_6X_5X_4X_3X_2X_1 \text{ (SEQ ID NO: 1)} \qquad \text{(I)}$$

wherein $X_1$ is selected from the group consisting of: leucine (L), L-$NH_2$, isoleucine (I), alanine (A), and $X_{1a}$; wherein $X_{1a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, =$CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein $X_2$ is selected from the group consisting of: leucine (L), isoleucine (I), alanine (A), tyrosine (Y), histidine (H), and $X_{2a}$, wherein $X_{2a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, =$CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein $X_3$ is selected from the group consisting of: glutamic acid (E), glutamine (Q), homoglutamic acid (hGlu), aspartic acid (D), alanine (A) and $X_{3a}$, wherein $X_{3a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, =$CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein $X_4$ is selected from the group consisting of: tyrosine (Y), 2-naphthalanine (2-Nal), 1-naphthalanine (1-Nal), alanine (A) and $X_{4a}$, wherein $X_{4a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein $X_5$ is selected from the group consisting of: glutamine (Q), cysteine (C), olefinic amino acids, amino acids containing a carboxylic acid group side chain, amino acids containing an amine side-chain, amino acid containing an alkynyl side-chain, and amino acids containing an azidated side-chain; and

wherein $X_6$ is selected from the group consisting of: arginine (R), C, olefinic amino acids, amino acids containing a carboxylic acid group side chain, amino acids containing an amine side-chain, amino acid containing an alkynyl side-chain, and amino acids containing an azidated side-chain,

wherein at least one of $X_1$, $X_2$, $X_3$ and $X_4$ is an alanine analogue, phenylalanine (F) or tryptophan (W) and/or wherein said peptidomimetic comprises at least one covalent tether, said covalent tether being formed from the reaction of an amino acid containing an amine side-chain with an amino acid containing a carboxylic acid group side-chain, or from the reaction between two olefinic amino acids, or from the reaction of an amino acid containing an azidated side-chain with an amino acid containing an alkynyl side-chain, or from the reaction between two amino acids each containing a thiol group side-chain, or from the reaction of an olefinic amino acid with an amino acid containing a thiol group side-chain, preferably said covalent tether is formed from the reaction of an amino acid containing an azidated side-chain with an amino acid containing an alkynyl side-chain.

Aspect 3. The G protein peptidomimetic or salt thereof according to aspect 1 or 2 comprising or consisting of the structure shown in formula (II):

$X_7HLX_6X_5X_4X_3X_2X_1$ (SEQ ID NO: 2) (II)

wherein $X_7$ is selected from the group consisting of: M, C, olefinic amino acids, amino acids containing a carboxylic acid group side chain, amino acids containing an amine side-chain, amino acid containing an alkynyl side-chain, and amino acids containing an azidated side-chain.

Aspect 4. The G protein peptidomimetic or salt thereof according to any one of aspects 1 to 3 comprising or consisting of the structure shown in formula (III):

$RX_7HLX_6X_5X_4X_3X_2X_1$ (SEQ ID NO: 3) (III).

Aspect 5. The G protein peptidomimetic or salt thereof according to any one of aspects 1 to 4 comprising or consisting of the structure shown in formula (IV):

$QRX_7HLX_6X_5X_4X_3X_2X_1$ (SEQ ID NO: 4) (IV).

Aspect 6. The G protein peptidomimetic or salt thereof according to any one of aspects 1 to 5 comprising or consisting of the structure shown in formula (V):

$IQRX_7HLX_6X_5X_4X_3X_2X_1$ (SEQ ID NO: 5) (V).

Aspect 7. The G protein peptidomimetic or salt thereof according to any one of aspects 1 to 6 comprising or consisting of the structure shown in formula (VI):

$X_8IQRX_7HLX_6X_5X_4X_3X_2X_1$ (SEQ ID NO: 6) (VI)

wherein $X_8$ is selected from the group consisting of: I, C, olefinic amino acids, amino acids containing a carboxylic acid group side chain, amino acids containing an amine side-chain, amino acid containing an alkynyl side-chain, and amino acids containing an azidated side-chain.

Aspect 8. The G protein peptidomimetic or salt thereof according to any one of aspects 1 to 7 comprising or consisting of the structure shown in formula (VII):

$DX_8IQRX_7HLX_6X_5X_4X_3X_2X_1$ (SEQ ID NO: 7) (VII).

Aspect 9. The G protein peptidomimetic or salt thereof according to any one of aspects 1 to 8 comprising or consisting of the structure shown in formula (VIII):

$RDX_8IQRX_7HLX_6X_5X_4X_3X_2X_1$ (SEQ ID NO: 8) (VIII).

Aspect 10. The G protein peptidomimetic or salt thereof according to any one of aspects 7 to 9, wherein
$X_1$ is selected from the group consisting of: leucine (L), L-$NH_2$, isoleucine (I), and $X_{1a}$; wherein $X_{1a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;
wherein $X_2$ is selected from the group consisting of: leucine (L), isoleucine (I), tyrosine (Y), histidine (H), and $X_{2a}$, wherein $X_{2a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;
wherein $X_3$ is selected from the group consisting of: glutamic acid (E), glutamine (Q), homoglutamic acid (hGlu), aspartic Acid (D), alanine (A) and $X_{3a}$, wherein $X_{3a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;
wherein $X_4$ is selected from the group consisting of: tyrosine (Y), 2-naphthalanine (2-Nal), 1-naphthalanine (1-Nal), and $X_{4a}$, wherein $X_{4a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;
wherein the sequence $X_4X_3X_2X_1$ (SEQ ID NO:18) is selected from the group consisting of $X_4X_3X_{2a}X_1$ (SEQ ID NO:137133), $X_4X_3X_2X_{1a}$ (SEQ ID NO:134), $X_{4a}X_3X_2X_1$ (SEQ ID NO:135) and $X_4X_{3a}X_2X_1$ (SEQ ID NO:136), preferably wherein the sequence $X_4X_3X_2X_1$ (SEQ ID NO:18) is selected from the group consisting of: $YEX_{2a}L$ (SEQ ID NO:19), $YELX_{1a}$ (SEQ ID NO:20), $X_{4a}ELL$ (SEQ ID NO:21) and $YX_{3a}LL$ (SEQ ID NO:22);
wherein the sequence $X_4X_3X_2X_1$ (SEQ ID NO:18) is not YELL (SEQ ID NO:23),
wherein $X_5$ is selected from the group consisting of: glutamine (Q), cysteine (C), olefinic amino acids, amino acids containing a carboxylic acid group side chain, amino acids containing an amine side-chain, amino acid containing an alkynyl side-chain, and amino acids containing an azidated side-chain,
wherein $X_6$ is selected from the group consisting of: arginine (R), C, olefinic amino acids, amino acids containing a carboxylic acid group side chain, amino acids containing an amine side-chain, amino acid containing an alkynylside-chain, and amino acids containing an azidated side-chain,

wherein $X_7$ is selected from the group consisting of: methionine (M), C, olefinic amino acids, amino acids containing a carboxylic acid group side chain, amino acids containing an amine side-chain, amino acid containing an alkynyl side-chain, and amino acids containing an azidated side-chain, and

wherein $X_8$ is selected from the group consisting of: isoleucine (I), C, olefinic amino acids, amino acids containing a carboxylic acid group side chain, amino acids containing an amine side-chain, amino acid containing an alkynyl side-chain, and amino acids containing an azidated side-chain,

and optionally wherein said peptidomimetic comprises at least one covalent tether, said covalent tether being formed from the reaction of an amino acid containing an amine side-chain with an amino acid containing a carboxylic acid group side-chain, or from the reaction between two olefinic amino acids, or from the reaction of an amino acid containing an azidated side-chain with an amino acid containing an alkynyl side-chain, or from the reaction between two amino acids each containing a thiol group side-chain, or from the reaction of an olefinic amino acid with an amino acid containing a thiol group side-chain, preferably said covalent tether is formed from the reaction of an amino acid containing an azidated side-chain with an amino acid containing an alkynyl side-chain.

Aspect 11. A G protein peptidomimetic or salt thereof comprising the sequence of formula (VIII):

$$RDX_8IQRX_7HLX_6X_5X_4X_3X_2X_1 \text{ (SEQ ID NO:8)} \qquad \text{(VIII)}$$

wherein $X_1$ is selected from the group consisting of: leucine (L), L-$NH_2$, isoleucine (I), and $X_{1a}$; wherein $X_{1a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein $X_2$ is selected from the group consisting of: leucine (L), isoleucine (I), tyrosine (Y), histidine (H), and $X_{2a}$, wherein $X_{2a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein $X_3$ is selected from the group consisting of: glutamic acid (E), glutamine (Q), homoglutamic acid (hGlu), aspartic Acid (D), alanine (A) and $X_{3a}$, wherein $X_{3a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein $X_4$ is selected from the group consisting of: tyrosine (Y), 2-naphthalanine (2-Nal), 1-naphthalanine (1-Nal), and $X_{4a}$, wherein $X_{4a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein the sequence $X_4X_3X_2X_1$ (SEQ ID NO:18) is selected from the group consisting of $X_4X_3X_{2a}X_1$ (SEQ ID NO:137133), $X_4X_3X_2X_{1a}$ (SEQ ID NO:134), $X_{4a}X_3X_2X_1$ (SEQ ID NO:135) and $X_4X_{3a}X_2X_1$ (SEQ ID NO:136), preferably wherein the sequence $X_4X_3X_2X_1$ (SEQ ID NO:18) is selected from the group consisting of: $YEX_{2a}L$ (SEQ ID NO:19), $YELX_{1a}$ (SEQ ID NO:20), $X_{4a}ELL$ (SEQ ID NO:21) and $YX_{3a}LL$ (SEQ ID NO:22);

wherein the sequence $X_4X_3X_2X_1$ (SEQ ID NO:18) is not YELL (SEQ ID NO:23),

wherein $X_5$ is selected from the group consisting of: glutamine (Q), cysteine (C), olefinic amino acids, amino acids containing a carboxylic acid group side chain, amino acids containing an amine side-chain, amino acid containing an alkynyl side-chain, and amino acids containing an azidated side-chain,

wherein $X_6$ is selected from the group consisting of: arginine (R), C, olefinic amino acids, amino acids containing a carboxylic acid group side chain, amino acids containing an amine side-chain, amino acid containing an alkynylside-chain, and amino acids containing an azidated side-chain,

wherein $X_7$ is selected from the group consisting of: methionine (M), C, olefinic amino acids, amino acids containing a carboxylic acid group side chain, amino acids containing an amine side-chain, amino acid containing an alkynyl side-chain, and amino acids containing an azidated side-chain, and

wherein $X_8$ is selected from the group consisting of: isoleucine (I), C, olefinic amino acids, amino acids containing a carboxylic acid group side chain, amino acids containing an amine side-chain, amino acid containing an alkynyl side-chain, and amino acids containing an azidated side-chain,

and optionally wherein said peptidomimetic comprises at least one covalent tether, said covalent tether being formed from the reaction of an amino acid containing an amine side-chain with an amino acid containing a carboxylic acid group side-chain, or from the reaction between two olefinic amino acids, or from the reaction of an amino acid containing an azidated side-chain with an amino acid containing an alkynyl side-chain, or from the reaction between two amino acids each containing a thiol group side-chain, or from the reaction of an olefinic amino acid with an amino acid containing a thiol group side-chain, preferably said covalent tether is formed from the reaction of an amino acid containing an azidated side-chain with an amino acid containing an alkynyl side-chain.

Aspect 12. The G protein peptidomimetic or salt thereof according to any one of aspects 1 to 11 comprising or consisting of the structure shown in formula (IX):

$X_9RDX_8IQRX_7HLX_6X_5X_4X_3X_2X_1$ (SEQ ID NO: 9) (IX)

wherein $X_9$ is C or an amino acid without a thiol side-chain.

Aspect 13. The G protein peptidomimetic or salt thereof according to any one of aspects 1 to 12 comprising or consisting of the structure shown in formula (X):

$X_{10}$ $X_9RDX_8IQRX_7HLX_6X_5X_4X_3X_2X_1$ (SEQ ID NO: 10) (X)

wherein $X_{10}$ is selected from the group consisting of: D, C, olefinic amino acids, amino acids containing a carboxylic acid group side chain, amino acids containing an amine side-chain, amino acid containing an alkynyl side-chain, and amino acids containing an azidated side-chain.

Aspect 14. The G protein peptidomimetic or salt thereof according to any one of aspects 1 to 13 comprising or consisting of the structure shown in formula (XI):

$NX_{10}$ $X_9RDX_8IQRX_7HLX_6X_5X_4X_3X_2X_1$ (SEQ ID NO: 11) (XI).

Aspect 15. The G protein peptidomimetic salt thereof according to any one of aspects 1 to 14 comprising or consisting of the structure shown in formula (XII):

$FNX_{10}X_9RDX_8IQRX_7HLX_6X_5X_4X_3X_2X_1$ (SEQ ID NO: 12) (XII).

Aspect 16. The G protein peptidomimetic or salt thereof according to any one of aspects 1 to 15 comprising or consisting of the structure shown in formula (XIII):

$IFNX_{10}X_9RDX_8IQRX_7HLX_6X_5X_4X_3X_2X_1$ (SEQ ID NO: 13) (XIII).

Aspect 17. The G protein peptidomimetic or salt thereof according to any one of aspects 1 to 16 comprising or consisting of the structure shown in formula (XIV):

$RIFNX_{10}X_9RDX_8IQRX_7HLX_6X_5X_4X_3X_2X_1$ (SEQ ID NO: 14) (XIV).

Aspect 18. The G protein peptidomimetic or salt thereof according to any one of aspects 1 to 17 comprising or consisting of the structure shown in formula (XV):

$RRIFNX_{10}X_9RDX_8IQRX_7HLX_6X_5X_4X_3X_2X_1$ (SEQ ID NO: 15) (XV).

Aspect 19. The G protein peptidomimetic or salt thereof according to any one of aspects 1 to 18 comprising or consisting of the structure shown in formula (XVI):

$ARRIFNX_{10}X_9RDX_8IQRX_7HLX_6X_5X_4X_3X_2X_1$ (SEQ ID NO: 16) (XVI).

Aspect 20. The G protein peptidomimetic or salt thereof according to any one of aspects 1 to 19 comprising or consisting of the structure shown in formula (XVII):

$NARRIFNX_{10}X_9RDX_8IQRX_7HLX_6X_5X_4X_3X_2X_1$ (SEQ ID NO: 17) (XVII).

Aspect 21. The G protein peptidomimetic or salt thereof according to any one of aspects 2 to 20, wherein $X_5$ is Q, wherein $X_6$ is R and wherein $X_7$ is M.

Aspect 22. The G protein peptidomimetic or salt thereof according to any one of aspects 2 to 21 comprising a sequence $X_4X_3X_2X_1$ (SEQ ID NO:18) selected from the group consisting of $X_4X_3X_{2a}X_1$ (SEQ ID NO:133), $X_4X_3X_2X_{1a}$ (SEQ ID NO:134), $X_{4a}X_3X_2X_1$ (SEQ ID NO:135) and $X_4X_{3a}X_2X_1$ (SEQ ID NO:136), preferably a sequence selected from $YEX_{2a}L$ (SEQ ID NO:19), $YELX_{1a}$ (SEQ ID NO:20), $X_{4a}ELL$ (SEQ ID NO:21) or $YX_{3a}LL$ (SEQ ID NO:22), even more preferably $YEX_{2a}L$ (SEQ ID NO:19), wherein $X_{1a}$, $X_{2a}$, $X_{3a}$ and $X_{4a}$ are each independently selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl; and wherein $X_1$, $X_2$, $X_3$ and $X_4$ have the same meaning as defined in aspect 2.

Aspect 23. The G protein peptidomimetic or salt thereof according to any one of aspects 2 to 22, wherein the sequence $X_4X_3X_2X_1$ (SEQ ID NO:18) is not YELL (SEQ ID NO:23).

Aspect 24. The G protein peptidomimetic or salt thereof according to any one of aspects 2 to 23, wherein the sequence $X_4X_3X_2X_1$ (SEQ ID NO:18) is $X_4X_3X_{2a}X_1$ (SEQ ID NO:133), preferably wherein the sequence $X_4X_3X_2X_1$ (SEQ ID NO:18) is $YEX_{2a}L$ (SEQ ID NO:19), wherein:

$X_1$ is selected from the group consisting of: leucine (L), $L\text{-}NH_2$ and isoleucine (I);

$X_{2a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or

$C_{6-12}$aryl may be optionally substituted by one or more $C_{1-6}$alkyl;

$X_3$ is selected from the group consisting of: glutamic acid (E), glutamine (Q), homoglutamic acid (hGlu), aspartic Acid (D) and alanine (A);

$X_4$ is selected from the group consisting of: tyrosine (Y), 2-naphthylalanine (2-Nal), and 1-naphthylalanine (1-Nal).

Aspect 25. The G protein peptidomimetic or salt thereof according to any one of aspects 2 to 23,, wherein the sequence $X_4X_3X_2X_1$ (SEQ ID NO:18) is $X_4X_3X_2X_{1a}$ (SEQ ID NO:134), wherein:

$X_{1a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

$X_2$ is selected from the group consisting of: leucine (L), isoleucine (I), alanine (A), tyrosine (Y) and histidine (H);

$X_3$ is selected from the group consisting of: glutamic acid (E), glutamine (Q), homoglutamic acid (hGlu), aspartic acid (D) and alanine (A);

$X_4$ is selected from the group consisting of: tyrosine (Y), 2-naphthalanine (2-Nal), 1-naphthalanine (1-Nal) and alanine (A).

Aspect 26. The G protein peptidomimetic or salt thereof according to any one of aspects 2 to 23, wherein the sequence $X_4X_3X_2X_1$ (SEQ ID NO:18) is $X_{4a}X_3X_2X_1$ (SEQ ID NO:135), wherein:

$X_1$ is selected from the group consisting of: leucine (L), L-$NH_2$, isoleucine (I) and alanine (A);

$X_2$ is selected from the group consisting of: leucine (L), isoleucine (I), alanine (A), tyrosine (Y) and histidine (H);

$X_3$ is selected from the group consisting of: glutamic acid (E), glutamine (Q), homoglutamic acid (hGlu), aspartic acid (D) and alanine (A);

$X_{4a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl

Aspect 27. The G protein peptidomimetic or salt thereof according to any one of aspects 2 to 23, wherein the sequence $X_4X_3X_2X_1$ (SEQ ID NO:18) is $X_4X_{3a}X_2X_1$ (SEQ ID NO:136), wherein:

$X_1$ is selected from the group consisting of: leucine (L), L-$NH_2$, isoleucine (I) and alanine (A);

$X_2$ is selected from the group consisting of: leucine (L), isoleucine (I), alanine (A), tyrosine (Y) and histidine (H);

$X_{3a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2

substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

$X_4$ is selected from the group consisting of: tyrosine (Y), 2-naphthalanine (2-Nal), 1-naphthalanine (1-Nal) and alanine (A) .

Aspect 28. The G protein peptidomimetic or salt thereof according to any one of aspects 2 to 23 comprising a sequence $X_4X_3X_2X_1$ (SEQ ID NO:18) selected from $YEX_{2a}X_{1a}$ (SEQ ID NO:24), $YX_{3a}X_{2a}L$ (SEQ ID NO:25), $X_{4a}X_{3a}LL$ (SEQ ID NO:26), $X_{4a}EX_{2a}L$(SEQ ID NO:27), $X_{4a}ELX_{1a}$ (SEQ ID NO: 28), or $YX_{3a}LX_{1a}$ (SEQ ID NO: 29), wherein $X_{1a}$, $X_{2a}$, $X_{3a}$ and $X_{4a}$ are each independently selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl. In particular embodiments of this aspect, $X_4X_3X_2X_1$ (SEQ ID NO:18) is not YELL (SEQ ID NO:23).

Aspect 29. The G protein peptidomimetic or salt thereof according to any one of aspects 2 to 28, wherein
$X_{1a}$ is a moiety of formula (Ia) or (I'a),
each of $X_{2a}$, $X_{3a}$ and $X_{4a}$ can be independently selected from a moiety of formula (Ib):

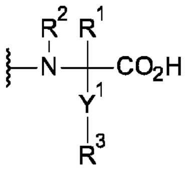 (Ia) 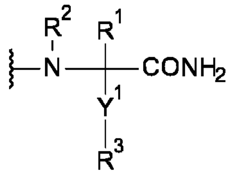 (I'a) 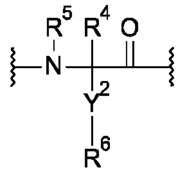 (Ib)

wherein

$R^1$ is hydrogen or $C_{1-6}$alkyl; $R^2$ is hydrogen or $C_{1-6}$alkyl;

$R^3$ is a moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

$Y^1$ is $-C(R^7)R^8-$ or $-C(=O)-$;

$R^7$ is selected from the group comprising hydrogen, OH, SH, $C_{1-6}$alkyl, $C_{3-12}$cycloalkyl, $C_{1-6}$alkoxy, amino, and halo;

$R^8$ is hydrogen or $C_{1-6}$alkyl;

or $R^7$ and at least one substituent of $R^3$ together with the carbon atom to which they are attached form a $C_{3-12}$cycloalkyl, wherein said $C_{3-12}$cycloalkyl can be optionally substituted with one or more substituents independently selected from the group comprising $C_{1-6}$alkyl, OH, halo, $C_{3-12}$cycloalkyl, $C_{2-6}$alkenyl, $C_{1-6}$alkoxy, oxo, $=CH_2$, amino, mono- or di-$C_{1-6}$alkylamino, amino $C_{1-6}$alkyl, and halo$C_{1-6}$alkyl, or two substituents together with the atom to which they are attached may form a $C_{3-12}$cycloalkyl, a $C_{5-12}$cycloalkenyl, a heterocycloalkyl or an $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

$R^4$ is hydrogen or $C_{1-6}$alkyl; $R^5$ is hydrogen or $C_{1-6}$alkyl;

$R^6$ is a moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$ alkoxy, oxo, $=CH_2$, amino, mono- or di- $C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

$Y^2$ is $-C(R^7)R^8-$ or $-C(=O)-$;

$R^7$ is selected from the group comprising hydrogen, OH, SH, $C_{1-6}$alkyl, $C_{3-12}$cycloalkyl, $C_{1-6}$ alkoxy, amino, and halo;

$R^8$ is hydrogen or $C_{1-6}$alkyl;

or $R^7$ and at least one substituent of $R^6$ together with the carbon atom to which they are attached form a $C_{3-12}$cycloalkyl, wherein said $C_{3-12}$cycloalkyl can be optionally substituted with one or more substituents independently selected from the group comprising $C_{1-6}$alkyl, OH, halo, $C_{3-12}$cycloalkyl, $C_{2-6}$alkenyl, $C_{1-6}$alkoxy, oxo, $=CH_2$, amino, mono- or di-$C_{1-6}$alkylamino, amino $C_{1-6}$alkyl, and halo$C_{1-6}$alkyl, or two substituents together with the atom to which they are attached may form a $C_{3-12}$cycloalkyl, a $C_{5-12}$cycloalkenyl, a heterocycloalkyl or an $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

preferably $X_4X_3X_2X_1$ (SEQ ID NO:18) is selected from the group consisting of: $X_4X_3X_{2a}X_1$ (SEQ ID NO:133), $X_4X_3X_2X_{1a}$ (SEQ ID NO:134), $X_{4a}X_3X_2X_1$ (SEQ ID NO:135) and $X_4X_{3a}X_2X_1$ (SEQ ID NO:136)" and $X_{1a}$, $X_{2a}$, $X_{3a}$ and $X_{4a}$ are each independently cyclohexylalanine (Cha), more preferably $X_4X_3X_2X_1$ (SEQ ID NO:18) is $X_4X_3X_{2a}X_1$ (SEQ ID NO:133), and $X_{2a}$ is cyclohexylalanine (Cha).

Aspect 30. The G protein peptidomimetic or salt thereof according to any one of aspects 2 to 29, wherein $X_{1a}$ is a moiety of formula (Ie), and/or $X_{2a}$, $X_{3a}$ and/or $X_{4a}$ can be each independently selected from a moiety of formula (If):

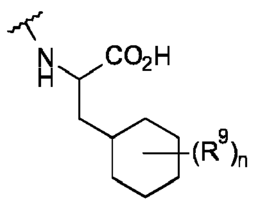
(Ie),
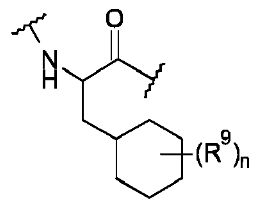
(If)

wherein $R^9$ is selected from the group comprising OH, halo, $C_{1-6}$alkyl, $C_{3-12}$cycloalkyl, $C_{2-6}$alkenyl, $C_{1-6}$ alkoxy, oxo, $=CH_2$, amino, mono- or di- $C_{1-6}$alkylamino, halo$C_{1-6}$alkyl, or two $R^9$ together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl.

Aspect 31. The G protein peptidomimetic or salt thereof according to any one of aspect 2 to 30, wherein $X_{1a}$ , $X_{2a}$, $X_{3a}$ and/or $X_{4a}$ can be each independently selected from phenylalanine (F) or tryptophan (W), preferably tryptophan (W).

Aspect 32. The G protein peptidomimetic or salt thereof according to any one of aspect 2 to 24, 29 to31, comprising a sequence $X_4X_3X_2X_1$ (SEQ ID NO:18) selected from YEChaL (SEQ ID NO:30), YEFL (SEQ ID NO: 31), YEWL (SEQ ID NO: 32), YELCha (SEQ ID NO:33), YELF (SEQ ID NO:34) or YELW (SEQ ID NO:35), preferably selected from YEChaL (SEQ ID NO:30), YEFL (SEQ ID NO: 31) or YEWL (SEQ ID NO: 32), more preferably YEChaL (SEQ ID NO:30).

Aspect 33. The G protein peptidomimetic or salt thereof according to any one of aspects 1 to 32, which is a macrocycle.

Aspect 34. The G protein peptidomimetic or salt thereof according to any one of aspects 3 to 33 comprising at least one covalent tether between $X_8$ and $X_{10}$, between $X_7$ and $X_8$, between $X_5$ and $X_7$, between $X_6$ and $X_8$ or between

$X_6$ and $X_7$, wherein said covalent tether is not part of the linear peptide backbone.

Aspect 35. The G protein peptidomimetic or salt thereof according to any one of aspects 3 to 34 comprising a covalent tether between $X_8$ and $X_{10}$, between $X_7$ and $X_8$, or between $X_5$ and $X_7$, preferably between $X_8$ and $X_{10}$, wherein said covalent tether is not part of the linear peptide backbone.

Aspect 36. The G protein peptidomimetic or salt thereof according to any one of aspects 13 to 35, comprising a covalent tether between $X_8$ and $X_{10}$, wherein said covalent tether is not part of the linear peptide backbone.

Aspect 37. The G protein peptidomimetic or salt thereof according to any one of aspects 13 to 36, comprising a covalent tether between $X_8$ and $X_{10}$, wherein $X_8$ is an amino acid containing an azidated side-chain and $X_{10}$ is an amino acid containing an alkynyl side-chain or wherein $X_{10}$ is an amino acid containing an azidated side-chain and $X_8$ is an amino acid containing an alkynyl side-chain, preferably wherein $X_8$ is azidolysine (Azk) and wherein $X_{10}$ is propargylglycine (Pra) or wherein $X_8$ is propargylglycine (Pra) and wherein $X_{10}$ is azidolysine (Azk), wherein said covalent tether is not part of the linear peptide backbone.

Aspect 38. The G protein peptidomimetic or salt thereof according to any one of aspects 1 to 37 comprising at least one covalent tether, said covalent tether being formed from the reaction of an amino acid containing an amine side-chain with an amino acid containing a carboxylic acid group side-chain, or from the reaction between two olefinic amino acids, or from the reaction of an amino acid containing an azidated side-chain with an amino acid containing an alkynyl side-chain, or from the reaction between two amino acids each containing a thiol group side-chain, or from the reaction of an olefinic amino acid with an amino acid containing a thiol group side-chain, preferably said covalent tether is formed from the reaction of an amino acid containing an azidated side-chain with an amino acid containing an alkynyl side-chain.

Aspect 39. The G protein peptidomimetic or salt thereof according to any one of aspects 13 to 38 comprising at least one covalent tether formed from the reaction of the side-chain of $X_8$ with the side-chain of $X_{10}$, wherein $X_8$ is an amino acid containing an azidated side-chain and $X_{10}$ is an amino acid containing an alkynyl side-chain or wherein $X_{10}$ is an amino acid containing an azidated side-chain and $X_8$ is an amino acid containing an alkynyl side-chain; or formed from the reaction of the side-chain of $X_7$ with the side-chain of $X_8$, wherein $X_7$ is an amino acid containing an azidated side-chain and $X_8$ is an amino acid containing an alkynyl side-chain or wherein $X_8$ is an amino acid containing an azidated side-chain and $X_7$ is an amino acid containing an alkynyl side-chain; or formed from the reaction of the side-chain of $X_5$ with the side-chain of $X_7$, wherein $X_5$ is an amino acid containing an azidated side-chain and $X_7$ is an amino acid containing an alkynyl side-chain or wherein $X_7$ is an amino acid containing an azidated side-chain and $X_5$ is an amino acid containing an alkynyl side-chain, preferably formed from the reaction of the side-chain of $X_8$ with the side-chain of $X_{10}$, wherein $X_8$ is an amino acid containing an azidated side-chain and $X_{10}$ is an amino acid containing an alkynyl side-chain or wherein $X_{10}$ is an amino acid containing an azidated side-chain and $X_8$ is an amino acid containing an alkynyl side-chain.

Aspect 40. The G protein peptidomimetic or salt thereof according to any one of aspects 1 to 39 comprising a covalent tether formed between an amino acid containing an azidated side-chain and an amino acid containing an alkynyl side-chain, wherein said amino acid containing an azidated side-chain is azidolysine (Azk) and wherein said amino acid containing an alkynyl side-chain is propargylglycine (Pra).Aspect 41. The G protein peptidomimetic or salt thereof according to any one of aspects 1, or 13 to 40 comprising the structure shown in formula (X):

$X_{10}X_9RDX_8IQRX_7$ (SEQ ID NO:36) (X)

wherein $X_7$ is selected from the group consisting of: M, C, olefinic amino acids, amino acids containing a carboxylic acid group side chain, amino acids containing an amine side-chain, amino acid containing an alkynyl side-chain, and amino acids containing an azidated side-chain;

wherein $X_8$ is selected from the group consisting of: I, C, olefinic amino acids, amino acids containing a carboxylic acid group side chain, amino acids containing an amine side-chain, amino acid containing an alkynyl side-chain, and amino acids containing an azidated side-chain;

wherein $X_9$ is C or an amino acid without a thiol side-chain;

wherein $X_{10}$ is selected from the group consisting of: D, C, olefinic amino acids, amino acids containing a

carboxylic acid group side chain, amino acids containing an amine side-chain, amino acid containing an alkynyl side-chain, and amino acids containing an azidated side-chain;

wherein:

when $X_8$ is an amino acid containing a thiol group side chain, $X_{10}$ or $X_7$ is an amino acid containing a thiol group side chain,

when $X_8$ is an olefinic amino acid, $X_{10}$ or $X_7$ is an olefinic amino acid,

when $X_8$ is an amino acid containing an amine side-chain, $X_{10}$ or $X_7$ is an amino acid containing a carboxylic acid group side-chain,

when $X_8$ is an amino acid containing a carboxylic acid group side-chain, $X_{10}$ or $X_7$ is an amino acid containing an amine side-chain,

when $X_8$ is an amino acid containing an azidated side-chain, $X_{10}$ or $X_7$ is an amino acid containing an alkynyl side-chain,

when $X_8$ is an amino acid containing an alkynyl side-chain, $X_{10}$ or $X_7$ is an amino acid containing an azidated side-chain; and

wherein said peptidomimetic comprises a covalent tether formed from the reaction of the side chain of $X_8$ with the side chain of $X_{10}$ or with the side chain of $X_7$.

Aspect 42. The G protein peptidomimetic or salt thereof according to aspect 41, wherein said peptidomimetic comprises a covalent tether formed from the reaction of the side chain of $X_8$ with the side chain of $X_{10}$.

Aspect 43. The G protein peptidomimetic or salt thereof according to aspect 41 or 42, wherein $X_7$ is M.

Aspect 44. The G protein peptidomimetic or salt thereof according to any one of aspects 41 to 43, wherein $X_8$ is an amino acid containing an azidated side-chain and $X_{10}$ is an amino acid containing an alkynyl side-chain or wherein $X_8$ is an amino acid containing an alkynyl side-chain and $X_{10}$ an amino acid containing an azidated side-chain, preferably wherein $X_8$ is azidolysine (Azk) and wherein $X_{10}$ is propargylglycine (Pra) or wherein $X_8$ is propargylglycine (Pra) and wherein $X_{10}$ is azidolysine (Azk).

Aspect 45. The G protein peptidomimetic or salt thereof according to any one of aspects 41 to 44 comprising a sequence $X_{10}X_9RDX_8IQRX_7$ (SEQ ID NO: 36) selected from PraXgRDAzklQRM (SEQ ID NO:37), AzkXgRDPralQRM (SEQ ID NO:38), $EX_9RDKIQRM$ (SEQ ID NO:39), $KX_9RDEIQRM$ (SEQ ID NO:40), DXgRDPralQRAzk (SEQ ID NO:41), DXgRDAzklQRPra (SEQ ID NO:42), preferably PraXgRDAzklQRM (SEQ ID NO:37) or AzkXgRDPralQRM (SEQ ID NO:38).

Aspect 46. The G protein peptidomimetic or salt thereof according to any one of aspects 1 to 45 comprising a sequence selected from $FNPraX_9RDAzklQRMHLRQX_4X_3X_2X_1$ (SEQ ID NO:43), $FNAzkX_9RDPralQRMHLRQX_4X_3X_2X_1$ (SEQ ID NO:44), $FNEX_9RDKIQRMHLRQX_4X_3X_2X_1$ (SEQ ID NO:45), $FNKX_9RDEIQRMHLRQX_4X_3X_2X_1$ (SEQ ID NO:46), $FNDX_9RDPralQRAzkHLRQX_4X_3X_2X_1$ (SEQ ID NO:47), $FNDX_9RDAzklQRPraHLRQX_4X_3X_2X_1$ (SEQ ID NO:48), preferably $FNPraX_9RDAzklQRMHLRQX_4X_3X_2X_1$ (SEQ ID NO:43) or $FNAzkX_9RDPralQRMHLRQX_4X_3X_2X_1$ (SEQ ID NO:44).Aspect 47. The G protein peptidomimetic or salt thereof according to any one of aspects 1 to 46, comprising a sequence selected from the group consisting of: FNPraCRDAzklQRMHLRQYEChaL (SEQ ID NO:122), KKKFNPraCRDAzklQRMHLRQYEChaL (SEQ ID NO:123), RDIIQRMHLRQYEChaL (SEQ ID NO:124), FNPraCRDAzklQRMHLRQYEFL (SEQ ID NO:125), KKKFNPraCRDAzklQRMHLRQYEFL (SEQ ID NO:126), FNDCRDIIQRMHLRQYEChaL (SEQ ID NO:127), FNDCRDIIQRMHLRQYEFL (SEQ ID NO:128), FNDCRDIIQRMHLRQYEWL (SEQ ID NO:129), KKFNPraCRDAzklQRMHLRQYEChaL (SEQ ID NO:130), and KFNPraCRDAzklQRMHLRQYEChaL (SEQ ID NO:131).

Aspect 48. The G protein peptidomimetic or salt thereof according to any one of aspects 2 to 47, wherein $X_9$ is C.

Aspect 49. The G protein peptidomimetic or salt thereof according to any one of aspects 1 to 32, 41 to 48, which is

a linear peptide.

Aspect 50. The G protein peptidomimetic or salt thereof according to any one of aspects 1 to 49 comprising a sequence $X_{10}X_9RDX_8IQRX_7$ (SEQ ID NO:36), which is $DX_9RDIIQRM$ (SEQ ID NO:49), wherein $X_9$ is C or an amino acid without a thiol side-chain.

Aspect 51. The G protein peptidomimetic or salt thereof according to any one of aspects 1 to50 comprising the sequence $DX_9RDIIQRMHLRQX_4X_3X_2X_1$ (SEQ ID NO:50).

Aspect 52. The G protein peptidomimetic or salt thereof according to any one of aspects 1 to 51 comprising the sequence $FNDX_9RDIIQRMHLRQX_4X_3X_2X_1$ (SEQ ID NO:51).

Aspect 53. The G protein peptidomimetic or salt thereof according to any one of aspects 1 to 52 comprising the sequence $NARRIFNDX_9RDIIQRMHLRQX_4X_3X_2X_1$ (SEQ ID NO:52).

Aspect 54. The G protein peptidomimetic or salt thereof according to any one of aspects 1 to 53, wherein $X_9$ is A or G, preferably A.

Aspect 55. The G protein peptidomimetic or salt thereof according to any one of aspects 1 to 54 comprising at least one basic amino acid at its N-terminus, preferably from 1 to 6 basic amino acid at its N-terminus, such as at least 2, for example at least 3, for example at least 4, for example at least 5, for example at least 6, more preferably 2 or 3, basic amino acid at its N-terminus.

Aspect 56. The G protein peptidomimetic or salt thereof according to any one of aspects 1 to 55, further comprising at least one lysine at its N-terminus, preferably comprising a triple lysine at its N-terminus.

Aspect 57. The G protein peptidomimetic or salt thereof according to any one of aspects 1 to 56, comprising at least one basic amino acid at its N-terminus, wherein the basic amino acid is selected from lysine (K), histidine (H) and arginine (R), preferably lysine (K).

Aspect 58. The G protein peptidomimetic or salt thereof according to any one of aspects 1 to 57, wherein said G protein peptidomimetic comprises an N-terminal modification.

Aspect 59. The G protein peptidomimetic or salt thereof according to any one of aspects 1 to 58, wherein said G protein peptidomimetic comprises an N-terminal acetylation.

Aspect 60. The G protein peptidomimetic or salt thereof according to any one of aspects 1 to 59, wherein said G protein peptidomimetic is capable of stabilizing a GPCR in an active conformational state.

Aspect 61. The G protein peptidomimetic or salt thereof according to any one of aspects 1 to 60, wherein said G protein peptidomimetic is capable of stabilizing a GPCR in an active conformational state, wherein said GPCR is selected from a Gs protein coupled receptor or a Golf protein coupled receptor, preferably a Gs protein coupled receptor, more preferably an adrenergic receptor or a dopamine receptor, even more preferably a β2 adrenergic receptor or a D1 receptor.

Aspect 62. The G protein peptidomimetic or salt thereof according to any one of aspects 1 to 61, wherein said G protein peptidomimetic induces a shift in $IC_{50}$ value of more than 5, preferably more than 10 or more than 20, more preferably more than 25, more than 30, more than 35, more than 40 or more than 45, even more preferably more than 50, wherein $IC_{50}$ values are determined in a radioligand binding assay (RLA) wherein the GPCR is human β2AR, the radioligand is 3H-DHA, and the unlabelled ligand is isoproterenol.

Aspect 63. The G protein peptidomimetic or salt thereof according to any one of aspects 1 to 62, wherein said G protein peptidomimetic induces a shift in $IC_{50}$ value of an agonist that binds to the GPCR of more than 20, preferably more than 50,, wherein said shift is the $IC_{50}$ value of the agonist for binding to the GPCR in the absence of the G protein peptidomimetic divided by the $IC_{50}$ value of the agonist for binding to the GPCR in the presence of the G protein peptidomimetic, wherein said $IC_{50}$ values are determined in a radioligand binding assay (RLA) wherein the GPCR is human β2AR, the radioligand is 3H-DHA, and the agonist is isoproterenol.

Aspect 64. The G protein peptidomimetic or salt thereof according to any one of aspects 1 to 63, wherein said G protein peptidomimetic is capable of inducing an active conformational state in at least 20%, preferably at least 25%, more preferably at least 50%, of the GPCRs in a population of GPCRs, wherein said fraction of GPCRs in an active conformational state (fraction HI or fraction high) is determined in a radioligand binding assay (RLA) wherein the GPCR is human β2AR, the radioligand is 3H-DHA, and the unlabelled ligand is isoproterenol.

Aspect 65. The G protein peptidomimetic or salt thereof according to any one of aspects 1 to 64, wherein said G protein peptidomimetic is selected from Table C.

Aspect 66. A fusion polypeptide comprising a G protein peptidomimetic according to any one of aspects 1 to 65 and a GPCR, wherein said G protein peptidomimetic and GPCR are optionally fused through a linker.

Aspect 67. A complex comprising a G protein peptidomimetic according to any one of aspects 1 to 65 and a GPCR.

Aspect 68. The complex according to aspect 67 further comprising a receptor ligand.

Aspect 69. A composition comprising a fusion polypeptide according to aspect 66 or a complex according to aspect 67 or 68.

Aspect 70. Use, preferably *in vitro* use, of a G protein peptidomimetic according to any one of aspects 1 to 65, a fusion polypeptide according to aspect 66, a complex according to aspect 67 or 68, or a composition according to aspect 69 to capture a GPCR in an active conformation.

Aspect 71. A method of capturing a GPCR in an active conformation, said method comprising the steps of:

a) bringing a G protein peptidomimetic according to anyone of aspects 1 to 65 into contact with a GPCR, and
b) allowing the G protein peptidomimetic to bind to the GPCR, whereby the GPCR is captured in an active conformation.

Aspect 72. Use of a G protein peptidomimetic according to any one of aspects 1 to 65 for crystallizing a complex of the G protein peptidomimetic and a GPCR and optionally a ligand of the GPCR.

Aspect 73. A method of crystallizing a complex of a G protein peptidomimetic according to any one of aspects 1 to 65 and a GPCR and optionally a ligand of the GPCR, the method comprising the steps of:

a) providing a G protein peptidomimetic according to any one of aspects 1 to 65 and a GPCR, and optionally a ligand of the GPCR,
b) allowing the formation of a complex of the G protein peptidomimetic, the GPCR and optionally the ligand, and
c) crystallizing said complex of step b) to form a crystal.

Aspect 74. A method of determining the crystal structure of a GPCR in an active conformation, the method comprising the steps of:

a. crystallizing a complex of a G protein peptidomimetic according to any one of aspects 1 to 65 and a GPCR, and optionally a ligand of the GPCR according to the method defined in aspect 73 to form a crystal, and
b. obtaining the atomic coordinates of the crystal.

Aspect 75. Use of a G protein peptidomimetic according to any one of aspects 1 to 65, a complex according to aspect 67 or 68, a fusion polypeptide according to aspect 66, or a composition according to aspect 69 for identifying compounds that are capable of interacting with the GPCR, preferably active conformation-selective ligands of the GPCR.

Aspect 76. A screening method for identifying compounds capable of interacting with a GPCR, preferably active conformation-selective ligands of the GPCR, the method comprising the steps:

a) contacting the GPCR with a test compound and a G protein peptidomimetic according to any one of aspect 1 to 65, a complex according to aspect 67 or 68, a fusion polypeptide according to aspect 66, or a composition according to aspect 69;

b) evaluating binding of the test compound to the GPCR; and

c) optionally selecting a test compound that binds to the GPCR as a compound capable of interacting with the GPCR.

The term "peptidomimetic" generally refers to any compound that biologically mimics a peptide or protein. Therefore, a suitable definition of a peptidomimetic as described herein may be 'compounds whose essential elements mimic a natural peptide or protein in 3D space and which retain the ability to interact with the biological target and produce the same biological effect' as formulated by Vagner et al. (2008, Current Opinion in Chemical Biology 292:296). A skilled person readily appreciates that peptidomimetics are commonly designed by modification of an existing peptide, although this is not a prerequisite. Thus, the design process of a peptidomimetic is not particularly limited, and may therefore be generated by various strategies including but by no means limited to approaches such as rational engineering, directed evolution, random mutagenesis, (alanine or D-amino acid) scanning approaches, or any combination thereof.

By means of guidance, the G protein peptidomimetics as described herein would generally be considered a type I or type II mimetic when using the classification system of Ripka and Rich (2008 Current Opinion in Chemical Biology 2:441:452). Therefore, in preferred embodiments, the G protein peptidomimetics as described herein are type I (i.e. structural) mimetics or type II (i.e. functional) mimetics. A skilled person appreciates from the cited art that type I mimetics show a strict analogy with the native substrate and carry all the functionalities in the same spatial orientation. Type II mimetics do not show apparent structural analogies with the native substrate, but are able to mimic its function by interacting similarly with the target receptor or enzyme. In an alternative embodiment, the peptidomimetic may be a type III (functional-structural) mimetic that possesses a scaffold significantly different from the native substrate while displaying the interacting elements in the same spatial orientation.

More recently, a new classification system for peptidomimetics has been formulated by Pelay-Gimeno et al. (2015 Angewandte Chemie International Edition 54:8896:8927). This classification system differs from the one of Ripka and Rich in that it is centered around the degree of peptide character. When using this classification system, peptidomimetics may be stratified in four classes (A-D):

- Class A mimetics contain a limited number of local modifications, which are mainly introduced to stabilize the conformation and/or limit the proteolysis degradation rate. The backbone and side-chains of the mimetics show a close alignment with the topography of the native peptide.

- Class B mimetics contain more extensive modifications in their sequence, said modifications being present in both the backbone and side-chains. Non-natural amino acids are envisaged, as well as isolated small-molecule building blocks and backbone mimetics.

- Class C mimetics have an increased small-molecule character when compared to class A and class B peptidomimetics and are characterized by a non-peptide unnatural frame replacing the backbone of the native substrate. The interacting elements are still presented in the same topological manner, but the peptide backbone is globally altered.

- Class D mimetics mimic the mode of action of the natural substrate but do no longer share a direct link to the side-chain functionalities. Class D mimetics are considered the least similar to the original peptide.

In the present disclosure, the peptidomimetics as described herein are generally considered to have a peptide or peptide-like backbone structure and would therefore classify as either a class A peptidomimetic or class B peptidomimetic. It is therefore understood that the peptidomimetics as described herein still have a certain degree of sequence similarity to the native substrate, herein the G protein or mini Gs protein unless explicitly indicated otherwise. Hence, in certain preferred embodiments the G protein peptidomimetic is a class A peptidomimetic. In alternative embodiments, the G protein peptidomimetic is a class B peptidomimetic.

The term "protein" as used throughout this specification generally encompasses macromolecules comprising one or more polypeptide chains, i.e., polymeric chains of amino acid residues linked by peptide bonds. The term may encompass naturally, recombinantly, semi-synthetically or synthetically produced proteins. The term also encompasses proteins that carry one or more co- or post-expression-type modifications of the polypeptide chain(s), such as, without limitation, glycosylation, acetylation, guanidinylation, phosphorylation, sulfonation, methylation, ubiquitination, signal peptide removal, N-terminal Met removal, conversion of pro-enzymes or pre-hormones into active forms, etc. The term further also includes protein variants or mutants which carry amino acid sequence variations vis-à-vis a corresponding native proteins, such as, e.g., amino acid deletions, additions and/or substitutions. The term contemplates both full-length proteins and protein parts or fragments, e.g., naturally-occurring protein parts that ensue from processing of such full-length proteins.

The term "polypeptide" as used throughout this specification generally encompasses polymeric chains of amino acid residues linked by peptide bonds. Hence, especially when a protein is only composed of a single polypeptide chain, the terms "protein" and "polypeptide" may be used interchangeably herein to denote such a protein. The term is not limited to any minimum length of the polypeptide chain. The term may encompass naturally, recombinantly, semi-synthetically or synthetically produced polypeptides. The term also encompasses polypeptides that carry one or more co- or post-expression-type modifications of the polypeptide chain, such as, without limitation, glycosylation, acetylation, phosphorylation, sulfonation, methylation, ubiquitination, signal peptide removal, N-terminal Met removal, conversion of pro-enzymes or pre-hormones into active forms, etc. The term further also includes polypeptide variants or mutants which carry amino acid sequence variations vis-à-vis a corresponding native polypeptide, such as, e.g., amino acid deletions, additions and/or substitutions. The term contemplates both full-length polypeptides and polypeptide parts or fragments, e.g., naturally-occurring polypeptide parts that ensue from processing of such full-length polypeptides.

The term "peptide" as used throughout this specification preferably refers to a short chain of amino acid residues linked by peptide bonds comprising 50 amino acids or less, e.g., 45 amino acids or less, preferably 40 amino acids or less, e.g., 35 amino acids or less, more preferably 30 amino acids or less, e.g., 25 or less, 20 or less, 15 or less or 10 or less amino acids. No strict maximal length is attributed to a peptide to still be considered a peptide. The term peptide may encompass naturally, recombinantly, semi-synthetically or synthetically produced peptides such as discussed for polypeptides above.

The term "amino acid" encompasses naturally occurring amino acids, naturally encoded amino acids or proteinogenic amino acids, non-naturally encoded amino acids, non-naturally occurring amino acids, amino acid analogues and amino acid mimetics that function in a manner similar to the naturally occurring amino acids, all in their D- and L-stereoisomers, provided their structure allows such stereoisomeric forms. Amino acids are referred to herein by either their name, their commonly known three letter codes or by the one-letter codes recommended by the IUPAC-IUB Biochemical Nomenclature Commission. A "naturally encoded amino acid" refers to an amino acid that is one of the 20 common amino acids or pyrrolysine, pyrroline-carboxy-lysine or selenocysteine. The 20 common amino acids are: alanine (A or Ala), cysteine (C or Cys), aspartic acid (D or Asp), glutamic acid (E or Glu), phenylalanine (F or Phe), glycine (G or Gly), histidine (H or His), isoleucine (I or Ile), lysine (K or Lys), leucine (L or Leu), methionine (M or Met), asparagine (N or Asn), proline (P or Pro), glutamine (Q or Gln), arginine (R or Arg), serine (S or Ser), threonine (T or Thr), valine (V or Val), tryptophan (W or Trp), and tyrosine (Y or Tyr). Also included are amino acid analogues, in which one or more individual atoms have been replaced either with a different atom, an isotope of the same atom, or with a different functional group.

"Side-chain" as used herein and spelled interchangeably in the art by "side chain" or "sidechain" refers to a chemical group that is attached to a main chain or backbone of a molecule. Side-chain as used herein is to be interpreted in accordance with this definition unless specified otherwise. Side-chains of amino acids are attached to the alpha-carbon of the amide backbone. Certain side-chains or groups of side-chain may be annotated or simplified in the art by the letter "R". Amino acid side-chains determine both charge and polarity of amino acids.

The terms "backbone", "(poly)peptide backbone", or "protein backbone" as used interchangeably herein are to be interpreted in their generally accepted meaning in the art. The peptide backbone is thus indicative for the peptide bonds between a first amino acid to a second consecutive amino acid. Peptide bonds are thus amide bonds that link the non-side-chain or alpha-carboxyl group of one amino acid with the non-side chain or alpha-amino group of the other amino acid. Peptide bond formation is a dehydration synthesis reaction.

In accordance with the above, the term peptidomimetic as used herein is used to describe peptide or peptide-like molecules that do not have a 100% sequence identity to the naturally occurring substrate peptide or protein yet nevertheless exert a similar or identical function to said peptide or protein. Hence, the sequence of a peptidomimetic does not occur in natural peptides or proteins, but contains at least one residue that has been substituted, chemically modified, deleted, and/or added when compared to the naturally occurring sequence. A peptidomimetic may therefore comprise one or more mutated amino acids and/or one or more non-naturally occurring (i.e. artificial) amino acids as part of its protein or protein-like chain compared to the native substrate peptide. Optionally, the peptidomimetics as described herein may have a higher stability towards proteolysis, better permeability properties, better transport properties, and/or improved selectivity against non-target receptors compared to the naturally occurring peptide. It is evident that many of the herein described mutations and modifications may be replaced by amino acid analogues known to a skilled person. Peptidomimetic molecules comprising one or more of such amino acid analogues are also envisaged by the inventors.

The peptidomimetics disclosed herein can be readily prepared using standard techniques known in the art, including chemical synthesis (Merrifield, 1963) and genetic engineering. When non-proteinogenic amino acids are contained in the peptidomimetics disclosed herein, they may be either added directly to the growing chain during peptide synthesis or prepared by chemical modification of the complete synthesized peptide, depending on the nature of the desired non-proteinogenic amino acid. Those of skill in the chemical synthesis art are well aware of which non-proteinogenic amino acids may be added directly and which must be synthesized by chemically modifying the complete peptide

chain following peptide synthesis (reviewed in Jaradat 2018 Amino Acids 50:39-68). Alternatively, where the peptidomimetic is synthesized by a cellular expression system, certain codons may be reprogrammed and allocated in said expression system to encode non-naturally occurring amino acids (see e.g. Xie and Schultz 2005 Current Opinion Chemical Biology 548:554 and Kuo et al. 2018 Current Genetics 327-333). The occurrence of non-naturally occurring amino acids in the peptidomimetics therefore does not exclude synthesis by expression systems.

The term "G protein peptidomimetic" as used herein refers to a compound that biologically mimics a G protein, in particular the α-subunit of a G protein. In particular, the G protein peptidomimetics disclosed herein produce and/or stabilize a conformational change of a GPCR upon binding or interaction with the GPCR, which mimics the conformational state of the GPCR upon interaction or binding with the G protein. It is understood that "a G protein" is not to be regarded in a limiting singular "G protein" interpretation, and a single G protein peptidomimetic may therefore biologically mimic one or more different (a-subunits of) G proteins.

With "G proteins" are meant the family of guanine nucleotide-binding proteins involved in transmitting chemical signals outside the cell and causing changes inside the cell. G proteins are key molecular components in the intracellular signal transduction following ligand binding to the extracellular domain of a GPCR. They are also referred to as "heterotrimeric G proteins", or "large G proteins". G proteins consist of three subunits: alpha (α), beta (β), and gamma (γ) and their classification is largely based on the identity of their distinct α subunits, and the nature of the subsequent transduction event. Further classification of G proteins has come from cDNA sequence homology analysis. G proteins bind either guanosine diphosphate (GDP) or guanosine triphosphate (GTP) and possess highly homologous guanine nucleotide binding domains and distinct domains for interactions with receptors and effectors. Different subclasses of Gα proteins, such as Gas, Gai, Gaq and Gα12, amongst others, signal through distinct pathways involving second messenger molecules such as cAMP, inositol triphosphate (IP3), diacylglycerol, intracellular $Ca^{2+}$ and RhoA GTPases. To illustrate this further, the α subunit (39 - 46 kDa) contains the guanine nucleotide binding site and possesses GTPase activity; the β (37 kDa) and γ (8 kDa) subunits are tightly associated and function as a βγ heterodimer. There are 23 types (including some splicing isoforms) of α subunits, 6 of β, and 11 of γ currently described. The classes of G protein and subunits are subscripted: thus, for example, the α subunit of Gs protein (which activates adenylate cyclase) is Gsα; other G proteins include Gi, which differs from Gs structurally (different type of α subunit) and inhibits adenylate cyclase. Further examples are provided in Table A.

Table A. Non-limiting examples of G proteins and their relationship with G protein-coupled receptors and signalling pathways.

| G protein family | α subunit | Effectors/Signalling pathways | Use/Receptors |
|---|---|---|---|
| **Gi family** | | | |
| Gi Go | αi αo | Inhibition of adenylate cyclase (cAMP ↓) Closing $Ca^{2+}$ channels | Acetylcholine M2 & M4 receptors<br>Adenosine A1 & A3 receptors<br>Adrenergic α2A, α2B, & α2C receptors<br>Apelin receptors<br>Calcium-sensing receptor<br>Chemokine CXCR4 receptor<br>Dopamine D2, D3, D4<br>GABAB receptor<br>Glutamate mGluR2, mGluR3, mGluR4, mGluR6,<br>mGluR7, & mGluR8 receptors<br>Histamine H2 & H3 & H4 receptors<br>Melatonin MT1, MT2, & MT3 receptors<br>Muscarinic M2 & M4 receptors<br>Opioid δ, κ, μ, & nociceptin receptors<br>Prostaglandin EP1, EP3, FP, & TP receptors<br>Serotonin 5-HT1 & 5-HT5 receptors |

(continued)

| G protein family | α subunit | Effectors/Signalling pathways | Use/Receptors |
|---|---|---|---|
| **Gi family** | | | |
| Gt | αt (transducin) | Activation phosphodiesterase 6 (vision) | Rhodopsin |
| Ggust | αgust (gustducin) | Activation phosphodiesterase 6 (vision) | Taste receptors |
| Gz | αz | Inhibition adenylate cyclase (cAMP ↓) | unknown |
| **Gs family** | | | |
| Gs | αs | Activation adenylate cyclase (cAMP ↑) | 5-HT receptors types 5-HT4 and 5-HT7<br>ACTH receptor<br>Adenosine receptor types A2a and A2b<br>Arginine vasopressin receptor 2<br>β-adrenergic receptors types $\beta_1$, $\beta_2$ and $\beta_3$<br>Calcitonin receptor<br>Calcitonin gene-related peptide receptor<br>Corticotropin-releasing hormone receptor<br>Dopamine receptors D1-like family (D1 and D5)<br>FSH-receptor<br>Gastric inhibitory polypeptide receptor<br>Glucagon receptor<br>Histamine H2 receptor<br>Luteinizing hormone/ choriogonadotropin receptor<br>Melanocortin receptor<br>Parathyroid hormone receptor 1<br>Prostaglandin receptor types D2 and I2<br>Secretin receptor |
| | | | Thyrotropin receptor |
| Golf | αolf | Activation adenylate cyclase (cAMP ↑) | Olfactory receptors |

(continued)

| | | | |
|---|---|---|---|
| **Gq family** | | | |
| Gq | $\alpha q$ | Activation of phospholipase C ($IP_3$ ↑) | 5-HT2 serotonergic receptors<br>Alpha-1 adrenergic receptor<br>Vasopressin type 1 receptor<br>Angiotensin II receptor type 1<br>Calcitonin receptor<br>Histamine H1 receptor<br>Metabotropic glutamate receptor, Group I<br>M1, M3, and M5 muscarinic receptors |
| **G12/13 family** | | | |
| G12 | $\alpha 12$ | | |
| G13 | $\alpha 13$ | $Na^+/H^+$ exchange ↑ | |
| | | | |
| | **$\beta\gamma$ subunit** | **Effectors/Signalling pathways** | |
| | $\beta\gamma$ | Opening $K^+$ channels ($K^+$ ↑) | |
| | $\beta\gamma$ | Adenylate cyclase (cAMP) ↑ or ↓ | |
| | $\beta\gamma$ | Phospholipase C ($IP_3$) | |

Typically, in nature, G proteins are in a nucleotide-bound form. More specifically, G proteins (or at least the $\alpha$ subunit) are bound to either GTP or GDP depending on the activation status of a particular GPCR. Agonist binding to a GPCR promotes interactions with the GDP-bound $G\alpha\beta\gamma$ heterotrimer leading to the exchange of GDP for GTP on Ga, and the functional dissociation of the G protein into $G\alpha$-GTP and $G\beta\gamma$ subunits. The separate $G\alpha$-GTP and $G\beta\gamma$ subunits can modulate, either independently or in parallel, downstream cellular effectors (channels, kinases or other enzymes, see Table A). The intrinsic GTPase activity of $G\gamma$ leads to hydrolysis of GTP to GDP and the re-association of $G\alpha$-GDP and $G\beta\gamma$ subunits, and the termination of signalling. Thus, G proteins serve as regulated molecular switches capable of eliciting bifurcating signals through $\alpha$ and $\beta\gamma$ subunit effects. The switch is turned on by the receptor and it turns itself off within a few seconds, a time sufficient for considerable amplification of signal transduction. Methods for assessing GPCR signal transduction have been described in the art (e.g. Ratnayake et al. 2017 Methods Cellular Biology, 1:25)

The G protein peptidomimetics disclosed herein can be, without limitation, Gs protein mimetics, Golf protein mimetics, Gi protein mimetics, Go protein mimetics, Gt protein mimetics, Ggust protein mimetics, Gz protein mimetics, Gq protein mimetics, G12 protein mimetics or G13 protein mimetics. In particular embodiments, the G protein peptidomimetics disclosed herein are Gs protein mimetics or Golf protein mimetics, preferably Gs protein mimetics.

The G protein peptidomimetics described herein arise from modifications of the $\alpha_5$ helix of $G\alpha_s$ protein, in particular from modifications of peptides comprising or consisting of the amino acid sequence set forth in SEQ ID NO:117 [FNDCRDIIQRMHLRQYELL] or fragments thereof, and are characterized in that they are capable of stabilizing a G protein coupled receptor in an active conformational state.

In preferred embodiments, the G protein peptidomimetics are peptides or peptide-like molecules whose amino acid sequence is derived from the amino acid sequence set forth in SEQ ID NO:117 or a fragment thereof. Preferably, the peptidomimetics are not fragments of the $\alpha_5$ helix of $G\alpha_s$ protein, although their amino acid sequence is derived from the linear sequence of said $\alpha_5$ helix.

In particular, the G protein peptidomimetics described herein comprise or consist of the structure shown in formula (I):

$$HLX_6X_5X_4X_3X_2X_1 \text{ (SEQ ID NO: 1)} \qquad (I)$$

wherein $X_1$ is selected from the group consisting of: leucine (L), L-$NH_2$, isoleucine (I), alanine (A) and $X_{1a}$; wherein

$X_{1a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein $X_2$ is selected from the group consisting of: leucine (L), isoleucine (I), alanine (A), tyrosine (Y), histidine (H), and $X_{2a}$, wherein $X_{2a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein $X_3$ is selected from the group consisting of: glutamic acid (E), glutamine (Q), homoglutamic acid (hGlu), aspartic acid (D), alanine (A) and $X_{3a}$, wherein $X_{3a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein $X_4$ is selected from the group consisting of: tyrosine (Y), 2-naphthalanine (2-Nal), 1-naphthalanine (1-Nal), alanine (A) and $X_{4a}$, wherein $X_{4a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein $X_5$ is selected from the group consisting of: Q, C, olefinic amino acids, amino acids containing a carboxylic acid group side-chain, amino acids containing an amine side-chain, amino acid containing an alkynyl side-chain, and amino acids containing an azidated side-chain;

wherein $X_6$ is selected from the group consisting of: R, C, olefinic amino acids, amino acids containing a carboxylic acid group side-chain, amino acids containing an amine side-chain, amino acid containing an alkynyl side-chain, and amino acids containing an azidated side-chain; and

wherein $X_1$ is $X_{1a}$, $X_2$ is $X_{2a}$, $X_3$ is $X_{3a}$ and/or $X_4$ is $X_{4a}$ and/or wherein said peptidomimetic comprises at least one covalent tether, said covalent tether being formed from the reaction of an amino acid containing an amine side-chain with an amino acid containing a carboxylic acid group side-chain, or from the reaction between two olefinic amino acids, or from the reaction of an amino acid containing an azidated side-chain with an amino acid containing an alkynyl side-chain, or from the reaction between two amino acids each containing a thiol group side-chain, or from the reaction of an olefinic amino acid with an amino acid containing a thiol group side-chain, preferably said covalent tether is formed from the reaction of an amino acid containing an azidated side-chain with an amino acid containing an alkynyl side-chain.

In embodiments of the G protein peptidomimetic,
$X_1$ is selected from the group consisting of: leucine (L), L-$NH_2$, isoleucine (I) and $X_{1a}$;

$X_2$ is selected from the group consisting of: leucine (L), isoleucine (I), tyrosine (Y), histidine (H), and $X_{2a}$;
$X_3$ is selected from the group consisting of: glutamic acid (E), glutamine (Q), homoglutamic acid (hGlu), aspartic acid (D), alanine (A) and $X_{3a}$; and
$X_4$ is selected from the group consisting of: tyrosine (Y), 2-naphthalanine (2-Nal), 1-naphthalanine (1-Nal), and $X_{4a}$;
wherein $X_{1a}$, $X_{2a}$, $X_{3a}$ and $X_{4a}$ are as defined above.

In embodiments, the G protein peptidomimetics comprise or consist of the structure shown in formula (II):

$X_7HLX_6X_5X_4X_3X_2X_1$ (SEQ ID NO: 2) (II)

wherein $X_1$, $X_2$, $X_3$, $X_4$, $X_5$ and $X_6$ are as defined above; and

wherein $X_7$ is selected from the group consisting of: M, C, olefinic amino acids, amino acids containing a carboxylic acid group side-chain, amino acids containing an amine side-chain, amino acid containing an alkynyl side-chain, and amino acids containing an azidated side-chain.

In embodiments, the G protein peptidomimetics comprise or consist of the structure shown in formula (III):

$RX_7HLX_6X_5X_4X_3X_2X_1$ (SEQ ID NO: 3) (III)

wherein $X_1$, $X_2$, $X_3$, $X_4$, $X_5$, $X_6$ and $X_7$ are as defined above.

In embodiments, the G protein peptidomimetics comprise or consist of the structure shown in formula (IV):

$QRX_7HLX_6X_5X_4X_3X_2X_1$ (SEQ ID NO: 4) (IV)

wherein $X_1$, $X_2$, $X_3$, $X_4$, $X_5$, $X_6$ and $X_7$ are as defined above.

In embodiments, the G protein peptidomimetics comprise or consist of the structure shown in formula (V):

$IQRX_7HLX_6X_5X_4X_3X_2X_1$ (SEQ ID NO: 5) (V)

wherein $X_1$, $X_2$, $X_3$, $X_4$, $X_5$, $X_6$ and $X_7$ are as defined above.

In embodiments, the G protein peptidomimetics comprise or consist of the structure shown in formula (VI):

$X_8IQRX_7HLX_6X_5X_4X_3X_2X_1$ (SEQ ID NO: 6) (VI)

wherein $X_1$, $X_2$, $X_3$, $X_4$, $X_5$, $X_6$ and $X_7$ are as defined above; and

wherein $X_8$ is selected from the group consisting of: I, C, olefinic amino acids, amino acids containing a carboxylic acid group side-chain, amino acids containing an amine side-chain, amino acid containing an alkynyl side-chain, and amino acids containing an azidated side-chain.

In embodiments, the G protein peptidomimetics comprise or consist of the structure shown in formula (VII):

$DX_8IQRX_7HLX_6X_5X_4X_3X_2X_1$ (SEQ ID NO: 7) (VII)

wherein $X_1$, $X_2$, $X_3$, $X_4$, $X_5$, $X_6$, $X_7$ and $X_8$ are as defined above.

In embodiments, the G protein peptidomimetics comprise or consist of the structure shown in formula (VIII):

$RDX_8IQRX_7HLX_6X_5X_4X_3X_2X_1$ (SEQ ID NO: 8) (VIII)

wherein $X_1$, $X_2$, $X_3$, $X_4$, $X_5$, $X_6$, $X_7$ and $X_8$ are as defined above.

In embodiments, the G protein peptidomimetics comprise or consist of the structure shown in formula (IX):

$X_9RDX_8IQRX_7HLX_6X_5X_4X_3X_2X_1$ (SEQ ID NO: 9) (IX)

wherein $X_1$, $X_2$, $X_3$, $X_4$, $X_5$, $X_6$, $X_7$ and $X_8$ are as defined above; and

wherein $X_9$ is C or an amino acid without a thiol side-chain.

In embodiments, the G protein peptidomimetics comprise or consist of the structure shown in formula (X):

$X_{10}$ $X_9RDX_8IQRX_7HLX_6X_5X_4X_3X_2X_1$ (SEQ ID NO: 10) (X)

wherein $X_1$, $X_2$, $X_3$, $X_4$, $X_5$, $X_6$, $X_7$, $X_8$ and $X_9$ are as defined above; and

wherein $X_{10}$ is selected from the group consisting of: D, C, olefinic amino acids, amino acids containing a carboxylic acid group side-chain, amino acids containing an amine side-chain, amino acid containing an alkynyl side-chain, and amino acids containing an azidated side-chain.

In embodiments, the G protein peptidomimetics comprise or consist of the structure shown in formula (XI):

$NX_{10}$ $X_9RDX_8IQRX_7HLX_6X_5X_4X_3X_2X_1$ (SEQ ID NO: 11) (XI)

wherein $X_1$, $X_2$, $X_3$, $X_4$, $X_5$, $X_6$, $X_7$, $X_8$, $X_9$ and $X_{10}$ are as defined above.

In embodiments, the G protein peptidomimetics comprise or consist of the structure shown in formula (XII):

$FNX_{10}X_9RDX_8IQRX_7HLX_6X_5X_4X_3X_2X_1$ (SEQ ID NO: 12) (XII)

wherein $X_1$, $X_2$, $X_3$, $X_4$, $X_5$, $X_6$, $X_7$, $X_8$, $X_9$ and $X_{10}$ are as defined above.

In embodiments, the G protein peptidomimetics comprise or consist of the structure shown in formula (XIII):

$IFNX_{10}X_9RDX_8IQRX_7HLX_6X_5X_4X_3X_2X_1$ (SEQ ID NO: 13) (XIII)

wherein $X_1$, $X_2$, $X_3$, $X_4$, $X_5$, $X_6$, $X_7$, $X_8$, $X_9$ and $X_{10}$ are as defined above.

In embodiments, the G protein peptidomimetics comprise or consist of the structure shown in formula (XIV):

$RIFNX_{10}X_9RDX_8IQRX_7HLX_6X_5X_4X_3X_2X_1$ (SEQ ID NO: 14) (XIV)

wherein $X_1$, $X_2$, $X_3$, $X_4$, $X_5$, $X_6$, $X_7$, $X_8$, $X_9$ and $X_{10}$ are as defined above.

In embodiments, the G protein peptidomimetics comprise or consist of the structure shown in formula (XV):

$RRIFNX_{10}X_9RDX_8IQRX_7HLX_6X_5X_4X_3X_2X_1$ (SEQ ID NO: 15) (XV)

wherein $X_1$, $X_2$, $X_3$, $X_4$, $X_5$, $X_6$, $X_7$, $X_8$, $X_9$ and $X_{10}$ are as defined above.

In embodiments, the G protein peptidomimetics comprise or consist of the structure shown in formula (XVI):

$ARRIFNX_{10}X_9RDX_8IQRX_7HLX_6X_5X_4X_3X_2X_1$ (SEQ ID NO: 16) (XVI)

wherein $X_1$, $X_2$, $X_3$, $X_4$, $X_5$, $X_6$, $X_7$, $X_8$, $X_9$ and $X_{10}$ are as defined above.

In embodiments, the G protein peptidomimetics comprise or consist of the structure shown in formula (XVII):

$NARRIFNX_{10}X_9DX_8IQRX_7HLX_6X_5X_4X_3X_2X_1$ (SEQ ID NO: 17) (XVII)

wherein $X_1$, $X_2$, $X_3$, $X_4$, $X_5$, $X_6$, $X_7$, $X_8$, $X_9$ and $X_{10}$ are as defined above.

In embodiments of the G protein peptidomimetics according to any one of formula (II) to (XVII),

when $X_5$ is an amino acid containing a thiol group side-chain, $X_7$ is an amino acid containing a thiol group side-chain,

when $X_5$ is an olefinic amino acid, $X_7$ is an olefinic amino acid,

when $X_5$ is an amino acid containing an amine side-chain, $X_7$ is an amino acid containing a carboxylic acid group side-chain,

when $X_5$ is an amino acid containing a carboxylic acid group side-chain, $X_7$ is an amino acid containing an amine

side-chain,

when $X_5$ is an amino acid containing an azidated side-chain, $X_7$ is an amino acid containing an alkynyl side-chain,

when $X_5$ is an amino acid containing an alkynyl side-chain, $X_7$ is an amino acid containing an azidated side-chain.

In embodiments of the G protein peptidomimetics according to any one of formula (II) to (XVII),

when $X_7$ is an amino acid containing a thiol group side-chain, $X_8$ or $X_5$ is an amino acid containing a thiol group side-chain,

when $X_7$ is an olefinic amino acid, $X_8$ or $X_5$ is an olefinic amino acid,

when $X_7$ is an amino acid containing an amine side-chain, $X_8$ or $X_5$ is an amino acid containing a carboxylic acid group side-chain,

when $X_7$ is an amino acid containing a carboxylic acid group side-chain, $X_8$ or $X_5$ is an amino acid containing an amine side-chain,

when $X_7$ is an amino acid containing an azidated side-chain, $X_8$ or $X_5$ is an amino acid containing an alkynyl side-chain,

when $X_7$ is an amino acid containing an alkynyl side-chain, $X_8$ or $X_5$ is an amino acid containing an azidated side-chain.

In embodiments of the G protein peptidomimetics according to any one of formula (VI) to (XVII),

when $X_8$ is an amino acid containing a thiol group side-chain, $X_{10}$ or $X_7$ is an amino acid containing a thiol group side-chain,

when $X_8$ is an olefinic amino acid, $X_{10}$ or $X_7$ is an olefinic amino acid,

when $X_8$ is an amino acid containing an amine side-chain, $X_{10}$ or $X_7$ is an amino acid containing a carboxylic acid group side-chain,

when $X_8$ is an amino acid containing a carboxylic acid group side-chain, $X_{10}$ or $X_7$ is an amino acid containing an amine side-chain,

when $X_8$ is an amino acid containing an azidated side-chain, $X_{10}$ or $X_7$ is an amino acid containing an alkynyl side-chain,

when $X_8$ is an amino acid containing an alkynyl side-chain, $X_{10}$ or $X_7$ is an amino acid containing an azidated side-chain.

In embodiments of the G protein peptidomimetics according to any one of formula (X) to (XVII),

when $X_{10}$ is an amino acid containing a thiol group side-chain, $X_8$ is an amino acid containing a thiol group side-chain,

when $X_{10}$ is an olefinic amino acid, $X_8$ is an olefinic amino acid,

when $X_{10}$ is an amino acid containing an amine side-chain, $X_8$ is an amino acid containing a carboxylic acid group side-chain,

when $X_{10}$ is an amino acid containing a carboxylic acid group side-chain, $X_8$ is an amino acid containing an amine side-chain,

when $X_{10}$ is an amino acid containing an azidated side-chain, $X_8$ is an amino acid containing an alkynyl side-chain,

when $X_{10}$ is an amino acid containing an alkynyl side-chain, $X_8$ is an amino acid containing an azidated side-chain.

In embodiments of the G protein peptidomimetics according to any one of formula (II) to (XVII),

when $X_6$ is an amino acid containing a thiol group side-chain, $X_7$ or $X_8$ is an amino acid containing a thiol group side-chain,

when $X_6$ is an olefinic amino acid, $X_7$ or $X_8$ is an olefinic amino acid,

when $X_6$ is an amino acid containing an amine side-chain, $X_7$ or $X_8$ is an amino acid containing a carboxylic acid group side-chain,

when $X_6$ is an amino acid containing a carboxylic acid group side-chain, $X_7$ or $X_8$ is an amino acid containing an amine side-chain,

when $X_6$ is an amino acid containing an azidated side-chain, $X_7$ or $X_8$ is an amino acid containing an alkynyl side-chain,

when $X_6$ is an amino acid containing an alkynyl side-chain, $X_7$ or $X_8$ is an amino acid containing an azidated side-chain.

In particular embodiments, the G protein peptidomimetics have a peptide backbone length of at least 8 amino acids, at least 11 amino acids or at least 13 amino acids, preferably at least 15 amino acids, at least 16 amino acids, at least 17 amino acids or at least 18 amino acids, more preferably at least 19 amino acids, at least 20 amino acids or at least 21 amino acids, even more preferably at least 22 amino acids or at least 23 amino acids, most preferably at least 24 amino acids. In certain embodiments, the G protein peptidomimetics have a peptide backbone length of between 8 and 30 amino acids, preferably between 8 and 28, preferably between 8 and 25 amino acids, preferably between 8 and 24 amino acids, preferably between 8 and 22 amino acids, preferably between 8 and 20 amino acids, preferably between 10 and 20 amino acids. In certain embodiments, the G protein peptidomimetics have a peptide backbone length of between 15 and 30 amino acids, between 19 and 30 amino acids, between 22 and 30 amino acids or between 24 and 30 amino acids.

In the following paragraphs, different suitable, more specific, modifications that may be comprised in the peptidomimetics are described. It is evident that these different modifications may be combined into a peptidomimetic depending on the needs of individual examples and their objectives. In certain embodiments, the combination of multiple modifications is causative for a synergistic effect on the final peptidomimetic when compared to peptidomimetics comprising less modifications. However, by no means a generalization may be made that addition of modifications de facto lead to an improved peptidomimetic, and each combination should be assessed on its own merits.

G protein peptidomimetics with C-terminal modifications

In preferred embodiments of the G protein peptidomimetics disclosed herein, at least one of $X_1$, $X_2$, $X_3$ and $X_4$, is an alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl. In embodiments, one of $X_1$, $X_2$, $X_3$ and $X_4$, preferably $X_2$, is an alanine analogue as defined herein, phenylalanine (F) or tryptophan (W). In other embodiments at least two, for example, two, three or all of $X_1$, $X_2$, $X_3$ and $X_4$ are an alanine analogue as defined herein, phenylalanine (F) or tryptophan (W). In a particularly preferred embodiment, $X_2$ is an alanine analogue as defined herein, phenylalanine (F) or tryptophan (W).

The term "alanine" (code Ala or A) refers to an amino acid containing an amino group and a carboxylic acid group, both attached to the central carbon atom which also carries a methyl group side-chain.

As used herein, the term "alanine analogue" refers to a molecule resulting from the replacement of any hydrogen of alanine by at least one moiety selected from the group comprising $C_{1-6}$alkyl, $C_{6-12}$cycloalkyl, $C_{6-12}$aryl, heteroaryl, $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo $C_{1-6}$alkyl; or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or aryl may be optionally substituted by one or more $C_{1-6}$alkyl. Preferably, the alanine analogue refers to a molecule resulting from the replacement of at least one hydrogen of the methyl group side-chain of alanine by at least one moiety

selected from the group comprising $C_{1-6}$alkyl, $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo $C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or aryl may be optionally substituted by one or more $C_{1-6}$alkyl. Preferably, an alanine analogue as referred to herein comprises at least one cyclohexyl group, one phenyl group or one indole group, preferably at least one cyclohexyl group. Preferably, said cyclohexyl, phenyl or indole group is a substituent of a hydrogen of the methyl group of alanine. Optionally, said cyclohexyl, phenyl or indole group may be substituted at any position with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo $C_{1-6}$alkyl; or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or aryl may be optionally substituted by one or more $C_{1-6}$alkyl. The alanine analogues referred to herein may in addition comprise a replacement of another hydrogen of the methyl group and/or a hydrogen of the amino group of the main chain or the hydrogen atom on the alpha carbon atom.

In some embodiments, the alanine analogue, $X_1$, and/or $X_{1a}$ is a moiety of formula (Ia) or (I'a),

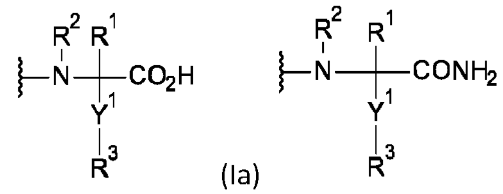

(Ia) (I'a)

wherein

$R^1$ is hydrogen or $C_{1-6}$alkyl; $R^2$ is hydrogen or $C_{1-6}$alkyl;

$R^3$ is a moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

$Y^1$ is $-C(R^7)R^8-$ or $-C(=O)-$;

$R^7$ is selected from the group comprising hydrogen, OH, SH, $C_{1-6}$alkyl, $C_{3-12}$cycloalkyl, $C_{1-6}$alkoxy, amino, and halo;

$R^8$ is hydrogen or $C_{1-6}$alkyl;

or $R^7$ and at least one substituent of $R^3$ together with the carbon atom to which they are attached form a $C_{3-12}$cycloalkyl, wherein said $C_{3-12}$cycloalkyl can be optionally substituted with one or more substituents independently selected from the group comprising $C_{1-6}$alkyl, OH, halo, $C_{3-12}$cycloalkyl, $C_{2-6}$alkenyl, $C_{1-6}$alkoxy, oxo, $=CH_2$, amino, mono- or di-$C_{1-6}$alkylamino, amino $C_{1-6}$alkyl, and halo$C_{1-6}$alkyl, or two substituents together with the atom to which they are attached may form a $C_{3-12}$cycloalkyl, a $C_{5-12}$cycloalkenyl, a heterocycloalkyl or an $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl.

In some embodiments, the alanine analogue, $X_2$, $X_3$, $X_4$, $X_{2a}$, $X_{3a}$ and/or $X_{4a}$ can be each independently selected from moiety of formula (Ib):

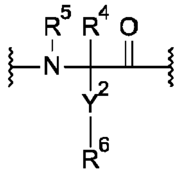

(Ib)

wherein

$R^4$ is hydrogen or $C_{1-6}$alkyl;

$R^5$ is hydrogen or $C_{1-6}$alkyl;

$R^6$ is a moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$ alkoxy, oxo, $=CH_2$, amino, mono- or di- $C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

$Y^2$ is $-C(R^7)R^8-$ or $-C(=O)-$;

$R^7$ is selected from the group comprising hydrogen, OH, SH, $C_{1-6}$alkyl, $C_{3-12}$cycloalkyl, $C_{1-6}$ alkoxy, amino, and halo;

$R^8$ is hydrogen or $C_{1-6}$alkyl;

or $R^7$ and at least one substituent of $R^6$ together with the carbon atom to which they are attached form a $C_{3-12}$cycloalkyl, wherein said $C_{3-12}$cycloalkyl can be optionally substituted with one or more substituents independently selected from the group comprising $C_{1-6}$alkyl, OH, halo, $C_{3-12}$cycloalkyl, $C_{2-6}$alkenyl, $C_{1-6}$alkoxy, oxo, $=CH_2$, amino, mono- or di-$C_{1-6}$alkylamino, amino $C_{1-6}$alkyl, and halo$C_{1-6}$alkyl, or two substituents together with the atom to which they are attached may form a $C_{3-12}$cycloalkyl, a $C_{5-12}$cycloalkenyl, a heterocycloalkyl or an $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl.

In some embodiments, each of $R^3$ and $R^6$ can be a cyclic moiety selected from the group comprising

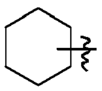 , 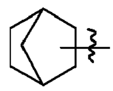 , 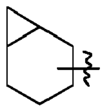 , 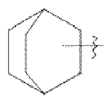 , 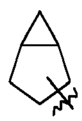 , 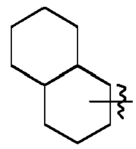 , 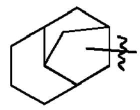 , 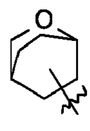 ,

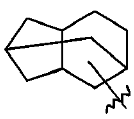 , 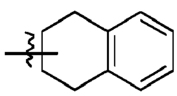 , 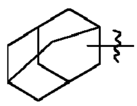 , 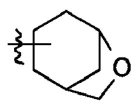 , 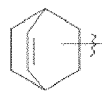 , 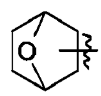 ;

each of said cyclic moiety being optionally substituted with one or more substituents each independently selected from OH, halo, $C_{1-6}$alkyl, $C_{3-12}$cycloalkyl, $C_{2-6}$alkenyl, $C_{1-6}$ alkoxy, oxo, $=CH_2$, amino, mono- or di- $C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl.

Preferably, in some embodiments, each of $R^3$ and $R^6$ can be a cyclic moiety selected from the group comprising

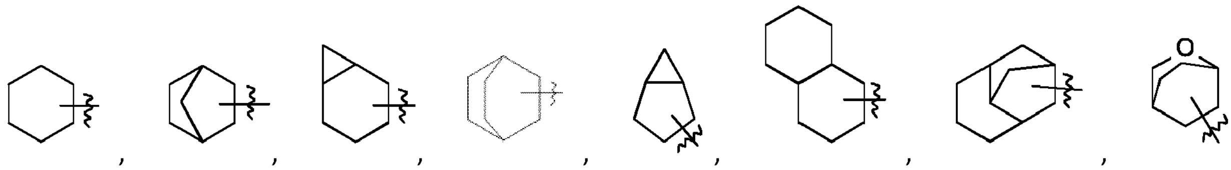

, , , , , , , ,

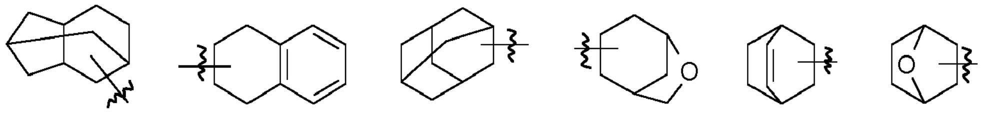

, , , , , ;

each of said cyclic moiety being optionally substituted with one or more substituents each independently selected from OH, halo, $C_{1-6}$alkyl, $C_{3-12}$cycloalkyl, $C_{2-6}$alkenyl, $C_{1-6}$ alkoxy, oxo, $=CH_2$, amino, mono- or di- $C_{1-6}$alkylamino, halo$C_{1-6}$alkyl.

Preferably, in some embodiments, each of $R^3$ and $R^6$ can be a cyclic moiety selected from the group comprising

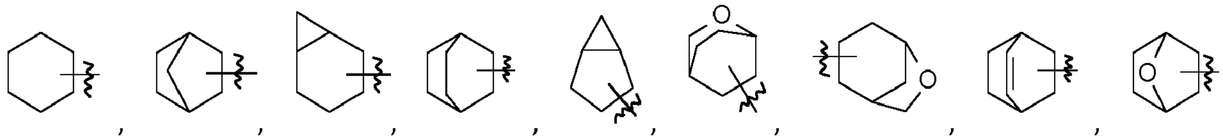

, , , , , , , , ;

each of said cyclic moiety being optionally substituted with one or more substituents each independently selected from OH, halo, $C_{1-6}$alkyl, $C_{3-12}$cycloalkyl, $C_{2-6}$alkenyl, $C_{1-6}$ alkoxy, oxo, $=CH_2$, amino, mono- or di- $C_{1-6}$alkylamino, halo$C_{1-6}$alkyl.

Preferably, in some embodiments, each of $R^3$ and $R^6$ can be a cyclic moiety selected from the group comprising

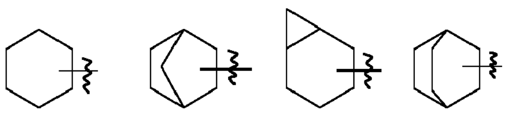

, , , ,

each of said cyclic moiety being optionally substituted with one or more substituents each independently selected from OH, halo, $C_{1-6}$alkyl, $C_{1-6}$ alkoxy, oxo, $=CH_2$, halo$C_{1-6}$alkyl.

Non-limiting examples of cyclohexylalanine analogues to be used in the preparation of the peptidomimetic are shown in Table B:

Table B: Non-limiting examples of suitable cyclohexylalanine analogues.

| | | | | |
|---|---|---|---|---|
| 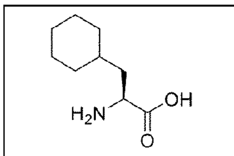 | 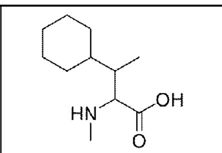 | 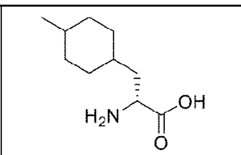 | 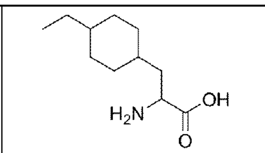 | 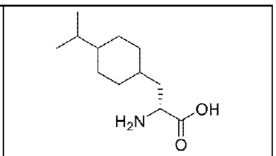 |
| 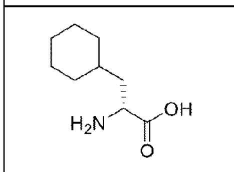 | 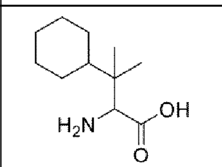 | 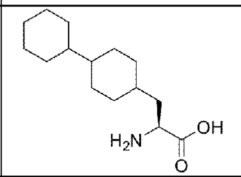 | 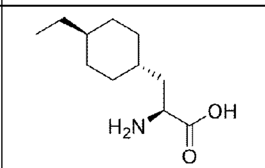 | 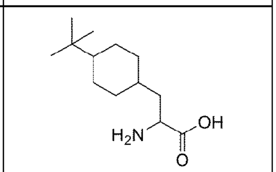 |
| 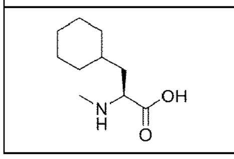 | 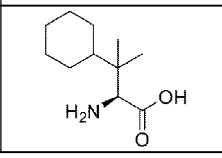 | 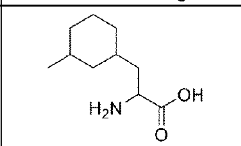 | 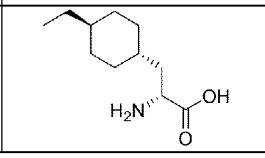 | 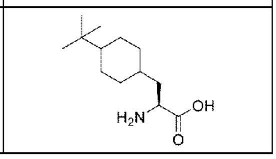 |

| | | | | |
|---|---|---|---|---|
| 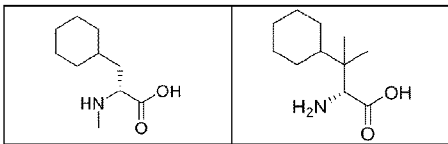 | | 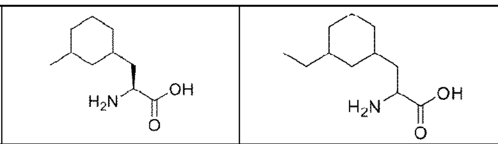 | | 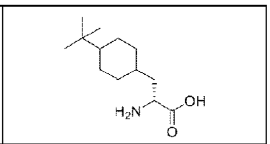 |
| 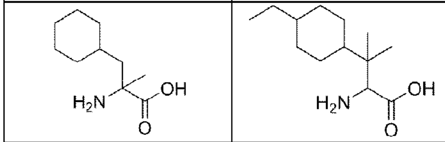 | | 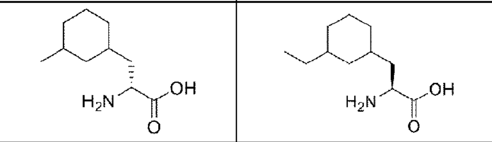 | | 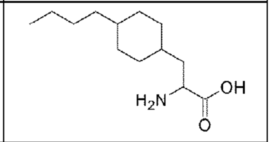 |
| 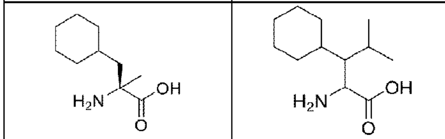 | | 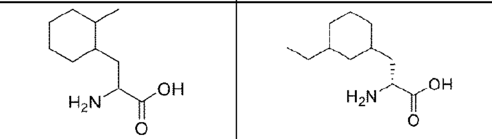 | | 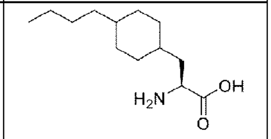 |
| 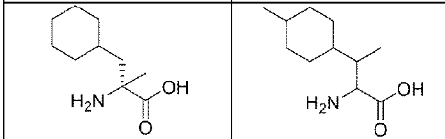 | | 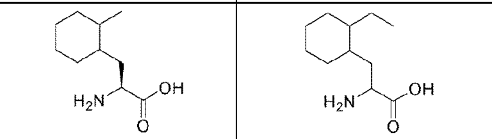 | | 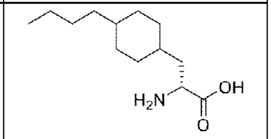 |
| 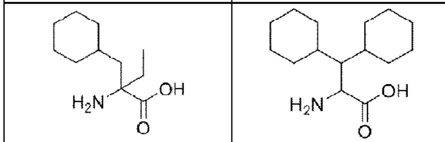 | | 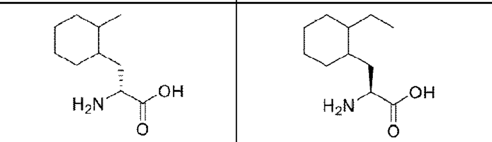 | | 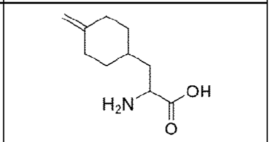 |
| 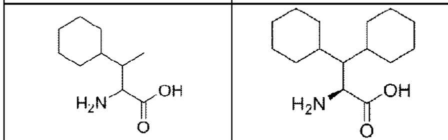 | | 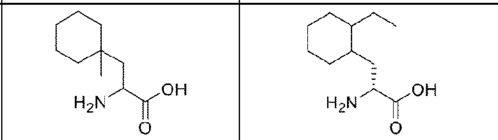 | | 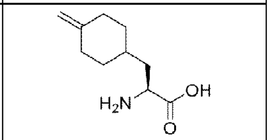 |
| 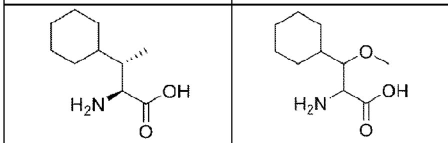 | | 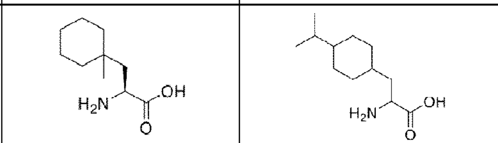 | | 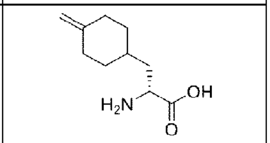 |
| 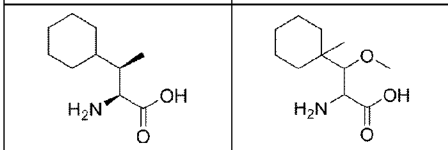 | | 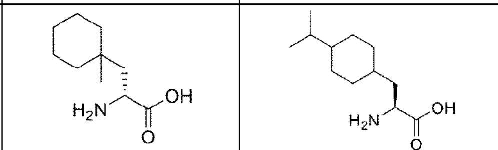 | | 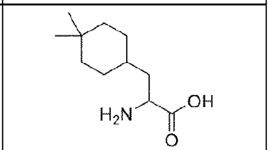 |
| 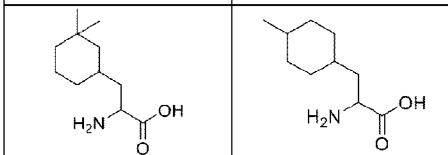 | | 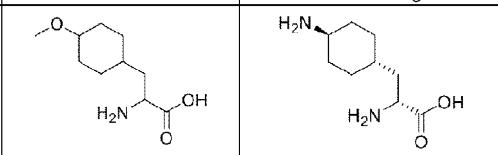 | | 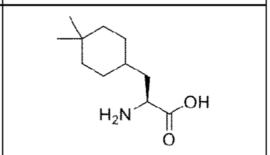 |
| 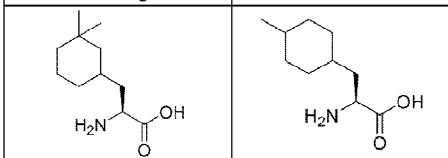 | | 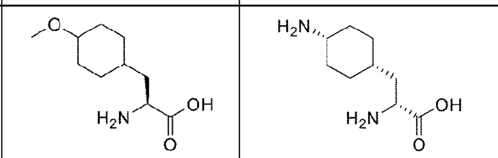 | | 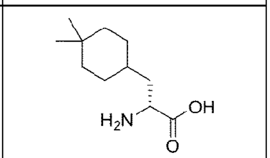 |
| 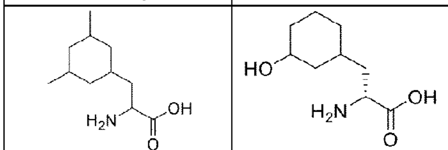 | | 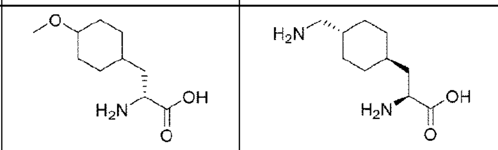 | | 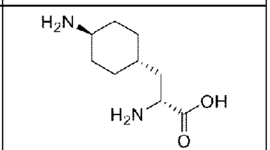 |
| 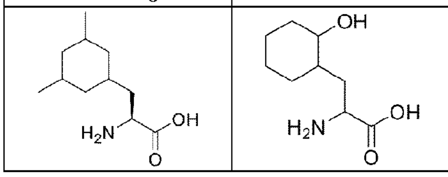 | | 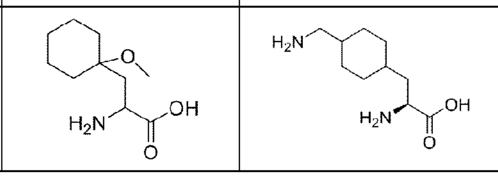 | | 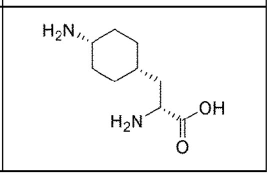 |

| | | | | |
|---|---|---|---|---|
| 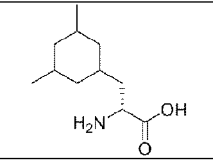 | 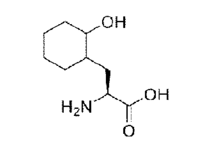 | 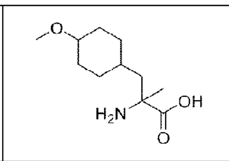 | 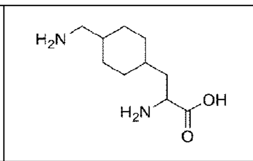 | 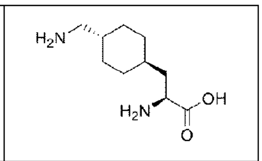 |
| 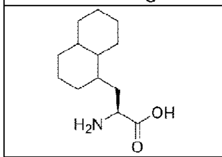 | 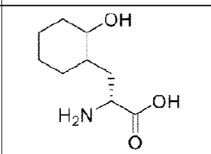 | 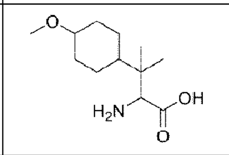 | 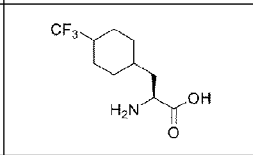 | 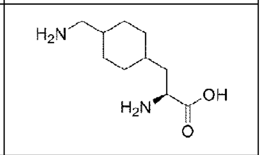 |
| 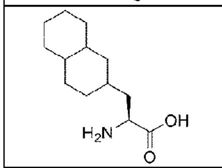 | 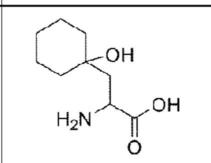 | 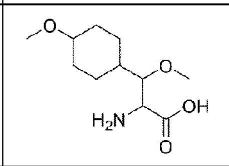 | 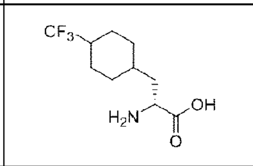 | 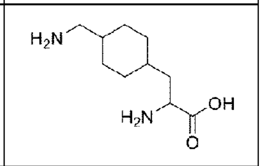 |
| 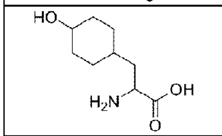 | 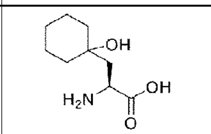 | 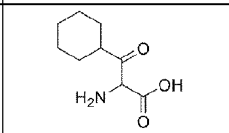 | 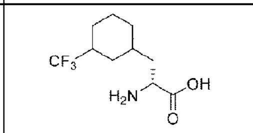 | 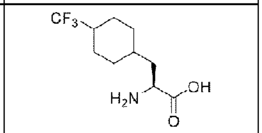 |
| 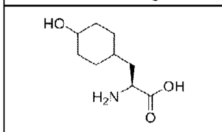 | 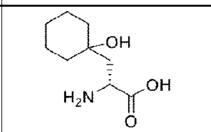 | 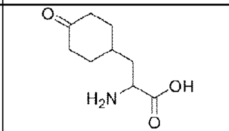 | 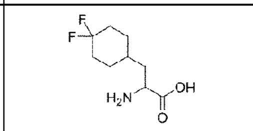 | 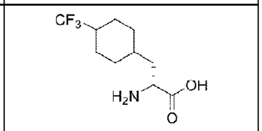 |
| 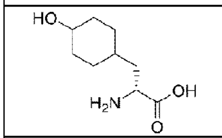 | 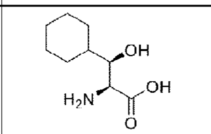 | 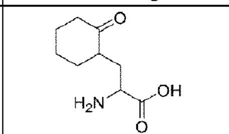 | 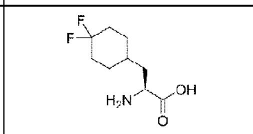 | 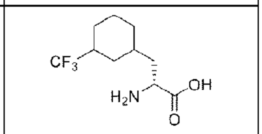 |
| 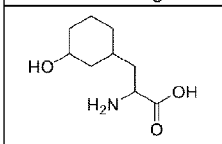 | 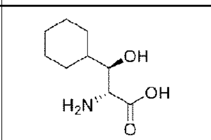 | 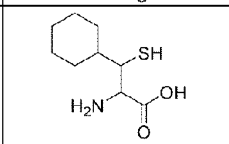 | 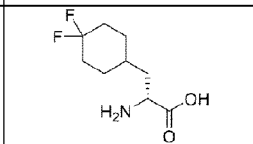 | 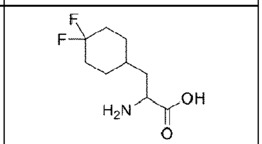 |
| 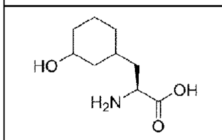 | 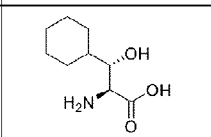 | 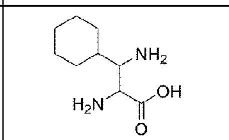 | 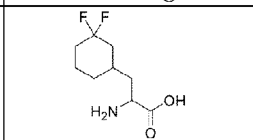 | 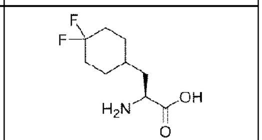 |
| | | | | |
| | | | | |
| | | | | |

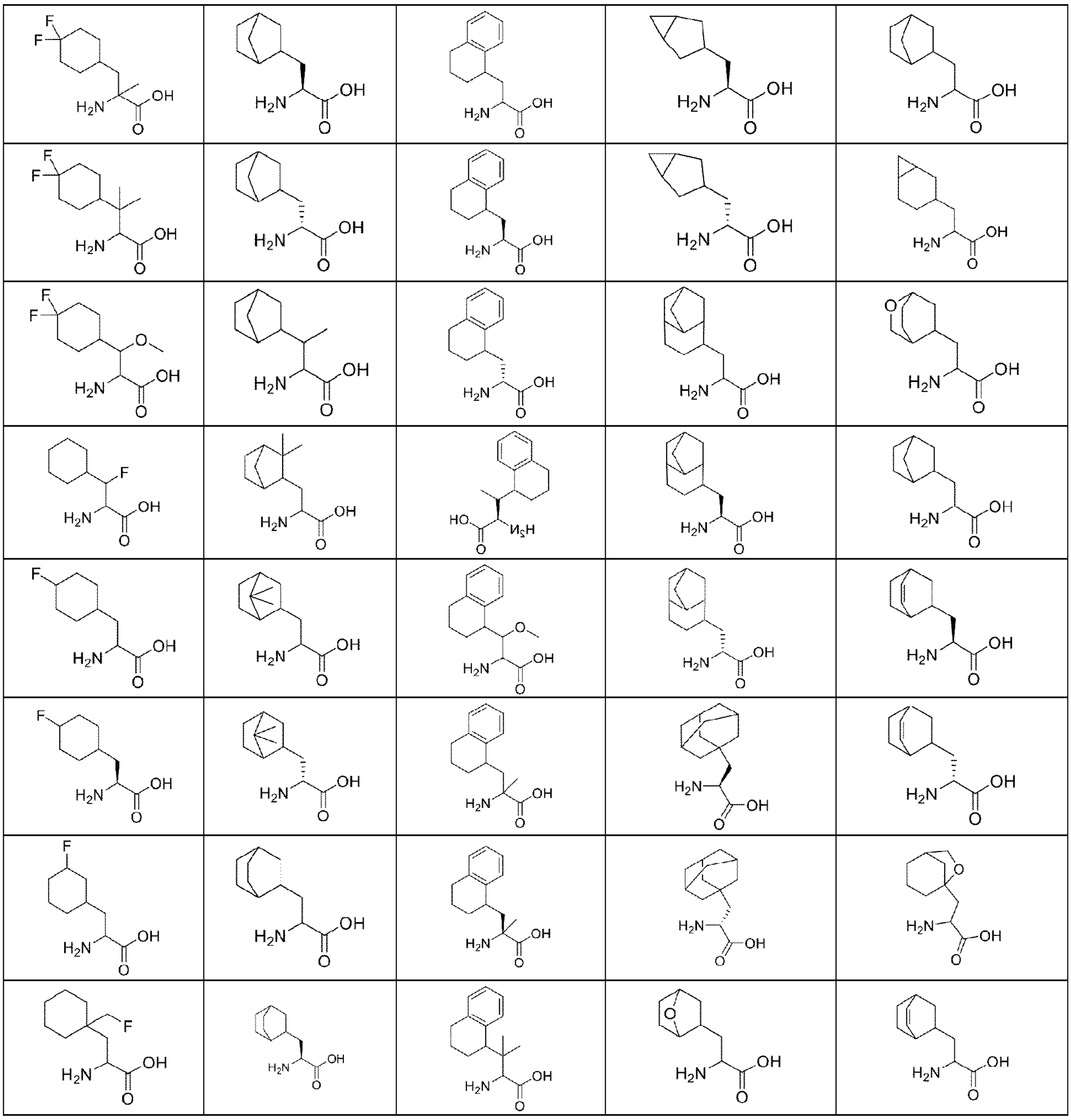

In some embodiments, the alanine analogue, $X_1$ and/or $X_{1a}$ is a moiety of formula (Ic), and/or the alanine analogue, $X_2$, $X_3$, $X_4$, $X_{2a}$, $X_{3a}$ and/or $X_{4a}$ can be each independently selected from a moiety of formula (Id):

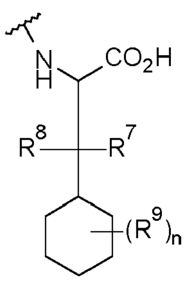
(Ic),

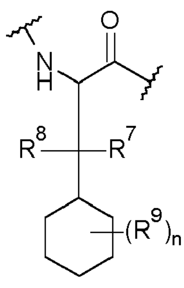
(Id)

wherein $R^7$ is selected from the group comprising hydrogen, OH, SH, $C_{1-6}$alkyl, $C_{3-12}$cycloalkyl, $C_{1-6}$ alkoxy, amino,

and halo;

$R^8$ is hydrogen or $C_{1-6}$alkyl;

n is an integer selected from 0, 1, 2, 3, 4, or 5; preferably 1, 2, 3 or 4, preferably 1, 2 or 3;

$R^9$ is selected from the group comprising OH, halo, $C_{1-6}$alkyl, $C_{3-12}$cycloalkyl, $C_{2-6}$alkenyl, $C_{1-6}$ alkoxy, oxo, $=CH_2$, amino, mono- or di- $C_{1-6}$alkylamino, halo$C_{1-6}$alkyl, or two $R^9$ together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

or $R^7$ and at least one $R^9$ together with the carbon atom to which they are attached form a $C_{3-12}$cycloalkyl, wherein said $C_{3-12}$cycloalkyl can be optionally substituted with one or more substituents independently selected from the group comprising $C_{1-6}$alkyl, OH, halo, $C_{3-12}$cycloalkyl, $C_{2-6}$alkenyl, $C_{1-6}$alkoxy, oxo, $=CH_2$, amino, mono- or di-$C_{1-6}$alkylamino, amino $C_{1-6}$alkyl, and halo$C_{1-6}$alkyl, or two substituents together with the atom to which they are attached may form a $C_{3-12}$cycloalkyl, a $C_{5-12}$cycloalkenyl, a heterocycloalkyl or an $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl.

In some embodiments, the alanine analogue, $X_1$ and/or $X_{1a}$ is a moiety of formula (Ie), and/or the alanine analogue, $X_2$, $X_3$, $X_4$, $X_{2a}$, $X_{3a}$ and/or $X_{4a}$ can be each independently selected from a moiety of formula (If):

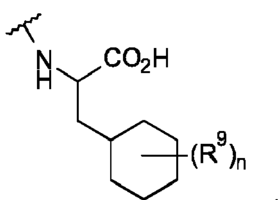
(Ie),

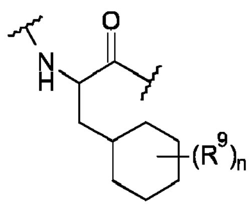
(If)

wherein $R^9$ is selected from the group comprising OH, halo, $C_{1-6}$alkyl, $C_{3-12}$cycloalkyl, $C_{2-6}$alkenyl, $C_{1-6}$ alkoxy, oxo, $=CH_2$, amino, mono- or di- $C_{1-6}$alkylamino, halo$C_{1-6}$alkyl, or two $R^9$ together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl.

In embodiments, at least one of $X_1$, $X_2$, $X_3$, and $X_4$ is independently selected from the group consisting of cyclohexylalanine, phenylalanine, and tryptophan, each of said cyclohexylalanine, phenylalanine, and tryptophan being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl. Preferably, at least one of $X_1$, $X_2$, $X_3$, and $X_4$ is independently selected from cyclohexylalanine, or phenylalanine, each of said cyclohexylalanine, and phenylalanine, being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl. More preferably, at least one of $X_1$, $X_2$, $X_3$, and $X_4$ is cyclohexylalanine, said cyclohexylalanine being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl. In embodiments, at least one of $X_1$, $X_2$, $X_3$, and $X_4$ is selected from the group consisting of cyclohexylalanine, phenylalanine, and tryptophan, preferably cyclohexylalanine or phenylalanine, more preferably cyclohexylalanine.

In embodiments, the G protein peptidomimetics disclosed herein comprise a sequence $X_4X_3X_2X_1$ (SEQ ID NO:18) selected from $X_4X_3X_{2a}X_1$ (SEQ ID NO:133), $X_4X_3X_2X_{1a}$ (SEQ ID NO:134), $X_{4a}X_3X_2X_1$ (SEQ ID NO:135) and $X_4X_{3a}X_2X_1$ (SEQ ID NO:136), wherein $X_1$, $X_2$, $X_3$, $X_4$, $X_{1a}$, $X_{2a}$, $X_{3a}$ and $X_{4a}$ have the same meaning as defined herein.

In particular embodiments, the G protein peptidomimetics disclosed herein comprise a sequence $X_4X_3X_2X_1$

(SEQ ID NO:18) selected from YE$X_{2a}$L (SEQ ID NO:19), YEL$X_{1a}$ (SEQ ID NO:20), $X_{4a}$ELL (SEQ ID NO:21) and Y$X_{3a}$LL (SEQ ID NO:23), preferably selected from YE$X_{2a}$L (SEQ ID NO:19), YEL$X_{1a}$ (SEQ ID NO:20) and $X_{4a}$ELL (SEQ ID NO:21), more preferably YE$X_{2a}$L (SEQ ID NO:19) or YEL$X_{1a}$ (SEQ ID NO:20), even more preferably YE$X_{2a}$L (SEQ ID NO:19), wherein $X_{1a}$, $X_{2a}$, $X_{3a}$ and $X_{4a}$ have the same meaning as defined herein.

In preferred embodiments, the G protein peptidomimetics disclosed herein comprise a sequence $X_4X_3X_2X_1$ (SEQ ID NO:18) selected from the group consisting of YEChaL (SEQ ID NO:30), YEFL (SEQ ID NO: 31) and YEWL (SEQ ID NO: 32), preferably YEChaL (SEQ ID NO:30).

In embodiments, the G protein peptidomimetics disclosed herein comprise a sequence $X_4X_3X_2X_1$ (SEQ ID NO:18) selected from the group consisting of YELCha (SEQ ID NO:33), YELF (SEQ ID NO:34) and YELW (SEQ ID NO:35), preferably YELCha (SEQ ID NO:33).

In embodiments, the G protein peptidomimetics disclosed herein comprise a sequence $X_4X_3X_2X_1$ (SEQ ID NO:18) selected from the group consisting of: YE$X_{2a}X_{1a}$ (SEQ ID NO:24), $X_{4a}$E$X_{2a}$L (SEQ ID NO:27) and $X_{4a}$EL$X_{1a}$ (SEQ ID NO:28), wherein $X_{1a}$, $X_{2a}$, $X_{3a}$ and $X_{4a}$ can be as defined herein above.

## Covalentlv tethered G protein peptidomimetics

In embodiments, the G protein peptidomimetics are (macro)cyclized or covalently tethered ('stapled'), i.e. an intramolecular covalent bond, tether or linkage is formed between two non-adjacent (amino acid) residues of the peptide or peptidomimetic. These cyclized peptides have also been coined "macrocycles" in the art. Both peptidomimetics comprising a staple and peptidomimetics comprising suitable (amino acid) residues arranged to allow (macro)cyclization are envisaged herein. Any of the sequences disclosed herein can refer to a peptide or a peptidomimetic wherein (side-chains of) residues have been reacted to form a covalent tether as described herein, i.e. a stapled peptide or peptidomimetic.

(Macro)cyclization or stapling of the peptide or peptidomimetic disclosed herein is aimed to stabilize and/or mimic peptide α-helices. The α-helical secondary structure is well defined in the art. Briefly, they comprise a right-handed spiral that is maintained by hydrogen bond interactions between the hydrogen from the backbone amino group of an amino acid of the peptide and the backbone carbonyl group of the amino acid in a further position (3 or 4 residues) of the peptide chain. Methods to measure the helicity of a peptide are known to a person skilled in the art, such as but not limited to circular dichroism, nuclear magnetic resonance (NMR) spectroscopy, and X-ray crystallography. Stapling of the peptides may confer certain advantages over their non-stapled counterparts, or improve certain advantages observed to a lesser degree in the non-stapled counterparts. Such advantages include but are not limited to (improved) protease resistance and/or (improved) cellular uptake.

Different combinations of functional group to achieve macrocyclization have been described in the art and include head-to-side-chain (i.e. between the N-terminus of the peptide and a functional group on a side-chain of an amino acid), head-to-tail (i.e. between the N-terminus and C-terminus), side-chain-to-tail (i.e. between the C-terminus and a functional group on a side-chain of an amino acid), and side-chain-to-side-chain (between two functional groups on the side-chain of an amino acid). In a preferred embodiment, G protein peptidomimetics comprise at least one side-chain-to-side-chain cyclization that stabilizes an α-helical conformation. The cyclization may be formed between two natural occurring amino acids, between two non-naturally occurring amino acids, or between a naturally occurring and a non-naturally occurring amino acid.

Cyclization may be achieved by methods well-known to those in the art (described inter alia in detail in White and Yudin (2011. Nature Chemistry 3:509-524). Non-limiting examples of cyclization reactions include Ugi reaction, lactamization, ring-closing metathesis (RCM), triazole formation by copper-catalyzed azide-alkyne cycloaddition (CuAAC) (also referred to as click chemistry), Staudinger ligation, thiol-ene addition, thiazolidine formation, cross-coupling, disulfide formation, and azobenzene formation. In view of the preferred side-to-side-chain cyclization manner of peptidomimetics as described herein, non-limiting examples of preferred cyclization reactions include cross-coupling, ring-closing metathesis, lactamization, disulfide formation, and azobenzene formation. In further preferred embodiments, the macrocyclizations are generated by ring-closing metathesis, lactamization, disulfide bridge formation, or click chemistry.

Macrocyclization by a lactamization reaction is based on the formation of an amide bond between two side-chains. Exemplary pairs of amino acids that are suitable for this reaction are aspartic acid or glutamic acid (providing the carboxylic group) and lysine (providing the amine group). It is common in the art to discriminate lactamization reactions wherein the amino acid providing the amine functional group is positioned as the C-terminal or N-terminal amino acid in the reaction. In the latter case, such a lactam bridge is typically referred to as a "reverse" lactam bridge. Both "standard" lactam bridges and "reverse" lactam bridges are envisaged by the present disclosure, as both induce a secondary structure on the peptide showing similarity to a α-helix. In a lactamization reaction, an amide is formed by condensation between a carboxylic acid and an amine wherein a water molecule is eliminated. Lactamization reactions require a condensation agent in order to activate the carboxylic group. Numerous suitable condensation agents have been described in the art and include but are by no means limited to carbodiimides (e.g. N,N'-dicyclohexylcarbodiimide (DCC) and N,N'-diisopropylcarbodiimide (DIC), phosphonium salts (e.g. (Benzotriazol-1-yloxy)tris(dimethylamino)phosphonium hexafluorophosphate (BOP) or (Benzotriazol-1-yloxy)tripyrrolidinophosphonium hexafluorophosphate (PyBOP)), uronium salts, or thiouronium salts (HBTU, TOTT). While one of the strengths of lactamization is the possibility to rely on natural amino acids, a skilled person appreciates that this cyclization method may also be used between any combination of naturally or non-naturally occurring amino acids that are able to form an amide bond by condensation of a carboxylic acid-containing side-chain of a first amino acid and an amine-containing side-chain of a second amino acid. Additionally, functional groups involved in stapling should be protected during peptide synthesis by protective groups orthogonal to the protective groups used for the N- and or C-termini.

Macrocyclization by ring-closing metathesis is based on coupling of two terminal alkenes that form a macrocycle linked by a double bond with the loss of an ethylene molecule. Central to ring-closing metathesis is a metal catalyst. Different catalysts have been described in the art and include but are not limited to so-called first and second generation Grubbs catalysts, and Shrock catalysts. First generation Grubbs catalysts have a ruthenium core substituted with two phosphine groups, two chlorine atoms and a carbene compound and have the advance of being air-stable and therefore easy to handle. Second generation Grubbs catalysts comprise an N-heterocyclic carbene (NHC) replacing a phosphine substituent. NHC provides enhanced catalyst activity while still providing adequate air and water stability. The reaction relies on a double 2+2 cycloaddition - cycloelimination between an olefin (as envisaged herein an olefinic substituted amino acid) and the carbene-metal complex. While the thermal cycloaddition between olefinic compounds require high activation energies since they are symmetry forbidden, interaction with the metal catalysts substantially lower the activation energy, allowing the reaction to occur at room temperature. As indicated above, ring-closing metathesis requires two olefinic-substituted amino acids.

A non-limiting manner to generate such amino acids is by allylation of serine by a nucleophilic substitution reaction between the hydroxyl group of serine and an allyl halide. Alternatively, insertion of a terminal olefinic hydrocarbon chain on glycine or alanine residues may be achieved by usage of a chiral nickel catalyst. Non-limiting examples of suitable olefinic amino acids include alanine derivatives "S5", "R5" and "R8" as described herein. In the art, the terms "alkene" and "olefin" are often used interchangeably.

Macrocyclization by disulfide formation can also be envisaged. Disulfide bridges may be formed between amino acids that have a thiol-side-chain such as e.g. cysteine. Disulfide bridges are typically formed by oxidation of the sulfhydryl groups.

Copper-catalyzed azide-alkyne cycloaddition (CuAAC) is a further preferred macrocyclization reaction and the reaction as such is alternatively known in the art as "Huisgen cycloaddition" or Click-chemistry. An advantage of this approach is that the functional groups that are involved are orthogonal to any other functionality in a cellular milieu. Additionally, the copper catalysis is typically performed under mild conditions. CuAAC relies on regioselective 3+2 cycloaddition between an azide and a terminal alkyne leading to a 1,4-disubstituted 1,2,3-triazole ring having aromatic properties. In a CuAAC reaction, copper is linked to an alkyne. The subsequent elimination of the terminal proton is responsible for formation of a copper-acetylide complex. In a next step, the azido group is linked to the copper atom, eventually forming a triazolic ring which is then released. Methods to generate alkynyl-amino acids have been described in the art. A non-limiting suitable method is nucleophilic substitution on a propargyl bromide or homolog thereof by a nucleophilic amino acid. The nucleophilic amino acid may be a natural nucleophilic amino acid such as serine, cysteine, glutamate, glutamine, aspartic acid, or asparagine. Alternatively, a chiral nickel catalyst can be employed to obtain (all-hydrocarbon) alkynyl amino acids. Methods to generate azidated amino acids are ubiquitous in the art (and are *inter alia* summarized in Johansson and Pedersen 2012 European Journal of Organic Chemistry 4267-4281). Illustrative methods include direct insertion of the azido group on a serine residue by using Mitsunobu coupling conditions, mesylation of the serine Weinreb amide and subsequent insertion of the azido group by nucleophilic substitution on the mesylated hydroxyl group, Hoffmann rearrangement of an asparagine followed by a diazotransfer, or Ullmann coupling of p-iodophenylalanine. A non-limiting example of a suitable amino acid containing an azidated side-chain is azidolysine (also referred to herein as "Azk"), a non-limiting example of a suitable amino acid containing an alkynyl side-chain is propargylglycine (also referred to herein as "Pra").

The terms "covalent tether", "tether", "staple", "braces", "bridges" may be used interchangeably herein and are to be interpreted in the current disclosure in accordance with their generally accepted meaning in the technical field, i.e. a covalent tether or bond that is not part of the linear peptide backbone and that mediates macrocycle formation.

For example, and without limitation, in the G protein peptidomimetics disclosed herein, a staple can be formed from the reaction/coupling of the side-chain of $X_8$ with the side-chain of $X_{10}$ or from the reaction/coupling of the side-chain of $X_7$ with the side-chain of $X_8$, from the reaction/coupling of the side-chain of $X_5$ with the side-chain of $X_7$, from the reaction/coupling of the side-chain of $X_6$ with the side-chain of $X_8$ or from the reaction/coupling of the side-chain of $X_6$ with the side-chain of $X_7$.

In preferred embodiments, the G protein peptidomimetics comprise a covalent tether between $X_8$ and $X_{10}$, between $X_7$ and $X_8$, or between $X_5$ and $X_7$, preferably between $X_8$ and $X_{10}$, wherein said covalent tether is not part of

the linear peptide backbone.

In further embodiments, said covalent tether is formed between an amino acid containing an amine side-chain and an amino acid containing a carboxylic acid group side-chain. In certain embodiments, the covalent tether is a lactam bridge formed between a glutamic acid and a lysine or between an aspartic acid and a lysine. In alternative further embodiments, said covalent tether is formed between two olefinic amino acids. In yet alternative further embodiments, said covalent tether is formed between an amino acid containing an azidated side-chain and an amino acid containing an alkynyl-bearing side-chain. In certain embodiments, said covalent tether is formed between an azidolysine (Azk) and a propargylglycine (Pra). In yet alternative further embodiments, the staple is a disulfide bridge connecting two amino acids each comprising a thiol functional group in their side-chains. In certain embodiments, a disulfide bridge is formed between two cysteine residues. In yet alternative further embodiments, a staple is formed between an amino acid comprising a thiol functional group and an olefinic amino acid. The order of disclosure in the above embodiments does by no means imply that such an arrangement is fixed in the herein disclosed peptidomimetics.

In particular embodiments, a staple is formed from the reaction/coupling of the side-chain of $X_8$ with the side-chain of $X_{10}$, wherein $X_8$ is an amino acid containing an azidated side-chain and $X_{10}$ is an amino acid containing an alkynyl side-chain or wherein $X_{10}$ is an amino acid containing an azidated side-chain and $X_8$ is an amino acid containing an alkynyl side-chain; from the reaction/coupling of the side-chain of $X_7$ with the side-chain of $X_{8,}$, wherein $X_7$ is an amino acid containing an azidated side-chain and $X_8$ is an amino acid containing an alkynyl side-chain or wherein $X_8$ is an amino acid containing an azidated side-chain and $X_7$ is an amino acid containing an alkynyl side-chain; or from the reaction/coupling of the side-chain of $X_5$ with the side-chain of $X_7$, wherein $X_5$ is an amino acid containing an azidated side-chain and $X_7$ is an amino acid containing an alkynyl side-chain or wherein $X_7$ is an amino acid containing an azidated side-chain and $X_5$ is an amino acid containing an alkynyl side-chain. In yet further particular embodiments, a staple is formed from the reaction/coupling of the side-chain of $X_8$ with the side chain of $X_{10}$, wherein $X_8$ is an azidolysine (Azk) and $X_{10}$ is a propargylglycine (Pra) or wherein $X_{10}$ is an azidolysine (Azk) and $X_8$ is a propargylglycine (Pra); from the reaction/coupling of the side-chain of $X_7$ with the side-chain of $X_{8,}$, wherein $X_7$ is an azidolysine (Azk) and $X_8$ is a propargylglycine (Pra) or wherein $X_8$ is an azidolysine (Azk) and $X_7$ is a propargylglycine (Pra); or between $X_5$ and $X_7$, wherein $X_5$ is an azidolysine (Azk) and $X_7$ is a propargylglycine (Pra) or wherein $X_7$ is azidolysine (Azk) and $X_5$ is a propargylglycine (Pra). Particularly preferred embodiments are peptidomimetics wherein a staple is formed from the reaction/coupling of the side-chain of $X_8$ with the side chain of $X_{10}$, wherein $X_8$ is an azidolysine (Azk) and $X_{10}$ is a propargylglycine (Pra) or wherein $X_{10}$ is an azidolysine (Azk) and $X_8$ is a propargylglycine (Pra).

The G protein peptidomimetics disclosed herein can also comprise two or more staples. For example, a peptidomimetic may comprise a side-chain-to-side-chain macrocyclization in addition to a second side-chain-to-side-chain macrocyclization formed by identical, similar, or unrelated functional groups of a side-chain of an amino acid. In such an example, four amino acids of the peptidomimetic would be used to generate a double staple (i.e. two braces). Furthermore, a peptidomimetic may comprise a side-chain-to-side-chain macrocyclization combined with any other macrocyclization of any head, tail, or side-chain combination. Thus, any type of staple able to stabilize the linear peptidomimetic in a helix conformation as described herein may be used in connection with any of the macrocyclization methods known in the art.

Linear G protein peptidomimetics

In other embodiments, the G protein peptidomimetics are linear. Linear G protein peptidomimetics may be, amongst other, preferred for the fusion to a GPCR as described herein.

In particular embodiments, $X_5$ is Q; $X_6$ is R; $X_7$ is M; $X_8$ is I and $X_{10}$ is D.

Intermolecular disulfide bridges

Additionally, the G protein peptidomimetics may comprise one or more modifications to reduce or avoid the formation of intermolecular disulfide bridges. For example, the G protein peptidomimetics may be modified by substitution of cysteine residues by another amino acid that does not allow the formation of disulfide bridges such as e.g. an alanine residue or a glycine residue. Hence, in embodiments, the peptidomimetic does not comprise an amino acid that is capable of forming a disulfide bridge. In certain embodiments, the peptidomimetic does not comprise an amino acid comprising a thiol side chain. In preferred embodiments, the peptidomimetic does not comprise a cysteine. In particular embodiments, $X_9$ is A or G.

G protein peptidomimetics with additional basic amino acids at the N-terminus

In embodiments, the G protein peptidomimetics comprise at least one additional basic amino acid at their amino-terminus (N-terminus). Addition of one or more basic amino acids may improve the solubility of the G protein peptidomimetic.

In particular embodiments, the G protein peptidomimetics described herein are modified by the addition of a single (K), a double (KK) or triple (KKK) lysine at their N-terminus.

The G protein peptidomimetics may comprise between 1 and 10, preferably between 1 and 5, more preferably between 1 and 3 such as 1, 2 or 3 additional basic amino acids.

As used herein the term "basic amino acid" refers to an amino acid that is positively charged at physiological pH. Alternatively worded, the term "basic amino acid" as used herein refers to any amino acid that behaves as a Bronsted/Lowry and Lewis base. The term encompasses both natural and non-natural amino acids. Non-limiting examples of basic amino acids that can be added to the G protein peptidomimetic disclosed herein include lysine (K), histidine (H), arginine (R), hydroxylysine, ornithine, 2,4-diamino-butyric acid, (guanidino)-acetic acid or other (guanidino)alkyl-acetic acids.

In embodiments, the basic amino acid is selected from lysine (K), histidine (H) and arginine (R), preferably lysine (K).

Optionally, said additional basic amino acid(s) may be linked to the G protein peptidomimetic via a spacer or linker, as known in the art. Non-limiting examples of suitable linkers or spacers include betaAla, Gly (repeats), aminohexanoic acid (Ahx), etc.

G protein peptidomimetics with additional modifications

Any of the peptides and peptidomimetics described herein can include various (chemical) modifications as long as the biological activity (i.e. the ability to stabilize a GPCR in an active conformational state) is not affected.

For example, any of the G protein peptidomimetics can be amidated (i.e. addition of an amide or substituted amide group) at its carboxy-terminus.

For example, any of the G protein peptidomimetics can be modified at its amino-terminus. Non-limiting examples of N-terminal modifications include acylation (e.g. acetyl, formyl, pyroglutamyl, fatty acids), guanidinylation, attachment of urea, carbamate, sulfonamide, alkylamine, radioligand molecules (e.g. DOTA, NOTA, NODAGA), dyes and quencher molecules, etc. In particular embodiments, the G protein peptidomimetics comprise an N-terminal acetylation. In particular embodiments, the G protein peptidomimetics comprise an N-terminal guanidinylation.

Other modifications can also be made to any of the G protein peptidomimetics described herein. For example, the peptide or peptidomimetic can be phosphorylated, glycosylated, PEGylated, lipidated, or any combination thereof.

Yet another modification may comprise the introduction of one or more detectable labels or other signal-generating groups or moieties, depending on the intended use of the labelled G protein peptidomimetic. Suitable labels and techniques for attaching, using and detecting them will be clear to the skilled person, and for example include, but are not limited to, fluorescent labels, (such as IRDye800, VivoTag800, fluorescein, isothiocyanate, rhodamine, phycoerythrin, phycocyanin, allophycocyanin, o-phthaldehyde, and fluorescamine and fluorescent metals such as Eu or others metals from the lanthanide series), phosphorescent labels, chemiluminescent labels or bioluminescent labels (such as luminal, isoluminol, theromatic acridinium ester, imidazole, acridinium salts, oxalate ester, dioxetane or GFP and its analogues ), radio-isotopes, metals, metal chelates or metallic cations or other metals or metallic cations that are particularly suited for use in in vivo, in vitro or in situ diagnosis and imaging, as well as chromophores and enzymes (such as malate dehydrogenase, staphylococcal nuclease, delta- V- steroid isomerase, yeast alcohol dehydrogenase, alpha-glycerophosphate dehydrogenase, triose phosphate isomerase, biotinavidin peroxidase, horseradish peroxidase, alkaline phosphatase, asparaginase, glucose oxidase, beta-galactosidase, ribonuclease, urease, catalase, glucose-VI-phosphate dehydrogenase, glucoamylase and acetylcholine esterase). Other suitable labels will be clear to the skilled person, and for example include moieties that can be detected using NMR or ESR spectroscopy. Such labelled G protein peptidomimetics of the invention may for example be used for in vitro, in vivo or in situ assays (including immunoassays known per se such as ELISA, RIA, EIA and other "sandwich assays", etc.) as well as in vivo diagnostic and imaging purposes, depending on the choice of the specific label. As will be clear to the skilled person, another modification may involve the introduction of a chelating group, for example to chelate one of the metals or metallic cations referred to above. Suitable chelating groups for example include, without limitation, 2,2',2"-(10-(2-((2,5-dioxopyrrolidin-1-yl)oxy)-2-oxoethyl)-1,4,7,10-tetraazacyclododecane-1,4,7-triyl)triacetic acid (DOTA), 2,2'-(7-(2-((2,5-dioxopyrrolidin-1-yl)oxy)-2-oxoethyl)-1,4,7-triazonane-1,4-diyl)diacetic acid (NOTA), diethyl- enetriaminepentaacetic acid (DTPA) or ethylenediaminetetraacetic acid (EDTA). Yet another modification may comprise the introduction of a functional group that is one part of a specific binding pair, such as the biotin-(strept)avidin binding pair. Such a functional group may be used to link the G protein peptidomimetic to another protein, polypeptide or chemical compound that is bound to the other half of the binding pair, i.e. through formation of the binding pair. For example, a G protein peptidomimetic as described herein may be conjugated to biotin, and linked to another protein, polypeptide, compound or carrier conjugated to avidin or streptavidin. For example, such a conjugated G protein peptidomimetic may be used as a reporter, for example in a diagnostic system where a detectable signal-producing agent is conjugated to avidin or streptavidin. Such binding pairs may for example also be

used to bind the G protein peptidomimetic to a carrier, including carriers suitable for pharmaceutical purposes. Such binding pairs may also be used to link a therapeutically active agent to the G protein peptidomimetic of the invention.

Variants of G protein peptidomimetics

The G protein peptidomimetics disclosed herein also encompass functional variants thereof. Functionally variant G protein peptidomimetics include peptides and peptidomimetics having one or more conservative or non-conservative amino acid substitutions as compared to the sequences of the peptides and peptidomimetics described herein, but still retain substantially the same biological activity as the peptide or peptidomimetic described herein that does not have the substitution.

In particular, the terms "variant" of a peptide or peptidomimetic refers to peptides or peptidomimetics the sequence (i.e., amino acid sequence) of which is substantially identical (i.e., largely but not wholly identical) to the sequence of said recited peptide or peptidomimetic, e.g., at least about 80% identical or at least about 85% identical, e.g., preferably at least about 90% identical, e.g., at least 91% identical, 92% identical, more preferably at least about 93% identical, e.g., at least 94% identical, even more preferably at least about 95% identical, e.g., at least 96% identical, yet more preferably at least about 97% identical, e.g., at least 98% identical, and most preferably at least 99% identical. Preferably, a variant may display such degrees of identity to a recited peptide or peptidomimetic when the whole sequence of the recited peptide or peptidomimetic is queried in the sequence alignment (i.e., overall sequence identity). Also included among variants of a peptide or peptidomimetic are fusion products of said peptide or peptidomimetic with another, usually unrelated, peptide or peptidomimetic. Sequence identity may be determined using suitable algorithms for performing sequence alignments and determination of sequence identity as know per se. Exemplary but non-limiting algorithms include those based on the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250), for example using the published default settings or other suitable settings (such as, e.g., for the BLASTP algorithm: matrix = Blosum62, cost to open a gap = 11, cost to extend a gap = 1, expectation value = 10.0, word size = 3).

Amino acid substitutions may be generally based on the relative similarity of the amino acid side-chain substituents, for example, their hydrophobicity, hydrophilicity, charge, size and the like. Within the scope of the invention, conservative amino acid changes means an amino acid change at a particular position which may be of the same type as originally present; i.e. a hydrophobic amino acid exchanged for a hydrophobic amino acid, a basic amino acid for a basic amino acid, etc. Examples of conservative substitutions may include, without limitation, the substitution of non-polar (hydrophobic) residues such as isoleucine, valine, leucine or methionine for another, the substitution of one polar (hydrophilic) residue for another such as between arginine and lysine, between glutamine and asparagine, between threonine and serine, the substitution of one basic residue such as lysine, arginine or histidine for another, or the substitution of one acidic residue, such as aspartic acid or glutamic acid for another, the substitution of a branched chain amino acid, such as isoleucine, leucine, or valine for another, the substitution of one aromatic amino acid, such as phenylalanine, tyrosine or tryptophan for another. Examples of such conservative changes are well-known to the skilled artisan and are within the scope of the present invention.

Conservative substitution may also include the use of a chemically derivatized residue in place of a non-derivatized residue provided that the resulting peptide or peptidomimetic is a biologically functional equivalent to the peptides and peptidomimetics described herein.

Other substitutions that are contemplated herein are non-natural amino acids that are substituted for natural amino acids of the peptidomimetics described herein, so long as the peptidomimetic having substituted amino acid(s) retains substantially the same activity as the peptidomimetic in which amino acid(s) have not been substituted. Examples of non-natural amino acids include, but are not limited to, ornithine, citrulline, hydroxyproline, homoserine, phenylglycine, taurine, iodotyrosine, 2,4-diaminobutyric acid, $\alpha$-amino isobutyric acid, 4-aminobutyric acid, 2-amino butyric acid, $\gamma$-amino butyric acid, $\varepsilon$-amino hexanoic acid, 6-amino hexanoic acid, 2-amino isobutyric acid, 3-amino propionic acid, norleucine, norvaline, sarcosine, homocitrulline, cysteic acid, $\tau$-butylglycine, $\tau$-butylalanine, phenylglycine, cyclohexylalanine, $\beta$-alanine, fluoro-amino acids, designer amino acids such as $\beta$-methyl amino acids, C-methyl amino acids, N-methyl amino acids, and amino acid analogues in general.

Furthermore, any of the amino acids in the protein can be of the D (dextrorotary) form or L (levorotary) form.

Illustrative G protein peptidomimetics are shown in Table C. "[]" denotes cyclic peptides.

Table C

| Code | Sequence | SEQ ID NO: |
|---|---|---|
| compound 2 | Ac-FNDCRDIIQRMHLRQYELL-OH | 53 |
| compound 3 | Ac-FNDCRD**E**IQR**K**HLRQYELL-OH | 54 |
| compound 4a | H-FNDCRD**E**IQR**K**HLRQYELL-OH | 55 |
| compound 4 | H-FNDCRD[**E**IQR**K**]HLRQYELL-OH: 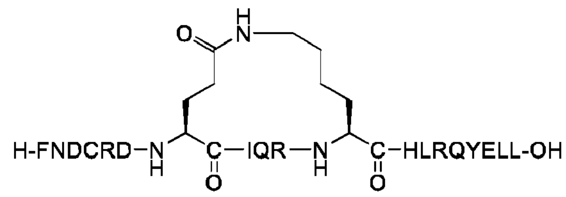 | 137 |
| compound 5 | Ac-FNDCRD[**E**IQR**K**]HLRQYELL-OH 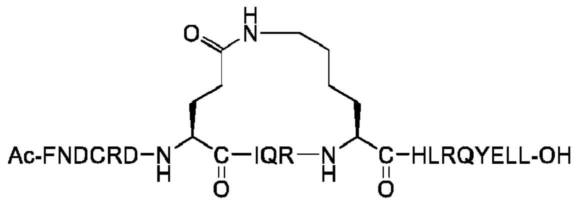 | 138 |
| | 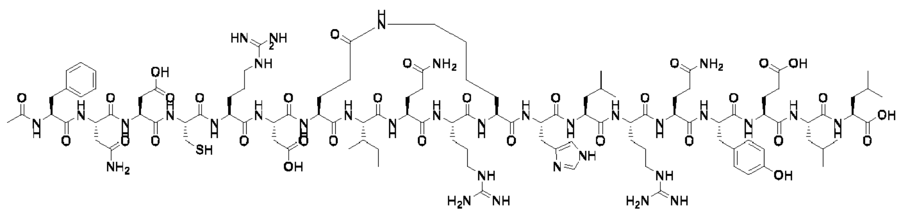 | |
| compound 6 | Ac-FNDCRD**S**$_5$IQR**S**$_5$HLRQYELL-OH | 56 |
| Compound 7a | H-FNDCRD**S**$_5$IQR**S**$_5$LRQYELL-OH | 57 |

(continued)

| Code | Sequence | SEQ ID NO: |
|---|---|---|
| compound 7 | H-FNDCRD[**S$_5$**IQR**S**$_5$]HLRQYELL-OH 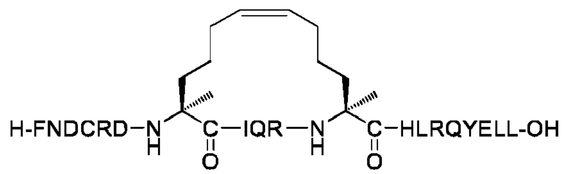 | 139 |
| compound 8 | Ac-FNDCRD[**S**$_5$IQR**S**$_5$]HLRQYELL-OH 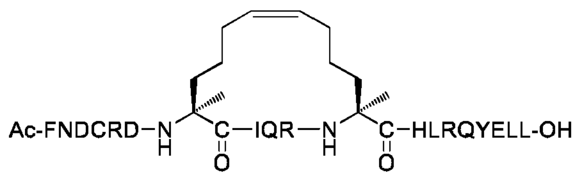 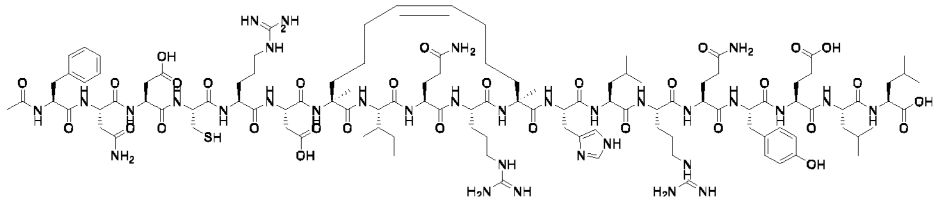 | 140 |
| compound 9 | Ac-FNDCRD**Pra**IQR**Azk**HLRQYELL-OH | 58 |
| Compound 10a | H-FNDCRD**Pra**IQR**Azk**HLRQYELL-OH | 59 |
| compound 10 | H-FNDCRD[**Pra**IQR**Azk**]HLRQYELL-OH 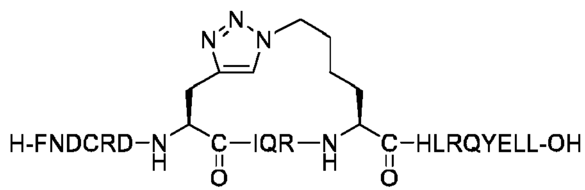 | 141 |

(continued)

| Code | Sequence | SEQ ID NO: |
|---|---|---|
| compound 11 | Ac-FNDCRD[**Pra**IQR**Azk**]HLRQYELL-OH 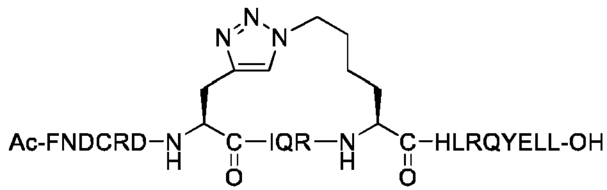 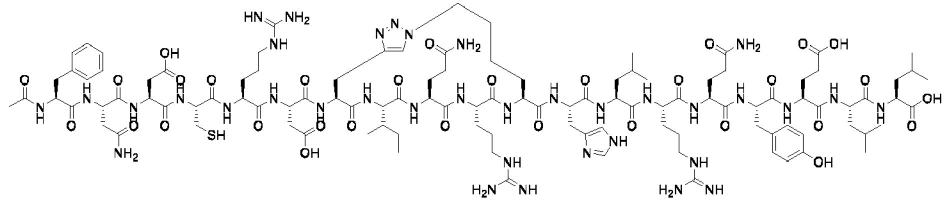 | 142 |
| compound 22 | Ac-FN**Pra**CRD**Azk**IQRMHLRQYELL-OH | 60 |
| compound 12 | Ac-FN[**Pra**CRD**Azk**]IQRMHLRQYELL-OH | 143 |
| | 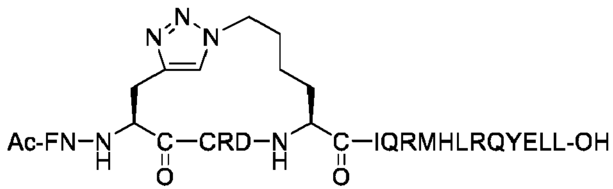 | |
| compound 23 | Ac-FN**E**CRD**K**IQRMHLRQYELL-OH | 61 |

(continued)

| Code | Sequence | SEQ ID NO: |
|---|---|---|
| compound 24 | Ac-FN[**E**CRD**K**]IQRMHLRQYELL-OH 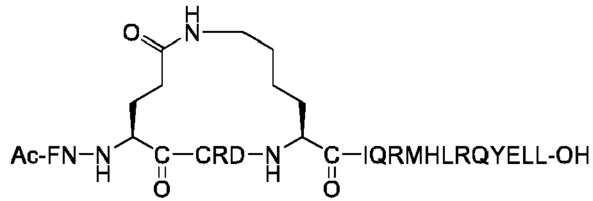 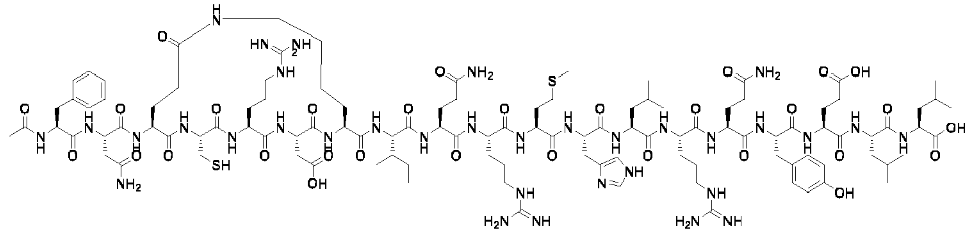 | 144 |
| compound 25 | Ac-FN**S**$_5$CRD**S**$_5$IQRMHLRQYELL-OH | 62 |
| compound 26 | Ac-FN[**S**$_5$CRD**S**$_5$]IQRMHLRQYELL-OH 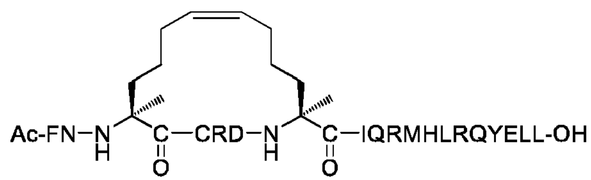 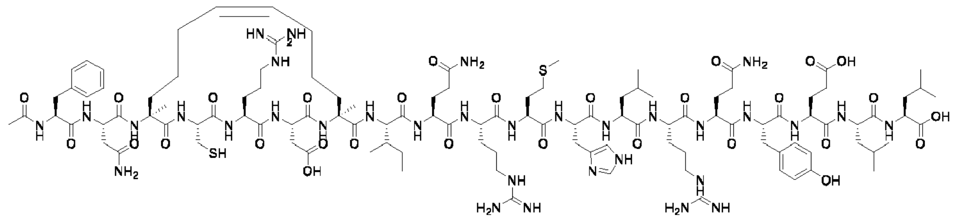 | 145 |
| compound 13a | Ac-FNDCRDIIQR**R**$_5$HL**S**$_5$QYELL-OH | 63 |

(continued)

| Code | Sequence | SEQ ID NO: |
|---|---|---|
| compound 13 | Ac-FNDCRDIIQR[**R**$_5$HL**S**$_5$]QYELL-OH 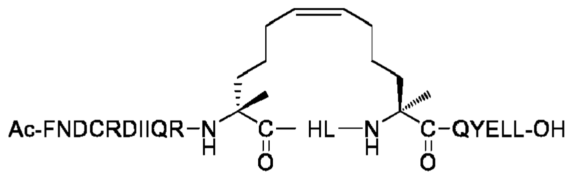 | 146 |
| compound 14a | Ac-FNDCRD**R**$_8$IQRMHL**S**$_5$QYELL-OH | 64 |
| compound 14 | Ac-FNDCRD[**R**$_8$IQRMHL**S**$_5$]QYELL-OH 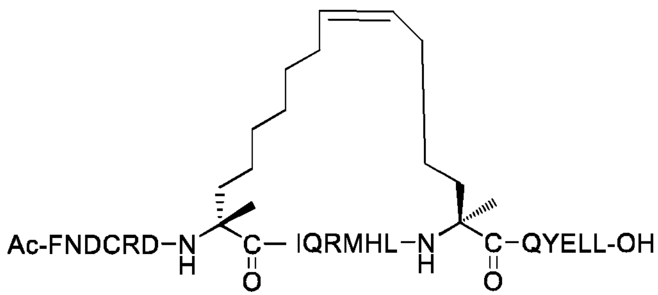 | 147 |
| compound 16 | Ac-RDIIQRMHLRQYELL-OH | 65 |
| compound 17 | Ac-RDIIQR**Azk**HLR**Pra**YELL-OH | 66 |
| compound 19 | Ac-RDIIQR[**Azk**HLR**Pra**]YELL-OH 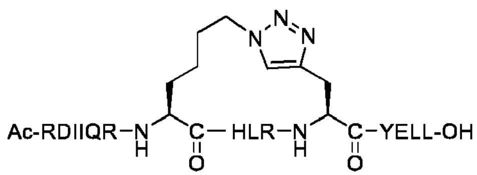 | 148 |
| compound 21 | Ac-QRMHLRQYELL-OH | 67 |
| compound 27 | Ac-FN**Pra**CRD**Azk**IQRMHLRQYQLL-OH | 68 |

(continued)

| Code | Sequence | SEQ ID NO: |
|---|---|---|
| compound 28 | Ac-FN[**Pra**CRD**Azk**]IQRMHLRQYQLL-OH 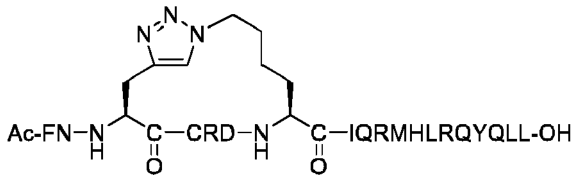 | 149 |
| compound 29 | Ac-FN**Pra**CRD**Azk**IQRMHLRQ**W**ELL-OH | 69 |
| compound 30 | Ac-FN[**Pra**CRD**Azk**]IQRMHLRQWELL-OH 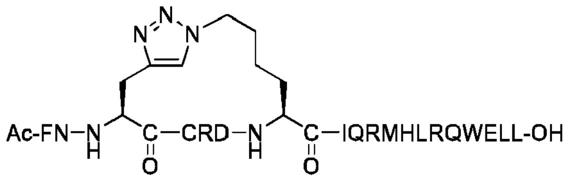 | 150 |
| compound 31 | Ac-FN**Pra**CRD**Azk**IQRMHLRQYE**I**L-OH | 70 |
| compound 32 | Ac-FN[**Pra**CRD**Azk**]IQRMHLRQYEIL-OH 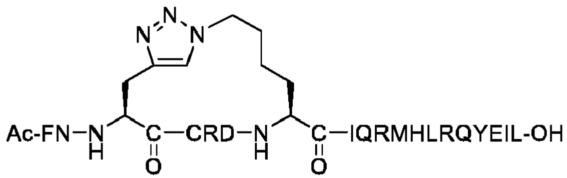 | 151 |
| compound 33 | Ac-FN**Pra**CRD**AzkI**QRMHLRQYEL**I**-OH | 71 |
| compound 34 | Ac-FN[**Pra**CRD**Azk**]IQRMHLRQYELI-OH 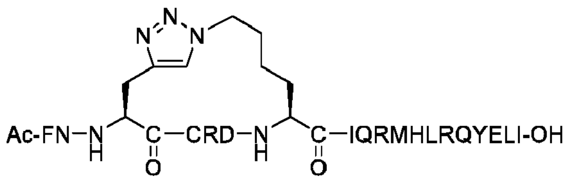 | 152 |
| compound 35a | Ac-FN**Azk**CRD**PraI**QRMHLRQYELL-OH | 72 |

(continued)

| Code | Sequence | SEQ ID NO: |
|---|---|---|
| compound 35 | Ac-FN[**Azk**CRD**Pra**]IQRMHLRQYELL-OH 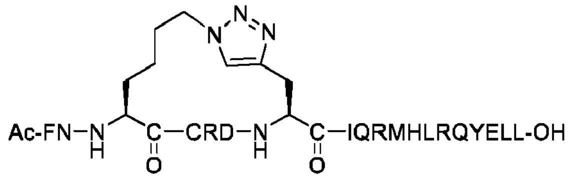 | 153 |
| compound | Ac-FN**Pra**CRD**Azk**IQRMHLRQYELL-$NH_2$ | 73 |
| 36a | | |
| compound 36 | Ac-FN[**Pra**CRD**Azk**]IQRMHLRQYELL-$NH_2$ 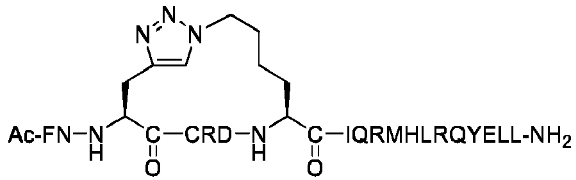 | 154 |
| compound 37a | Ac-FN**Pra**CRD**Azk**IQRMHLRQY**hGlu**LL-OH | 74 |
| compound 37 | Ac-FN[**Pra**CRD**Azk**]IQRMHLRQY**hGlu**LL-OH 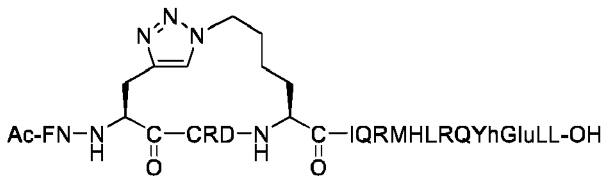 | 155 |
| Compound 38a | Ac-FN**Pra**CRN**Azk**IQRMHLRQYELL-OH | 75 |
| compound 38 | Ac-FN[**Pra**CR**NAzk**]IQRMHLRQYELL-OH 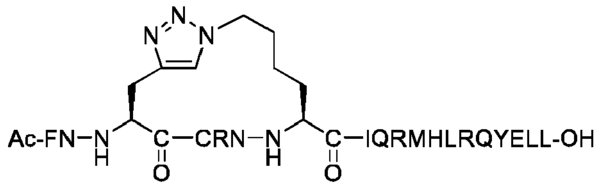 | 156 |

(continued)

| Code | Sequence | SEQ ID NO: |
|---|---|---|
| Compound 39a | Ac-FN**Pra**CRD**AzkI**QRMHLRQY**D**LL-OH | 76 |
| compound 39 | Ac-FN[**Pra**CRD**Azk**]IQRMHLRQY**D**LL-OH 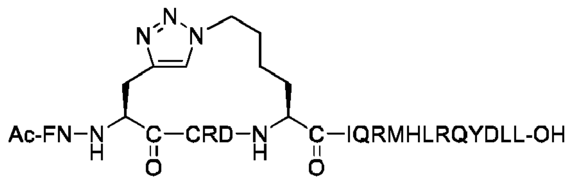 | 157 |
| compound 43a | Ac-FN**Pra**CRD**Azk**IQRMHLRQ**1**-**Nal**ELL-OH | 77 |
| compound 43 | Ac-FN[**Pra**CRD**Azk**]IQRMHLRQ**1**-**Nal**ELL-OH 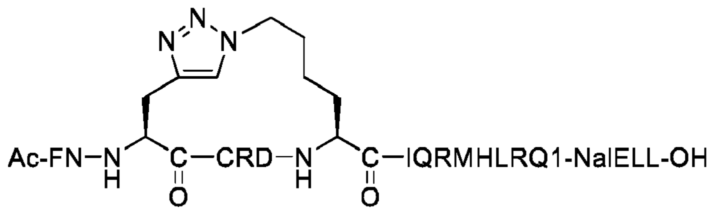 | 158 |
| compound 40a | Ac-**R**FN**Pra**CRD**AzkI**QRM HLRQYELL-OH | 78 |
| compound 40 | Ac-**R**FN[**Pra**CRD**Azk**]IQRMHLRQYELL-OH 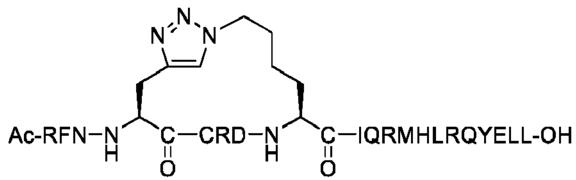 | 159 |
| compound 41a | Ac-**K**FN**Pra**CRD**AzkI**QRM HLRQYELL-OH | 79 |
| compound 41 | Ac-**K**FN[**Pra**CRD**Azk**]IQRMHLRQYELL-OH | 160 |

(continued)

| Code | Sequence | SEQ ID NO: |
|---|---|---|
| | 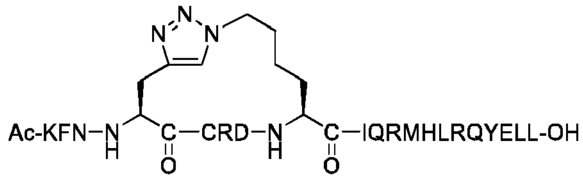 | |
| compound 42a | Ac-**KKK**FN**Pra**CRD**Azkl**QRM HLRQYELL-OH | 80 |
| compound 42 | Ac-**KKK**FN[**Pra**CRD**Azk**]IQRMHLRQYELL-OH 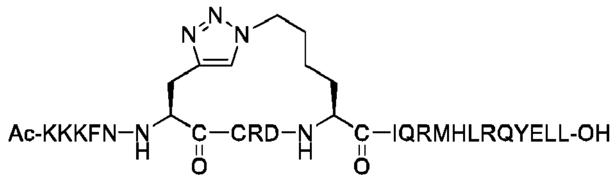 | 161 |
| compound 46a | Ac-**K**FN**Pra**CRD**Azkl**QRMHLRQYEL**I**-OH | 81 |
| compound 46 | Ac-**K**FN[**Pra**CRD**Azk**]IQRMHLRQYELI-OH 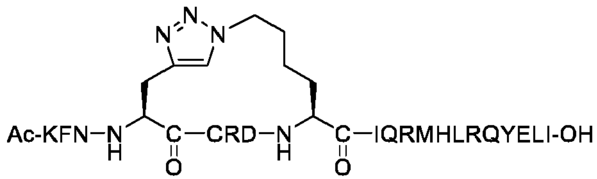 | 162 |
| compound 47a | Ac-**KKK**FN**Pra**CRD**Azk**IQRMHLRQYEL**I**-OH | 82 |
| compound 47 | Ac-**KKK**FN[**Pra**CRD**Azk**]IQRMHLRQYELI-OH 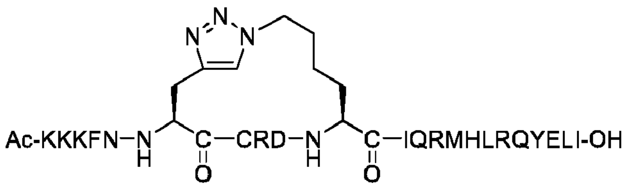 | 163 |
| compound 48a | Ac-**K**FN**Pra**CRD**Azkl**QRMHLRQYE**I**L-OH | 83 |

(continued)

| Code | Sequence | SEQ ID NO: |
|---|---|---|
| compound 48 | Ac-**K**FN[**Pra**CRD**Azk**]IQRMHLRQYE**I**L-OH 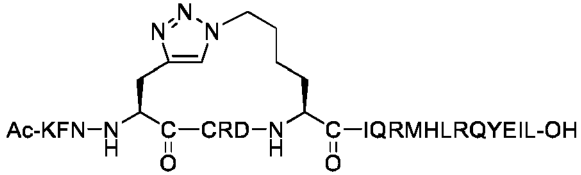 | 164 |
| compound 49a | Ac-**KKK**FN**Pra**CRD**AzkI**QRMHLRQYE**I**L-OH | 84 |
| compound 49 | Ac-**KKK**FN[**Pra**CRD**Azk**]IQRMHLRQYEIL-OH 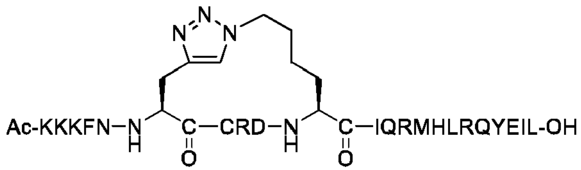 | 165 |
| compound 50a | Ac-**K**FN**Pra**CRD**AzkI**QRMHLRQYE**Cha**L-OH | 85 |
| compound 50 | Ac-**K**FN[**Pra**CRD**Azk**]IQRMHLRQYE**Cha**L-OH 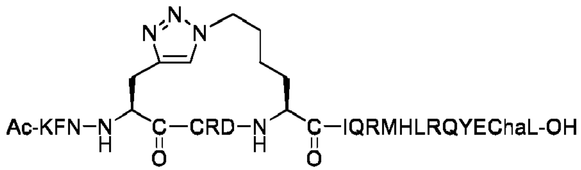 | 166 |
| compound 51a | Ac-**KKK**FN**Pra**CRD**AzkI**QRMHLRQYE**Cha**L-OH | 86 |
| compound 51 | Ac-**KKK**FN[**Pra**CRD**Azk**]IQRMHLRQYE**Cha**L-OH 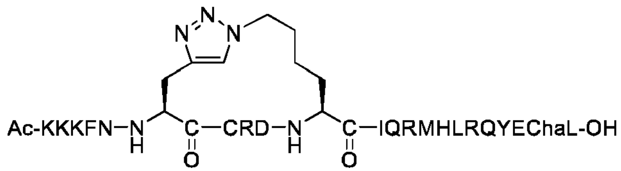 | 167 |
| compound 52a | Ac-**K**FN**Pra**CRD**AzkI**QRMHLRQY**hGlu**LL-OH | 87 |

(continued)

| Code | Sequence | SEQ ID NO: |
|---|---|---|
| compound 52 | Ac-**K**FN[**Pra**CRD**Azk**]IQRMHLRQY**hGlu**LL-OH 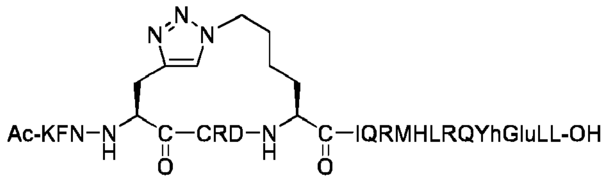 | 168 |
| compound 53a | Ac-**KKK**FN**Pra**CRD**Azkl**QRMHLRQY**hGlu**LL-OH | 88 |
| compound 53 | Ac-**KKK**FN[**Pra**CRD**Azk**]IQRMHLRQYhGluLL-OH 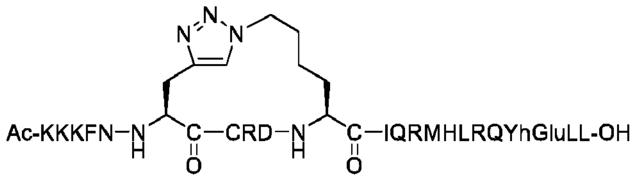 | 169 |
| compound 54a | Ac-**K**FN**Pra**CRD**Azkl**QRMHLRQY**D**LL-OH | 89 |
| compound 54 | Ac-**K**FN[**Pra**CRD**Azk**]IQRMHLRQYDLL-OH 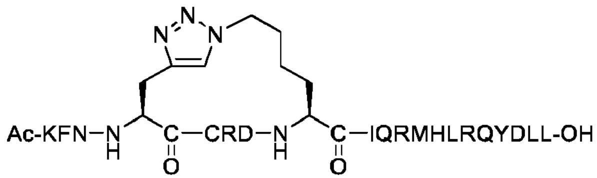 | 170 |
| compound 55a | Ac-**KKK**FN**Pra**CRD**Azkl**QRMHLRQY**D**LL-OH | 90 |
| compound 55 | Ac-**KKK**FN**[Pra**CRD**Azk**]IQRMHLRQY**D**LL-OH 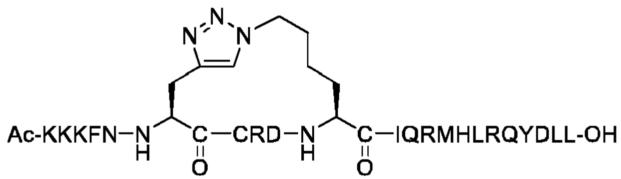 | 171 |
| compound 56a | Ac-**KKK**FN**Pra**CRD**Azkl**QRMHLRQ**W**ELL-OH | 91 |

(continued)

| Code | Sequence | SEQ ID NO: |
|---|---|---|
| compound 56 | Ac-**KKK**FN**[Pra**CRD**Azk]**IQRMHLRQ**W**ELL-OH 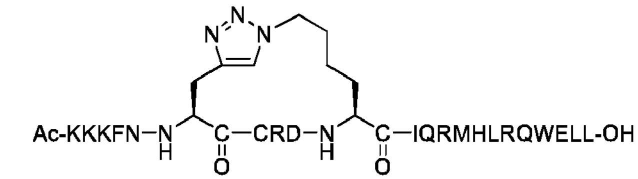 | 172 |
| compound 57a | Ac-**KKK**FN**Pra**CRD**AzkI**QRMHLRQ**1-Nal**ELL-OH | 92 |
| compound 57 | Ac-**KKK**FN**[Pr**aCRD**Azk]**IQRMHLRQ**1-Nal**ELL-OH | 173 |
| | 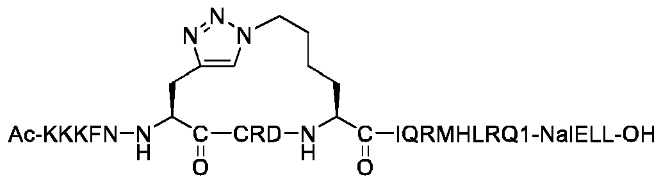 | |
| compound 58a | Ac-**KKK**FN**Pra**CRD**AzkI**QRMHLRQ**2-Nal**ELL-OH | 93 |
| compound 58 | Ac-**KKK**FN**[Pra**CRD**Azk]**IQRMHLRQ**2-Nal**ELL-OH 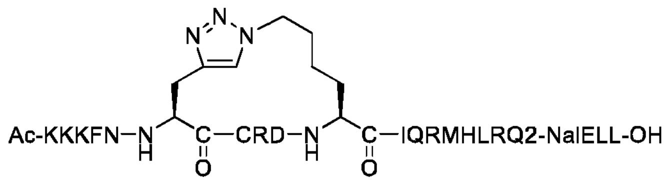 | 174 |
| compound 62a | Ac-**KKK**FN**Pra**CRD**AzkI**QRMHLRQYE**F**L-OH | 94 |
| compound 62 | Ac-**KKK**FN[**Pra**CRD**Azk**]IQRMHLRQYE**F**L-OH | 175 |
| compound 63 | Ac-FNDCRDIIQRMHLRQYE**Cha**L-OH | 95 |
| compound 64a | Ac-RDIIQR**Azk**HLR**Pra**YE**Cha**L-OH | 96 |
| compound 64 | Ac-RDIIQR[**Azk**HLR**Pra**]YE**Cha**L-OH | 176 |
| compound 65a | Ac-**KKK**RDIIQR**Azk**HLR**Pra**YE**Cha**L-OH | 97 |
| compound 65 | Ac-**KKK**RDIIQR[**Azk**HLR**Pra**]YE**Cha**L-OH | 177 |

(continued)

| Code | Sequence | SEQ ID NO: |
|---|---|---|
| compound 66 | Ac-QRMHLRQYE**Cha**L-OH | 98 |
| compound 67 | Ac-RDIIQRMHLRQYE**Cha**L-OH | 99 |
| compound 68 | Ac-FNDCRDIIQRMHLRQYE**F**L-OH | 100 |
| compound 69 | Ac-FNDCRDIIQRMHLRQYE**WL**-OH | 101 |
| compound 70 | Ac-FNDCRDIIQRMHLRQYE**YL**-OH | 102 |
| compound 71 | Ac-FNDCRDIIQRMHLRQYE**H**L-OH | 103 |
| compound 72 | Ac-**NARRI**FNDCRDIIQRMHLRQYELL-OH | 104 |
| compound 73 | Ac-**RRI**FNDCRDIIQRMHLRQYELL-OH | 105 |
| compound 74a | Ac-**KK**FN**Pra**CRD**AzkI**QRMHLRQYE**Cha**L-OH | 106 |
| compound 74 | Ac-**KK**FN[**Pra**CRD**Azk**]IQRMHLRQYE**Cha**L-OH | 178 |
| compound 75 | Ac-FND**A**RDIIQRMHLRQYELL-OH | 107 |
| compound 76 | Ac-FNDCRDIIQRMHLRQYE**A**L-OH | 108 |
| compound 77 | Ac-FNDCRDIIQRMHLRQY**A**LL-OH | 109 |
| compound 78 | Ac-FNDCRDIIQRMHLRQ**A**ELL-OH | 110 |
| compound 79 | Ac-FNDCRDIIQRMHLRQYEL**A**-OH | 111 |
| compound 80 | Ac-IIQRMHLRQYE**Cha**L-OH | 112 |
| compound 81 | Ac-**NARRI**FND**A**RDIIQRMHLRQYELL-OH | 113 |
| compound 82 | Ac-FNDCRDIIQRMHLRQ**Cha**ELL-OH | 114 |
| compound 83 | Ac-**NARRI**FND**A**RDIIQRMHLRQYE**W**L-OH | 115 |
| compound 84 | Ac-FNDCRDIIQRMHLRQYEL**Cha**-OH | 116 |
| compound 18a | H-RDIIQR**Azk**HLR**Pra**YELL-OH | 119 |
| compound 18 | H-RDIIQR[**Azk**HLR**Pra**]YELL-OH | 179 |
| Compound 44a | Ac-FN**Pra**CRD**AzkI**QRMHLRQ**2-Nal**ELL-OH | 121 |
| compound 44 | Ac-FN**[Pra**CRD**Azk]**IQRMHLRQ**2-Nal**ELL-OH | 180 |

Salts of the G protein peptidomimetics

Salts of the G protein peptidomimetics disclosed herein include those which are prepared with acids or bases, depending on the particular substituents present on the subject peptides and peptidomimetics described herein. Examples of a base addition salts include sodium, potassium, calcium, ammonium, or magnesium salt. Examples of acid addition salts include hydrochloric, hydrobromic, nitric, phosphoric, carbonic, sulphuric, and organic acids like acetic, trifluoroacetic, propionic, benzoic, succinic, fumaric, mandelic, oxalic, citric, tartaric, maleic, and the like.

Functional characterization

The G protein peptidomimetics disclosed herein are "capable of stabilizing a GPCR in an active conformational state".

The term "conformation" or "conformational state" of a protein refers generally to the range of structures that a protein may adopt at any instant in time. One of skill in the art will recognize that determinants of conformation or conformational state include a protein's primary structure as reflected in a protein's amino acid sequence (including modified amino acids) and the environment surrounding the protein. The conformation or conformational state of a protein also relates to structural features such as protein secondary structures (e.g., α-helix, β-sheet, among others), tertiary structure (e.g., the three dimensional folding of a polypeptide chain), and quaternary structure (e.g., interactions of a polypeptide chain with other protein subunits). Post-translational and other modifications to a polypeptide chain such as ligand binding, phosphorylation, sulfation, glycosylation, or attachments of hydrophobic groups, among others, can influence the conformation of a protein. Furthermore, environmental factors, such as pH, salt concentration, ionic strength, and osmolality of the surrounding solution, and interaction with other proteins and co-factors, among others, can affect protein conformation. The conformational state of a protein may be determined by either functional assay for activity or binding to another molecule or by means of physical methods such as X-ray crystallography, NMR, or spin labelling, among other methods. For a general discussion of protein conformation and conformational states, one is referred to Cantor and Schimmel, Biophysical Chemistry, Part I: The Conformation of Biological. Macromolecules, W.H. Freeman and Company, 1980, and Creighton, Proteins: Structures and Molecular Properties, W.H. Freeman and Company, 1993. A "specific conformational state" is any subset of the range of conformations or conformational states that a protein may adopt.

A "functional conformation" or a "functional conformational state", as used herein, refers to the fact that proteins possess different conformational states having a dynamic range of activity, in particular ranging from no activity to maximal activity. It should be clear that "a functional conformational state" is meant to cover any conformational state of a GPCR, having any activity, including no activity; and is not meant to cover the denatured states of proteins. For example, a "basal conformational state" can be defined as a low energy state of the receptor in the absence of a ligand (e.g. effector molecules, agonists, antagonists, inverse agonists). An "active conformational state" of a GPCR as used herein refers to a spectrum of receptor conformations that allows signal transduction towards an intracellular effector system, including G protein dependent signalling and G protein-independent signalling (e.g. β-arrestin signalling). Typically, an active conformational state of a GPCR is in the presence of a ligand and an "active conformation" thus encompasses a range of ligand-specific conformations, including an agonist conformation, a partial agonist conformation or a biased agonist conformation.

The term "stabilizing" or "stabilized", with respect to a functional conformational state of a GPCR, refers to an increased stability of a GPCR with respect to the structure (e.g. conformational state) and/or particular biological activity (e.g. intracellular signalling activity, ligand binding affinity, ...). In relation to increased stability with respect to structure and/or biological activity, this may be readily determined by either a functional assay for activity (e.g. $Ca^{2+}$ release, cAMP generation or transcriptional activity, β-arrestin recruitment, ...) or ligand binding or by means of physical methods such as X-ray crystallography, NMR, or spin labelling, among other methods.

Within this context, a G protein peptidomimetic capable of stabilizing a GPCR in an active conformational state may also be referred to as a G protein peptidomimetic "capable of specifically or selectively binding to a GPCR in an active conformational state". A binding agent, in particular a G protein peptidomimetic, that selectively binds to a specific conformation or conformational state of a GPCR generally refers to a binding agent that binds with a higher affinity to a GPCR in a subset of conformations or conformational states than to other conformations or conformational states that the GPCR may assume.

Within the context of the spectrum of conformational states of GPCRs, the terms "specifically bind" and "specific binding", as used herein, refer to the ability of a G protein peptidomimetic as disclosed herein to preferentially recognize and/or bind to a particular conformational state of a GPCR as compared to another conformational state.

The term "affinity", as used herein, refers to the degree to which a ligand or a binding agent (e.g. a G protein peptidomimetic) binds to a target protein so as to shift the equilibrium of target protein and ligand/binding agent toward the presence of a complex formed by their binding. Thus, for example, where a GPCR and a ligand are combined in

relatively equal concentration, a ligand of high affinity will bind to the available antigen on the GPCR so as to shift the equilibrium toward high concentration of the resulting complex. The dissociation constant is commonly used to describe the affinity between a ligand or a binding agent and a target protein. Typically, the dissociation constant is lower than $10^{-5}$ M. Preferably, the dissociation constant is lower than $10^{-6}$ M, more preferably, lower than $10^{-7}$ M. Most preferably, the dissociation constant is lower than $10^{-8}$ M. Other ways of describing the affinity between a ligand or a binding agent and its target protein are the association constant ($K_a$), the inhibition constant ($K_i$), or indirectly by evaluating the potency of ligands by measuring the half maximal inhibitory concentration ($IC_{50}$) or half maximal effective concentration ($EC_{50}$). It will be appreciated that within the scope of the present invention, the term "affinity" is used in the context of a binding agent, in particular a G protein peptidomimetic as disclosed herein, as well as in the context of a ligand or test compound that binds to a target GPCR.

Various methods may be used to determine specific binding (as defined herein before) between a G protein peptidomimetic and a target GPCR, including for example, enzyme linked immunosorbent assays (ELISA), flow cytometry, radioligand binding assays (also referred to as radioligand displacement assay or RLA), surface plasmon resonance assays, phage display, and the like, which are common practice in the art and are further illustrated in the Example section.

A radioligand displacement assay can quantify the pharmacological stabilization of the GPCR in the active conformation by comparing the affinities of an agonist for the basal versus the active GPCR conformer. In particular, stabilization of a GPCR in an active conformational state or specific binding to a GPCR in an active conformational state by a G protein peptidomimetic as disclosed herein can be determined based on the shift in $IC_{50}$ value of a ligand, in particular an agonist more particularly an orthosteric agonist, which binds to the GPCR when tested in the presence and the absence of the G protein peptidomimetic. During the assay, the binding affinity of an orthosteric agonist for the receptor in presence and absence of the (allosteric) peptidomimetic is determined. Therefore, the GPCR, e.g. embedded in membrane extracts, is incubated with a radioligand and different concentrations of the agonist. By increasing the agonist concentration, radioligand will be displaced by the agonist (Fig. 20). A leftward shift of the curve in the presence of the peptidomimetic is indicative of a peptidomimetic capable of stabilizing a GPCR in an active conformational state. A "shift" in $IC_{50}$ value or $IC_{50}$ shift is defined herein as the $IC_{50}$ ratio of a ligand, in particular an agonist, more particularly an orthosteric agonist, which binds to the GPCR in the absence of a G protein peptidomimetic relative to the presence of the G protein peptidomimetic, or the $IC_{50}$ value of a ligand, in particular an agonist, for binding to the GPCR in the absence of a G protein peptidomimetic divided by the $IC_{50}$ value of the ligand in the presence of the G protein peptidomimetic in the same preparation, wherein said $IC_{50}$ values are determined in a radioligand binding assay or radioligand displacement assay (RLA) as known to the skilled person. For example, a human β2AR radioligand binding assay using 3H-dihydroalprenolol (DHA) as the radioligand and increasing concentrations of isoproterenol (agonist) as cold competitor may be used.

In particular embodiments, the G protein peptidomimetics induce a shift in $IC_{50}$ value of more than 5, preferably more than 10, more preferably more than 20, 25, 30, 35, 40 or 45, even more preferably more than 50, wherein said shift is determined in a radioligand binding assay wherein the GPCR is human β2AR, the radioligand is 3H-DHA, and the unlabelled ligand is isoproterenol.

In particular embodiments, the G protein peptidomimetics are capable of inducing an active conformational state in at least 20%, preferably in at least 25%, 30%, 35%, 40% or 45%, more preferably in at least 50%, 55%, 60%, 65%, 70%, 75% or 80% of the GPCRs in a population of GPCRs. A radioligand binding assay as described above can be used to determine the fraction of GPCRs in an active conformational state (fraction Hi or fraction high) or basal conformational state (fraction Lo or fraction low) of a population of GPCRs as described elsewhere herein.

In further particular embodiments, the G protein peptidomimetics induce a shift in $IC_{50}$ value of more than 50, and at least 20% of the GPCRs are induced in the active conformation; or a shift in $IC_{50}$ value of more than 20, and at least 50% of the GPCRs are induced in the active conformation, as determined in a radioligand binding assay wherein the GPCR is human β2AR, the radioligand is 3H-DHA, and the unlabelled ligand is isoproterenol.

"G-protein coupled receptors", or "GPCRs", as used herein, are polypeptides that share a common structural motif, having seven regions of between 22 to 24 hydrophobic amino acids that form seven alpha helices, each of which spans the membrane. Each span is identified by number, i.e., transmembrane-1 (TM1), transmembrane-2 (TM2), etc. The transmembrane helices are joined by regions of amino acids between transmembrane-2 and transmembrane-3, transmembrane-4 and transmembrane-5, and transmembrane-6 and transmembrane-7 on the exterior, or "extracellular" side, of the cell membrane, referred to as "extracellular" regions 1, 2 and 3 (EC1, EC2 and EC3), respectively. The transmembrane helices are also joined by regions of amino acids between transmembrane-1 and transmembrane-2, transmembrane-3 and transmembrane-4, and transmembrane-5 and transmembrane-6 on the interior, or "intracellular" side, of the cell membrane, referred to as "intracellular" regions 1, 2 and 3 (IC1, IC2 and IC3), respectively. The "carboxy" ("C") terminus of the receptor lies in the intracellular space within the cell, and the "amino" ("N") terminus of the receptor lies in the extracellular space outside of the cell. Any of these regions are readily identifiable by analysis of the primary amino acid sequence of a GPCR.

GPCRs can be grouped on the basis of sequence homology into several distinct families. Although all GPCRs

have a similar architecture of seven membrane-spanning α- helices, the different families within this receptor class show no sequence homology to one another, thus suggesting that the similarity of their transmembrane domain structure might define common functional requirements. A comprehensive view of the GPCR repertoire was possible when the first draft of the human genome became available. Fredriksson and colleagues divided 802 human GPCRs into families on the basis of phylogenetic criteria. This showed that most of the human GPCRs can be found in five main families, termed Rhodopsin, Adhesion, Secretin, Glutamate, Frizzled/Taste2 (Fredriksson et al., 2003). Members of the Rhodopsin family (corresponding to class A (Kolakowski, 1994)) or Class 1 (Foord et al (2005) in older classification systems)) only have small extracellular loops and the interaction of the ligands occurs with residues within the transmembrane cleft. This is by far the largest group (>90% of the GPCRs) and contains receptors for odorants, small molecules such as catecholamines and amines, (neuro)peptides and glycoprotein hormones. Rhodopsin, a representative of this family, is the first GPCR for which the structure has been solved. β2AR, the first receptor interacting with a diffusible ligand for which the structure has been solved (Rosenbaum et al, 2007) also belongs to this family. Based on phylogenetic analysis, class B GPCRs or Class 2 (Foord et al, 2005) receptors have recently been subdivided into two families: adhesion and secretin (Fredriksson et al., 2003). Adhesion and secretin receptors are characterized by a relatively long amino terminal extracellular domain involved in ligand-binding. Little is known about the orientation of the transmembrane domains, but it is probably quite different from that of rhodopsin. Ligands for these GPCRs are hormones, such as glucagon, secretin, gonadotropin-releasing hormone and parathyroid hormone. The glutamate family receptors (Class C or Class 3 receptors) also have a large extracellular domain, which functions like a "Venus fly trap" since it can open and close with the agonist bound inside. Family members are the metabotropic glutamate, the $Ca^{2+}$-sensing and the γ- aminobutyric acid (GABA)-B receptors.

GPCRs can also be classified based on the G protein to which they are coupled. Particular non-limiting examples are provided in Table A provided elsewhere herein.

In embodiments, the GPCR that is stabilized in an active conformational state by the G protein peptidomimetic disclosed herein is selected from a Gs protein coupled receptor, a Golf protein coupled receptor, a Gi protein coupled receptor, a Go protein coupled receptor, a Gt protein coupled receptor, a Ggust protein coupled receptor, a Gz protein coupled receptor, a Gq protein coupled receptor, a G12 protein coupled receptor or a G13 protein coupled receptor. In preferred embodiments, the GPCR that is stabilized in an active conformational state by the G protein peptidomimetic disclosed herein is a Gs protein coupled receptor.

In embodiments, the GPCR is a GPCR from the Rhodopsin family (class A) or a class B GPCR, preferably a class A GPCR. In further embodiments, the GPCR is selected from the group comprising the adrenergic receptors (including the α adrenergic receptors, such as the α1 adrenergic receptors and the α2 adrenergic receptors, and the β adrenergic receptors, such as the β1 adrenergic receptors, the $\beta_2$ adrenergic receptors and the $\beta_3$ adrenergic receptors), preferably the β adrenergic receptors; the dopamine receptors (including the D1, D2, D3, D4, and D5 receptors), preferably the D1-like family of dopamine receptors, more preferably the D1 receptor; all of which are well known in the art. In further embodiments, the GPCR is selected from the $\beta_2$ adrenergic receptors, the D1-like family of dopamine receptors. In other further embodiments, the GPCR is selected from the group comprising glucagon-like peptide-1 receptor (GLP1R).

The GPCR that is stabilized in an active conformational state by the G protein peptidomimetic may be naturally occurring or non-naturally occurring (i.e., altered by man). The term "naturally-occurring", as used herein, means a GPCR that is naturally produced. In particular, wild type polymorphic variants and isoforms of GPCRs, as well as orthologues across different species are examples of naturally occurring proteins. Thus, such GPCRs are found in nature. The term "non-naturally occurring", as used herein, means a GPCR that is not naturally-occurring. In certain circumstances, it may be advantageous that the GPCR is a non-naturally occurring protein. For example, and for illustration purposes only, some protein engineering without or only minimally affecting ligand binding affinity might be performed to increase the probability of obtaining crystals of a GPCR stabilized in an active conformational state by a G protein peptidomimetic disclosed herein. Or, alternatively or additionally, to increase cellular expression levels of a GPCR, or to increase the stability, one might also consider introducing certain mutations in the GPCR of interest. Non-limiting examples of non-naturally occurring GPCRs include, without limitation, GPCRs that have been made constitutively active through mutation, GPCRs with a loop deletion, GPCRs with an N- and/or C-terminal deletion, GPCRs with a substitution, an insertion or addition, or any combination thereof, in relation to their amino acid or nucleotide sequence, or other variants of naturally-occurring GPCRs. Also comprised within the scope of the present invention are target GPCRs comprising a chimeric or hybrid GPCR, for example a chimeric GPCR with an N- and/or C-terminus from one GPCR and loops of a second GPCR, or comprising a GPCR fused to a moiety.

The nature of the GPCR is not critical to the invention and can be from any organism including a fungus (including yeast), nematode, virus, insect, plant, bird (e.g. chicken, turkey), reptile or mammal (e.g., a mouse, rat, rabbit, hamster, gerbil, dog, cat, goat, pig, cow, horse, whale, monkey, camelid, or human). Preferably, the GPCR is of mammalian origin, even more preferably of human origin.

Fusion polypeptides

The G protein peptidomimetics disclosed herein can be fused to the GPCR that they can stabilize in an active conformational state, optionally through use of a linker. In this way the constitutive stabilization of a unique active conformation of the GPCR can be obtained through an intramolecular reaction of both moieties. One key advantage of the fusion polypeptides disclosed herein is that a defined 1:1 stoichiometry of GPCR to G protein peptidomimetic is ensured in a single protein, forcing the physical proximity of the fusion partners, while maintaining the properties of the G protein to stabilize the receptor in an active conformational state. It is thus particularly envisaged that the fusion polypeptides described herein comprise a GPCR moiety that is stabilized in an active conformation upon binding of the G protein moiety in an intramolecular reaction, preferably without the need for an additional ligand.

Accordingly, an aspect relates to a fusion molecule or fusion polypeptide comprising i) a GPCR as defined herein and ii) a G protein peptidomimetic as disclosed herein that is capable of stabilizing said GPCR in an active conformational state. The G protein peptidomimetic is fused to the GPCR either directly or through a linker.

The terms "fusion polypeptide" or "fusion protein" are used interchangeably herein and refer to a protein that comprises at least two separate and distinct (poly)peptide components that may or may not originate from the same protein. The (poly)peptide components, while typically unjoined in their native state, are joined by their respective amino and carboxyl termini through a peptide linkage to form a single continuous polypeptide. The term "fused to", and other grammatical equivalents, when referring to a fusion polypeptide (as defined herein) refers to any chemical or recombinant mechanism for linking two or more (poly)peptide components. The fusion of the two or more (poly)peptide components may be a direct fusion of the sequences or it may be an indirect fusion, e.g. with intervening amino acid sequences or linker sequences.

The way the different moieties that form part of the fusion polypeptides as described, in particular the GPCR and the G protein peptidomimetic, are fused to each other will typically depend on both the type of GPCR and the characteristics of the G protein peptidomimetic (e.g. linear or stapled G protein peptidomimetic).

As is known by the person skilled in the art, GPCRs are characterized by an extracellular N-terminus, followed by seven transmembrane $\alpha$-helices connected by three intracellular and three extracellular loops, and finally an intracellular C-terminus. In preferred embodiments, the G protein peptidomimetic is fused to the C-terminus of the GPCR. The G protein peptidomimetic will preferably be fused with its N-terminal end to the C-terminal end of the GPCR.

Further, the fusion may be a direct fusion of the sequences or it may be an indirect fusion, e.g. with intervening amino acid sequences or linker sequences.

Linker molecules or linkers may be peptides of 1 to 200 amino acids length, and are typically, but not necessarily, chosen or designed to be unstructured and flexible. For instance, one can choose amino acids that form no particular secondary structure. Or, amino acids can be chosen so that they do not form a stable tertiary structure. Or, the amino acid linkers may form a random coil. Such linkers include, but are not limited to, synthetic peptides rich in Gly, Ser, Thr, Gln, Glu or further amino acids that are frequently associated with unstructured regions in natural proteins. IUPred: web server for the prediction of intrinsically unstructured regions of proteins based on estimated energy content. Non-limiting examples include $(G_xS_z)_b$ wherein x and z are independently chosen integers from 0 to 9 one of them having at least a value of 1 and wherein b is an integer from 1 to 9. Preferably, the amino acid linker sequence has a low susceptibility to proteolytic cleavage and does not interfere with the biological activity of the fusion polypeptide. Hence, a suitable linker should not provide sterical hindrance or impede proper folding of the functional portion of either the G protein peptidomimetic or the GPCR.

A person skilled in the art will know how to design a fusion construct. For general methods relating to the present disclosure, reference is made inter alia to well-known textbooks, including e.g. "Molecular Cloning: A Laboratory Manual, 4th Ed." (Green and Sambrook et al., 2012, Cold Spring Harbor Laboratory Press)". A convenient means for linking or fusing two (poly)peptides is by expressing them as a fusion protein from a recombinant nucleic acid molecule, which comprises a first polynucleotide encoding a first (poly)peptide operably linked to a second polynucleotide encoding the second (poly)peptide. This method is particularly preferably for G protein peptidomimetics disclosed herein that have a peptide backbone consisting of naturally occurring amino acids, or peptidomimetics that consist of naturally occurring amino acids. Otherwise, the (poly)peptides comprised in a fusion protein can be linked through peptide bonds that result from chemoenzymatic methods. In case the G protein peptidomimetic and the GPCR moiety are linked using chemoenzymatic methods for protein modification, the linker moiety may exist of different chemical entities, depending on the enzymes or the synthetic chemistry that is used to produce the covalent chimer *in vivo* or *in vitro.*

Complex

In an aspect, the invention provides a complex comprising a GPCR as defined herein and a G protein peptidomimetic as disclosed herein that specifically binds to said GPCR. In embodiments, the complex may further comprise at least one other receptor ligand.

A related aspect provides a complex comprising a fusion polypeptide disclosed herein and at least one other receptor ligand.

As used herein, the term "ligand" means a molecule that specifically binds to a GPCR. A ligand may be, without the purpose of being limitative, a polypeptide, a peptide, a peptidomimetic, a lipid, a small molecule, an antibody, an antibody fragment, a nucleic acid, a carbohydrate. A ligand may be synthetic or naturally occurring. A ligand includes a "native ligand" which is a ligand that is an endogenous, natural ligand for a native GPCR. Within the context of the present invention, a ligand may bind to a GPCR, either intracellularly or extracellularly. An "orthosteric ligand" as used herein, refers to a ligand that binds to the active site of a GPCR. Orthosteric ligands are further classified according to their efficacy or in other words to the effect they have on signalling through a specific pathway. As used herein, an "agonist" refers to a ligand that, by binding a receptor protein, increases the receptor's signalling activity. Full agonists are capable of maximal protein stimulation; partial agonists are unable to elicit full activity even at saturating concentrations. Partial agonists can also function as "blockers" by preventing the binding of more robust agonists. An "antagonist", also referred to as a "neutral antagonist", refers to a ligand that binds a receptor without stimulating any activity. An "antagonist" is also known as a "blocker" because of its ability to prevent binding of other ligands and, therefore, block agonist-induced activity. Further, an "inverse agonist" refers to an antagonist that, in addition to blocking agonist effects, reduces a receptor's basal or constitutive activity below that of the unliganded protein. Ligands as used herein may also be "biased ligands" (also known as "biased agonists" or "functionally selective agonists") with the ability to selectively stimulate a subset of a receptor's signalling activities, for example in the case of GPCRs the selective activation of G-protein or β-arrestin function. More particularly, ligand bias can be an imperfect bias characterized by a ligand stimulation of multiple receptor activities with different relative efficacies for different signals (non-absolute selectivity) or can be a perfect bias characterized by a ligand stimulation of one receptor protein activity without any stimulation of another known receptor protein activity. Another kind of ligands is known as allosteric regulators. "Allosteric regulators" or otherwise "allosteric modulators", "allosteric ligands" or "effector molecules", as used herein, refer to ligands that bind at an allosteric site (that is, a regulatory site physically distinct from the protein's active site) of a GPCR. In contrast to orthosteric ligands, allosteric modulators are non-competitive because they bind receptor proteins at a different site and modify their function even if the endogenous ligand also is binding. Allosteric regulators that enhance the protein's activity are referred to herein as "allosteric activators" or "positive allosteric modulators" (PAMs), whereas those that decrease the protein's activity are referred to herein as "allosteric inhibitors" or otherwise "negative allosteric modulators" (NAMs).

In particular embodiments, the ligand in the complexes described herein may be a "conformation-selective ligand" or "conformation-specific ligand", meaning that such a ligand binds the GPCR in a conformation-selective manner. A conformation-selective ligand binds with a higher affinity to a particular conformation of the GPCR than to other conformations the GPCR may adopt. In further particular embodiments, the ligand is an active conformation-selective ligand and the GPCR is an active conformational state in the complex described herein.

In embodiments, the ligand is an agonist (e.g. a partial agonist or full agonist) and the GPCR is in an active conformational state. The ligand may also be an inverse agonist, an antagonist or a biased ligand. Ligands also include allosteric modulators, potentiators, enhancers, negative allosteric modulators and inhibitors.

As a non-limiting example, a stable complex as described herein may be purified by size exclusion chromatography.

The complexes described herein may be crystalline. So, a crystal of the complex is also provided herein, as well as methods of making said crystal, which are described in greater detail below. Preferably, a crystalline form of a complex as described herein and a receptor ligand is envisaged.

Compositions

The fusion polypeptides and complexes described herein may be in a solubilized form, such as in a detergent. Alternatively, the fusion polypeptide or complex may be immobilized to a solid support. Non-limiting examples of solid supports as well as methods and techniques for immobilization are well known to the skilled person. Yet alternatively, the fusion polypeptide or complex may be in a cellular composition, including an organism, a tissue, a cell, a cell line, or in a membrane composition or liposomal composition derived from said organism, tissue, cell or cell line. Examples of membrane or liposomal compositions include, but are not limited to organelles, membrane preparations, viruses, virus like lipoparticles, and the like. It will be appreciated that a cellular composition, or a membrane-like or liposomal composition may comprise natural or synthetic lipids.

Accordingly, the present invention also relates to compositions comprising a fusion polypeptide or a complex as described herein. Particular embodiments relate to a membrane or liposomal composition comprising a fusion polypeptide or a complex as described herein. Membrane compositions may be derived from a tissue, cell or cell line and include organelles, membrane extracts or fractions thereof, VLPs, viruses, and the like, as long as sufficient functionality of the fusion polypeptides and complexes is retained.

Expression systems

Further disclosed herein is a nucleic acid molecule comprising one or more nucleic acid sequences encoding a G protein peptidomimetic of the invention.

Also disclosed herein is a nucleic acid molecule comprising a nucleic acid sequence encoding a fusion polypeptide of the invention.

Further disclosed herein are expression vectors comprising nucleic acid sequences encoding a G protein peptidomimetic or fusion polypeptide as described herein, as well as host cells expressing such expression vectors.

In certain embodiments, the expression vector encodes a cleavable concatenation of the G protein peptidomimetic, optionally separated by a protease cleavage site sequence.

It is evident that further regulatory sequences may be part of the expression vector such as but not limited to promoters, enhancers, selection markers, origins of replication, linker sequences, polyA sequences, and degradation sequences. The term "selection marker" as used herein is to be interpreted in accordance to its generally accepted meaning in the art, i.e. a gene that allow for artificial selection of cells comprising (a certain amount of concentration of) the expression vector(s) carrying the selection marker. Suitable selection markers include prokaryotic or eukaryotic antibiotic resistance genes or fluorescent proteins. In certain embodiments wherein the G protein peptidomimetic and GPCR are encoded by a distinct expression vector, each expression vector can be construed in order to express a separate selection marker. The selection marker(s) may be fused to the GPCR and/or the G protein peptidomimetic or may be expressed as separate moieties. In the latter embodiments, expression of the selection marker(s) and the GPCR/G protein peptidomimetic may be governed (i.e. regulated) by a single promoter or by distinct promoters. In embodiments where expression of the selection marker and the GPCR and/or G protein peptidomimetic is regulated by a single promoter, the different moieties may still be expressed as separate elements by inclusion of one or more e.g. internal ribosomal entry sites (IRES) sequences or alternatively one or more 2A self-cleaving peptide sequences. Hence, bicistronic and multicistronic expression vectors are envisaged by the inventors and part of the scope of the invention.

Suitable expression systems include constitutive and inducible expression systems in bacteria or yeasts, virus expression systems, such as baculovirus, semliki forest virus and lentiviruses, or transient transfection in insect or mammalian cells. The cloning and/or expression of the G protein peptidomimetics and fusion polypeptides can be done according to techniques known by the skilled person in the art. The expression of the GPCR and/or G protein peptidomimetic may be governed by a constitutive promoter sequence or an inducible promoter sequence. Non-limiting examples of inducible expression systems are the tetracycline- or doxycycline-induced Tet-On and Tet-off expression systems (Gossen et al. 1995 PNAS 5547:5551, and Gossen et al. 1995 Science 1766:1769).

The "host cell" can be of any prokaryotic or eukaryotic organism. Preferably, the host cell is a eukaryotic cell and can be of any eukaryotic organism, but in particular embodiments yeast, plant, mammalian and insect cells are envisaged. The nature of the cells used will typically depend on the ease and cost of producing the G protein peptidomimetics and fusion polypeptides, the desired glycosylation properties, the origin of the fusion polypeptide, the intended application, or any combination thereof. Mammalian cells may for instance be used for achieving complex glycosylation, but it may not be cost-effective to produce proteins in mammalian cell systems. Plant and insect cells, as well as yeast typically achieve high production levels and are more cost-effective, but additional modifications may be needed to mimic the complex glycosylation patterns of mammalian proteins. Yeast cells are often used for expression of proteins because they can be economically cultured, give high yields of (medium-secreted) protein, and when appropriately modified are capable of producing proteins having suitable glycosylation patterns. Further, yeast offers established genetics allowing for rapid transformations, tested protein localization strategies, and facile gene knock-out techniques. Insect cells are also an attractive system to express GPCRs because insect cells offer an expression system without interfering with mammalian GPCR signalling. Eukaryotic cell or cell lines for protein production are well known in the art, including cell lines with modified glycosylation pathways, and non-limiting examples will be provided hereafter.

Exemplary animal or mammalian host cells suitable for harboring, expressing, and producing proteins such the G protein peptidomimetics and fusion polypeptides disclosed herein, for subsequent isolation and/or purification include Chinese hamster ovary cells (CHO), such as CHO-K1 (ATCC CCL-61), DG44 (Chasin et al., 1986; Kolkekar et al., 1997), CHO-K1 Tet-On cell line (Clontech), CHO designated ECACC 85050302 (CAMR, Salisbury, Wiltshire, UK), CHO clone 13 (GEIMG, Genova, IT), CHO clone B (GEIMG, Genova, IT), CHO-K1/SF designated ECACC 93061607 (CAMR, Salisbury, Wiltshire, UK), RR-CHOK1 designated ECACC 92052129 (CAMR, Salisbury, Wiltshire, UK), dihydrofolate reductase negative CHO cells (CHO/-DHFR, Urlaub and Chasin, 1980), and dp12.CHO cells (U.S. Pat. No. 5,721,121); monkey kidney CV1 cells transformed by SV40 (COS cells, COS-7, ATCC CRL-1651); human embryonic kidney cells (e.g., 293 cells, or 293T cells, or 293 cells subcloned for growth in suspension culture, Graham et al., 1977, J. Gen. Virol., 36:59, or GnTI KO HEK293S cells, Reeves et al. 2002); baby hamster kidney cells (BHK, ATCC CCL-10); monkey kidney cells (CV1, ATCC CCL-70); African green monkey kidney cells (VERO-76, ATCC CRL-1587; VERO, ATCC CCL-81); mouse sertoli cells (TM4, Mather, 1980, Biol. Reprod., 23:243-251); human cervical carcinoma cells (HELA, ATCC CCL-2); canine kidney cells (MDCK, ATCC CCL-34); human lung cells (W138, ATCC CCL-75); human hepatoma cells

(HEP-G2, HB 8065); mouse mammary tumor cells (MMT 060562, ATCC CCL-51); buffalo rat liver cells (BRL 3A, ATCC CRL-1442); TRI cells (Mather, 1982); MCR 5 cells; FS4 cells. Preferably, the cells are mammalian cells selected from Hek293 cells or COS cells.

Exemplary non-mammalian cell lines include, but are not limited to, insect cells, such as Sf9 cells/baculovirus expression systems (e.g. review Jarvis, Virology Volume 310, Issue 1, 25 May 2003, Pages 1-7), plant cells such as tobacco cells, tomato cells, maize cells, algae cells, or yeasts such as *Saccharomyces* species, Schizosaccharomyces species, *Hansenula* species, *Yarrowia* species or *Pichia* species. According to particular embodiments, the eukaryotic cells are yeast cells from a *Saccharomyces* species (e.g. *Saccharomyces cerevisiae*), Schizosaccharomyces sp. (for example Schizosaccharomyces pombe), a *Hansenula* species (e.g. *Hansenula polymorpha*), a *Yarrowia* species (e.g. *Yarrowia lipolytica*), a *Kluyveromyces* species (e.g. *Kluyveromyces lactis*), a *Pichia* species (e.g. *Pichia pastoris*), or a *Komagataella* species (e.g. *Komagataella pastoris*).

Transfection of target cells (e.g. mammalian cells) can be carried out following principles outlined by Sambrook and Russel (Molecular Cloning, A Laboratory Manual, 3rd Edition, Volume 3, Chapter 16, Section 16.1-16.54). In addition, viral transduction can also be performed using reagents such as adenoviral vectors. Selection of the appropriate viral vector system, regulatory regions and host cell is common knowledge within the level of ordinary skill in the art. The resulting transfected cells are maintained in culture or frozen for later use according to standard practices.

Applications

The above described G protein peptidomimetics as well as the complexes and fusion polypeptides comprising these G protein peptidomimetics are particularly useful in a variety of contexts and applications. For example, and without limitation, (1) for capturing and/or purification of a GPCR whereby upon binding, the G protein peptidomimetic maintains the receptor in a particular conformation, in particular an active conformation; (2) for co-crystallization studies and high-resolution structural analysis of a GPCR in complex with the G protein peptidomimetic, and optionally additionally bound to another conformation-selective receptor ligand; (3) for ligand characterization, compound screening, and (structure-based) drug discovery;, all of which will be described into further detail below.

*Capturing, separation and purification methods for GPCR in a functional conformation*

In an aspect, the invention provides a method for capturing and/or purifying a GPCR in a functional conformation, preferably an active conformation, by making use of any of the above described G protein peptidomimetics. Capturing and/or purifying a receptor in a particular conformation such as an active conformation will allow amongst others subsequent crystallization, ligand characterization, compound screening, immunizations, etc.

Thus, the invention relates to the use, preferably an *in vitro* or *ex vivo* use, of a G protein peptidomimetic as described herein to capture a GPCR in a functional conformation, in particular an active conformation. Optionally, but not necessarily, capturing of a GPCR in an active conformation may include capturing a GPCR in complex with another conformation-selective receptor ligand (e.g. an orthosteric ligand, an allosteric ligand, a natural binding partner such as an arrestin, and the like).

In accordance, the invention also provides a method of capturing a GPCR in a functional conformation, in particular an active conformation, said method comprising the steps of:

a) bringing a G protein peptidomimetic as described herein into contact with a GPCR, and

b) allowing the G protein peptidomimetic to specifically bind to the GPCR, whereby GPCR is captured in a functional conformation, in particular an active conformation.

In embodiments, the invention also envisages a method of capturing a GPCR in a functional conformation, in particular an active conformation, said method comprising the steps of:

a) applying a solution containing GPCR in a plurality of conformations to a solid support possessing an immobilized G protein peptidomimetic as described herein, and
b) allowing the G protein peptidomimetic to specifically bind to the GPCR, whereby the GPCR is captured in a functional conformation, in particular an active conformation and
c) optionally removing weakly bound or unbound molecules.

It will be appreciated that any of the methods as described above may further comprise the step of isolating the complex formed in step (ii) of the above described methods, said complex comprising the G protein peptidomimetic and the GPCR in a particular conformation. Suitable techniques for isolating/purifying GPCRs include, without limitation,

affinity-based methods such as affinity chromatography, affinity purification, immunoprecipitation, protein detection, immunochemistry, surface-display, size exclusion chromatography, ion exchange chromatography, amongst others, and are all well-known in the art.

*Crystallography and applications in structure-based drug design*

The G protein peptidomimetics disclosed herein are particularly useful in X-ray crystallography of GPCRs and applications thereof in structure-based drug design.

Agonist-bound receptor crystals may provide three-dimensional representations of the active states of GPCRs, which structures can help clarifying the conformational changes connecting the ligand-binding and G protein-interaction sites, and lead to more precise mechanistic hypotheses and eventually new therapeutics. Given the conformational flexibility inherent to ligand-activated GPCRs, stabilizing such a state, e.g. for crystal formation, is not easy. Such efforts can benefit from the stabilization of the agonist-bound receptor conformation by the addition of binding agents that are specific for an active conformational state of the receptor. It is thus a particular advantage of the G protein peptidomimetics that upon binding to the GPCR, they can stabilize the receptor in an active conformation, thereby reducing its conformational flexibility and increasing its polar surface, facilitating the crystallization of a receptor:G protein peptidomimetic complex. The G protein peptidomimetics of the present invention are therefore valuable tools to increase the probability of obtaining well-ordered crystals by minimizing the conformational heterogeneity in the target GPCR.

The so-obtained crystals will also be of great advantage to help guide drug discovery. Especially methods for acquiring structures of receptors bound to lead compounds that have pharmacological or biological activity and whose chemical structure is used as a starting point for chemical modifications in order to improve potency, selectivity, or pharmacokinetic parameters are very valuable and are provided herein. Persons of ordinary skill in the art will recognize that the G protein peptidomimetics disclosed herein are particularly suited for co-crystallization of receptor:G protein peptidomimetic with lead compounds that are selective for the druggable conformation induced by the G protein peptidomimetic because this G protein peptidomimetic is able to substantially increase the affinity of conformation-selective receptor ligands.

Thus, it is envisaged herein to use the G protein peptidomimetics disclosed herein for crystallization purposes. Advantageously, crystals can be formed of a complex of a G protein peptidomimetic as disclosed herein and a GPCR to which the G protein peptidomimetic specifically binds (as disclosed herein), wherein the receptor is trapped in a particular receptor conformation, more particularly a therapeutically relevant receptor conformation (e.g. an active conformation). The G protein peptidomimetic will also reduce the flexibility of extracellular regions upon binding the receptor to grow well-ordered crystals.

Accordingly, provided herein is the use of G protein peptidomimetics as described herein for crystallizing a complex of a G protein peptidomimetic and a GPCR to which the G protein peptidomimetic can specifically bind, and eventually to solve the structure of the complex. Particular embodiments relate to crystallization of a complex of a G protein peptidomimetic as described herein, a GPCR to which the G protein peptidomimetic will specifically bind, and another conformation-selective receptor ligand (as defined hereinbefore).

In accordance, provided herein is a method of crystallizing a complex of a G protein peptidomimetic and a GPCR to which the G protein peptidomimetic can specifically bind and optionally determining the crystal structure of a GPCR in a functional conformation, in particular an active conformation, the method comprising the steps of:

a) providing a G protein peptidomimetic as described herein and a GPCR to which the G protein peptidomimetic can specifically bind, and optionally a receptor ligand, and

b) allowing the formation of a complex of the G protein peptidomimetic, the GPCR and optionally a receptor ligand,

c) crystallizing said complex of step b) to form a crystal, and

d) optionally obtaining the atomic coordinates of the crystal.

"Crystal" or "crystalline structure", as used herein, refers to a solid material, whose constituent atoms, molecules, or ions are arranged in an orderly repeating pattern extending in all three spatial dimensions. The process of forming a crystalline structure from a fluid or from materials dissolved in the fluid is often referred to as "crystallization" or "crystallogenesis". Protein crystals are almost always grown in solution. The most common approach is to lower the solubility of its component molecules gradually. Crystal growth in solution is characterized by two steps: nucleation of a microscopic crystallite (possibly having only 100 molecules), followed by growth of that crystallite, ideally to a diffraction-quality crystal.

Any of a variety of specialized crystallization methods for membrane proteins can be used, many of which are reviewed in Caffrey (2003 & 2009). In general terms, the methods are lipid-based methods that include adding lipid to

the complex prior to crystallization. Many of these methods, including the lipidic cubic phase crystallization method and the bicelle crystallization method, exploit the spontaneous self-assembling properties of lipids and detergent as vesicles (vesicle-fusion method), discoidal micelles (bicelle method), and liquid crystals or mesophases (in meso or cubic-phase method). Lipidic cubic phases crystallization methods are described in, for example: Landau et al. 1996; Gouaux 1998; Rummel et al. 1998; Nollert et al. 2004, Rasmussen et al. 2011a and b, which publications are incorporated by reference for disclosure of those methods. Bicelle crystallization methods are described in, for example: Faham et al. 2005; Faham et al. 2002, which publications are incorporated by reference for disclosure of those methods.

"Solving the structure" as used herein refers to determining the arrangement of atoms or the atomic coordinates of a protein, and is often done by a biophysical method, such as X-ray crystallography. In many cases, obtaining a diffraction-quality crystal of a protein is the key barrier to solving its atomic-resolution structure. The herein described G protein peptidomimetics can be used to improve the diffraction quality of the crystals so that the crystal structure of the receptor:G protein peptidomimetic complex can be solved/determined.

The term "atomic coordinates", as used herein, refers to a position of atoms within the space of a molecular structure, typically expressed by a set of X, Y, and Z coordinates. In certain embodiments, the atomic coordinates contain additional information. A skilled person appreciates that a 3D rigid body rotation of the atomic coordinates or a translation of the atomic coordinates do not alter the structure of the described structure. An illustrative example to conduct similarity analyses is by means of software application such as the molecular similarity program QUANTA (Molecular Simulations Inc., San Diego).

In one embodiment, atomic coordinates are obtained using X-ray crystallography according to methods well-known to those of ordinarily skill in the art of biophysics. "X-ray crystallography", as used herein, is a method of determining the arrangement of atoms within a crystal, in which a beam of X-rays strikes a crystal and diffracts into many specific directions. From the angles and intensities of these diffracted beams, a crystallographer can produce a three-dimensional picture of the density of electrons within the crystal. From this electron density, the mean positions of the atoms in the crystal can be determined, as well as their chemical bonds, their disorder and various other information. Those skilled in the art understand that a set of structure co-ordinates determined by X-ray crystallography contains standard errors. In other embodiments, atomic coordinates can be obtained using other experimental biophysical structure determination methods that can include electron diffraction (also known as electron crystallography) and nuclear magnetic resonance (NMR) methods. In yet other embodiments, atomic coordinates can be obtained using molecular modelling tools which can be based on one or more of ab initio protein folding algorithms, energy minimization, and homology-based modelling. These techniques are well known to persons of ordinary skill in the biophysical and bioinformatic arts.

*Ligand screening and drug discovery*

Other applications are particularly envisaged that can make use of the G protein peptidomimetics of the invention, including compound or fragment screening, which will be described further herein. The G protein peptidomimetics disclosed herein are particularly useful for the screening of compounds or fragments that selectively recognize structural features of orthosteric or allosteric sites that are unique to the active conformation of a GPCR (leading to G protein coupled signalling).

In the process of compound screening, lead optimization and drug discovery (including peptide and antibody discovery), there is a requirement for faster, more effective, less expensive and especially information-rich screening assays that provide simultaneous information on various compound characteristics and their effects on various cellular pathways (i.e. efficacy, specificity, toxicity and drug metabolism). Thus, there is a need to quickly and inexpensively screen large numbers of compounds in order to identify new specific ligands of a protein of interest, preferably conformation-selective ligands, which may be potential new drug candidates.

Alternatively, fragment-based drug discovery (FBDD) is a method to generate hits for selected drug targets. FBDD is based on the concept that the chemical space is easier filled by low molecular weight fragments than larger molecules, as used in high-throughput screenings (HTS). Fragments identified by screening techniques (functional screening, nuclear magnetic resonance, mass spectrometry and X-ray crystallography) may be optimized by elongation or combination in order to improve the affinity and reach the criteria for drug leads. In order to discover novel conformation-selective drugs, there is a need for tools that allow the identification of low-molecular weight fragments with a moderate affinity for the targeted receptors in a particular conformation such as an active conformation.

The present invention provides G protein peptidomimetics that stabilize or lock a GPCR in a functional conformation, preferably in an active conformation. This will allow to quickly and reliably screen for and differentiate between receptor agonists, inverse agonists, antagonists and/or modulators as well as inhibitors of GPCRs, so increasing the likelihood of identifying a ligand with the desired pharmacological properties. Further, as shown herein, the G protein peptidomimetics described herein can be used for fragment-based screening to identify low-molecular weight fragments with a desired affinity for the active GPCR conformer. In particular, the G protein peptidomimetics, the complexes and fusion polypeptides comprising the same, and compositions, including cellular compositions, comprising said G protein

peptidomimetics, complexes or fusion polypeptides, for which specific preferences have been described herein before, are particularly suitable for this purpose, and can then be used as selection reagents for screening in a variety of contexts.

Thus, the present invention encompasses the use of the G protein peptidomimetics described herein, complexes comprising the same, fusion polypeptides comprising the same, or compositions comprising said G protein peptidomimetics, complexes orfusion polypeptides as described hereinbefore, in screening and/or identification programs for binding partners or ligands of a GPCR, in particular a GPCR to which the G protein specifically binds. This might ultimately lead to potential new drug candidates.

In accordance, the invention method provides a (screening) method for identifying a compound capable of interacting with a GPCR, comprising:

- contacting the GPCR with a test compound(s) and a G protein peptidomimetic, fusion polypeptide, complex or composition as described herein;

- evaluating binding of the test compound to the GPCR; and

- optionally selecting a test compound(s) that binds to the GPCR as a compound capable of interacting with the GPCR.

In particular embodiments, the compound capable of interacting with the GPCR is a conformation-selective compound of the GPCR, in particular an active conformation-selective compound of the GPCR.

Also disclosed herein is a method of identifying conformation-selective compounds of a GPCR, the method comprising the steps of

a) providing a complex or fusion polypeptide comprising a GPCR and a G protein peptidomimetic capable of stabilizing the GPCR in an active conformational state, and
b) providing a test compound, and
c) evaluating whether the test compound is a conformation-selective compound for the GPCR.

Specific preferences for the G protein peptidomimetics, complexes, fusion polypeptides, and compositions are as defined above with respect to earlier aspects of the invention.

In embodiments, the G protein peptidomimetic, the GPCR or the complex or fusion polypeptide comprising the G protein peptidomimetic and the GPCR, as used in any of the screening methods described herein, are provided as whole cells, or cell (organelle) extracts such as membrane extracts or fractions thereof, or may be incorporated in lipid layers or vesicles (comprising natural and/or synthetic lipids), high-density lipoparticles, or any nanoparticle, such as nanodisks, or are provided as virus or virus-like particles (VLPs), so that sufficient functionality of the respective proteins is retained. Methods for preparations of GPCRs from membrane fragments or membrane-detergent extracts are reviewed in detail in Cooper (2004). Alternatively, the GPCR and/or the complex or fusion polypeptide may also be solubilized in detergents. High-throughput screening for binding partners or ligands of receptors may be preferred, and optionally the screening methods disclosed herein may be miniaturized in view hereof. The use of both new and known compound libraries is envisaged in the present invention. Also envisaged herein is the use of low-molecular weight fragment libraries. The size of the compound or fragment library is not limiting.

This may be facilitated by immobilization of either the G protein peptidomimetic, the complex or the fusion polypeptide as described herein onto a suitable solid surface or support that can be arrayed or otherwise multiplexed. Accordingly, in embodiments, the G protein peptidomimetic, the complex or the fusion polypeptide are immobilized to a solid support.

Non-limiting examples of suitable solid supports include beads, columns, slides, chips or plates. More particularly, the solid supports may be particulate (e. g. beads or granules, generally used in extraction columns) or in sheet form (e. g. membranes or filters, glass or plastic slides, microtiter assay plates, dipstick, capillary fill devices or such like) which can be flat, pleated, or hollow fibres or tubes. The following matrices are given as examples and are not exhaustive, such examples could include silica (porous amorphous silica), e.g. the FLASH series of cartridges containing 60A irregular silica (32-63 um or 35-70 um) supplied by Biotage (a division of Dyax Corp.); agarose or polyacrylamide supports, for example the Sepharose range of products supplied by Amersham Pharmacia Biotech, or the Affi-Gel supports supplied by Bio-Rad. In addition, there are macroporous polymers, such as the pressure-stable Affi-Prep supports as supplied by Bio-Rad. Other supports that could be used include, without limitation, dextran, collagen, polystyrene, methacrylate, calcium alginate, controlled pore glass, aluminium, titanium and porous ceramics. Alternatively, the solid surface may comprise part of a mass dependent sensor, for example, a surface plasmon resonance detector. Further examples of commercially available supports are discussed in, for example, Protein Immobilization, R.F. Taylor ed.., Marcel Dekker, Inc., New York, (1991).

Immobilization may be either non-covalent or covalent. In particular, non-covalent immobilization or adsorption

on a solid surface of the G protein peptidomimetic, or the complex or the fusion polypeptide comprising the G protein peptidomimetic and the GPCR, may occur via a surface coating with any of an antibody, or streptavidin or avidin, or a metal ion, recognizing a molecular tag attached to the G protein peptidomimetic, according to standard techniques known by the skilled person (e.g. biotin tag, histidine tag, etc.). Alternatively, G protein peptidomimetic, or the complex or fusion polypeptide comprising the G protein peptidomimetic and the GPCR, may be attached to a solid surface by covalent cross-linking using conventional coupling chemistries. A solid surface may naturally comprise cross-linkable residues suitable for covalent attachment or it may be coated or derivatized to introduce suitable cross-linkable groups according to methods well known in the art. Sufficient functionality of the immobilized protein can be retained following direct covalent coupling to the desired matrix via a reactive moiety that does not contain a chemical spacer arm. Advances in molecular biology, particularly through site-directed mutagenesis, enable the mutation of specific amino acid residues in a protein sequence. The mutation of a particular amino acid (in a protein with known or inferred structure) to a lysine or cysteine (or other desired amino acid) can provide a specific site for covalent coupling, for example. It is also possible to reengineer a specific protein to alter the distribution of surface available amino acids involved in the chemical coupling (Kallwass et al, 1993), in effect controlling the orientation of the coupled protein. A similar approach can be applied to the G protein peptidomimetics, thereby minimizing disruption to the GPCR-binding activity of the G protein peptidomimetic, so providing a means of oriented immobilization without the addition of other peptide tails or domains containing either natural or unnatural amino acids.

Conveniently, the immobilized proteins described herein may be used in immunoadsorption processes such as immunoassays, for example ELISA, or immunoaffinity purification processes by contacting the immobilized proteins with a test sample according to standard methods conventional in the art. Alternatively, and particularly for high-throughput purposes, the immobilized proteins can be arrayed or otherwise multiplexed.

In other embodiments, the test compound (or a library of test compounds) may be immobilized on a solid surface, such as a chip surface, whereas the G protein peptidomimetic and GPCR, the complex or the fusion polypeptide as described herein are provided, for example, in a detergent solution or in a membrane-like preparation or composition.

In yet other embodiments, neither the G protein peptidomimetic, nor the GPCR, nor the test compound is immobilized, for example in phage-display selection protocols in solution, or radioligand binding assays. For example, the GPCR, as used in any of the screening methods described herein, may be provided as whole cells, or cell (organelle) extracts such as membrane extracts or fractions thereof, wherein the GPCR is embedded in the cell wall or cell membrane fragment, or the GPCR may be incorporated in lipid layers or vesicles (comprising natural and/or synthetic lipids), high-density lipoparticles, or any nanoparticles, such as nanodisks, or as virus or virus-like particles (VLPs) as described above. Typically, the G protein peptidomimetic and its binding epitope (which typically comprises amino acid residues from the intracellular loops of the GPCR) are on one side (which may be referred to as the "intracellular" side) of respectively, the cell wall, the cell membrane, the lipid layer or vesicle, lipoparticle, nanoparticle, etc. whereas the test compound(s) is on the other side (which may be referred to as the "extracellular" side).

Screening assays for drug discovery can be solid phase (e.g. beads, columns, slides, chips or plates) or solution phase assays, e.g. a binding assay, such as radioligand binding assays.

In high-throughput assays, it is possible to screen up to several thousand different compounds or low-molecular weight fragments, in a single day in 96-, 384- or 1536-well formats. For example, each well of a microtiter plate can be used to run a separate assay against a selected test compound, or, if concentration or incubation time effects are to be observed, every 5-10 wells can test a single test compound. Thus, a single standard microtiter plate can assay about 96 test compounds. It is possible to assay many plates per day; assay screens for up to about 6.000, 20.000, 50.000 or more different compounds are possible today.

Various methods may be used to determine binding between the (active conformation stabilized) GPCR and a test compound, including for example, flow cytometry, radioligand binding assays, enzyme linked immunosorbent assays (ELISA), surface plasmon resonance assays, chip-based assays, immunocytofluorescence, yeast two-hybrid technology and phage display which are common practice in the art, for example, in Sambrook et al. (2001), Molecular Cloning, A Laboratory Manual. Third Edition. Cold Spring Harbor Laboratory Press, Cold Spring Harbor, NY. Other methods of detecting binding between a test compound and a GPCR include ultrafiltration with ion spray mass spectroscopy/HPLC methods or other (bio)physical and analytical methods. Fluorescence Energy Resonance Transfer (FRET) methods, for example, well known to those skilled in the art, may also be used. It will be appreciated that a bound test compound can be detected using a unique label or tag associated with the compound, such as a peptide label, a nucleic acid label, a chemical label, a fluorescent label, or a radioactive isotope label, as described further herein.

The test compound may thus optionally be covalently or non-covalently linked to a detectable label. Suitable detectable labels and techniques for attaching, using and detecting them will be clear to the skilled person. Non-limiting examples include detection by spectroscopic, photochemical, biochemical, immunochemical, electrical, optical or chemical means. Useful labels include magnetic beads (e.g. dynabeads), fluorescent dyes (e.g. all Alexa Fluor dyes, fluorescein isothiocyanate, Texas red, rhodamine, green fluorescent protein and the like), radiolabels (e.g. $^{3}H$, $^{125}I$, $^{35}S$, $^{14}C$, or $^{32}P$), enzymes (e.g. horse radish peroxidase, alkaline phosphatase), and colorimetric labels such as colloidal gold or

coloured glass or plastic (e.g. polystyrene, polypropylene, latex, etc.) beads. Means of detecting such labels are well known to those of skill in the art. Thus, for example, radiolabels may be detected using photographic film or scintillation counters, fluorescent markers may be detected using a photodetector to detect emitted illumination. Enzymatic labels are typically detected by providing the enzyme with a substrate and detecting the reaction product produced by the action of the enzyme on the substrate, and colorimetric labels are detected by simply visualizing the coloured label.

The compounds to be tested can be any small chemical compound, a macromolecule (such as a protein, a sugar, nucleic acid or lipid), as well as a low-molecular weight fragment. In embodiments, the test compound used in any of the screening methods described herein is selected from the group comprising a polypeptide, a peptide, a small molecule, a natural product, a peptidomimetic, a nucleic acid, a lipid, a lipopeptide, a carbohydrate, an antibody or any fragment derived thereof, such as Fab, Fab' and F(ab')2, Fd, single-chain Fvs (scFv), single-chain antibodies, disulfide-linked Fvs (dsFv) and fragments comprising either a VL or VH domain, a heavy chain antibody (hcAb), a single domain antibody (sdAb), a minibody, the variable domain derived from camelid heavy chain antibodies (VHH or Nanobody), the variable domain of the new antigen receptors derived from shark antibodies (VNAR), a protein scaffold including an alphabody, protein A, protein G, designed ankyrin-repeat domains (DARPins), fibronectin type III repeats, anticalins, knottins, engineered and CH2 domains (nanoantibodies). For example, test compounds may be small chemical compounds, peptides, antibodies, or (low-molecular weight) fragments thereof.

It will be appreciated that in some embodiments the test compound may be a library of test compounds. For example, high-throughput screening assays for therapeutic compounds such as agonists, antagonists or inverse agonists and/or modulators are envisaged herein. For high-throughput purposes, compound libraries or combinatorial libraries may be used such as allosteric compound libraries, peptide libraries, antibody libraries, fragment-based libraries, synthetic compound libraries, natural compound libraries, phage-display libraries and the like. Methodologies for preparing and screening such libraries are known to those of skill in the art. For example, high-throughput screening methods may involve providing a combinatorial chemical or peptide library containing a large number of potential therapeutic ligands. Such "combinatorial libraries" or "compound libraries" are then screened in one or more assays, as described herein, to identify those library members (particular chemical species or subclasses) that display a desired characteristic activity. A "compound library" as used herein refers to a collection of stored chemicals usually used ultimately in high-throughput screening A "combinatorial library" refers to a collection of diverse chemical compounds generated by either chemical synthesis or biological synthesis, by combining a number of chemical "building blocks" such as reagents. Preparation and screening of combinatorial libraries are well known to those of skill in the art.

The compounds thus identified can serve as conventional "lead compounds" or can themselves be used as potential or actual therapeutics. Thus, in one further embodiment, the screening methods as described herein further comprises a step of modifying a test compound which has been shown to selectively bind to a GPCR in a particular conformation, in particular an active conformation, and determining whether the modified test compound binds to the GPCR when residing in the particular conformation.

In embodiments, it is determined whether the test compound alters the binding of a receptor ligand (as defined herein) to the GPCR. Preferably, the receptor ligand is chosen from the group comprising a small molecule, a polypeptide, an antibody or any fragment derived thereof, a natural product, and the like. More preferably, the receptor ligand is a full agonist, or a partial agonist, a biased agonist, an antagonist, or an inverse agonist, as described hereinbefore. Binding of a ligand to this receptor can be assayed using standard ligand binding methods known in the art as described elsewhere herein. For example, a ligand may be radiolabelled or fluorescently labelled. The compound will be characterized by its ability to alter the binding of the labelled ligand. The compound may decrease the binding between the ligand and the receptor, or may increase the binding between the ligand and the receptor, for example by a factor of at least 2 fold, 3 fold, 4 fold, 5 fold, 10 fold, 20 fold, 30 fold, 50 fold, 100 fold.

In embodiments, the test compound as used in any of the herein described screening methods is provided as a biological sample. In particular, the sample can be any suitable sample taken from an individual. For example, the sample may be a body fluid sample such as blood, serum, plasma, spinal fluid.

In addition to establishing binding to a GPCR in a particular conformation of interest, it will also be desirable to determine the functional effect of a compound on the receptor. For example, the compounds may bind to the GPCR resulting in the modulation (activation or inhibition) of the biological function of the receptor, in particular the downstream receptor signalling. This modulation of intracellular signalling can occur ortho- or allosterically. The compounds may bind to the GPCR so as to activate or increase receptor signalling; or alternatively so as to decrease or inhibit receptor signalling. The compounds may also bind to the GPCR in such a way that they block off the constitutive activity of the receptor. The compounds may also bind to the GPCR in such a way that they mediate allosteric modulation (e.g. bind to the receptor at an allosteric site). In this way, the compounds may modulate the receptor function by binding to different regions in the receptor (e.g. at allosteric sites). Reference is for example made to George et al. 2002; Kenakin 2002; Rios et al. 2001. The compounds may also bind to the GPCR in such a way that they prolong the duration of the receptor-mediated signalling or that they enhance receptor signalling by increasing receptor-ligand affinity. Further, the compounds may also bind to the GPCR in such a way that they inhibit or enhance the assembly of receptor functional homomers

or heteromers. The efficacy of the compounds and/or compositions comprising the same, can be tested using any suitable in vitro assay, cell-based assay, in vivo assay and/or animal model known per se, or any combination thereof, depending on the specific disease or disorder involved.

It will be appreciated that the G protein peptidomimetics, complexes, fusion polypeptides, and compositions comprising the same as described herein, may be further engineered and are thus particularly useful tools for the development or improvement of cell-based assays. Cell-based assays are critical for assessing the mechanism of action of new biological targets and biological activity of chemical compounds. For example, without the purpose of being limitative, current cell-based assays for GPCRs include measures of pathway activation ($Ca^{2+}$ release, cAMP generation or transcriptional activity); measurements of protein trafficking by tagging GPCRs and downstream elements with GFP; and direct measures of interactions between proteins using Forster resonance energy transfer (FRET), bioluminescence resonance energy transfer (BRET) or yeast two-hybrid approaches.

In embodiments, the complex or fusion polypeptide described herein comprising the GPCR and the G protein peptidomimetic that specifically binds to the GPCR may be used for the selection of binding agents including antibodies or antibody fragments that bind the receptor by any of the screening methods as described above. Persons of ordinary skill in the art will recognize that such binding agents, as a non-limiting example, can be selected by screening a set, collection or library of cells that express binding agents on their surface, or bacteriophages that display a fusion of genIII and binding agent at their surface, or yeast cells that display a fusion of the mating factor protein Aga2p, or by ribosome display amongst others.

*Kit of parts*

Still another aspect of the invention relates to a kit comprising a G protein peptidomimetic capable of stabilizing a GPCR in an active conformational state, optionally as a fusion polypeptide with the GPCR, or a kit comprising a composition as described herein comprising such G protein peptidomimetic or fusion polypeptide. In further examples, the kit of parts may comprise a cellular expression system comprising an oligonucleotide sequence encoding the G protein peptidomimetic as described herein, optionally as a fusion polypeptide with the GPCR. In certain embodiments, the G protein peptidomimetic is encoded in the genome of the cellular expression system. The kit may further comprise a combination of reagents such as buffers, molecular tags, vector constructs, reference sample material, as well as a suitable solid supports, and the like. Such a kit may be useful for any of the applications of the present invention as described herein. For example, the kit may further comprise (a library of) test compounds useful for compound screening applications.

The present invention will now be further illustrated by means of the following non-limiting examples.

## EXAMPLES

### Example 1: General procedures

I. Materials and Reagents

The natural (Fmoc-protected) amino acids, the resins (preloaded Fmoc-Leu-Wang and Rink Amide resin) and the coupling reagent O-(benzotriazol-1-yl)-*N,N,N',N'*-tetramethyluronium hexafluorophosphate (HBTU) were purchased from Chem-Impex. Also, the unnatural (Fmoc-protected) 1-naphthyl-L-alanine (1-Nal), 2-naphthyl-L-alanine (2-Nal) and cyclohexyl-L-alanine (Cha) were ordered at Chem-Impex. The Fmoc-protected $\alpha,\alpha$-disubstituted unnatural amino acids for RCM cyclization, (S)-$\alpha$-methyl,$\alpha$-pentenylglycine ($S_5$), (R)-$\alpha$-methyl,$\alpha$-pentenylglycine ($R_5$) and (R)-$\alpha$-methyl,$\alpha$-octenylglycine ($R_8$), were obtained from Fluorochem, while the catalyst for cyclization, Hoveyda Grubbs 2$^{nd}$ generation, was from Carbosynth. The Fmoc-protected propargylglycine (Pra) and azidolysine (Azk), were ordered from Carbolution and IRIS Biotech GMBH resp. The copper(I) bromide (CuBr) for the Cu(I)-catalyzed azide-alkyne cycloaddition was originated from abcr GmbH. The reagents 4-methylpiperidine, diisopropylethylamine (DIPEA) and dimethyl sulfoxide (DMSO) were bought from Sigma-Aldrich, as well as Tetrakis (triphenylphosphine) Palladium(0), sodium diethyldithiocarbamate and pyridine hydrochloride. Trifluoroacetic acid (TFA), triisopropylsilane (TIS), ethyl cyano(hydroxyimino)acetate (Oxyma), N,N'-diisopropylcarbodiimide (DIC), phenylsilane and 2,2,2-trifluoroethanol (TFE) were purchased from Fluorochem. Acetic anhydride and 1-hydroxybenzotriazole hydrate (HOBt.$H_2O$) were obtained from Fluka. The reagent 2-(tert-butoxycarbonyloxyimino)-2-phenylacetonitrile (Boc-ON) was purchased from Janssen Chimica. The solvents were from Sigma-Aldrich for dichloromethane (DCM), acetonitrile and 1,2-dichloroethane, and from Acros Organics for *N,N*-dimethyl formamide (DMF) and methanol (MeOH). The Milli-Q water was obtained after purification through a Millipore Simplicity UV system.

The radioligands, [$^3$H]-dihydroalprenolol ([$^3$H]-DHA, 105 Ci/mmol) and [$^3$H]-SCH23390 (83,6 Ci/mmol), as well as the Whatman GF/C and GF/B unifilters and scintillation liquid were obtained from Perkin Elmer. The agonist A-77636

was purchased from Tocris Bioscience, while the agonist Isoproterenol was from Sigma-Aldrich, as well as ascorbic acid. For preparation of buffers, trisaminomethane (Tris), magnesium chloride ($MgCl_2$), Ethylenediaminetetraacetic acid (EDTA), sucrose and the Albumin fraction (BSA) were bought from Carl Roth. The Pierce BCA Protein Assay Kit and the 96-well plates were purchased from Thermo Scientific and sodium dihydrogen phosphate hydrate ($NaH_2PO_4.H_20$) was obtained from Merck.

II. Peptide synthesis and cyclization

All peptides were synthesized using Fmoc-based solid phase peptide synthesis (SPPS) on an automated synthesizer and/or manually, depending on the sequence. The synthesis was performed on preloaded Fmoc-Leu-Wang resin (loading 0.6 - 0.75 mmol/g) or Rink Amide resin (0.92 mmol/g) depending on the desired C-terminal end of the peptide, being a carboxylic acid or carboxamide. The resin was first swollen during 20 min in dichloromethane (DCM) followed by the Fmoc deprotection twice using a solution of 20 % 4-methylpiperidine in *N'-,N'*-dimethylformamide (DMF), for 5 min and 15 min, respectively. Then the resin was washed with DMF and DCM. During the manual synthesis 3 equiv. of Fmoc-protected amino acid (1.5 equiv. for unnatural amino acids) was added to the coupling mixture, consisting of 3 equiv. of O-(benzotriazol-1-yl)-*N,N,N',N'*-tetramethyluronium hexafluorophosphate (HBTU) and 4 equiv. of *N,N*-diisopropylethylamine (DIPEA) in DMF and let shaking for 40 min (1.5 h when 1.5 equiv. is used). After each coupling, the mixture was filtered off and the resin was washed with DMF and DCM. Before every coupling, the resin was treated with 20 % 4-methylpiperidine for Fmoc-deprotection. Using the automatic synthesizer (Activotec P11 or CEM Liberty Blue), the coupling was performed with 5 equiv. of Fmoc-protected amino acid (2 equiv. for unnatural amino acids) in a solution of 0.5 M HBTU and 1 M DIPEA in DMF for the Activotec P11 and 0.5 M DIC and 1 M Oxyma in DMF for the Liberty Blue. At the end of the synthesis the resin was removed from the synthesizer and washed several times with DCM. The acetylation of the *N*-terminus was performed manually with 10 equiv. of acetic anhydride and 5 equiv. of DIPEA during 1 h in DMF. The peptides with free *N*-terminus, were Boc-protected with 4 equiv. of 2-(tert-butoxycarbonyloxyimino)-2-phenylacetonitrile (Boc-ON) and 5 equiv. of DIPEA in DMF during 2 h, before cyclization.

Cyclization via lactamization: the orthogonal protecting groups (Allyl/Alloc) were removed after treatment with phenylsilane (24 equiv.) and Tetrakis (triphenylphosphine) Palladium(0) (0.2 equiv.) in DCM for two times, during 15 min. The resin was then washed with a solution of 11.6 mM sodium diethyldithiocarbamate and 0.01 % DIPEA in DMF, followed by 3 washing steps with DCM. The cyclization was then performed overnight with a solution of HBTU (6 equiv.), HOBt (6 equiv.) and DIPEA (12 equiv.) in DMF, followed by washing with DMF and DCM.

Cyclization via Cu(I)-catalyzed azide-alkyne cycloaddition: the macrocyclization was performed using 24 equiv. of CuBr and 24 equiv. DIPEA in DMF, during 7 h. The copper was removed by washing the resin with a solution of 1 M pyridine hydrochloride in DCM/methanol (95:5), followed by washing steps with DMF and DCM.

Cyclization via Ring-closing metathesis: the microwave assisted ring-closing metathesis was performed with 0.2 equiv. Hoveyda Grubbs catalyst 2nd generation in 1,2-dichloroethane, during 3 h at 85°C under Argon atmosphere (Biotage® Initiator+ Microwave Synthesizer). The resin beads were then washed with DMF and DCM.

After completion of the peptides, they were cleaved from the resin using a cocktail solution consisting of 95 % TFA, 2.5 % triisopropylsilane (TIS) and 2.5 % distilled water during 4 h. Finally, the crude peptides were obtained after freeze-drying. The purification of the crude products was performed using a preparative HPLC to obtain the peptide (TFA salt) as a powder with a high purity (> 97 %).

III. Receptor expression and membrane preparation

$\beta_2$AR365N constructs were expressed in Sf9 insect cells, using the Bac-to-Bac® Baculovirus Expression System. The membrane extracts were prepared from cells that expressed $\beta_2$AR. Subsequently, the cell pellet was resuspended in a buffer (1 ml buffer/2x1E7cells) containing 75 mM Tris pH 7.4, 12.5 mM $MgCl_2$, 1 mM EDTA, Leupeptin and PMSF as protease inhibitors. Afterwards the cells were vortexed and homogenized in ice using a small volume Ultraturrax (6x10 sec). The cells were then centrifuged for 15 min at 16000 rcf, in a pre-cooled centrifuge (4°C) and resuspended in the previous buffer containing 10 % sucrose. Finally, the membranes were again homogenized in ice with the small volume Ultraturrax (3x10 sec). The protein concentration in the membrane fragments was determined using a Pierce BCA Protein Assay Kit and the protein concentration was extrapolated from the Bovine Serum Albumine standard curve.

IV. Radioligand binding assay

To perform the radioligand competition binding assays, 96-well plates were used. For the assays on $\beta_2$AR, the membrane extracts as well as the peptides were prepared in a binding buffer (75 mM Tris pH 7.4,12.5 mM $MgCl_2$, 1 mM EDTA, 0.2 % BSA). The ligand (isoproterenol) and radioligand ([$^3$H]-dihydroalprenolol) were dissolved in ligand buffer (75 mM Tris pH 7.4, 12.5 mM $MgCl_2$, 1 mM EDTA, 0.2 % BSA and 0.1 % ascorbic acid), at 1 mM and 2 nM, respectively.

A ten-fold dilution series of the ligand was prepared to obtain a dose response curve. For each reaction 100 μl membrane extract, 50 μl ligand, 50 μl peptide or binding buffer containing 1 % DMSO and 50 μl of radioligand was added to the V-bottom storage plate, with a total volume of 250 μl per well. Afterwards, the plates were incubated at room temperature for 2 h on a plate shaker (150 rpm). The samples were harvested into filter plates and washed with ice cold washing buffer (75 mM Tris pH 7,4) using the 96-well harvester. The plates were dried for 1 h at 50 °C and then sealed to allow the addition of 30 μl scintillation liquid to each well. Finally, the top of each plate was sealed and the plates were placed in a Wallac MICROBETA® Trilux scintillation counter and incubated for 30 min in the dark.

For the radioligand assays on D1R a similar buffer, as for $\beta_2$AR, was used for the ligands, membrane extracts and peptides (+ 1 % DMSO). The competition assay was performed with [$^3$H]-SCH23390 as radiolabelled antagonist, at a concentration of 2 nM. A ten-fold dilution series of the agonist, A-77636, was prepared to obtain a dose response curve, starting from 100 μM for the receptor alone and 10 μM in the presence of the peptidomimetics. After incubation, the samples were harvested into filter plates, pre-soaked with 0.5 % polyethylenimine (PEI), and washed with ice cold washing buffer using the 96-well harvester. After drying in the oven during 1 h and the addition of scintillation liquid, the plates were placed in the MICROBETA® scintillation counter and incubated for 30 min in the dark before reading.

After the Microbeta scintillation counter, the experimental data was further analyzed using Graphpad Prism 6.0. After normalization of the raw data, a nonlinear regression was used to fit the data in a one-site binding model, with the total binding set as 100 % and the non-specific binding between 0 and 5 %. The $IC_{50}$ values, obtained from the competition curves, were used to determine the shift for each peptide, which is the ratio of $IC_{50}$ of the agonist test compound on the basal conformation (e.g. $IC_{50}$ $\beta_2$AR with no peptidomimetic) versus the active conformation (e.g. $IC_{50}$ $\beta_2$AP + peptidomimetic) of the receptor. For certain peptide analogues, a biphasic binding curve was observed and therefore fitted by a two-site model (nonlinear regression), resulting in two $IC_{50\ values}$ ($IC_{50Hi}$ and $IC_{50Lo}$) and a FractionHi, the fraction of receptors in the active state.

V. Circular dichroism (CD) spectroscopy

CD spectra were measured using a quartz cuvette of 1 mm path length at 20 °C on a Jasco J-715 spectropolarimeter with a scan rate of 50 nm $min^{-1}$, a bandwidth of 1.0 nm for a resolution of 0.5 nm. The peptides were dissolved at a concentration of 100 μM in a $NaH_2PO_4$ buffer (pH 6) without or with 25 % 2,2,2-trifluoroethanol (TFE). Five baseline scans of buffer were taken and averaged at T = 20 °C, followed by five scans of each peptide. These spectra were averaged, baseline-subtracted from the average blank scans and converted to the mean residue ellipticity. The mean residue ellipticity $[\theta]_{222}$ was calculated according to the following equation:

$$[\theta]_{222} = (\theta_{222} * M_W)/(10 * n * C * l)$$

With $\theta_{222}$ the measured ellipticity at 222 nm, $M_w$ the molecular weight of the peptide (g $mol^{-1}$), n the number of amino acids in the peptide, C the peptide concentration (mg/mL) and l the path length. The maximum mean ellipticity was then calculated using undermentioned formula:[30]

$$[\theta]_{max} = (-44\,000 + 250T) * (1 - k/n)$$

Where T is the temperature (°C) and k is 3 for N-acetylated peptides, corresponding to the non-hydrogen-bonded peptide carbonyls. The percentage α-helicity was then determined using following formula:

$$\%\ helicity = ([\theta]_{222}/[\theta]_{max}) * 100$$

**VI.** Fragment screening

Fragment screening was performed on 175 fragments of the Maybridge Ro3 library, to identify fragments that preferentially binds to the active and/or basal state, for drug discovery. This assay was done on $\beta_2$AR and D1R under similar conditions. A single point radioligand binding assay was carried out in parallel on the receptor and the conformational constrained receptor, in presence of the peptidomimetic. For $\beta_2$AR, the membrane extracts as well as the peptides were prepared in a binding buffer (75 mM Tris pH 7.4, 12.5 mM $MgCl_2$, 1 mM EDTA and 0.2 % BSA). The fragments, stored in DMSO (20 mM), and radioligand ([$^3$H]-dihydroalprenolol) were dissolved in ligand buffer (75 mM Tris pH 7.4, 12.5 mM $MgCl_2$, 1 mM EDTA, 0.2 % BSA and 0.1 % ascorbic acid), to a final concentration of resp. 200 μM and 2 nM. For D1R, the same buffer (50 mM Tris pH 7.4, 10 mM $MgCl_2$, 0.5 mM EDTA and 0.1 % BSA) was used

for the membrane extracts, peptide, fragments and radioligand. The fragments were divided over 96-well plates, each containing alprenolol ($\beta_2$AR) or A77636 (D1R) at 10 $\mu$M, to define non-specific binding, buffer to determine total binding and a dose-response curve of isoproterenol ($\beta_2$AR) or A77636 (D1R) as control. Each fragment (50 $\mu$l) was added to the wells containing, the receptor in membrane extracts (100 $\mu$l), peptidomimetic or buffer (50 $\mu$l) and the radioligand (50 $\mu$l). After incubation on a plate shaker for 2 h, the plates were washed with ice cold wash buffer (75 mM Tris pH 7.4 for $\beta_2$AR and 50 mM Tris pH 7.4 for D1R) and filtered using the harvester. While no specific treatment was required for the filter plates (B) of $\beta_2$AR, the plates (C) for D1R were pre-soaked with 0.5 % PEI, before harvesting to reduce non-specific binding. Each plate was then dried for 1 h in the oven. After addition of the scintillation fluid, the radioactivity was determined with a MICROBETA® scintillation counter. Each point was normalized relative to its maximal binding and the fragments were ranked based on their residual binding on the active and basal state.

**Example 2: Interaction of the G protein $\alpha$5 helix with the GPCR**

The present inventors synthesized peptides that mimic the G protein, in particular the $G_\alpha$ subunit of the G protein, from the G protein epitope interacting with the GPCR: the $\alpha_5$ helix comprising the following sequence F$^{376}$NDCRDIIQRMHLRQYELL$^{394}$ (SEQ ID NO:117). Without wishing to be bound by the theory, the identified interacting residues of the $\alpha_5$ helix are underlined in the linear sequence, and figure 21 shows the interaction map of a peptide representing the $\alpha_5$ helix with the $\beta_2$AR. Figure 21 shows that the peptide representing the $\alpha$5 helix interacts with the receptor through one face only, mostly via non-polar interactions with a participation of the four C-terminal amino acids (YELL), forming a reverse turn at the Gs' C-terminus. The two last leucine residues appear to be contact points with TM6, and potentially responsible for its outward movement upon receptor activation, while the C-terminal tyrosine is interacting with an arginine in TM3.

**Example 3: RLA**

Synthesised peptidomimetics were evaluated using a radioligand binding assay (RLA) with a human $\beta_2$AR, using $^3$H-dihydroalprenolol (DHA) as the radioligand and increasing concentrations of isoproterenol (agonist) as cold competitor. In particular, the pharmacological stabilization of the receptor in the active conformation was evaluated by performing the radioligand displacement assay (RLA) in the presence and absence of the peptidomimetics. This validated assay quantifies the pharmacological stabilization of the receptor in the active conformation by comparing the affinities of an agonist for the basal versus the active GPCR conformer (Pardon et al. (2018) Angewandte Chemie International Edition 57, 5292-5295). During the assay, the binding affinity of an orthosteric agonist for the receptor, in particular isoproterenol for human $\beta$2AR, in presence and absence of the allosteric peptidomimetic was determined. Therefore, the receptor embedded in membrane extracts was incubated with a radiolabelled antagonist, in particular $^3$H-dihydroalprenolol (DHA), and different concentrations of the agonist. By increasing the agonist concentration, radioligand was displaced by the agonist (Fig. 20, black curve). A leftward shift of the curve caused by the presence of the peptidomimetic was indicative of a peptidomimetic capable of stabilizing a GPCR in an active conformational state. Because the RLAs were performed on a population of receptors in membrane extracts, not all receptors showed the same affinity for the ligand, resulting in biphasic binding curve (Fig. 20, light grey curve) with a fraction of receptors showing a high affinity for the agonist ($IC_{50}$Hi, Fraction Hi) and a percentage of receptors displaying a low affinity for the agonist ($IC_{50}$Lo, Fraction Lo).

Tables 1-18 show the results of the radioligand displacements assays. The half maximal inhibitory concentration ($IC_{50}$) represents the affinity of the agonist for the receptor; The selectivity is quantified by the shift. "[]" denotes cyclic peptides.

I. Evaluation of the peptide length

The results are shown in Figures 1 and 2, and Tables 1 and 2.

Table 1

| Peptidomimetic | Sequence | SEQ ID NO: | $IC_{50}$ (nM) | Shift |
|---|---|---|---|---|
| $\beta_2$AP | | | 658 | 1 |
| $\beta_2$AP + compound 2 | Ac-FNDCRDIIQRMHLRQYELL-OH | 53 | 118 | 6 |
| $\beta_2$AP + compound 16 | Ac-RDIIQRMHLRQYELL-OH | 65 | 614 | 1 |
| $\beta_2$AP + compound 21 | Ac-QRMHLRQYELL-OH | 67 | 965 | 1 |

Table 2

| Peptidomimetic | Sequence | SEQ ID NO: | $IC_{50}$ (nM) | Shift |
|---|---|---|---|---|
| $\beta_2$AP | | | 1 104 | 1 |
| $\beta_2$AP + compound 2 | Ac-FNDCRDIIQRMHLRQYELL-OH | 53 | 77.1 | 13 |
| $\beta_2$AP + compound 72 | Ac-**NARRI**FNDCRDIIQRMHLRQYELL-OH | 104 | 22.2 | 46 |
| $\beta_2$AP + compound 73 | Ac-**RRI**FNDCRDIIQRMHLRQYELL-OH | 105 | 52.6 | 19 |

**II.** Evaluation of stapling and comparison with Confobodies

The tether positioning and cyclization method were evaluated by RLA. The results are shown in Figures 3-7 and Tables 3-7.

Table 3

| Code | Sequence | SEQ ID NO: | $IC_{50}$ (nM) | shift |
|---|---|---|---|---|
| $\beta_2$AP | | | 792 | 1 |
| $\beta_2$AP + compound 3 | Ac-FNDCRD**E**IQR**K**HLRQYELL-OH | 54 | 408 | 2 |
| $\beta_2$AP + compound 4 | H-FNDCRD[**E**IQR**K**]HLRQYELL-OH | 137 | 202 | 4 |
| $\beta_2$AP + compound 5 | Ac-FNDCRD[**E**IQR**K**]HLRQYELL-OH | 138 | 197 | 4 |

**Cyclization:** Lactamization, ($X_7$-$X_8$)

Table 4

| Code | Sequence | SEQ ID NO: | $IC_{50}$ (nM) | shift |
|---|---|---|---|---|
| $\beta_2$AP | | | 884.2 | 1 |
| $\beta_2$AP + compound 6 | Ac-FNDCRD**$S_5$**IQR**$S_5$**HLRQYELL-OH | 56 | 104.1 | 8 |
| $\beta_2$AP + compound 7 | H-FNDCRD**[$S_5$**IQR**$S_5$]**HLRQYELL-OH | 139 | 417.3 | 2 |
| $\beta_2$AP + compound 8 | Ac-FNDCRD**[$S_5$**IQR**$S_5$]**HLRQYELL-OH | 140 | 373 | 2 |

**Cyclization:** RCM, ($X_7$-$X_8$)

Table 5

| Code | Sequence | SEQ ID NO: | $IC_{50}$ (nM) | shift |
|---|---|---|---|---|
| $\beta_2$AP | | | 798 | 1 |
| $\beta_2$AP + compound 9 | Ac-FNDCRD**Pra**IQR**Azk**HLRQYELL-OH | 58 | 211 | 4 |
| $\beta_2$AP + compound 10 | H-FNDCRD**[Pra**IQR**Azk]**HLRQYELL-OH | 141 | 122 | 7 |
| $\beta_2$AP + compound 11 | Ac-FNDCRD**[Pra**IQR**Azk]**HLRQYELL-OH | 142 | 116 | 7 |

**Cyclization:** CuAAC, ($X_7$-$X_8$)

Table 6

| Code | Sequence | SEQ ID NO: | $IC_{50}$ (nM) | shift |
|---|---|---|---|---|
| $\beta_2$AR | | | 727 | 1 |
| $\beta_2$AR + compound 23 | Ac-FN**E**CRD**K**IQRMHLRQYELL-OH | 61 | 270 | 3 |
| $\beta_2$AR + compound 24 | Ac-FN**[E**CRD**K]**IQRMHLRQYELL-OH | 144 | 97.3 | 7 |
| $\beta_2$AR + compound 25 | Ac-FN**S**$_5$CRD**S**$_5$IQRMHLRQYELL-OH | 62 | 485 | 1 |
| $\beta_2$AR + compound 26 | Ac-FN[**S**$_5$CRD**S**$_5$]IQRMHLRQYELL-OH | 145 | 305 | 2 |

**Cyclization:** Lactamization + RCM, ($X_8$-$X_{10}$)

Table 7

| Code | Sequence | SEQ ID NO: | $IC_{50}$ (nM) | shift |
|---|---|---|---|---|
| $\beta_2$AR | | | 929 | 1 |
| $\beta_2$AR + compound 12 | Ac-FN[**Pra**CRD**Azk**]IQRMHLRQYELL-OH | 143 | 47.3 | 20 |
| $\beta_2$AR + compound 13 | Ac-FNDCRDIIQR**[R**$_5$HL**S**$_5$**]**QYELL-OH | 146 | 312 | 3 |
| $\beta_2$AR + compound 14 | Ac-FNDCRD**[R**$_8$IQRMHL**S**$_5$**]**QYELL-OH | 147 | 433 | 2 |

**Cyclization:** CuAAC, ($X_8$-$X_{10}$) + RCM (($X_6$-$X_7$) and ($X_6$-$X_8$))

**Compound 12** containing a triazolyl tether, located near the *N*-terminus, was compared with Nb80, a well-established allosteric modulator of the $\beta_2$AR. The results are shown in Figure 8 and Table 8.

Table 8

| Code | Sequence | SEQ ID NO: | $IC_{50}$ (nM) | Shift |
|---|---|---|---|---|
| $\beta_2$AP | | | 1425 | 1 |
| $\beta_2$AP + compound 12 | Ac-FN[**Pra**CRD**Azk**]IQRMHLRQYELL-OH | 143 | 38.9 | 37 |
| $\beta_2$AP + Nb80 | | | 9.7 | 146 |

**III.** Alanine scanning on the C-terminus

Alanine scanning of the 4 C-terminal amino acids. The results are shown in Figure 9 and Table 9.

Table 9

| Code | Sequence | SEQ ID NO: | $IC_{50}$ (nM) | Shift |
|---|---|---|---|---|
| $\beta_2$AR | | | 859 | 1 |
| compound 76 | Ac-FNDCRDIIQRMHLRQYE**A**L-OH | 108 | 1 073 | 1 |
| compound 77 | Ac-FNDCRDIIQRMHLRQY**A**LL-OH | 109 | 175 | 5 |
| compound 78 | Ac-FNDCRDIIQRMHLRQ**A**ELL-OH | 110 | 616 | 1 |
| compound 79 | Ac-FNDCRDIIQRMHLRQYEL**A**-OH | 111 | 849 | 1 |
| compound 2 | Ac-FNDCRDIIQRMHLRQYELL-OH | 53 | 97.6 | 9 |

**Conclusion:** Replacing the last Leu, penultimate Leu and Tyr residues of compound 2 had a greater effect on radioligand binding to the receptor than the substitution of Glu.

**IV.** Evaluation of Cha-containing peptide

The results are shown in Figures 10, 11 and Tables 10, 11.

Table 10

| Code | Sequence | SEQ ID NO: | $IC_{50}$ (nM) | Shift | Fraction HI (%) |
|---|---|---|---|---|---|
| $\beta_2$AP | | | 1 168 | 1 | |
| $\beta_2$AP + compound 51 100 μM | Ac-KKKFN**[Pra**CRD**Azk]**IQRMHLRQYE**Cha**L-OH | 167 | 3.4 | 345 | 77 |
| $\beta_2$AP + compound 51 50 μM | | | 6.9 | 170 | 79 |
| $\beta_2$AP + compound 51 10 μM | | | 6.7 | 175 | 76 |
| $\beta_2$AR + compound 51 1 μM | | | 56.4 | 21 | 54 |

Table 11

| Code | Sequence | SEQ ID NO: | $IC_{50}$ (nM) | Shift |
|---|---|---|---|---|
| $\beta_2$AP | | | 809 | 1 |
| compound 2 | Ac-FNDCRDIIQRMHLRQYELL-OH | 53 | 79.4 | 10 |
| compound 63 | Ac-FNDCRDIIQRMHLRQYE**Cha**L-OH | 95 | 688 | 118 |
| compound 82 | Ac-FNDCRDIIQRMHLRQ**Cha**ELL-OH | 114 | 25.6 | 32 |
| compound 84 | Ac-FNDCRDIIQRMHLRQYEL**Cha**-OH | 116 | 66.2 | 12 |

V. Evaluation of cyclohexylalanine analogues on linear peptides

The results are shown in Figure 12 and Table 12.

Table 12

| Code | Sequence | SEQ ID NO: | $IC_{50}$ (nM) | Shift | Fraction HI (%) |
|---|---|---|---|---|---|
| $\beta_2$AR | | | 1 587 | 1 | |
| $\beta_2$AR + compound 63 | Ac-FNDCRDIIQRMHLRQYE**Cha**L-OH | 95 | 7.3 | 217 | 81 |
| $\beta_2$AR + compound 68 | Ac-FNDCRDIIQRMHLRQYE**F**L-OH | 100 | 54.6 | 29 | 62 |
| $\beta_2$AR + compound 69 | Ac-FNDCRDIIQRMHLRQYE**W**L-OH | 101 | 28.9 | 55 | 51 |
| $\beta_2$AR + compound 2 | Ac-FNDCRDIIQRMHLRQYELL-OH | 53 | 124 | 13 | 21 |
| $\beta_2$AR + compound 62 | Ac-**KKK**FN**[Pra**CRD**Azk]**IQRMHLRQYE**F**L-OH | 175 | 17.1 | 93 | 91 |

**Conclusion:** The penultimate Leu of compound 2 was replaced by cyclohexylalanine, F or W and tested. These

data show that linear peptides comprising Cha, but also proteinogenic amino acids, such as Phe and Trp, at the penultimate position, are able to stabilize the receptor in an active state.

The effect of the staple is illustrated when comparing compound 68 with compound 62.

**VI.** Evaluation of Cha on shorter (linear) peptides

The results are shown in Figure 13 and Table 13.

Table 13

| Code | Sequence | SEQ ID NO: | $IC_{50}$(nM) | Shift |
|---|---|---|---|---|
| $\beta_2$AR | | | 786 | 1 |
| $\beta_2$AR + compound 63 | Ac-FNDCRDIIQRMHLRQYEChaL-OH | 95 | 3.96 | 198 |
| $\beta_2$AR + compound 67 | Ac-RDIIQRMHLRQYEChaL-OH | 99 | 45.4 | 17 |
| $\beta_2$AR + compound 80 | Ac-IIQRMHLRQYEChaL-OH | 112 | 551 | 1 |
| $\beta_2$AR + compound 66 | Ac-QRMHLRQYEChaL-OH | 98 | 551 | 1 |

**VII.** Evaluation of *N*-terminal additions of basic amino acids

The results are shown in Figures 14, 15 and Tables 14, 15.

Table 14

| Code | Sequence | SEQ ID NO: | $IC_{50}$(nM) | Shift |
|---|---|---|---|---|
| $\beta_2$AR | | | 1 150 | 1 |
| $\beta_2$AR + compound 40 | Ac-**R**FN[**Pra**CRD**Azk**]IQRMHLRQYELL-OH | 159 | 31.3 | 37 |
| $\beta_2$AR + compound 41 | Ac-**K**FN[**Pra**CRD**Azk**]IQRMHLRQYELL-OH | 160 | 26.0 | 44 |
| $\beta_2$AR + compound 42 | Ac-**KKK**FN[**Pra**CRD**Azk**]IQRMHLRQYELL-OH | 161 | 27.4 | 42 |
| $\beta_2$AR + compound 12 | Ac-FN[**Pra**CRD**Azk**]IQRMHLRQYELL-OH | 143 | 37.3 | 31 |

Table 15

| Code | Sequence | SEQ ID NO: | $IC_{50}$ (nM) | Shift | Fraction HI (%) |
|---|---|---|---|---|---|
| $\beta_2$AR | | | 1 626 | | |
| $\beta_2$AR + compound 51 | Ac-**KKK**FN**[Pra**CRD**Azk]**IQRMHLRQYE**Cha**L-OH | 167 | 2.8 | 580 | 83 |
| $\beta_2$AR + compound 74 | Ac-**KK**FN**[Pra**CRD**Azk]**IQRMHLRQYE**Cha**L-OH | 178 | 4.7 | 350 | 89 |
| $\beta_2$AR + compound 50 | Ac-KFN[PraCRDAzk]IQRMHLRQYEChaL-OH | 166 | 2.6 | 616 | 86 |

**Conclusion:** Additional arginine and lysine residues were inserted at the *N*-terminus of the peptides. By adding lysine residues, preferably more than 1 lysine residue such as 3 lysine residues, the stapled peptides became completely soluble in the aqueous buffer, with even enhanced modulatory activity of the receptor.

**Example 4: Circular dichroism (CD) spectroscopy**

Evaluation of the $\alpha$-helicity of the (stapled) peptides was assessed using CD spectroscopy. CD analysis was performed on several peptide analogues in a $NaH_2PO_4$ Buffer (pH 6). In most cases, a stabilization of the peptide by

stapling induced a significant increase in helicity. When comparing the 3 different macrocyclization strategies, the highest stabilization was obtained for the copper-catalyzed azide-alkyne cycloaddition followed by the lactamization. A decrease in stability was observed for the RCM cyclized peptide, compared to the linear analogue (Figures 16**a-d**). The effect of the lysine residues at the N-terminus on the structure was evaluated. A slightly, but no significant, decrease in helicity was observed when increasing the number of lysines (Figure 16**e-g**).

**Example 5: Fragment screening**

Fragment-based screening was performed on peptidomimetic stabilized receptors, $\beta_2$AR and D1R. Single point radioligand displacement assay was performed in parallel on the basal state (receptor alone) and on the structurally constrained receptor (receptor + **compound 51).** Fragments from the Maybridge Ro3 library were screened by incubation with the radioligand [$^3$H]-DHA for $\beta_2$AP and [$^3$H]-SCH23390 for D1R in the presence and absence of the peptidomimetic. The results are shown in Figure 17 ($\beta_2$AR) and Figure 18 (D1R). The fragments were plotted in function of residual radioligand binding to the active and basal state. Fragments able to compete with the radioligand in the active state and less in the basal state were selected as agonist-like fragments ($f_b$ for $\beta_2$AR and $f_d$ for D1R). Alternatively, fragments binding equally to the basal and active conformation, or with a preference for the basal state, are more likely to form antagonists or inverse agonists.

For $\beta_2$AR, 8 fragments (fb01-fb08, Fig. 18) were selected that preferentially bind to the active state (low radioligand binding on active state) and less to the basal state. Many fragments picked up during screening were characterized by the presence of aromatic alkylamines. Most of the selected fragments were also identified as agonist-like fragments by screening on the $\beta_2$AR fused to Nb80 (e.g., fb01, fb02, fb03, fb06, fb07 and fb08) (Pardon et al. 2018).

For D1R, fragments that were identified as preferentially binding to the active state contained key structural elements of known agonists, like alkylamines and catechols.

**Example 6: General overview of the results is shown in Table 18**

Table 18a-c: All $IC_{50}$ values are represented as means ± SEM with number of replicates indicated between brackets, each performed in duplicate. The shifts are averaged values. "[]" denote cyclic peptide. ND: not determined. (standard amino acid one letter coding used; $S_5$: α-methyl,α-pentenylglycine; $R_5$: (*R*)-α-methyl,α-pentenylglycine; $R_8$: (*R*)-α-methyl,α-octenylglycine; Pra: propargylglycine; Azk: azidolysine; Cha: cyclohexylalanine).

Table 18a

| Bridge | Code | Sequence | SEQ ID NO: | $IC_{50}$ (nM) | Shift |
|---|---|---|---|---|---|
| Linear | compound 1 | H-F$^{1}$N$^{2}$D$^{3}$C$^{4}$R$^{5}$D$^{6}$I$^{7}$I$^{8}$Q$^{9}$R$^{10}$M$^{11}$H$^{12}$L$^{13}$R$^{14}$Q$^{15}$Y$^{16}$E$^{17}$L$^{18}$L$^{19}$-OH | 117 | ND | ND |
| | compound 2 | Ac-FNDCRDIIQRMHLRQYELL-OH | 53 | 104.8 ± 18.2 (10) | 10 |
| Lactamization | compound 3 | Ac-FNDCRD**E**$^{7}$IQR**K**$^{11}$HLRQYELL-OH | 54 | 412.2 ± 72.4 (3) | 2 |
| | compound 4 | H-FNDCRD**[E**$^{7}$IQR**K**$^{11}$IHLRQYELL-OH | 137 | 224.5 ± 124.4 (3) | 4 |
| | compound 5 | Ac-FNDCRD**[E**$^{7}$IQR**K**$^{11}$]HLRQYELL-OH | 138 | 197.1 ± 6.0 (3) | 4 |
| RCM | compound 6 | Ac-FNDCRD**S**$_{5}^{7}$IQR**S**$_{5}^{11}$HLRQYELL-OH | 56 | 104.2 ± 11.2 (3) | 9 |
| | compound 7 | H-FNDCRD**[S**$_{5}^{7}$IQR**S**$_{5}^{11}$]HLRQYELL-OH | 139 | 417.2 ± 9.9 (3) | 2 |
| | compound 8 | Ac-FNDCRD[**S**$_{5}^{7}$IQR**S**$_{5}^{11}$]HLRQYELL-OH | 140 | 400.8 ± 201.5 (3) | 3 |
| Click | compound 9 | Ac-FNDCRD**Pra**$^{7}$IQR**Azk**$^{11}$HLRQYELL-OH | 58 | 218.1 ± 15.8 (3) | 4 |
| | compound 10 | H-FNDCRD**[Pra**$^{7}$IQR**Azk**$^{11}$]HLRQYELL-OH | 141 | 121.4 ± 16.9 (3) | 7 |
| | compound 11 | Ac-FNDCRD**[Pra**$^{7}$IQR**Azk**$^{11}$]HLRQYELL-OH | 142 | 116.2 ± 10.8 (3) | 7 |
| Click | compound 22 | Ac-FN**Pra**$^{3}$CRD**Azk**$^{7}$IQRMHLRQYELL-OH | 60 | 97.1 ± 20.3 (3) | 9 |
| | compound 12 | Ac-FN**[Pra**$^{3}$CRD**Azk**$^{7}$**]**IQRMHLRQYELL-OH | 143 | 40.3 ± 26.2 (19) | 27 |
| Lactamization | compound 23 | Ac-FN**E**$^{3}$CRD**K**$^{7}$IQRMHLRQYELL-OH | 61 | 269.6 ± 10.5 (3) | 3 |
| | compound 24 | Ac-FN**[E**$^{3}$CRD**K**$^{7}$**]**IQRMHLRQYELL-OH | 144 | 98.0 ± 14.9 (3) | 7 |
| RCM | compound 25 | Ac-FN**S**$_{5}^{3}$CRD**S**$_{5}^{7}$IQRMHLRQYELL-OH | 62 | 485.4 ± 180.7 (3) | 2 |
| | compound 26 | Ac-FN**[S**$_{5}^{3}$CRD**S**$_{5}^{7}$**]**IQRMHLRQYELL-OH | 145 | 307.4 ± 68.3 (3) | 2 |
| RCM | compound 13 | Ac-FNDCRDIIQR**[R**$_{5}^{11}$HL**S**$_{5}^{14}$**]**QYELL-OH | 146 | 313.0 ± 35.5 (3) | 3 |
| RCM | compound 14 | Ac-FNDCRD**[R**$_{8}^{7}$IQRMHL**S**$_{5}^{14}$**]**QYELL-OH | 147 | 449.3 ± 131.9 (3) | 2 |

Table 18b

| Bridge | Code | Sequence | SEQ ID NO: | $IC_{50}$ (nM | shift |
|---|---|---|---|---|---|
| Linear | compound 15 | H-R$^5$D$^6$I$^7$I$^8$Q$^9$R$^{10}$M$^{11}$H$^{12}$L$^{13}$R$^{14}$Q$^{15}$Y$^{16}$E$^{17}$L$^{18}$L$^{19}$-OH | 118 | ND | ND |
| | compound 16 | Ac-RDIIQRMHLRQYELL-OH | 65 | 616.6 ± 178.3 (4) | 1 |
| Click | compound 17 | Ac-RDIIQR**Azk**$^{11}$HLR**Pra**$^{15}$YELL-OH | 66 | ND | ND |
| | compound 18 | H-RDIIQR**[Azk**$^{11}$HLR**Pra**$^{15}$]YELL-OH | 179 | ND | ND |
| | compound 19 | Ac-RDIIQR**[Azk**$^{11}$HLR**Pra**$^{15}$]YELL-OH | 148 | 169.1 ± 2.0 (3) | 5 |
| Linear | compound 20 | H-QRMHLRQYELL-OH | 120 | ND | ND |
| | compound 21 | Ac-QRMHLRQYELL-OH | 67 | 1 104.9 ± 294.5 (4) | 1 |

Table 18c

| Code | Sequence | SEQ ID NO: | $IC_{50}$ (nM) | Shift |
|---|---|---|---|---|
| compound 27 | Ac-FN**Pra**CRD**AzkI**QRMHLRQY**Q**LL-OH | 68 | 192.5 ± 64.4 (3) | 4 |
| compound 28 | Ac-FN**[Pra**CRD**Azk]**IQRMHLRQY**Q**LL-OH | 149 | 45.1 ± 12.8 (6) | 12 |
| compound 29 | Ac-FN **Pra**CRD**AzkI**QRMHLRQ**W**ELL-OH | 69 | 162.9 ± 41.1 (3) | 4 |
| compound 30 | Ac-FN**[Pra**CRD**Azk]**IQRMHLRQ**W**ELL-OH | 150 | 60.5 ± 16.2 (6) | 9 |
| compound 31 | Ac-FN**Pra**CRD**AzkI**QRMHLRQYE**I**L-OH | 70 | ND | ND |
| compound 32 | Ac-FN**[Pra**CRD**Azk]I**QRMHLRQYEIL-OH | 151 | 204.8 ± 36.7 (3) | 2 |
| compound 33 | Ac-FN**Pra**CRD**AzkI**QRMHLRQYE**LI**-OH | 71 | ND | ND |
| compound 34 | Ac-FN**[Pra**CRD**Azk]I**QRMHLRQYE**LI**-OH | 152 | 26.1 ± 8.6 (7) | 29 |
| compound 35 | Ac-FN**[Azk**CRD**Pra]I**QRMHLRQYELL-OH | 153 | 177.4 ± 60.9 (3) | 5 |
| compound 36 | Ac-FN**[Pra**CRD**Azk]I**QRMHLRQYELL**-NH$_2$** | 154 | 618.1 ± 112.9 (3) | 1 |
| compound 37 | Ac-FN**[Pra**CRD**Azk]**IQRMHLRQY**hGlu**LL-OH | 155 | 19.8 ± 2.7 (2) | 35 |
| compound 38 | Ac-FN**[Pra**CR**NAzk]I**QRMHLRQYELL-OH | 156 | 132.4 ± 7.2 (3) | 7 |
| compound 39 | Ac-FN**[Pra**CRD**Azk]**IQRMHLRQY**D**LL-OH | 157 | 14.3 ± 5.0 (2) | 52 |
| compound 43 | Ac-FN**[Pra**CRD**Azk]**IQRMHLRQ**1-Nal**ELL-OH | 158 | 13.8 ± 5.0 (2) | 54 |
| compound 44 | Ac-FN**[Pra**CRD**Azk]**IQRMHLRQ**2-Nal**ELL-OH | 180 | | |
| compound 40 | Ac-**R**FN**[Pra**CRD**Azk]**IQRMHLRQYELL-OH | 159 | 31.3 (1) | 37 |
| compound 41 | Ac-**K**FN**[Pra**CRD**Azk]**IQRMHLRQYELL-OH | 160 | 16.9 ± 7.1 (4) | 51 |
| compound 42 | Ac-**KKK**FN**[Pra**CRD**Azk]**IQRMHLRQYELL-OH | 161 | 17.3 ± 9.2 (11) | 49 |
| compound 46 | Ac-**K**FN**[Pra**CRD**Azk]**IQRMHLRQYELI-OH | 162 | 14.9 ± 5.0 (3) | 51 |
| compound 47 | Ac-**KKK**FN**[Pra**CRD**Azk]**IQRMHLRQYELI-OH | 163 | 11.2 ± 2.3 (3) | 64 |
| compound 48 | Ac-**K**FN**[Pra**CRD**Azk]**IQRMHLRQYEIL-OH | 164 | 136.7 ± 50.2 (3) | 8 |
| compound 49 | Ac-**KKK**FN**[Pra**CRD**Azk]**IQRMHLRQYEIL-OH | 165 | 112.1 ± 56.4 (3) | 11 |
| compound 50 | Ac-**K**FN**[Pra**CRD**Azk]I**QRMHLRQYE**Cha**L-OH | 166 | 5.4 ± 3.9 (2) | 373 |

(continued)

| Code | Sequence | SEQ ID NO: | $IC_{50}$ (nM) | Shift |
|---|---|---|---|---|
| compound 51 | Ac-**KKK**FN**[Pra**CRD**Azk]I**QRMHLRQYE**Cha**L-OH | 167 | 2.9 ± 2.1 (9) | 400 |
| compound 52 | Ac-**K**FN**[Pra**CRD**Azk**]IQRMHLRQY**hGlu**LL-OH | 168 | 15.8 ± 4.8 (3) | 53 |
| compound 53 | Ac-**KKK**FN**[Pra**CRD**Azk]I**QRMHLRQY**hGlu**LL-OH | 169 | 20.8 ± 3.8 (3) | 38 |
| compound 54 | Ac-**K**FN**[Pra**CRD**Azk]**IQRMHLRQY**D**LL-OH | 170 | 5.6 ± 2.0 (3) | 91 |
| compound 55 | Ac-**KKK**FN**[Pra**CRD**Azk]I**QRMHLRQY**D**LL-OH | 171 | 8.1 ± 1.6 (2) | 72 |
| compound 56 | Ac-**KKK**FN**[Pra**CRD**Azk]**IQRMHLRQ**W**ELL-OH | 91 | 18.3 ± 3.1 (3) | 53 |
| compound 57 | Ac-**KKK**FN**[Pra**CRD**Azk]**IQRMHLRQ**1-Nal**ELL-OH | 172 | 23.9 ± 9.4 (4) | 49 |
| compound 58 | Ac-**KKK**FN**[Pra**CRD**Azk]**IQRMHLRQ**2-NalELL**-OH | 174 | 24.4 ± 16.2 (2) | 39 |
| compound 62 | Ac-**KKK**FN**[Pra**CRD**Azk]**IQRMHLRQY**EF**L-OH | 175 | 15.7 ± 2.0 (2) | 87 |
| compound 63 | Ac-FNDCRDIIQRMHLRQYE**Cha**L-OH | 95 | 5.9 ± 1.9 (7) | 203 |
| compound 64 | Ac-RDIIQR**[Azk**HLRPra]YE**Cha**L-OH | 176 | 14.2 (1) | 81 |
| compound 65 | Ac-**KK**KRDIIQR**[Azk**HLR**Pra]**YE**ChaL**-OH | 177 | 18.3 (1) | 63 |
| compound 66 | Ac-QRMHLRQYE**Cha**L-OH | 98 | 637.3 ± 121.3 (2) | 2 |
| compound 67 | Ac-RDIIQRMHLRQYE**Cha**L-OH | 99 | 37.0 ± 11.8 (2) | 30 |
| compound 68 | Ac-FNDCRDIIQRMHLRQY**EF**L-OH | 100 | 36.9 ± 25.1 (2) | 35 |
| compound 69 | Ac-FNDCRDIIQRMHLRQY**EWL**-OH | 101 | 48.5 ± 17.9 (4) | 27 |
| compound 70 | Ac-FNDCRDIIQRMHLRQY**EYL**-OH | 102 | 657.4 (1) | 2 |
| compound 71 | Ac-FNDCRDIIQRMHLRQY**EHL**-OH | 103 | 1 141.0 (1) | 1 |
| compound 72 | Ac-**NARRI**FNDCRDIIQRMHLRQYELL-OH | 104 | 20.3 ± 2.7 (2) | 47 |
| compound 73 | Ac-**RRI**FNDCRDIIQRMHLRQYELL-OH | 105 | 52.6 (1) | 19 |
| compound 74 | Ac-**KK**FN**[Pra**CRD**Azk]**IQRMHLRQYE**Cha**L-OH | 178 | 4.6 (1) | 350 |
| compound 75 | Ac-FND**A**RDIIQRMHLRQYELL-OH | 107 | 270.4 ± 69.9 (2) | 5 |
| compound 76 | Ac-FNDCRDIIQRMHLRQYE**A**L-OH | 108 | 1073.0 (1) | 1 |
| compound 77 | Ac-FNDCRDIIQRMHLRQY**A**LL-OH | 109 | 174.8 (1) | 5 |
| compound 78 | Ac-FNDCRDIIQRMHLRQ**A**ELL-OH | 110 | 615.7 (1) | 1 |
| compound 79 | Ac-FNDCRDIIQRMHLRQYEL**A**-OH | 111 | 848.6 (1) | 1 |
| compound 80 | Ac-IIQRMHLRQYE**Cha**L-OH | 112 | 550.5 (1) | 1 |
| compound 81 | Ac-**NARR**IFNDARDIIQRMHLRQYELL-OH | 113 | 117.7 (1) | 8 |
| compound 82 | Ac-FNDCRDIIQRMHLRQ**Cha**ELL-OH | 114 | 25.6 (1) | 32 |
| compound 83 | Ac-**NARR**IFNDARDIIQRMHLRQY**EWL**-OH | 115 | 93.7 (1) | 9 |
| compound 84 | Ac-FNDCRDIIQRMHLRQYEL**Cha**-OH | 116 | 66.2 (1) | 12 |

The effect of replacing the four C-terminal amino acids by residues sharing structural similarities is illustrated in Table 18. For example, the two last leucine residues were replaced by isoleucine (compounds 34, 46 and 47; and compounds 32n 48 and 49). The glutamic acid ($E^{392}$) was replaced by other negatively charged amino acids with a different number of methylene units in the side chain, e.g. aspartic acid (compounds 39, 54 and 55) and homoglutamic acid (compounds 37, 52 and 53). The tyrosine residue ($Y^{391}$) was substituted by aromatic residues, such as tryptophan (compounds 30 and 56), 1-naphthylalanine (compounds 43 and 57) and 2-naphthylalanine (compounds 44 and 58).

**Example 7: Genericity**

**I.** RLA on D1R

Peptidomimetics were screened using GPCR-radioligand binding assay (RLA) with dopamine 1 receptor (D1R) binding, using [$^3$H]-SCH23390 as radiolabelled antagonist, and increasing concentrations of A-77636 (agonist). The results are shown in Figure 19 and Table 19. The half maximal inhibitory concentration ($IC_{50}$) represents the affinity of the agonist for the receptor. The selectivity is quantified by the shift. "[]" denotes cyclic peptides.

Table 19

| Code | Sequence | SEQ ID NO: | $IC_{50}$ (nM) | Shift |
|---|---|---|---|---|
| D1R | | | 13.1 | 1 |
| D1R + compound 2 10 μM | Ac-FNDCRDIIQRMHLRQYELL-OH | 53 | 5.8 | 2 |
| D1R + compound 63 10 μM | Ac-FNDCRDIIQRMHLRQYEChaL-OH | 95 | 0.55 | 24 |
| D1R + compound 42 10 μM | Ac-KKKFN[PraCRDAzk]IQRMHLRQYELL-OH | 161 | 0.41 | 32 |
| D1R + compound 51 10 μM | Ac-**KKK**FN**[Pra**CRD**Azk]**IQRMHLRQYE**Cha**L-OH | 167 | 0.25 | 52 |

**Conclusion:** The peptides were tested on the dopamine 1 receptor (D1R).

**Example 8: Analytical data of synthesized peptides (HPLC** purity > **97%).**

Analytical data of the synthesized peptides is provided in Table 20.

Table 20

| Compound | Sequence | SEQ ID NO: | Formula | Molecular weight (g/mol) | Yield (%) | $t_r$ HPLC[a] (min) | HRMS ($[M+2H]^{2+}$) | |
|---|---|---|---|---|---|---|---|---|
| | | | | | | | Found | Calculated |
| compound 1 | H-FNDCRDIIQRMHLRQYELL-OH | 117 | $C_{107}H_{171}N_{33}O_{30}S_2$ | 2462.23 | 5 | 2.13 | 1232.125 | 1232.123 |
| compound 2 | Ac-FNDCRDIIQRMHLRQYELL-OH | 53 | $C_{109}H_{173}N_{33}O_{31}S_2$ | 2504.24 | 5 | 2.30 | 1253.13 | 1253.13 |
| compound 3 | Ac-FNDCRD**E**IQR**K**HLRQYELL-OH | 54 | $C_{109}H_{172}N_{34}O_{33}S$ | 2517.25 | 10 | 1.99 | 1259.6359 | 1259.6349 |
| compound 4 | H-FNDCRD$_c$**[E**IQR**K]**HLRQYELL-OH | 137 | $C_{107}H_{168}N_{34}O_{31}S$ | 2457.23 | 17 | 1.92 | 1229.623 | 1229.6243 |
| compound 5 | Ac-FNDCRD$_c$**[**EIQR**K]**HLRQYELL-OH | 138 | $C_{109}H_{170}N_{34}O_{32}S$ | 2499.24 | 10 | 2.05 | 1250.6332 | 1250.6296 |
| compound 6 | Ac-FNDCRD**S**$_5$IQR**S**$_5$HLRQYELL-OH | 56 | $C_{114}H_{179}N_{33}O_{31}S$ | 2538.32 | 6 | 2.41 | 1270.1675 | 1270.1658 |
| compound 7 | H-FNDCRD$_c$**[S**$_5$IQR**S**$_5$**]**HLRQYELL-OH | 139 | $C_{110}H_{173}N_{33}O_{30}S$ | 2468.27 | 4 | 2.12 | 1235.1442 | 1235.1449 |
| compound 8 | Ac-FNDCRD$_c$**[S**$_5$IQR**S**$_5$**]**HLRQYELL-OH | 140 | $C_{112}H_{175}N_{33}O_{31}S$ | 2510.29 | 7 | 2.27 | 1256.1492 | 1256.1501 |
| compound 9 | Ac-FNDCRD**Pra**IQR**Azk**HLRQYELL-OH | 58 | $C_{109}H_{168}N_{36}O_{31}S$ | 2509.24 | 8 | 2.20 | 1255.6277 | 1255.6274 |
| compound 10 | H-FNDCRD$_c$**[Pra**IQR**Azk]**HLRQYELL-OH | 141 | $C_{107}H_{166}N_{36}O_{30}S$ | 2467.23 | 9 | 1.96 | 1234.614 | 1234.6221 |
| compound 11 | Ac-FNDCRD$_c$**[Pra**IQR**Azk]**HLRQYELL-OH | 142 | $C_{109}H_{168}N_{36}O_{31}S$ | 2509.24 | 5 | 2.10 | 1255.6278 | 1255.6274 |
| compound 12 | Ac-FN$_c$**[Pra**CRD**Azk]**IQRMHLRQYELL-OH | 143 | $C_{110}H_{172}N_{36}O_{29}S_2$ | 2525.25 | 15 | 2.30 | 1263.6346 | 1263.6342 |
| compound 13 | Ac-FNDCRDIIQR$_c$**[R**$_5$HL**S**$_5$**]**QYELL-OH | 146 | $C_{112}H_{174}N_{30}O_{31}S$ | 2467.27 | 3 | 2.53 | 1234.6407 | 1234.6420 |
| compoun 14 | Ac-FNDCRD$_c$**[R**$_8$IQRMHL**S**$_5$**]**QYELL-OH | 147 | $C_{114}H_{178}N_{30}O_{31}S_2$ | 2527.27 | 1 | 2.66 | 1264.6276 | 1264.6433 |
| compound 15 | H-RDIIQRMHLRQYELL-OH | 118 | $C_{87}H_{146}N_{28}O_{23}S$ | 1983.08 | 16 | 1.97 | 992.5193 | 992.5494 |
| compound 16 | Ac-RDIIQRMHLRQYELL-OH | 65 | $C_{89}H_{148}N_{28}O_{24}S$ | 2025.09 | 17 | 2.08 | 1013.527 | 1013.5546 |

(continued)

| Compound | Sequence | SEQ ID NO: | Formula | Molecular weight (g/mol) | Yield (%) | $t_r$ HPLC[a] (min) | HRMS ($[M+2H]^{2+}$) | |
|---|---|---|---|---|---|---|---|---|
| | | | | | | | Found | Calculated |
| compound 17 | Ac-RDIIQR**Azk**HLR**Pra**YELL-OH | 66 | $C_{90}H_{146}N_{30}O$ 23 | 2015.12 | 24 | 2.22 | 1008.5135 | 1008.5664 |
| compound 18 | H-RDIIQR$_c$**[Azk**HLR**Pra]**YELL-OH | 179 | $C_{88}H_{144}N_{30}O$ 22 | 1973.11 | 4 | 1.99 | 1974.1255 **$[M+H]^+$** | 1974.1144 **$[M+H]^+$** |
| compound 19 | Ac-RDIIQR$_c$**[Azk**HLR**Pra]**YELL-OH | 148 | $C_{90}H_{146}N_{30}O$ 23 | 2015.12 | 9 | 2.14 | 1008.5154 | 1008.5664 |
| compound 20 | H-QRMHLRQYELL-OH | 120 | $C_{65}H_{107}N_{21}O_{17}S$ | 1485.79 | 4 | 1.90 | 1486,8059 **$[M+H]^+$** | 1486,7947 **$[M+H]^+$** |
| compound 21 | Ac-QRMHLRQYELL-OH | 67 | $C_{67}H_{109}N_{21}O_{18}S$ | 1527.8 | 14 | 1.94 | 1528.8029 **$[M+H]^+$** | 1528.8053 **$[M+H]^+$** |
| compound 22 | Ac-FN**Pra**CRD**Azk**IQRMHLRQYELL-OH | 60 | $C_{110}H_{172}N_{36}O_{29}S_2$ | 2525.25 | 6 | 2.38 | 1263.6252 | 1263.6342 |
| compound 23 | Ac-FN**E**CRD**K**IQRMHLRQYELL-OH | 61 | $C_{110}H_{176}N_{34}O_{31}S_2$ | 2533.27 | 6 | 2.06 | 1267.6289 | 1267.6416 |
| compound 24 | Ac-FN$_c$**[E**CRD**K]**IQRMHLRQYELL-OH | 144 | $C_{110}H_{174}N_{34}O_{30}S_2$ | 2515.26 | 3 | 2.24 | 1258.6338 | 1258.6364 |
| compound 25 | Ac-FN**$S_5$**CRD**$S_5$**IQRMHLRQYELL-OH | 62 | $C_{115}H_{183}N_{33}O_{29}S_2$ | 2554.33 | 7 | 2.69 | 1277.9982 | 1278.1726 |
| compound 26 | Ac-FN$_c$**[$S_5$**CRD**$S_5$]**IQRMHLRQYELL-OH | 145 | $C_{113}H_{179}N_{33}O_{29}S_2$ | 2526.3 | 5 | 2.58 | 1264.1697 | 1264.1572 |
| compound 27 | Ac-FN**Pra**CRD**Azk**IQRMHLRQY**Q**LL-OH | 68 | $C_{110}H_{173}N_{37}O_{28}S_2$ | 2524.27 | 11 | 2.34 | 1263.1388 | 1263.1425 |
| compound 28 | Ac-FN$_c$**[Pra**CRD**Azk]**IQRMHLRQY**Q**LL-OH | 149 | $C_{110}H_{173}N_{37}O_{28}S_2$ | 2524.27 | 9 | 2.26 | 1263.1382 | 1263.1425 |
| compound 29 | Ac-FN**Pra**CRD**Azk**IQRMHLRQ**W**ELL-OH | 69 | $C_{112}H_{173}N_{37}O_{28}S_2$ | 2548.27 | 14 | 2.43 | 1275.1443 | 1275.1425 |
| compound 30 | Ac-FN$_c$**[Pra**CRD**Azk]**IQRMHLRQ**W**ELL-OH | 150 | $C_{112}H_{173}N_{37}O_{28}S_2$ | 2548.27 | 7 | 2.38 | 1275.1450 | 1275.1425 |
| compound 31 | Ac-FN**Pra**CRD**Azk**IQRMHLRQYE**I**L-OH | 70 | $C_{110}H_{172}N_{36}O_{29}S_2$ | 2525.25 | 18 | 2.30 | 1263.6351 | 1263.6344 |

(continued)

| Compound | Sequence | SEQ ID NO: | Formula | Molecular weight (g/mol) | Yield (%) | $t_r$ HPLC[a] (min) | HRMS ([M+2H]$^{2+}$) | |
|---|---|---|---|---|---|---|---|---|
| | | | | | | | Found | Calculated |
| compound 32 | Ac-FN$_c$**[Pra**CRD**Azk]**IQRMHLRQYE**I**L-OH | 151 | $C_{110}H_{172}N_{36}O_{29}S_2$ | 2525.25 | 12 | 2.26 | 1263.6638 | 1263.6344 |
| compound 33 | Ac-FN**Pra**CRD**Azk**IQRMHLRQYEL**I**-OH | 71 | $C_{110}H_{172}N_{36}O_{29}S_2$ | 2525.25 | 20 | 2.28 | 1263.6327 | 1263.6344 |
| compound 34 | Ac-FN$_c$**[Pra**CRD**Azk]**IQRMHLRQYEL**I**-OH | 152 | $C_{110}H_{172}N_{36}O_{29}S_2$ | 2525.25 | 14 | 2.22 | 1263.5989 | 1263.6344 |
| compound 35 | Ac-FN$_c$**[Azk**CRD**Pra]**IQRMHLRQYELL-OH | 153 | $C_{110}H_{172}N_{36}O_{29}S_2$ | 2525.25 | 10 | 2.23 | 1263.6389 | 1263.6344 |
| compound 36 | Ac-FN$_c$**[Pra**CRD**Azk]**IQRMHLRQYELL-**NH$_2$** | 154 | $C_{110}H_{173}N_{37}O_{28}S_2$ | 2524.27 | 11 | 2.26 | 1263.1075 | 1263.1425 |
| compound 37 | Ac-FN$_c$**[Pra**CRD**Azk]**IQRMHLRQY**HGlu**LL-OH | 155 | $C_{111}H_{174}N_{36}O_{29}S_2$ | 2539.27 | 8 | 2.29 | 1270.6431 | 1270.6422 |
| compound 38 | Ac-FN$_c$**[Pra**CR**NAzk]**IQRMHLRQYELL-OH | 156 | $C_{110}H_{173}N_{37}O_{28}S_2$ | 2524.27 | 11 | 2.29 | 1253.1625 | 1263.1425 |
| compound 39 | Ac-FN$_c$**[Pra**CRD**Azk]**IQRMHLRQY**D**LL-OH | 157 | $C_{109}H_{170}N_{36}O_{29}S_2$ | 2511.24 | 6 | 2.31 | 1256.574 | 1256.6266 |
| compound 40 | Ac-**R**FN$_c$**[Pra**CRD**Azk]**IQRMHLRQYELL-OH | 159 | $C_{116}H_{184}N_{40}O_{30}S_2$ | 2681.35 | 7 | 2.17 | 1341.682 | 1341.6851 |
| compound 41 | Ac-**K**FN$_c$**[Pra**CRD**Azk]**IQRMHLRQYELL-OH | 160 | $C_{116}H_{184}N_{38}O_{30}S_2$ | 2653.35 | 20 | 2.12 | 1327.6863 | 1327.6819 |

(continued)

| Compound | Sequence | SEQ ID NO: | Formula | Molecular weight (g/mol) | Yield (%) | $t_r$ HPLC[a] (min) | HRMS ($[M+2H]^{2+}$) | |
|---|---|---|---|---|---|---|---|---|
| | | | | | | | Found | Calculated |
| compound 42 | Ac-**KKK**FN$_c$**[Pra**CRD**Azk]**IQRMHLRQYELL-OH | 161 | $C_{128}H_{208}N_{42}O_{32}S_2$ | 2909.54 | 11 | 2.08 | 1455.8062 | 1455.7769 |
| compound 43 | Ac-FN$_c$**[Pra**CRD**Azk]**IQRMHLRQ**1-Nal**ELL-OH | 158 | $C_{114}H_{174}N_{36}O_{28}S_2$ | 2559.27 | 3.0 | 2.45 | 1280.6489 | 1280.6448 |
| compound 44 | Ac-FN$_c$**[Pra**CRD**Azk]**IQRMHLRQ**2-Nal**ELL-OH | 180 | $C_{114}H_{174}N_{36}O_{28}S_2$ | 2559.27 | 1 | 2.37 | 1280.6444 | 1280.6448 |
| compound 46 | Ac-**K**FN$_c$**[Pra**CRD**Azk]**IQRMHLRQYEL**I**-OH | 162 | $C_{116}H_{184}N_{38}O_{30}S_2$ | 2653.35 | 6 | 2.10 | 1327.6772 | 1327.6819 |
| compound 47 | Ac-**KKK**FN$_c$**[Pra**CRD**Azk]**IQRMHLRQYEL**I**-OH | 163 | $C_{128}H_{208}N_{42}O_{32}S_2$ | 2909.54 | 3 | 2.07 | 1455.7897 | 1455.7769 |
| compound 48 | Ac-**K**FN$_c$**[Pra**CRD**Azk]**IQRMHLRQYE**I**L-OH | 164 | $C_{116}H_{184}N_{38}O_{30}S_2$ | 2653.35 | 6 | 2.10 | 1327.6752 | 1327.6819 |
| compound 49 | Ac-**KKK**FN$_c$**[Pra**CRD**Azk]**IQRMHLRQYE**I**L-OH | 165 | $C_{128}H_{208}N_{42}O_{32}S_2$ | 2909.54 | 2 | 2.09 | 1455.7783 | 1455.7769 |
| compound 50 | Ac-**K**FN$_c$**[Pra**CRD**Azk]**IQRMHLRQYE**Cha**L-OH | 166 | $C_{119}H_{188}N_{38}O_{30}S_2$ | 2693.38 | 3 | 2.23 | 1347.6965 | 1347.6976 |
| compound 51 | Ac-**KKK**FN$_c$**[Pra**CRD**Azk]**IQRMHLRQYE**Cha**L-OH | 167 | $C_{131}H_{212}N_{42}O_{32}S_2$ | 2949.57 | 8 | 2.18 | 1475.7964 | 1475.7925 |
| compound 52 | Ac-**K**F$_c$**[Pra**CRD**Azk]**IQRMHLRQY**hGlu**LL-OH | 168 | $C_{117}H_{186}N_{38}O_{30}S_2$ | 2667.36 | 6 | 2.12 | 1334.6943 | 1334.6897 |

(continued)

| Compound | Sequence | SEQ ID NO: | Formula | Molecular weight (g/mol) | Yield (%) | $t_r$ HPLC[a] (min) | HRMS ($[M+2H]^{2+}$) | |
|---|---|---|---|---|---|---|---|---|
| | | | | | | | Found | Calculated |
| compound 53 | Ac-**KKK**FN$_c$**[Pra**CRD**Azk]**IQRMHLRQY**hGlu**LL-OH | 163 | $C_{129}H_{210}N_{42}O_{32}S_2$ | 2923.55 | 2 | 2.10 | 1462.7954 | 1462.7847 |
| compound 54 | Ac-**K**FN$_c$**[Pra**CRD**Azk]**MHLRQY**D**LL-OH | 170 | $C_{115}H_{182}N_{38}O_{30}S_2$ | 2639.33 | 4 | 2.15 | 1320.6718 | 1320.6741 |
| compound 55 | Ac-**KKK**FN$_c$**[Pra**CRD**Azk]**IQRMHLRQY**D**LL-OH | 171 | $C_{127}H_{206}N_{42}O_{32}S_2$ | 2895.52 | 2 | 2.11 | 1448.7567 | 1448.769 |
| compound 56 | Ac-**KKK**FN$_c$**[Pra**CRD**Azk]**IQRMHLRQ**W**ELL-OH | 172 | $C_{130}H_{209}N_{43}O_{31}S_2$ | 2932.55 | 2 | 2.16 | 1467.2988 | 1467.2849 |
| compound 57 | Ac-**KKK**FN$_c$**[Pra**CRD**Azk]**IQRMHLRQ**1-Nal**ELL-OH | 173 | $C_{132}H_{210}N_{42}O_{31}S_2$ | 2943.56 | 4 | 2.26 | 1472.7969 | 1472.7872 |
| compound 58 | Ac-**KKK**FN$_c$**[Pra**CRD**Azk]**IQRMHLRQ**2-Nal**ELL-OH | 174 | $C_{132}H_{210}N_{42}O_{31}S_2$ | 2943.56 | 1 | 2.26 | 1472.7831 | 1472.7872 |
| compound 62 | Ac-**KKK**FN$_c$**[Pra**CRD**Azk]**IQRMHLRQYE**F**L-OH | 175 | $C_{131}H_{206}N_{42}O_{32}S_2$ | 2943.52 | 3 | 2.06 | 1472.7677 | 1472.769 |
| compound 63 | Ac-FNDCRDIIQRMHLRQYEChaL-OH | 95 | $C_{112}H_{177}N_{33}O_{31}S_2$ $O_{31}S_2$ | 2544.27 | 18 | 2.25 | 1273.1405 | 1273.1443 |
| compound 64 | Ac-RDIIQR$_c$**[AzK**HLR**Pra]**YE**Cha**L-OH | 96 | $C_{93}H_{150}N_{30}O$ 23 | 2055.15 | 16 | 2.10 | 1028.5847 | 1028.5823 |
| compound 65 | Ac-**KKK**RDIIQR$_c$**[Azk**HLR**Pra]**YE**Cha**L-OH | 97 | $C_{111}H_{186}N_{36}O_{26}$ | 2439.43 | 8 | 1.97 | 1220.7202 | 1220.7247 |
| compound 66 | Ac-QRMHLRQYE**Cha**L-OH | 98 | $C_{70}H_{113}N_{21}O$ $_{18}S$ | 1567.83 | 8 | 1.94 | 1568.8345 $[M+H]^+$ | 1568.8372 $[M+H]^+$ |

(continued)

| Compound | Sequence | SEQ ID NO: | Formula | Molecular weight (g/mol) | Yield (%) | $t_r$ HPLC[a] (min) | HRMS ($[M+2H]^{2+}$) | |
|---|---|---|---|---|---|---|---|---|
| | | | | | | | Found | Calculated |
| compound 67 | Ac-RDIIQRMHLRQYE**Cha**L-OH | 99 | $C_{92}H_{152}N_{28}O_{24}S$ | 2065.13 I | 24 | 2.13 | 1033.5706 | 1033.5706 |
| compound 68 | Ac-FNDCRDIIQRMHLRQYE**F**L-OH | 100 | $C_{112}H_{171}N_{33}O_{31}S_2$ | 2538.23 | 18 | 2.21 | 1270.1227 | 1270.1208 |
| compound 69 | Ac-FNDCRDIIQRMHLRQYE**W**L-OH | 101 | $C_{114}H_{172}N_{34}O_{31}S_2$ | 2577.24 | 17 | 2.22 | 1289.6234 | 1289.6263 |
| compound 70 | Ac-FNDCRDIIQRMHLRQYE**Y**L-OH | 102 | $C_{112}H_{171}N_{33}O_{32}S_2$ | 2554.22 | 20 | 2.16 | 1278.1222 | 1278.1183 |
| compound 71 | Ac-FNDCRDIIQRMHLRQYE**H**L-OH | 103 | $C_{109}H_{169}N_{35}O_{31}S_2$ | 2528.22 | 19 | 2.09 | 1265.1135 | 1265.1161 |
| compound 72 | Ac-**NARRI**FNDCRDIIQRMHLRQYELL-OH | 104 | $C_{134}H_{219}N_{45}O_{37}S_2$ | 3114.61 | 9 | 2.37 | 1558.3101 | 1558.3118 |
| compound 73 | Ac-**RRI**FNDCRDIIQRMHLRQYELL-OH | 105 | $C_{127}H_{208}N_{42}O_{34}S_2$ | 2929.53 | 12 | 2.32 | 1465.775 | 1465.7719 |
| compound 74 | Ac-**KK**FN$_c$**[Pra**CRD**Azk]**IQRMHLRQYE**Cha**L-OH | 178 | $C_{125}H_{200}N_{40}O_{31}S_2$ | 2821.47 | 7 | 2.14 | 1411.7465 | 1411.7451 |
| compound 75 | Ac-FND**A**RDIIQRMHLRQYELL-OH | 107 | $C_{109}H_{173}N_{33}O_{31}S$ | 2472.27 | 21 | 2.17 | 1237.1433 | 1237.1426 |
| compound 76 | Ac-FNDCRDIIQRMHLRQYE**A**L-OH | 108 | $C_{106}H_{167}N_{33}O_{31}S_2$ | 2462.19 | 21 | 2.11 | 1232.1002 | 1232.1052 |
| compound 77 | Ac-FNDCRDIIQRMHLRQY**A**LL-OH | 109 | $C_{107}H_{171}N_{33}O_{29}S_2$ | 2446.24 | 14 | 2.20 | 1224.1282 | 1224.126 |
| compound 78 | Ac-FNDCRDIIQRMHLRQ**A**ELL-OH | 110 | $C_{103}H_{169}N_{33}O_{30}S_2$ | 2412.22 | 14 | 2.16 | 1207.116 | 1207.1156 |
| compound 79 | Ac-FNDCRDIIQRMHLRQYEL**A**-OH | 111 | $C_{106}H_{167}N_{33}O_{31}S_2$ | 2462.19 | 17 , | 2.10 | 1232.1007 | 1232.1052 |
| compound 80 | Ac-IIQRMHLRQYE**Cha**L-OH | 112 | $C_{82}H_{135}N_{23}O_{20}S$ | 1794.0 | 33 . | 2.15 . | 1795.0037 **$[M+H]^+$** | 1795.0052 **$[M+H]^+$** |
| compound 81 | Ac-**NARRI**FND**A**RDIIQRMHLRQYELL-OH | 113 | $C_{134}H_{219}N_{45}O_{37}S$ | 3082.64 | 13 | 2.34 | 1542.3235 | 1542.3258 |
| compound 82 | Ac-FNDCRDIIQRMHLRQ**Cha**ELL-OH | 114 | $C_{109}H_{179}N_{33}O_{30}S_2$ | 2494.29 | 13 | 2.34 | 1248.1549 | 1248.15 |
| compound 83 | Ac-**NARRI**FND**A**RDIIQRMHLRQYE**W**L-OH | 115 | $C_{139}H_{218}N_{46}O_{37}S$ | 3155.63 | 12 | 2.43 | 1578.8191 | 1578.8234 |
| compound 84 | Ac-FNDCRDIIQRMHLRQYEL**Cha**-OH | 116 | $C_{112}H_{177}N_{33}O_{31}S_2$ | 2544.27 | 5 | 2.27 | 1273.1467 | 1273.1443 |

## Claims

**1.** A G protein peptidomimetic or salt thereof comprising the sequence of formula (VIII):

$$RDX_8IQRX_7HLX_6X_5X_4X_3X_2X_1 \text{ (SEQ ID NO:8)} \qquad \text{(VIII)}$$

wherein $X_1$ is selected from the group consisting of: leucine (L), L-$NH_2$, isoleucine (I), and $X_{1a}$; wherein $X_{1a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;
wherein $X_2$ is selected from the group consisting of: leucine (L), isoleucine (I), tyrosine (Y), histidine (H), and $X_{2a}$, wherein $X_{2a}$ is selected from alanine analogue, phenylalanine (F) or tryptophan (W), wherein the alanine analogue is a molecule resulting from the replacement of at least one hydrogen of an alanine by at least one

moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein $X_3$ is selected from the group consisting of: glutamic acid (E), glutamine (Q), homoglutamic acid (hGlu), aspartic acid (D), alanine (A) and $X_{3a}$, wherein $X_{3a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein $X_4$ is selected from the group consisting of: tyrosine (Y), 2-naphthalanine (2-Nal), 1-naphthalanine (1-Nal), and $X_{4a}$, wherein $X_{4a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

wherein the sequence $X_4X_3X_2X_1$ (SEQ ID NO:18) is selected from the group consisting of $X_4X_3X_{2a}X_1$ (SEQ ID NO: 133), $X_4X_3X_2X_{1a}$ (SEQ ID NO:134), $X_{4a}X_3X_2X_1$ (SEQ ID NO:135) and $X_4X_{3a}X_2X_1$ (SEQ ID NO:136), preferably wherein the sequence $X_4X_3X_2X_1$ (SEQ ID NO:18) is selected from the group consisting of: $YEX_{2a}L$ (SEQ ID NO:19), $YELX_{1a}$ (SEQ ID NO:20), $X_{4a}ELL$ (SEQ ID NO:21) and $YX_{3a}LL$ (SEQ ID NO:22);

wherein the sequence $X_4X_3X_2X_1$ (SEQ ID NO:18) is not YELL (SEQ ID NO:23),

wherein $X_5$ is selected from the group consisting of: glutamine (Q), cysteine (C), olefinic amino acids, amino acids containing a carboxylic acid group side chain, amino acids containing an amine side-chain, amino acid containing an alkynyl side-chain, and amino acids containing an azidated side-chain,

wherein $X_6$ is selected from the group consisting of: arginine (R), C, olefinic amino acids, amino acids containing a carboxylic acid group side chain, amino acids containing an amine side-chain, amino acid containing an alkynyl side-chain, and amino acids containing an azidated side-chain,

wherein $X_7$ is selected from the group consisting of: methionine (M), C, olefinic amino acids, amino acids containing a carboxylic acid group side chain, amino acids containing an amine side-chain, amino acid containing an alkynyl side-chain, and amino acids containing an azidated side-chain, and

wherein $X_8$ is selected from the group consisting of: isoleucine (I), C, olefinic amino acids, amino acids containing a carboxylic acid group side chain, amino acids containing an amine side-chain, amino acid containing an alkynyl side-chain, and amino acids containing an azidated side-chain,

and optionally wherein said peptidomimetic comprises at least one covalent tether, said covalent tether being formed from the reaction of an amino acid containing an amine side-chain with an amino acid containing a carboxylic acid group side-chain, or from the reaction between two olefinic amino acids, or from the reaction of an amino acid containing an azidated side-chain with an amino acid containing an alkynyl side-chain, or from the reaction between two amino acids each containing a thiol group side-chain, or from the reaction of an olefinic amino acid with an amino acid containing a thiol group side-chain, preferably said covalent tether is formed from the reaction of an amino acid containing an azidated side-chain with an amino acid containing an alkynyl side-chain.

**2.** The G protein peptidomimetic or salt thereof according to claim 1, wherein the sequence $X_4X_3X_2X_1$ (SEQ ID NO:18) is $X_4X_3X_{2a}X_1$ (SEQ ID NO:133), preferably wherein the sequence $X_4X_3X_2X_1$ (SEQ ID NO:18) is $YEX_{2a}L$ (SEQ ID NO:19), wherein:

$X_1$ is selected from the group consisting of: leucine (L), L-$NH_2$ and isoleucine (I);

$X_{2a}$ is selected from alanine analogue, phenylalanine (F) or tryptophan (W), wherein the alanine analogue is a molecule resulting from the replacement of at least one hydrogen of an alanine by at least one moiety selected from the group comprising $C_{6-12}$cycloalkyl, $C_{6-12}$aryl, heteroaryl, and $C_{6-12}$cycloalkenyl; each moiety being

optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

$X_3$ is selected from the group consisting of: glutamic acid (E), glutamine (Q), homoglutamic acid (hGlu), aspartic acid (D) and alanine (A);

$X_4$ is selected from the group consisting of: tyrosine (Y), 2-naphthylalanine (2-Nal), and 1-naphthylalanine (1-Nal).

**3.** The G protein peptidomimetic or salt thereof according to any one of claims 1 or 2 comprising the sequence of formula (XII):

$$FNX_{10}X_9RDX_8IQRX_7HLX_6X_5X_4X_3X_2X_1 \text{ (SEQ ID NO: 12)} \quad \text{(XII)}$$

wherein $X_9$ is C or an amino acid without a thiol side-chain,

wherein $X_{10}$ is selected from the group consisting of: D, C, olefinic amino acids, amino acids containing a carboxylic acid group side chain, amino acids containing an amine side-chain, amino acid containing an alkynyl side-chain, and amino acids containing an azidated side-chain.

**4.** The G protein peptidomimetic or salt thereof according to any one of claims 1 to 3, wherein $X_5$ is Q, wherein $X_6$ is R and wherein $X_7$ is M.

**5.** The G protein peptidomimetic or salt thereof according to any one of claims 1 to 4, wherein $X_{1a}$ is a moiety of formula (Ia) or (I'a),
each of $X_{2a}$, $X_{3a}$ and $X_{4a}$ can be independently selected from a moiety of formula (Ib):

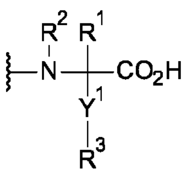
(Ia)
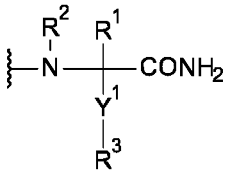
(I'a)
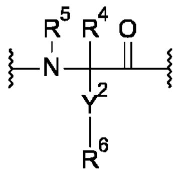
(Ib)

wherein

$R^1$ is hydrogen or $C_{1-6}$alkyl; $R^2$ is hydrogen or $C_{1-6}$alkyl;

$R^3$ is a moiety selected from the group comprising $C_{6-12}$cycloalkyl, $C_{6-12}$aryl, heteroaryl, and $C_{6-12}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$alkoxy, oxo, $=CH_2$, amino, mono- or di-$C_{1-6}$alkylamino, halo$C_{1-6}$alkyl, or 2 substituents together with the atom to which they are attached may form a $C_{3-12}$cycloalkyl, $C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

$Y^1$ is $-C(R^7)R^8-$ or $-C(=O)-$;

$R^7$ is selected from the group comprising hydrogen, OH, SH, $C_{1-6}$alkyl, $C_{3-12}$cycloalkyl, $C_{1-6}$alkoxy, amino, and halo;

$R^8$ is hydrogen or $C_{1-6}$alkyl;

or $R^7$ and at least one substituent of $R^3$ together with the carbon atom to which they are attached form a $C_{3-12}$cycloalkyl, wherein said $C_{3-12}$cycloalkyl can be optionally substituted with one or more substituents independently selected from the group comprising $C_{1-6}$alkyl, OH, halo, $C_{3-12}$cycloalkyl, $C_{2-6}$alkenyl, $C_{1-6}$alkoxy, oxo, $=CH_2$, amino, mono- or di-$C_{1-6}$alkylamino, amino $C_{1-6}$alkyl, and halo$C_{1-6}$alkyl, or two substituents together with the atom to which they are attached may form a $C_{3-12}$cycloalkyl, a $C_{5-12}$cycloalkenyl, a heterocycloalkyl or an $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

$R^4$ is hydrogen or $C_{1-6}$alkyl; $R^5$ is hydrogen or $C_{1-6}$alkyl;

$R^6$ is a moiety selected from the group comprising $C_{6-12}$cycloalkyl, $C_{6-12}$aryl, heteroaryl, and $C_{6-1}$cycloalkenyl; each moiety being optionally substituted with one or more substituents each independently selected from OH, halo, $C_{1-6}$alkyl, $C_{3-12}$cycloalkyl, $C_{2-6}$alkenyl, $C_{1-6}$ alkoxy, oxo, $=CH_2$, amino, mono- or di- $C_{1-6}$alkylamino, halo$C_{1-6}$alkyl, or 2 substituents together with the atom to which they are attached may form a $C_{3-12}$cycloalkyl,

$C_{5-12}$cycloalkenyl, heterocycloalkyl, or $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

$Y^2$ is $-C(R^7)R^8-$ or $-C(=O)-$;

$R^7$ is selected from the group comprising hydrogen, OH, SH, $C_{1-6}$alkyl, $C_{3-12}$cycloalkyl, $C_{1-6}$ alkoxy, amino, and halo;

$R^8$ is hydrogen or $C_{1-6}$alkyl;

or $R^7$ and at least one substituent of $R^6$ together with the carbon atom to which they are attached form a $C_{3-12}$cycloalkyl, wherein said $C_{3-12}$cycloalkyl can be optionally substituted with one or more substituents independently selected from the group comprising $C_{1-6}$alkyl, OH, halo, $C_{3-12}$cycloalkyl, $C_{2-6}$alkenyl, $C_{1-6}$alkoxy, oxo, $=CH_2$, amino, mono- or di-$C_{1-6}$alkylamino, amino $C_{1-6}$alkyl, and halo$C_{1-6}$alkyl, or two substituents together with the atom to which they are attached may form a $C_{3-12}$cycloalkyl, a $C_{5-12}$cycloalkenyl, a heterocycloalkyl or an $C_{6-12}$aryl; each of said formed $C_{3-12}$cycloalkyl, $C_{5-12}$cycloalkenyl, heterocycloalkyl or $C_{6-12}$aryl may be optionally substituted by one or more $C_{1-6}$alkyl;

preferably $X_4X_3X_2X_1$ (SEQ ID NO:18) is selected from the group consisting of: $X_4X_3X_{2a}X_1$ (SEQ ID NO:133), $X_4X_3X_2X_{1a}$ (SEQ ID NO:134), $X_{4a}X_3X_2X_1$ (SEQ ID NO:135) and $X_4X_{3a}X_2X_1$ (SEQ ID NO:136), and $X_{1a}$, $X_{2a}$, $X_{3a}$ and $X_{4a}$ are each independently cyclohexylalanine (Cha), more preferably $X_4X_3X_2X_1$ (SEQ ID NO:18) is $X_4X_3X_{2a}X_1$ (SEQ ID NO:133), and $X_{2a}$ is cyclohexylalanine (Cha).

**6.** The G protein peptidomimetic or salt thereof according to any one of claims 1 to 4, wherein $X_{1a}$, $X_{2a}$, $X_{3a}$ and $X_{4a}$ are each independently selected from phenylalanine (F) or tryptophan (W), preferably W.

**7.** The G protein peptidomimetic or salt thereof according to any one of claims 1 to 6, comprising a covalent tether formed between an amino acid containing an azidated side-chain and an amino acid containing an alkynyl side-chain, preferably wherein said amino acid containing an azidated side-chain is azidolysine (Azk) and said amino acid containing an alkynyl side-chain is propargylglycine (Pra).

**8.** The G protein peptidomimetic or salt thereof according to any one of claims 3 to 7, comprising a covalent tether between $X_8$ and $X_{10}$, wherein said covalent tether is not part of the linear peptide backbone.

**9.** The G protein peptidomimetic or salt thereof according to any one of claims 3 to 8, wherein $X_8$ is an amino acid containing an azidated side-chain and $X_{10}$ is an amino acid containing an alkynyl side-chain or wherein $X_{10}$ is an amino acid containing an azidated side-chain and $X_8$ is an amino acid containing an alkynyl side-chain, preferably wherein $X_8$ is azidolysine (Azk) and wherein $X_{10}$ is propargylglycine (Pra) or wherein $X_8$ is propargylglycine (Pra) and wherein $X_{10}$ is azidolysine (Azk).

**10.** The G protein peptidomimetic or salt thereof according to any one of claims 1 to 6, which is a linear peptide comprising the sequence FNDX$_9$RDIIQRMHLRQX$_4$X$_3$X$_2$X$_1$ (SEQ ID NO:51).

**11.** The G protein peptidomimetic or salt thereof according to any one of claims 1 to 6, or 10, comprising the sequence NARRIFNDX$_9$RDIIQRMHLRQX$_4$X$_3$X$_2$X$_1$ (SEQ ID NO:52).

**12.** The G protein peptidomimetic or salt thereof according to any one of claims 1 to 11, further comprising at least one lysine at its N-terminus, preferably comprising a triple lysine at its N-terminus.

**13.** The G protein peptidomimetic or salt thereof according to any one of claims 1 to 12, comprising a sequence selected from the group consisting of: FNPraCRDAzklQRMHLRQYEChaL (SEQ ID NO:122), KKKFNPraCRDAzklQRMHLRQYEChaL (SEQ ID NO:123), RDIIQRMHLRQYEChaL (SEQ ID NO:124), FNPraCRDAzklQRMHLRQYEFL (SEQ ID NO:125), KKKFNPraCRDAzklQRMHLRQYEFL (SEQ ID NO:126), FNDCRDIIQRMHLRQYEChaL (SEQ ID NO:127), FNDCRDIIQRMHLRQYEFL (SEQ ID NO:128), FNDCRDIIQRMHLRQYEWL (SEQ ID NO:129), KKFNPraCRDAzklQRMHLRQYEChaL (SEQ ID NO:130), and KFNPraCRDAzklQRMHLRQYEChaL (SEQ ID NO:131).

**14.** The G protein peptidomimetic or salt thereof according to any one of claims 1 to 13, wherein said G protein peptidomimetic is capable of stabilizing a GPCR in an active conformational state.

**15.** The G protein peptidomimetic or salt thereof according to any one of claims 1 to 14, wherein said G protein peptidomimetic induces a shift in $IC_{50}$ value of an agonist that binds to the GPCR of more than 20, preferably more than 50, wherein said shift is the $IC_{50}$ value of the agonist for binding to the GPCR in the absence of the G protein peptidomimetic divided by the $IC_{50}$ value of the agonist for binding to the GPCR in the presence of the G protein

peptidomimetic, wherein said $IC_{50}$ values are determined in a radioligand binding assay (RLA) wherein the GPCR is human P2AR, the radioligand is 3H-DHA, and the agonist is isoproterenol.

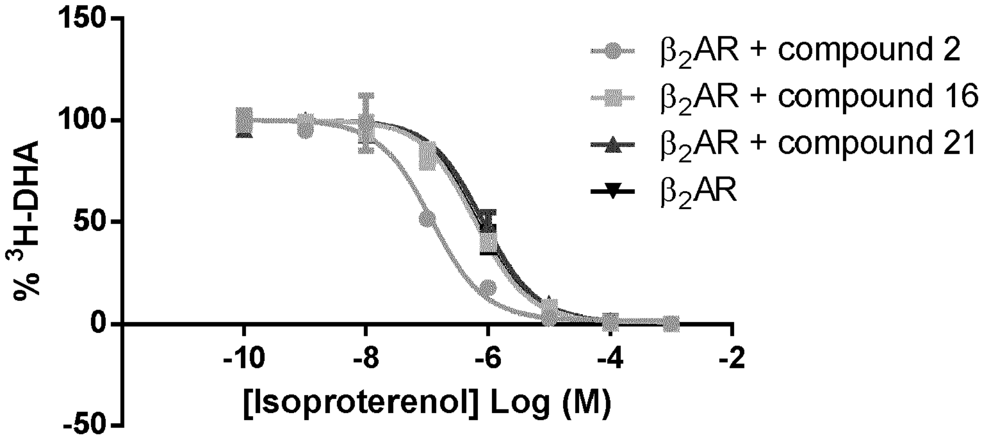


FIG. 1

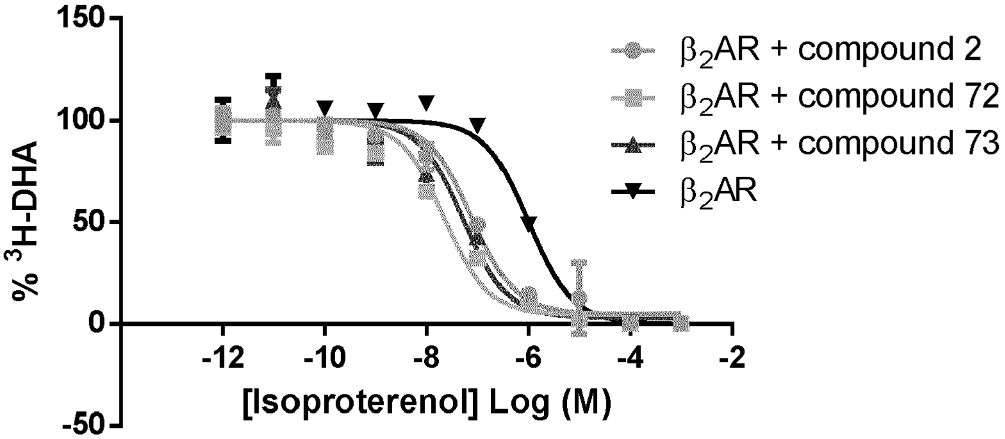


FIG. 2

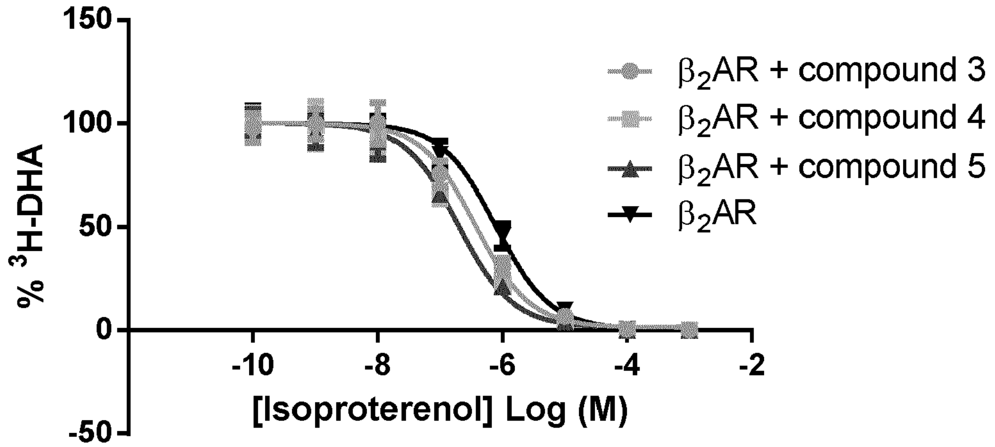


FIG. 3

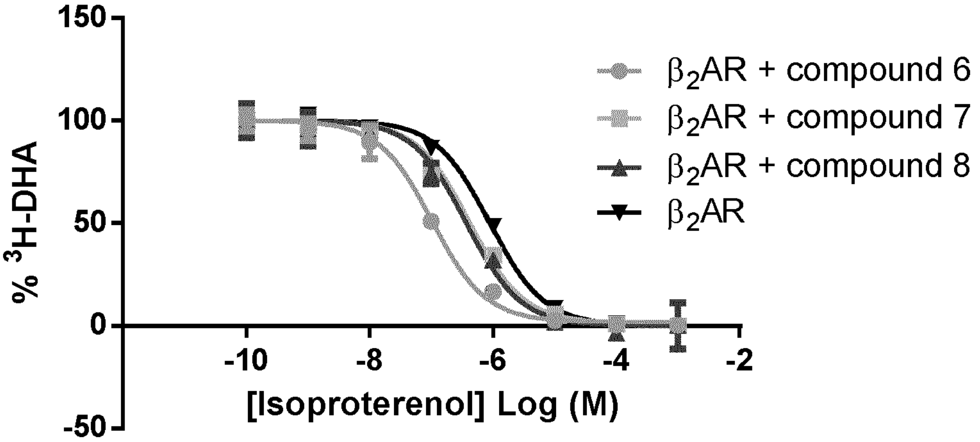


FIG. 4

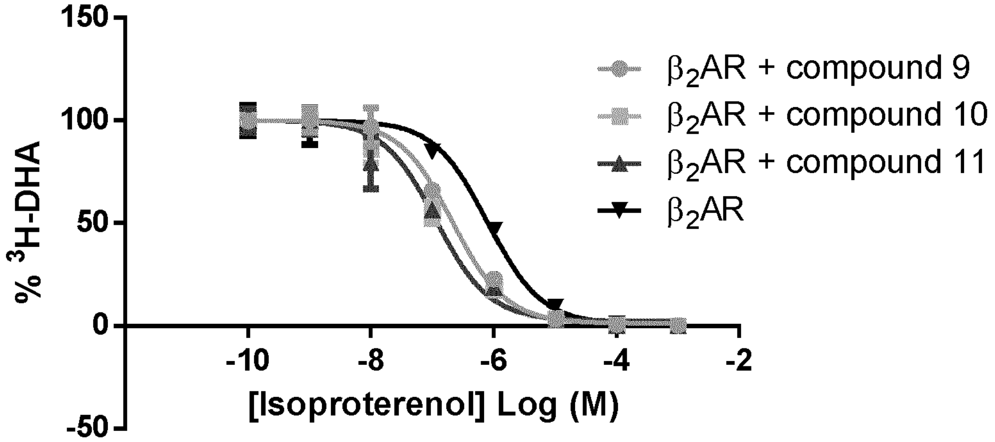


FIG.5

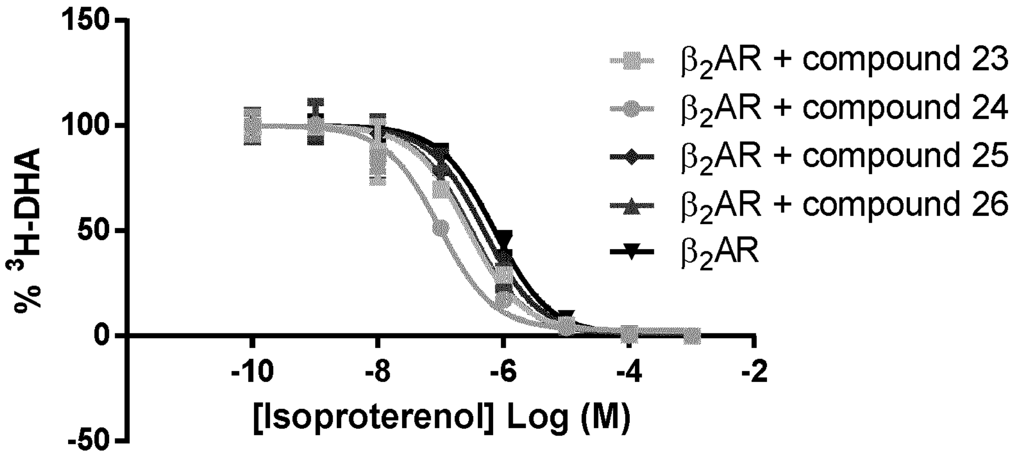


FIG. 6

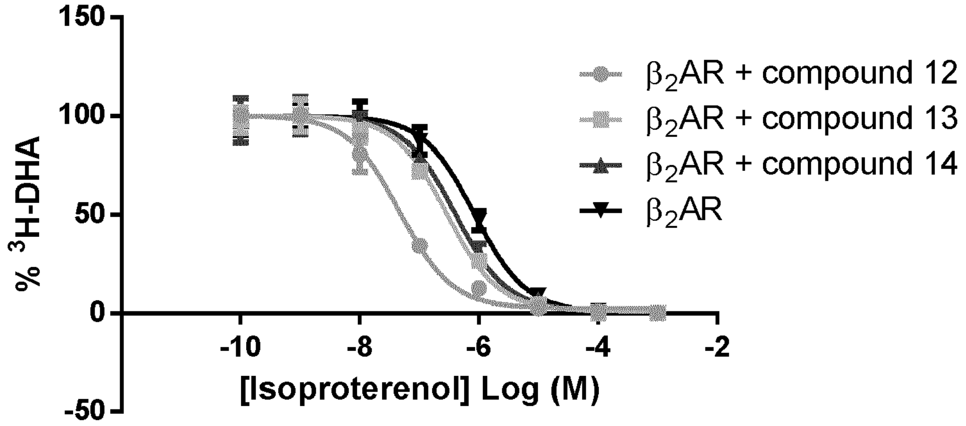


FIG. 7

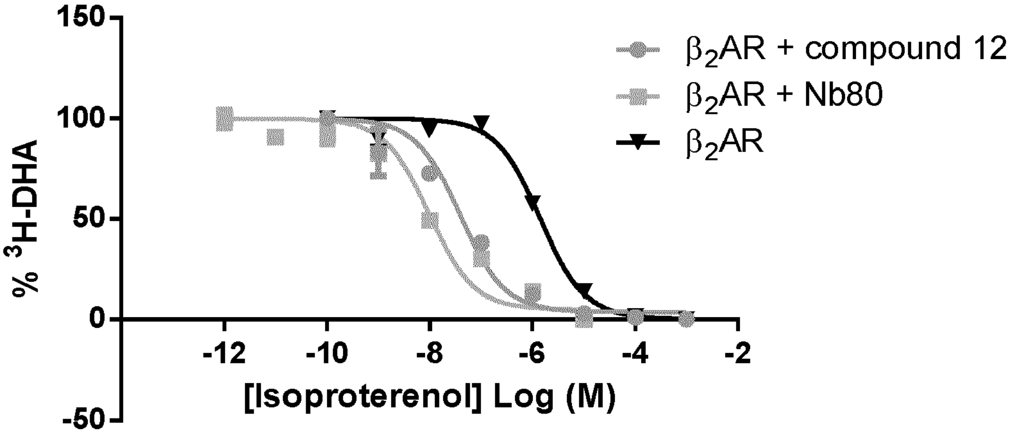


FIG. 8

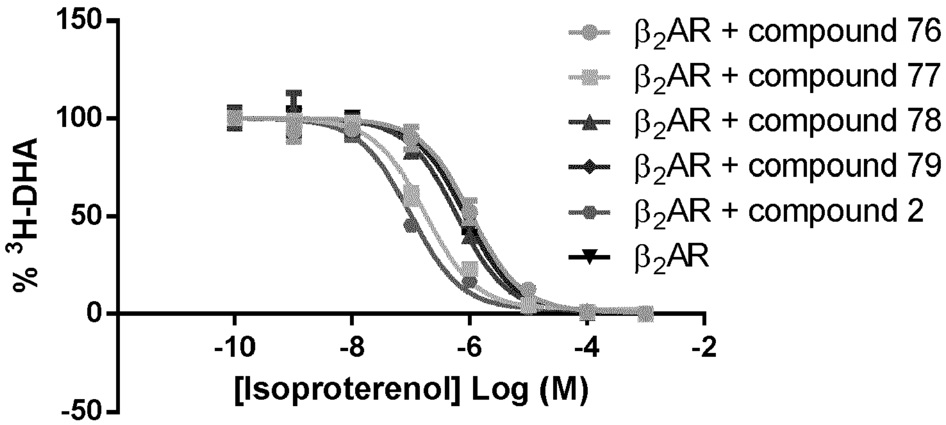


FIG. 9

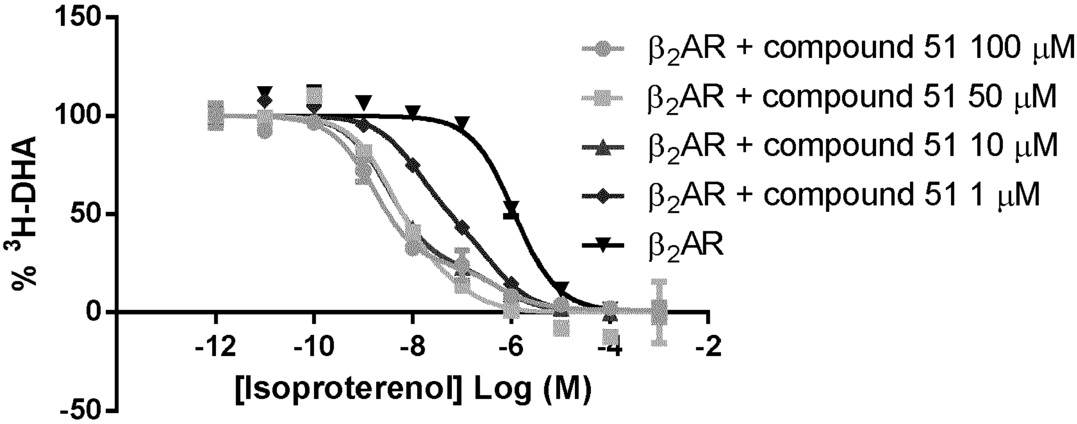


FIG. 10

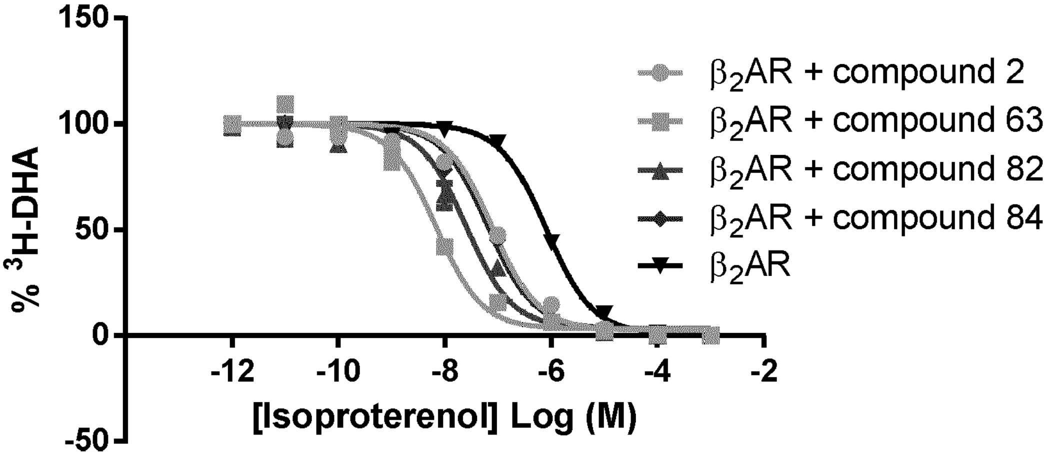


FIG. 11

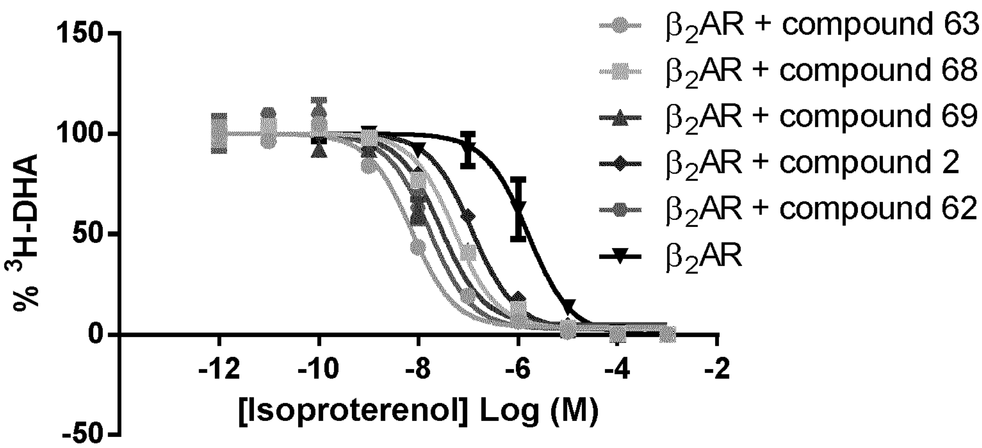


FIG. 12

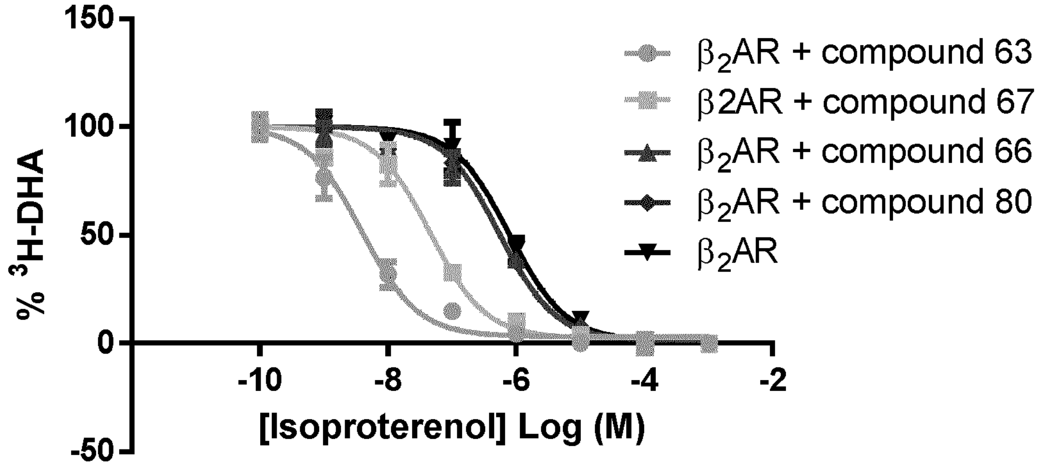


FIG. 13

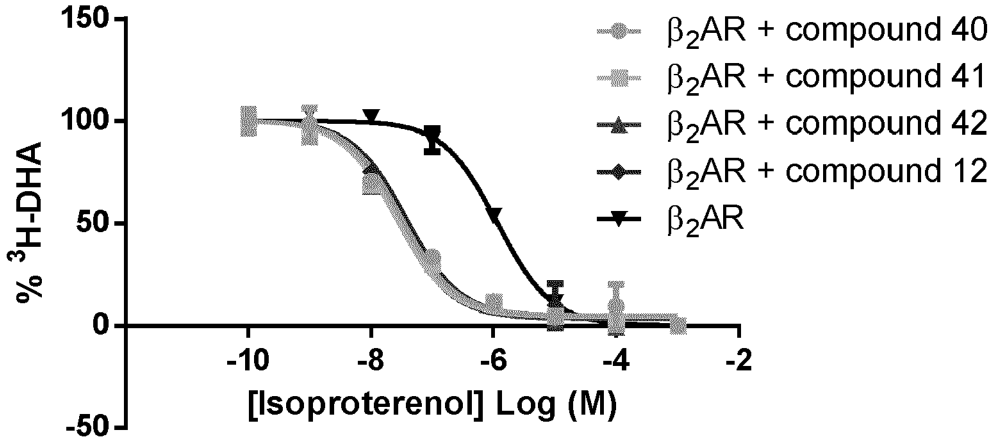


FIG. 14

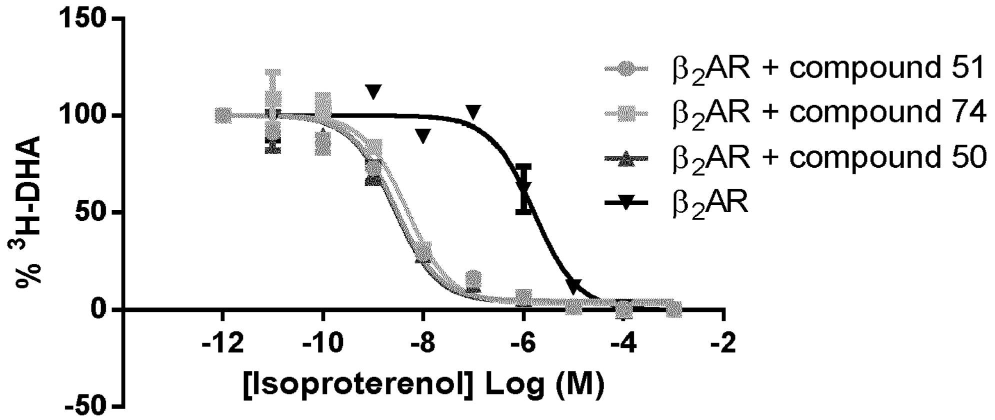


FIG. 15

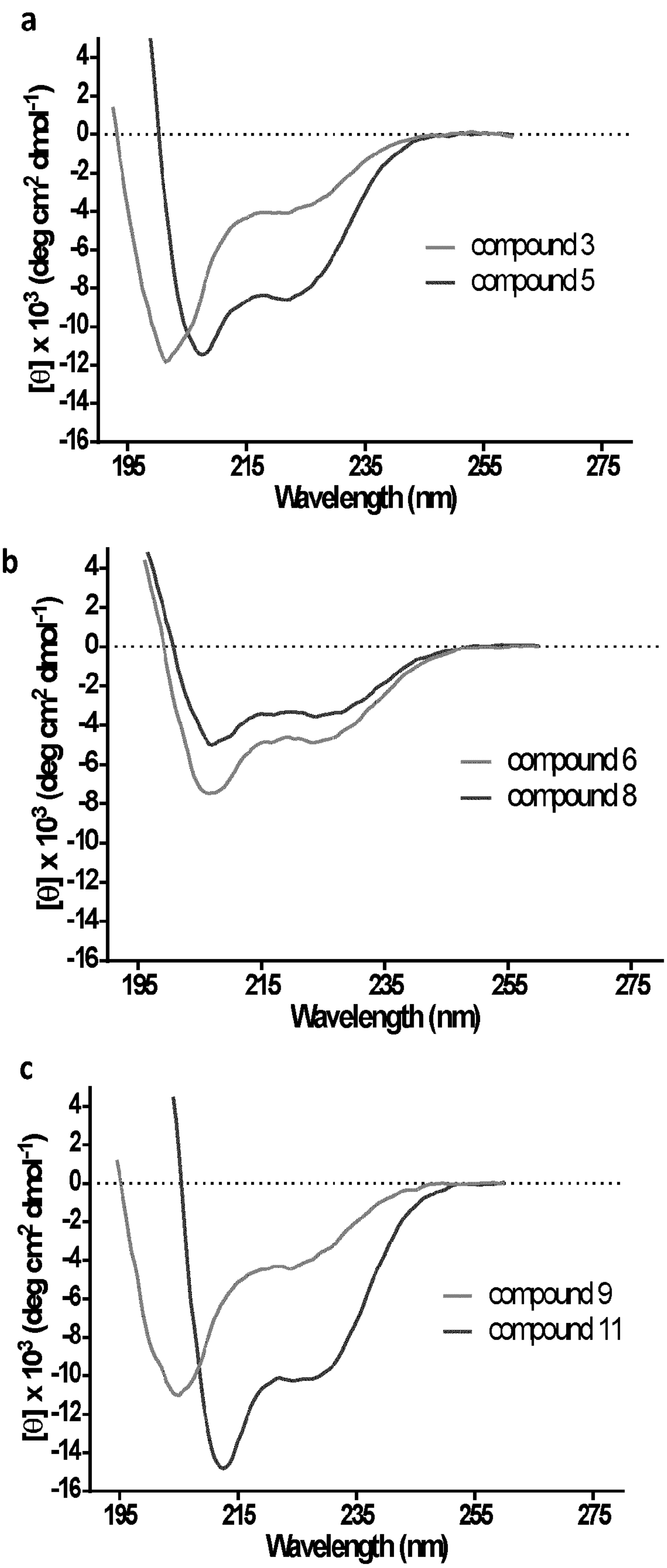
a
[θ] x 10³ (deg cm² dmol⁻¹)
Wavelength (nm)
compound 3
compound 5
b
compound 6
compound 8
c
compound 9
compound 11

FIG. 16

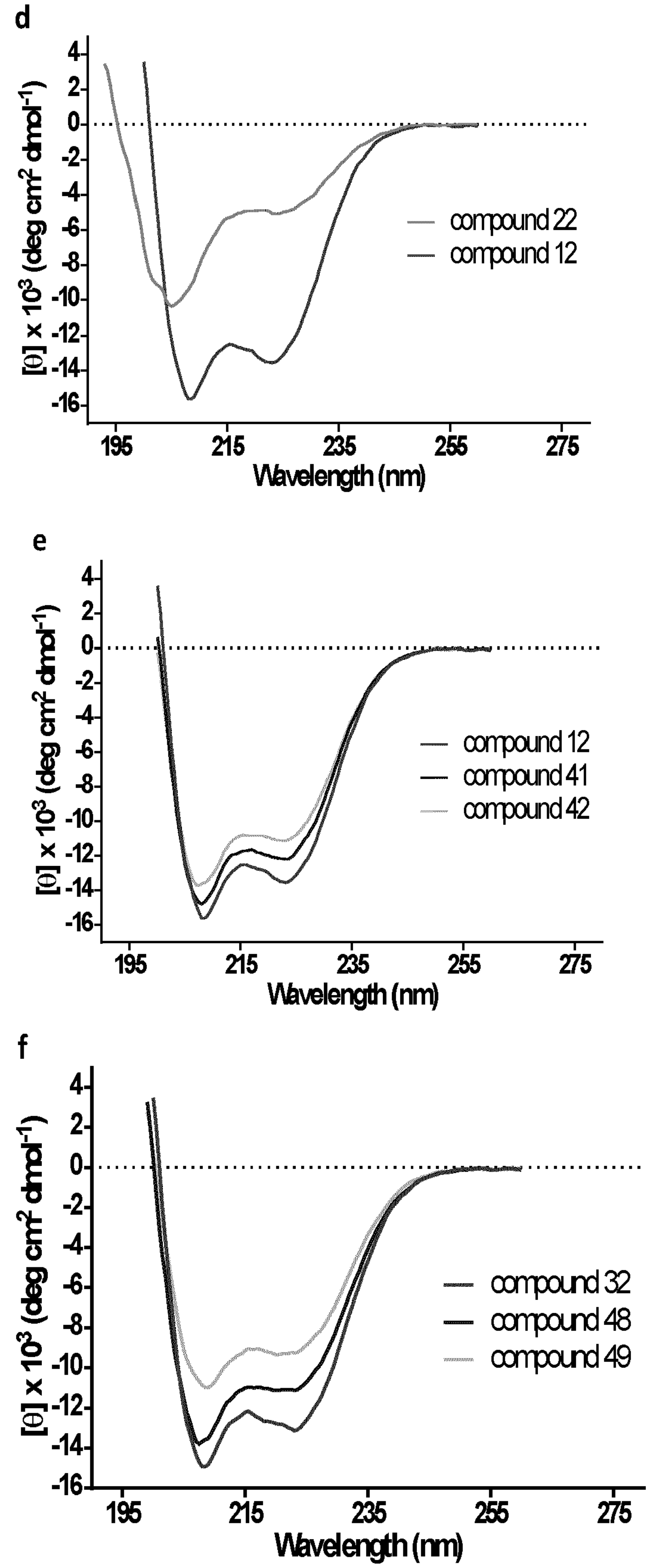
d
[θ] x 10^3 (deg cm^2 dmol^-1)
4
2
0
-2
-4
-6
-8
-10
-12
-14
-16
195
215
235
255
275
Wavelength (nm)
compound 22
compound 12
e
[θ] x 10^3 (deg cm^2 dmol^-1)
195
215
235
255
275
Wavelength (nm)
compound 12
compound 41
compound 42
f
[θ] x 10^3 (deg cm^2 dmol^-1)
195
215
235
255
275
Wavelength (nm)
compound 32
compound 48
compound 49

FIG. 16 (cont.)

g

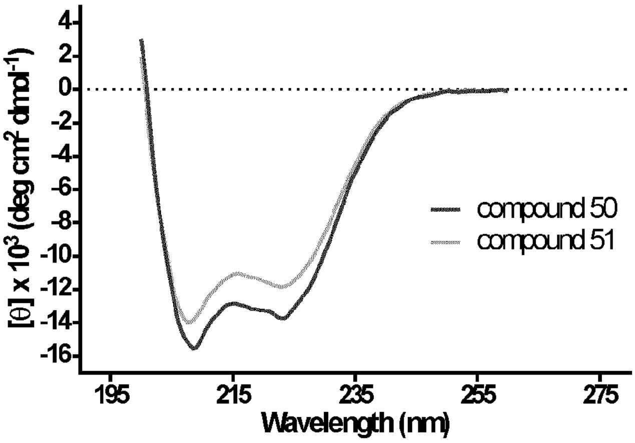


FIG. 16 (cont.)

$f_b01$ 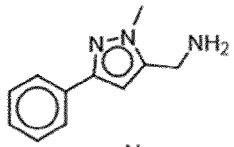

$f_b02$ 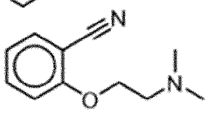

$f_b03$ 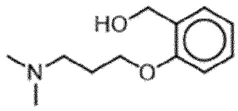

$f_b04$ 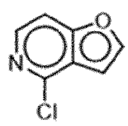

$f_b05$ 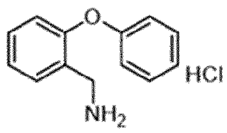

$f_b06$ 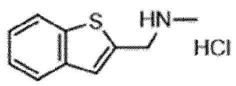

$f_b07$ 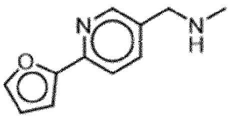

$f_b08$ 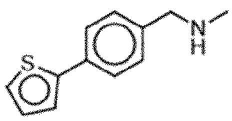

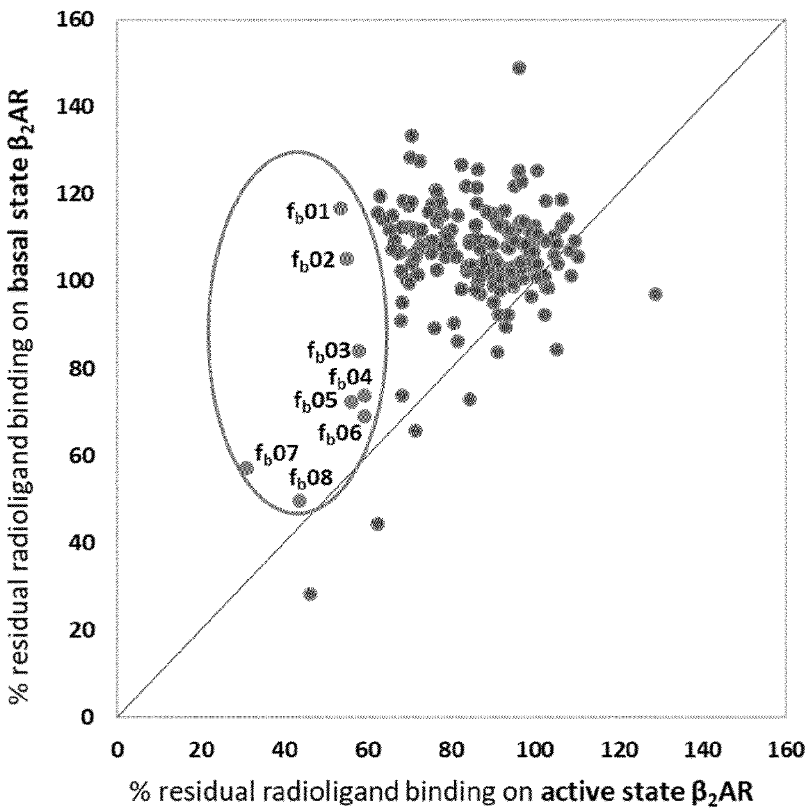


FIG. 17

$f_d01$ 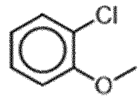

$f_d02$ 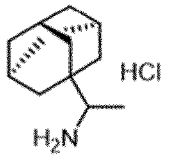

$f_d03$ 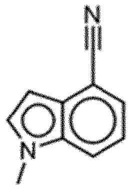

$f_d04$ 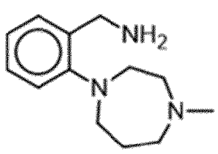

$f_d05$ 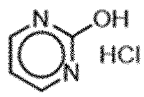

$f_d06$ 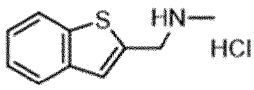

$f_d07$ 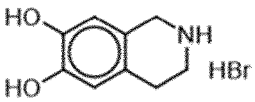

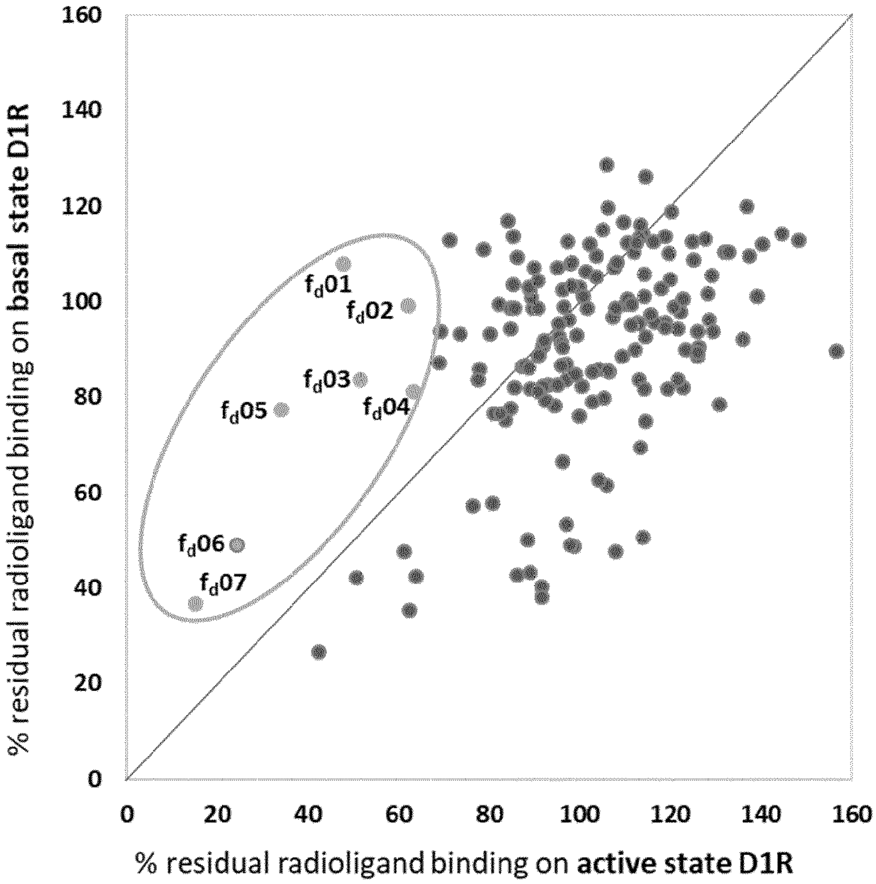


FIG. 18

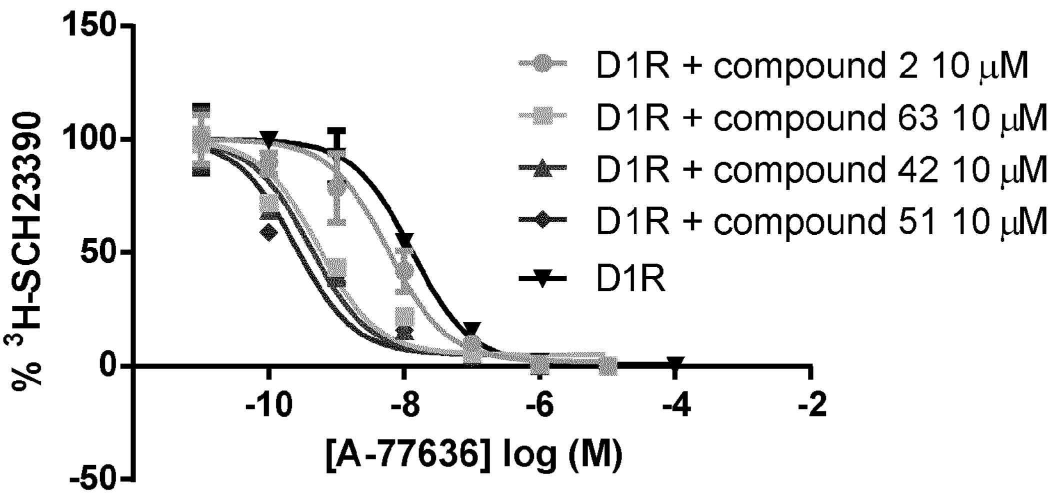


FIG. 19

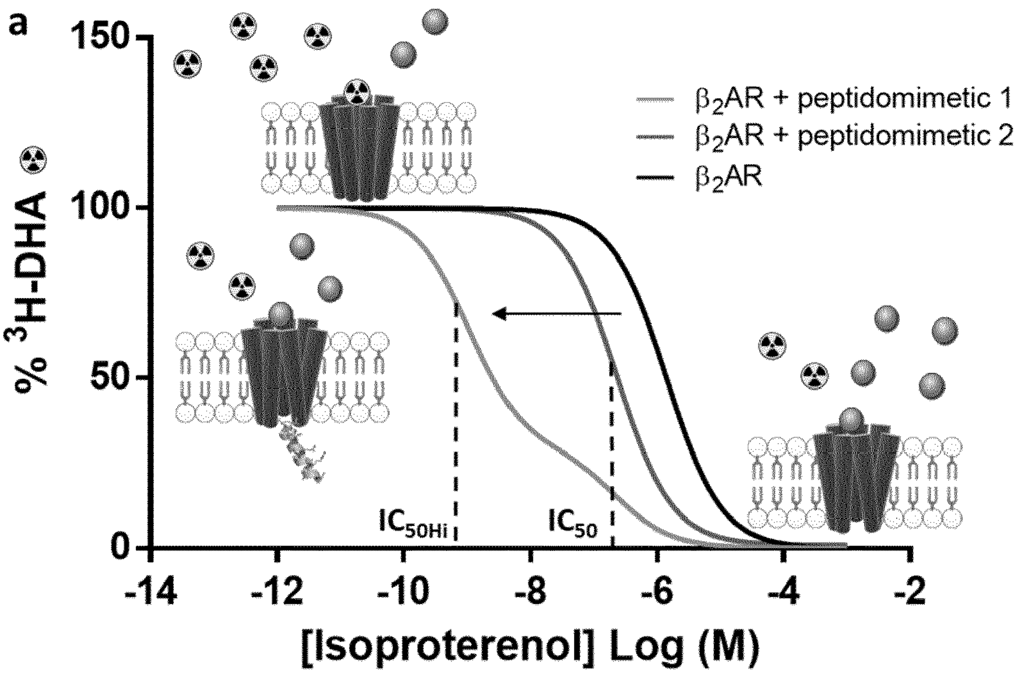
a
150
100
50
0
% $^3$H-DHA
β2AR + peptidomimetic 1
β2AR + peptidomimetic 2
β2AR
IC50Hi
IC50
-14
-12
-10
-8
-6
-4
-2
[Isoproterenol] Log (M)

FIG. 20

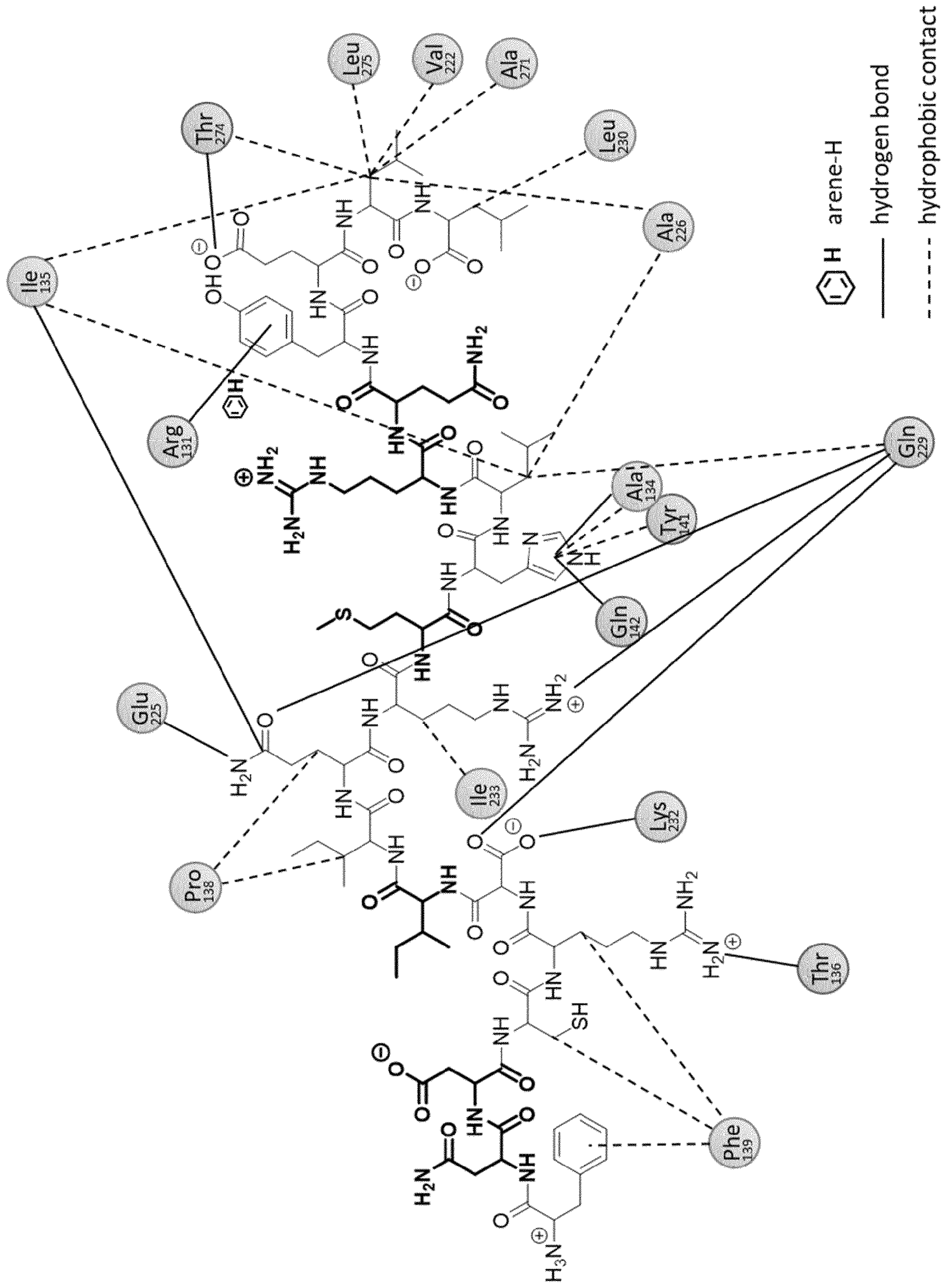

FIG. 21

Europäisches Patentamt
European Patent Office
Office européen des brevets

# EUROPEAN SEARCH REPORT

Application Number
EP 20 21 5815

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LOTTE-EMILIE BOYHUS ET AL: "G s protein peptidomimetics as allosteric modulators of the [beta] 2 -adrenergic receptor", RSC ADVANCES, vol. 8, no. 4, 9 January 2018 (2018-01-09), pages 2219-2228, XP055710297, DOI: 10.1039/C7RA11713B * the whole document * * figure 4; table 1 * ----- | 1-15 | INV. C07K14/72 |
| X | MAZZONI MARIA R. ET AL: "A G[alpha] s Carboxyl-Terminal Peptide Prevents G s Activation by the A 2A Adenosine Receptor", MOLECULAR PHARMACOLOGY, vol. 58, no. 1, July 2000 (2000-07), pages 226-236, XP055811369, US ISSN: 0026-895X, DOI: 10.1124/mol.58.1.226 * the whole document * * table 1 * ----- | 1-15 | |
| | | | TECHNICAL FIELDS SEARCHED (IPC) |
| X | RASENICK M.M. ET AL: "Synthetic peptides as probes for G protein function. Carboxyl-terminal G alpha s peptides mimic Gs and evoke high affinity agonist binding to beta-adrenergic receptors.", JOURNAL OF BIOLOGICAL CHEMISTRY, vol. 269, no. 34, August 1994 (1994-08), pages 21519-21525, XP055813988, US ISSN: 0021-9258, DOI: 10.1016/S0021-9258(17)31835-5 * table 1 * ----- | 1-15 | C07K A61K |

-/--

The present search report has been drawn up for all claims

| Place of search | Date of completion of the search | Examiner |
|---|---|---|
| Munich | 17 June 2021 | Cervigni, S |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 1 of 2

Europäisches Patentamt
European Patent Office
Office européen des brevets

# EUROPEAN SEARCH REPORT

Application Number
EP 20 21 5815

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | GRIECO P ET AL: "A structure-activity relationship study on position-2 of the G@a"s C-terminal peptide able to inhibit G"s activation by A"2"A adenosine receptor", EUROPEAN JOURNAL OF MEDICINAL CHEMISTRY, ELSEVIER, AMSTERDAM, NL, vol. 38, no. 1, January 2003 (2003-01), pages 13-18, XP004410841, ISSN: 0223-5234, DOI: 10.1016/S0223-5234(02)00010-7 * table 1 * ----- | 1-15 | |
| | | | TECHNICAL FIELDS SEARCHED (IPC) |

2

The present search report has been drawn up for all claims

| Place of search | Date of completion of the search | Examiner |
|---|---|---|
| Munich | 17 June 2021 | Cervigni, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5721121 A


### Non-patent literature cited in the description


- **PARK et al.** *Nature,* 2008, vol. 477, 549
- **SCHEERER et al.** *Nature,* 2008, vol. 455, 497
- **RASMUSSEN et al.** *Nature,* 2011, vol. 469, 175
- **KRUSE et al.** *Nature,* 2013, vol. 504, 101
- **SOUNIER et al.** *Nature,* 2015, vol. 524, 375
- **RASMUSSEN et al.** *Nature,* 2011, vol. 477, 175
- **LIANG et al.** *Nature,* 2017, vol. 546, 118
- **CARPENTER et al.** *Protein Engineering Design and Selection,* 2016, vol. 29, 583
- **CARPENTER et al.** *Nature,* 2016, vol. 536, 104
- **MARTIN et al.** *Chemistry-A European Journal,* 2017, vol. 23, 9632
- **BOYHUS et al.** *RSC Advances,* 2018, vol. 8, 2219
- **VAGNER et al.** *Current Opinion in Chemical Biology,* 2008, vol. 292, 296
- **RIPKA ; RICH.** *Current Opinion in Chemical Biology,* 2008, vol. 2 (441), 452
- **PELAY-GIMENO et al.** *Angewandte Chemie International Edition,* 2015, vol. 54 (8896), 8927
- **JARADAT.** *Amino Acids,* 2018, vol. 50, 39-68
- **XIE ; SCHULTZ.** *Current Opinion Chemical Biology,* 2005, vol. 548, 554
- **KUO et al.** *Current Genetics,* 2018, 327-333
- **RATNAYAKE et al.** *Methods Cellular Biology,* 2017, vol. 1, 25
- **WHITE ; YUDIN.** *Nature Chemistry,* 2011, vol. 3, 509-524
- **JOHANSSON ; PEDERSEN.** *European Journal of Organic Chemistry,* 2012, 4267-4281
- **ALTSCHUL et al.** *J Mol Biol,* 1990, vol. 215, 403-10
- **TATUSOVA ; MADDEN.** *FEMS Microbiol Lett,* 1999, vol. 174, 247-250
- The Conformation of Biological. Macromolecules. **CANTOR ; SCHIMMEL.** Biophysical Chemistry. W.H. Freeman and Company, 1980
- **CREIGHTON.** Proteins: Structures and Molecular Properties. W.H. Freeman and Company, 1993
- **GREEN ; SAMBROOK et al.** Molecular Cloning: A Laboratory Manual. Cold Spring Harbor Laboratory Press, 2012
- **GOSSEN et al.** *PNAS,* 1995, vol. 5547, 5551
- **GOSSEN et al.** *Science,* 1995, vol. 1766, 1769
- **GRAHAM et al.** *J. Gen. Virol.,* 1977, vol. 36, 59
- **MATHER.** *Biol. Reprod.,* 1980, vol. 23, 243-251
- **JARVIS.** *Virology,* 25 May 2003, vol. 310 (1), 1-7
- **SAMBROOK ; RUSSEL.** Molecular Cloning, A Laboratory Manual. vol. 3
- Protein Immobilization. Marcel Dekker, Inc, 1991
- **SAMBROOK et al.** Molecular Cloning, A Laboratory Manual. Cold Spring Harbor Laboratory Press, 2001
- **PARDON et al.** *Angewandte Chemie International Edition,* 2018, vol. 57, 5292-5295

SEQUENCE LISTING

<110> Vrije Universiteit Brussel

<120> G PROTEIN PEPTIDOMIMETICS

<130> VUB-086-EPPRIO

<160> 180

<170> PatentIn version 3.5

<210> 1
<211> 8
<212> PRT
<213> Artificial Sequence

<220>
<223> synthetic peptide

<220>
<221> MISC_FEATURE
<222> (3)..(3)
<223> Xaa can be arginine (R), cysteine (C), moiety R5, moiety S5, moiety R8, glutamic acid (E), lysine (K), moiety Pra or moiety Azk

<220>
<221> MISC_FEATURE
<222> (4)..(4)
<223> Xaa can be glutamine (Q), cysteine (C), moiety R5, moiety S5, moiety R8, glutamic acid (E), lysine (K), moiety Pra or moiety Azk

<220>
<221> MISC_FEATURE
<222> (5)..(5)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (6)..(6)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (7)..(7)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (8)..(8)
<223> Xaa can be leucine (L), L-NH2, isoleucine (I), alanine analogue of formula (Ia) or (I'a), phenylalanine (F) or tryptophan (W)

<400> 1

His Leu Xaa Xaa Xaa Xaa Xaa Xaa
1 5

<210> 2
<211> 9
<212> PRT
<213> Artificial Sequence

<220>
<223> synthetic peptide

<220>
<221> MISC_FEATURE
<222> (1)..(1)
<223> Xaa can be methionine (M), cysteine (C), moiety R5, moiety S5, moiety R8, glutamic acid (E), lysine (K), moiety Pra or moiety Azk

<220>
<221> MISC_FEATURE
<222> (4)..(4)
<223> Xaa can be arginine (R), cysteine (C), moiety R5, moiety S5, moiety R8, glutamic acid (E), lysine (K), moiety Pra or moiety Azk

<220>
<221> MISC_FEATURE
<222> (5)..(5)
<223> Xaa can be glutamine (Q), cysteine (C), moiety R5, moiety S5, moiety R8, glutamic acid (E), lysine (K), moiety Pra or moiety Azk

<220>
<221> MISC_FEATURE
<222> (6)..(6)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (7)..(7)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (8)..(8)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (9)..(9)
<223> Xaa can be leucine (L), L-NH2, isoleucine (I), alanine analogue of formula (Ia) or (I'a), phenylalanine (F) or tryptophan (W)

<400> 2

```
Xaa His Leu Xaa Xaa Xaa Xaa Xaa Xaa
1               5


<210>  3
<211>  10
<212>  PRT
<213>  Artificial Sequence

<220>
<223>  synthetic peptide


<220>
<221>  MISC_FEATURE
<222>  (2)..(2)
<223>  Xaa can be methionine (M), cysteine (C), moiety R5, moiety S5,
       moiety R8, glutamic acid (E), lysine (K), moiety Pra or moiety
       Azk

<220>
<221>  MISC_FEATURE
<222>  (5)..(5)
<223>  Xaa can be arginine (R), cysteine (C), moiety R5, moiety S5,
       moiety R8, glutamic acid (E), lysine (K), moiety Pra or moiety
       Azk

<220>
<221>  MISC_FEATURE
<222>  (6)..(6)
<223>  Xaa can be glutamine (Q), cysteine (C), moiety R5, moiety S5,
       moiety R8, glutamic acid (E), lysine (K), moiety Pra or moiety
       Azk

<220>
<221>  MISC_FEATURE
<222>  (7)..(7)
<223>  Xaa can be tyrosine (Y), 2-naphthalanine (2-Nal), 1-naphthalanine
       (1-Nal), alanine analogue of formula (Ib), phenylalanine (F) or
       tryptophan (W)

<220>
<221>  MISC_FEATURE
<222>  (8)..(8)
<223>  Xaa can be glutamic acid (E), glutamine (Q), homoglutamic acid
       (hGlu), aspartic Acid (D), alanine (A), alanine analogue of
       formula (Ib), phenylalanine (F) or tryptophan (W)

<220>
<221>  MISC_FEATURE
<222>  (9)..(9)
<223>  Xaa can be leucine (L), isoleucine (I), tyrosine (Y), histidine
       (H), alanine analogue of formula (Ib), phenylalanine (F) or
       tryptophan (W)

<220>
<221>  MISC_FEATURE
<222>  (10)..(10)
<223>  Xaa can be leucine (L), L-NH2, isoleucine (I), alanine analogue
       of formula (Ia) or (I'a), phenylalanine (F) or tryptophan (W)

<400>  3
```

```
Arg Xaa His Leu Xaa Xaa Xaa Xaa Xaa Xaa
1               5                   10


<210>  4
<211>  11
<212>  PRT
<213>  Artificial Sequence

<220>
<223>  synthetic peptide


<220>
<221>  MISC_FEATURE
<222>  (3)..(3)
<223>  Xaa can be methionine (M), cysteine (C), moiety R5, moiety S5,
       moiety R8, glutamic acid (E), lysine (K), moiety Pra or moiety
       Azk

<220>
<221>  MISC_FEATURE
<222>  (6)..(6)
<223>  Xaa can be arginine (R), cysteine (C), moiety R5, moiety S5,
       moiety R8, glutamic acid (E), lysine (K), moiety Pra or moiety
       Azk

<220>
<221>  MISC_FEATURE
<222>  (7)..(7)
<223>  Xaa can be glutamine (Q), cysteine (C), moiety R5, moiety S5,
       moiety R8, glutamic acid (E), lysine (K), moiety Pra or moiety
       Azk

<220>
<221>  MISC_FEATURE
<222>  (8)..(8)
<223>  Xaa can be tyrosine (Y), 2-naphthalanine (2-Nal), 1-naphthalanine
       (1-Nal), alanine analogue of formula (Ib), phenylalanine (F) or
       tryptophan (W)

<220>
<221>  MISC_FEATURE
<222>  (9)..(9)
<223>  Xaa can be glutamic acid (E), glutamine (Q), homoglutamic acid
       (hGlu), aspartic Acid (D), alanine (A), alanine analogue of
       formula (Ib), phenylalanine (F) or tryptophan (W)

<220>
<221>  MISC_FEATURE
<222>  (10)..(10)
<223>  Xaa can be leucine (L), isoleucine (I), tyrosine (Y), histidine
       (H), alanine analogue of formula (Ib), phenylalanine (F) or
       tryptophan (W)

<220>
<221>  MISC_FEATURE
<222>  (11)..(11)
<223>  Xaa can be leucine (L), L-NH2, isoleucine (I), alanine analogue
       of formula (Ia) or (I'a), phenylalanine (F) or tryptophan (W)

<400>  4
```

Gln Arg Xaa His Leu Xaa Xaa Xaa Xaa Xaa Xaa
1 5 10

<210> 5
<211> 12
<212> PRT
<213> Artificial Sequence

<220>
<223> synthetic peptide

<220>
<221> MISC_FEATURE
<222> (4)..(4)
<223> Xaa can be methionine (M), cysteine (C), moiety R5, moiety S5, moiety R8, glutamic acid (E), lysine (K), moiety Pra or moiety Azk

<220>
<221> MISC_FEATURE
<222> (7)..(7)
<223> Xaa can be arginine (R), cysteine (C), moiety R5, moiety S5, moiety R8, glutamic acid (E), lysine (K), moiety Pra or moiety Azk

<220>
<221> MISC_FEATURE
<222> (8)..(8)
<223> Xaa can be glutamine (Q), cysteine (C), moiety R5, moiety S5, moiety R8, glutamic acid (E), lysine (K), moiety Pra or moiety Azk

<220>
<221> MISC_FEATURE
<222> (9)..(9)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (10)..(10)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (11)..(11)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (12)..(12)
<223> Xaa can be leucine (L), L-NH2, isoleucine (I), alanine analogue of formula (Ia) or (I'a), phenylalanine (F) or tryptophan (W)

<400> 5

```
Ile Gln Arg Xaa His Leu Xaa Xaa Xaa Xaa Xaa Xaa
1               5                   10


<210>  6
<211>  13
<212>  PRT
<213>  Artificial Sequence

<220>
<223>  synthetic peptide


<220>
<221>  MISC_FEATURE
<222>  (1)..(1)
<223>  Xaa can be isoleucine (I), cysteine (C), moiety R5, moiety S5,
       moiety R8, glutamic acid (E), lysine (K), moiety Pra or moiety
       Azk

<220>
<221>  MISC_FEATURE
<222>  (5)..(5)
<223>  Xaa can be methionine (M), cysteine (C), moiety R5, moiety S5,
       moiety R8, glutamic acid (E), lysine (K), moiety Pra or moiety
       Azk

<220>
<221>  MISC_FEATURE
<222>  (8)..(8)
<223>  Xaa can be arginine (R), cysteine (C), moiety R5, moiety S5,
       moiety R8, glutamic acid (E), lysine (K), moiety Pra or moiety
       Azk

<220>
<221>  MISC_FEATURE
<222>  (9)..(9)
<223>  Xaa can be glutamine (Q), cysteine (C), moiety R5, moiety S5,
       moiety R8, glutamic acid (E), lysine (K), moiety Pra or moiety
       Azk

<220>
<221>  MISC_FEATURE
<222>  (10)..(10)
<223>  Xaa can be tyrosine (Y), 2-naphthalanine (2-Nal), 1-naphthalanine
       (1-Nal), alanine analogue of formula (Ib), phenylalanine (F) or
       tryptophan (W)

<220>
<221>  MISC_FEATURE
<222>  (11)..(11)
<223>  Xaa can be glutamic acid (E), glutamine (Q), homoglutamic acid
       (hGlu), aspartic Acid (D), alanine (A), alanine analogue of
       formula (Ib), phenylalanine (F) or tryptophan (W)

<220>
<221>  MISC_FEATURE
<222>  (12)..(12)
<223>  Xaa can be leucine (L), isoleucine (I), tyrosine (Y), histidine
       (H), alanine analogue of formula (Ib), phenylalanine (F) or
       tryptophan (W)
```

<220>
<221> MISC_FEATURE
<222> (13)..(13)
<223> Xaa can be leucine (L), L-NH2, isoleucine (I), alanine analogue of formula (Ia) or (I'a), phenylalanine (F) or tryptophan (W)

<400> 6

Xaa Ile Gln Arg Xaa His Leu Xaa Xaa Xaa Xaa Xaa Xaa
1               5                   10

<210> 7
<211> 14
<212> PRT
<213> Artificial Sequence

<220>
<223> synthetic peptide

<220>
<221> MISC_FEATURE
<222> (2)..(2)
<223> Xaa can be isoleucine (I), cysteine (C), moiety R5, moiety S5, moiety R8, glutamic acid (E), lysine (K), moiety Pra or moiety Azk

<220>
<221> MISC_FEATURE
<222> (6)..(6)
<223> Xaa can be methionine (M), cysteine (C), moiety R5, moiety S5, moiety R8, glutamic acid (E), lysine (K), moiety Pra or moiety Azk

<220>
<221> MISC_FEATURE
<222> (9)..(9)
<223> Xaa can be arginine (R), cysteine (C), moiety R5, moiety S5, moiety R8, glutamic acid (E), lysine (K), moiety Pra or moiety Azk

<220>
<221> MISC_FEATURE
<222> (10)..(10)
<223> Xaa can be glutamine (Q), cysteine (C), moiety R5, moiety S5, moiety R8glutamic acid (E), lysine (K), moiety Pra or moiety Azk

<220>
<221> MISC_FEATURE
<222> (11)..(11)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (12)..(12)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>

<221> MISC_FEATURE
<222> (13)..(13)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (14)..(14)
<223> Xaa can be leucine (L), L-NH2, isoleucine (I), alanine analogue of formula (Ia) or (I′a), phenylalanine (F) or tryptophan (W)

<400> 7

```
Asp Xaa Ile Gln Arg Xaa His Leu Xaa Xaa Xaa Xaa Xaa Xaa
1               5                   10
```

<210> 8
<211> 15
<212> PRT
<213> Artificial Sequence

<220>
<223> synthetic peptide

<220>
<221> MISC_FEATURE
<222> (3)..(3)
<223> Xaa can be isoleucine (I), cysteine (C), moiety R5, moiety S5, moiety R8, glutamic acid (E), lysine (K), moiety Pra or moiety Azk

<220>
<221> MISC_FEATURE
<222> (7)..(7)
<223> Xaa can be methionine (M), cysteine (C), moiety R5, moiety S5, moiety R8, glutamic acid (E), lysine (K), moiety Pra or moiety Azk

<220>
<221> MISC_FEATURE
<222> (10)..(10)
<223> Xaa can be arginine (R), cysteine (C), moiety R5, moiety S5, moiety R8, glutamic acid (E), lysine (K), moiety Pra or moiety Azk

<220>
<221> MISC_FEATURE
<222> (11)..(11)
<223> Xaa can be glutamine (Q), cysteine (C), moiety R5, moiety S5, moiety R8, glutamic acid (E), lysine (K), moiety Pra or moiety Azk

<220>
<221> MISC_FEATURE
<222> (12)..(12)
<223> Xaa can be tyrosine (Y), 2-naphthalanine (2-Nal), 1-naphthalanine (1-Nal), alanine analogue of formula (Ib), phenylalanine (F) or tryptophan (W)

<220>

<221> MISC_FEATURE
<222> (13)..(13)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (14)..(14)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (15)..(15)
<223> Xaa can be leucine (L), L-NH2, isoleucine (I), alanine analogue of formula (Ia) or (I'a), phenylalanine (F) or tryptophan (W)

<400> 8

```
Arg Asp Xaa Ile Gln Arg Xaa His Leu Xaa Xaa Xaa Xaa Xaa Xaa
1               5                   10                  15
```

<210> 9
<211> 16
<212> PRT
<213> Artificial Sequence

<220>
<223> synthetic peptide

<220>
<221> MISC_FEATURE
<222> (1)..(1)
<223> Xaa can be cysteine (C) or alanine (A)

<220>
<221> MISC_FEATURE
<222> (4)..(4)
<223> Xaa can be isoleucine (I), cysteine (C), moiety R5, moiety S5, moiety R8, glutamic acid (E), lysine (K), moiety Pra or moiety Azk

<220>
<221> MISC_FEATURE
<222> (8)..(8)
<223> Xaa can be methionine (M), cysteine (C), moiety R5, moiety S5, moiety R8, glutamic acid (E), lysine (K), moiety Pra or moiety Azk

<220>
<221> MISC_FEATURE
<222> (11)..(11)
<223> Xaa can be arginine (R), cysteine (C), moiety R5, moiety S5, moiety R8, glutamic acid (E), lysine (K), moiety Pra or moiety Azk

<220>
<221> MISC_FEATURE
<222> (12)..(12)

<223> Xaa can be glutamine (Q), cysteine (C), moiety R5, moiety S5, moiety R8, glutamic acid (E), lysine (K), moiety Pra or moiety Azk

<220>
<221> MISC_FEATURE
<222> (13)..(13)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (14)..(14)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (15)..(15)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (16)..(16)
<223> Xaa can be leucine (L), L-NH2, isoleucine (I), alanine analogue of formula (Ia) or (I'a), phenylalanine (F) or tryptophan (W)

<400> 9

```
Xaa Arg Asp Xaa Ile Gln Arg Xaa His Leu Xaa Xaa Xaa Xaa Xaa Xaa
1               5                   10                  15
```

<210> 10
<211> 17
<212> PRT
<213> Artificial Sequence

<220>
<223> synhtetic peptide

<220>
<221> MISC_FEATURE
<222> (1)..(1)
<223> Xaa can be aspartic acid (D), cysteine (C), moiety R5, moiety S5, moiety R8, glutamic acid (E), lysine (K), moiety Pra or moiety Azk

<220>
<221> MISC_FEATURE
<222> (2)..(2)
<223> Xaa can be cysteine (C) or alanine (A)

<220>
<221> MISC_FEATURE
<222> (5)..(5)
<223> Xaa can be isoleucine (I), cysteine (C), moiety R5, moiety S5, moiety R8, glutamic acid (E), lysine (K), moiety Pra or moiety

Azk

<220>
<221> MISC_FEATURE
<222> (9)..(9)
<223> Xaa can be methionine (M), cysteine (C), moiety R5, moiety S5, moiety R8, glutamic acid (E), lysine (K), moiety Pra or moiety Azk

<220>
<221> MISC_FEATURE
<222> (12)..(12)
<223> Xaa can be arginine (R), cysteine (C), moiety R5, moiety S5, moiety R8, glutamic acid (E), lysine (K), moiety Pra or moiety Azk

<220>
<221> MISC_FEATURE
<222> (13)..(13)
<223> Xaa can be glutamine (Q), cysteine (C), moiety R5, moiety S5, moiety R8, glutamic acid (E), lysine (K), moiety Pra or moiety Azk

<220>
<221> MISC_FEATURE
<222> (14)..(14)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (15)..(15)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (16)..(16)
<223> Xaa can be leucine (L), isoleucine (I), tyrosine (Y), histidine (H), alanine analogue of formula (Ib), phenylalanine (F) or tryptophan (W

<220>
<221> MISC_FEATURE
<222> (17)..(17)
<223> Xaa can be leucine (L), L-NH2, isoleucine (I), alanine analogue of formula (Ia) or (I'a), phenylalanine (F) or tryptophan (W)

<400> 10

```
Xaa Xaa Arg Asp Xaa Ile Gln Arg Xaa His Leu Xaa Xaa Xaa Xaa Xaa
1               5                   10                  15

Xaa
```

<210> 11
<211> 18
<212> PRT

<213> Artificial Sequence

<220>
<223> synthetic peptide

<220>
<221> MISC_FEATURE
<222> (2)..(2)
<223> Xaa can be aspartic acid (D), cysteine (C), moiety R5, moiety S5, moiety R8, glutamic acid (E), lysine (K), moiety Pra or moiety Azk

<220>
<221> MISC_FEATURE
<222> (3)..(3)
<223> Xaa can be cysteine (C) or alanine (A)

<220>
<221> MISC_FEATURE
<222> (6)..(6)
<223> Xaa can be isoleucine (I), cysteine (C), moiety R5, moiety S5, moiety R8, glutamic acid (E), lysine (K), moiety Pra or moiety Azk

<220>
<221> MISC_FEATURE
<222> (10)..(10)
<223> Xaa can be methionine (M), cysteine (C), moiety R5, moiety S5, moiety R8, glutamic acid (E), lysine (K), moiety Pra or moiety Azk

<220>
<221> MISC_FEATURE
<222> (13)..(13)
<223> Xaa can be arginine (R), cysteine (C), moiety R5, moiety S5, moiety R8, glutamic acid (E), lysine (K), moiety Pra or moiety Azk

<220>
<221> MISC_FEATURE
<222> (14)..(14)
<223> Xaa can be glutamine (Q), cysteine (C), moiety R5, moiety S5, moiety R8, glutamic acid (E), lysine (K), moiety Pra or moiety Azk

<220>
<221> MISC_FEATURE
<222> (15)..(15)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (16)..(16)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (17)..(17)

<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (18)..(18)
<223> Xaa can be leucine (L), L-NH2, isoleucine (I), alanine analogue of formula (Ia) or (I'a), phenylalanine (F) or tryptophan (W)

<400> 11

Asn Xaa Xaa Arg Asp Xaa Ile Gln Arg Xaa His Leu Xaa Xaa Xaa Xaa
1 5 10 15

Xaa Xaa

<210> 12
<211> 19
<212> PRT
<213> Artificial Sequence

<220>
<223> synthetic peptide

<220>
<221> MISC_FEATURE
<222> (3)..(3)
<223> Xaa can be aspartic acid (D), cysteine (C), moiety R5, moiety S5, moiety R8, glutamic acid (E), lysine (K), moiety Pra or moiety Azk

<220>
<221> MISC_FEATURE
<222> (4)..(4)
<223> Xaa can be cysteine (C) or alanine (A)

<220>
<221> MISC_FEATURE
<222> (7)..(7)
<223> Xaa can be isoleucine (I), cysteine (C), moiety R5, moiety S5, moiety R8, glutamic acid (E), lysine (K), moiety Pra or moiety Azk

<220>
<221> MISC_FEATURE
<222> (11)..(11)
<223> Xaa can be methionine (M), cysteine (C), moiety R5, moiety S5, moiety R8, glutamic acid (E), lysine (K), moiety Pra or moiety Azk

<220>
<221> MISC_FEATURE
<222> (14)..(14)
<223> Xaa can be arginine (R), cysteine (C), moiety R5, moiety S5, moiety R8, glutamic acid (E), lysine (K), moiety Pra or moiety Azk

<220>

<221> MISC_FEATURE
<222> (15)..(15)
<223> Xaa can be glutamine (Q), cysteine (C), moiety R5, moiety S5, moiety R8, glutamic acid (E), lysine (K), moiety Pra or moiety Azk

<220>
<221> MISC_FEATURE
<222> (16)..(16)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (17)..(17)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (18)..(18)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (19)..(19)
<223> Xaa can be leucine (L), L-NH2, isoleucine (I), alanine analogue of formula (Ia) or (I'a), phenylalanine (F) or tryptophan (W)

<400> 12

```
Phe Asn Xaa Xaa Arg Asp Xaa Ile Gln Arg Xaa His Leu Xaa Xaa Xaa
1               5                   10                  15


Xaa Xaa Xaa
```

<210> 13
<211> 20
<212> PRT
<213> Artificial Sequence

<220>
<223> synthetic peptide

<220>
<221> MISC_FEATURE
<222> (4)..(4)
<223> Xaa can be aspartic acid (D), cysteine (C), moiety R5, moiety S5, moiety R8, glutamic acid (E), lysine (K), moiety Pra or moiety Azk

<220>
<221> MISC_FEATURE
<222> (5)..(5)
<223> Xaa can be cysteine (C) or alanine (A)

<220>
<221> MISC_FEATURE
<222> (8)..(8)
<223> Xaa can be isoleucine (I), cysteine (C), moiety R5, moiety S5, moiety R8, glutamic acid (E), lysine (K), moiety Pra or moiety Azk

<220>
<221> MISC_FEATURE
<222> (12)..(12)
<223> Xaa can be methionine (M), cysteine (C), moiety R5, moiety S5, moiety R8, glutamic acid (E), lysine (K), moiety Pra or moiety Azk

<220>
<221> MISC_FEATURE
<222> (15)..(15)
<223> Xaa can be arginine (R), cysteine (C), moiety R5, moiety S5, moiety R8, glutamic acid (E), lysine (K), moiety Pra or moiety Azk

<220>
<221> MISC_FEATURE
<222> (16)..(16)
<223> Xaa can be glutamine (Q), cysteine (C), moiety R5, moiety S5, moiety R8, glutamic acid (E), lysine (K), moiety Pra or moiety Azk

<220>
<221> MISC_FEATURE
<222> (17)..(17)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (18)..(18)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (19)..(19)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (20)..(20)
<223> Xaa can be leucine (L), L-NH2, isoleucine (I), alanine analogue of formula (Ia) or (I'a), phenylalanine (F) or tryptophan (W)

<400> 13

```
Ile Phe Asn Xaa Xaa Arg Asp Xaa Ile Gln Arg Xaa His Leu Xaa Xaa
1               5                   10                  15

Xaa Xaa Xaa Xaa
```

20

<210> 14
<211> 21
<212> PRT
<213> Artificial Sequence

<220>
<223> synthetic peptide

<220>
<221> MISC_FEATURE
<222> (5)..(5)
<223> Xaa can be aspartic acid (D), cysteine (C), moiety R5, moiety S5, moiety R8, glutamic acid (E), lysine (K), moiety Pra or moiety Azk

<220>
<221> MISC_FEATURE
<222> (6)..(6)
<223> Xaa can be cysteine (C) or alanine (A)

<220>
<221> MISC_FEATURE
<222> (9)..(9)
<223> Xaa can be isoleucine (I), cysteine (C), moiety R5, moiety S5, moiety R8, glutamic acid (E), lysine (K), moiety Pra or moiety Azk

<220>
<221> MISC_FEATURE
<222> (13)..(13)
<223> Xaa can be methionine (M), cysteine (C), moiety R5, moiety S5, moiety R8, glutamic acid (E), lysine (K), moiety Pra or moiety Azk

<220>
<221> MISC_FEATURE
<222> (16)..(16)
<223> Xaa can be arginine (R), cysteine (C), moiety R5, moiety S5, moiety R8, glutamic acid (E), lysine (K), moiety Pra or moiety Azk

<220>
<221> MISC_FEATURE
<222> (17)..(17)
<223> Xaa can be glutamine (Q), cysteine (C), moiety R5, moiety S5, moiety R8, glutamic acid (E), lysine (K), moiety Pra or moiety Azk

<220>
<221> MISC_FEATURE
<222> (18)..(18)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (19)..(19)
<223> Xaa can be glutamic acid (E), glutamine (Q), homoglutamic acid

(hGlu), aspartic Acid (D), alanine (A), alanine analogue of formula (Ib), phenylalanine (F) or tryptophan (W)

<220>
<221> MISC_FEATURE
<222> (20)..(20)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (21)..(21)
<223> Xaa can be leucine (L), L-NH2, isoleucine (I), alanine analogue of formula (Ia) or (I'a), phenylalanine (F) or tryptophan (W)

<400> 14

Arg Ile Phe Asn Xaa Xaa Arg Asp Xaa Ile Gln Arg Xaa His Leu Xaa
1 5 10 15

Xaa Xaa Xaa Xaa Xaa
20

<210> 15
<211> 22
<212> PRT
<213> Artificial Sequence

<220>
<223> synthetic peptide

<220>
<221> MISC_FEATURE
<222> (6)..(6)
<223> Xaa can be aspartic acid (D), cysteine (C), moiety R5, moiety S5, moiety R8, glutamic acid (E), lysine (K), moiety Pra or moiety Azk

<220>
<221> MISC_FEATURE
<222> (7)..(7)
<223> Xaa can be cysteine (C) or alanine (A)

<220>
<221> MISC_FEATURE
<222> (10)..(10)
<223> Xaa can be isoleucine (I), cysteine (C), moiety R5, moiety S5, moiety R8, glutamic acid (E), lysine (K), moiety Pra or moiety Azk

<220>
<221> MISC_FEATURE
<222> (14)..(14)
<223> Xaa can be methionine (M), cysteine (C), moiety R5, moiety S5, moiety R8, glutamic acid (E), lysine (K), moiety Pra or moiety Azk

<220>
<221> MISC_FEATURE

<222> (17)..(17)
<223> Xaa can be arginine (R), cysteine (C), moiety R5, moiety S5, moiety R8, glutamic acid (E), lysine (K), moiety Pra or moiety Azk

<220>
<221> MISC_FEATURE
<222> (18)..(18)
<223> Xaa can be glutamine (Q), cysteine (C), moiety R5, moiety S5, moiety R8, glutamic acid (E), lysine (K), moiety Pra or moiety Azk

<220>
<221> MISC_FEATURE
<222> (19)..(19)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (20)..(20)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (21)..(21)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (22)..(22)
<223> Xaa can be leucine (L), L-NH2, isoleucine (I), alanine analogue of formula (Ia) or (I'a), phenylalanine (F) or tryptophan (W)

<400> 15

```
Arg Arg Ile Phe Asn Xaa Xaa Arg Asp Xaa Ile Gln Arg Xaa His Leu
1               5                   10                  15


Xaa Xaa Xaa Xaa Xaa Xaa
            20
```

<210> 16
<211> 23
<212> PRT
<213> Artificial Sequence

<220>
<223> synthetic peptide

<220>
<221> MISC_FEATURE
<222> (7)..(7)
<223> Xaa can be aspartic acid (D), cysteine (C), moiety R5, moiety S5, moiety R8, glutamic acid (E), lysine (K), moiety Pra or moiety

Azk

<220>
<221> MISC_FEATURE
<222> (8)..(8)
<223> Xaa can be cysteine (C) or alanine (A)

<220>
<221> MISC_FEATURE
<222> (11)..(11)
<223> Xaa can be isoleucine (I), cysteine (C), moiety R5, moiety S5, moiety R8, glutamic acid (E), lysine (K), moiety Pra or moiety Azk

<220>
<221> MISC_FEATURE
<222> (15)..(15)
<223> Xaa can be methionine (M), cysteine (C), moiety R5, moiety S5, moiety R8, glutamic acid (E), lysine (K), moiety Pra or moiety Azk

<220>
<221> MISC_FEATURE
<222> (18)..(18)
<223> Xaa can be arginine (R), cysteine (C), moiety R5, moiety S5, moiety R8, glutamic acid (E), lysine (K), moiety Pra or moiety Azk

<220>
<221> MISC_FEATURE
<222> (19)..(19)
<223> Xaa can be glutamine (Q), cysteine (C), moiety R5, moiety S5, moiety R8, glutamic acid (E), lysine (K), moiety Pra or moiety Azk

<220>
<221> MISC_FEATURE
<222> (20)..(20)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (21)..(21)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (22)..(22)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (23)..(23)
<223> Xaa can be leucine (L), L-NH2, isoleucine (I), alanine analogue of formula (Ia) or (I'a), phenylalanine (F) or tryptophan (W)

<400> 16

```
Ala Arg Arg Ile Phe Asn Xaa Xaa Arg Asp Xaa Ile Gln Arg Xaa His
1               5                   10                  15


Leu Xaa Xaa Xaa Xaa Xaa Xaa
            20
```

<210> 17
<211> 24
<212> PRT
<213> Artificial Sequence

<220>
<223> synthetic peptide

<220>
<221> MISC_FEATURE
<222> (8)..(8)
<223> Xaa can be aspartic acid (D), cysteine (C), moiety R5, moiety S5, moiety R8, glutamic acid (E), lysine (K), moiety Pra or moiety Azk

<220>
<221> MISC_FEATURE
<222> (9)..(9)
<223> Xaa can be cysteine (C) or alanine (A)

<220>
<221> MISC_FEATURE
<222> (12)..(12)
<223> Xaa can be isoleucine (I), cysteine (C), moiety R5, moiety S5, moiety R8, glutamic acid (E), lysine (K), moiety Pra or moiety Azk

<220>
<221> MISC_FEATURE
<222> (16)..(16)
<223> Xaa can be methionine (M), cysteine (C), moiety R5, moiety S5, moiety R8, glutamic acid (E), lysine (K), moiety Pra or moiety Azk

<220>
<221> MISC_FEATURE
<222> (19)..(19)
<223> Xaa can be arginine (R), cysteine (C), moiety R5, moiety S5, moiety R8, glutamic acid (E), lysine (K), moiety Pra or moiety Azk

<220>
<221> MISC_FEATURE
<222> (20)..(20)
<223> Xaa can be glutamine (Q), cysteine (C), moiety R5, moiety S5, moiety R8, glutamic acid (E), lysine (K), moiety Pra or moiety Azk

<220>
<221> MISC_FEATURE
<222> (21)..(21)
<223> Xaa can be tyrosine (Y), 2-naphthalanine (2-Nal), 1-naphthalanine (1-Nal), alanine analogue of formula (Ib), phenylalanine (F) or

tryptophan (W)

<220>
<221> MISC_FEATURE
<222> (22)..(22)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (23)..(23)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (24)..(24)
<223> Xaa can be leucine (L), L-NH2, isoleucine (I), alanine analogue of formula (Ia) or (I'a), phenylalanine (F) or tryptophan (W)

<400> 17

```
Asn Ala Arg Arg Ile Phe Asn Xaa Xaa Arg Asp Xaa Ile Gln Arg Xaa
1               5                   10                  15

His Leu Xaa Xaa Xaa Xaa Xaa Xaa
            20
```

<210> 18
<211> 4
<212> PRT
<213> Artificial Sequence

<220>
<223> synthetic peptide

<220>
<221> MISC_FEATURE
<222> (1)..(1)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (2)..(2)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (3)..(3)
<223> Xaa can be leucine (L), isoleucine (I), tyrosine (Y), histidine (H), alanine analogue of formula (Ib), phenylalanine (F) or tryptophan (W)

<220>

<221> MISC_FEATURE
<222> (4)..(4)
<223> Xaa can be leucine (L), L-NH2, isoleucine (I), alanine analogue of formula (Ia) or (I'a), phenylalanine (F) or tryptophan (W)

<400> 18

Xaa Xaa Xaa Xaa
1

<210> 19
<211> 4
<212> PRT
<213> Artificial Sequence

<220>
<223> synthetic peptide

<220>
<221> MISC_FEATURE
<222> (3)..(3)
<223> Xaa can be an alanine analogue of formula (Ib), phenylalanine (F) or tryptophan (W)

<400> 19

Tyr Glu Xaa Leu
1

<210> 20
<211> 4
<212> PRT
<213> Artificial Sequence

<220>
<223> synthetic peptide

<220>
<221> MISC_FEATURE
<222> (4)..(4)
<223> Xaa can be alanine analogue of formula (Ia) or (I'a), phenylalanine (F) or tryptophan (W)

<400> 20

Tyr Glu Leu Xaa
1

<210> 21
<211> 4
<212> PRT
<213> Artificial Sequence

<220>
<223> synthetic peptide

<220>

<221> MISC_FEATURE
<222> (1)..(1)
<223> Xaa can be an alanine analogue of formula (Ib), phenylalanine (F) or tryptophan (W)

<400> 21

Xaa Glu Leu Leu
1

<210> 22
<211> 4
<212> PRT
<213> Artificial Sequence

<220>
<223> synthetic peptide

<220>
<221> MISC_FEATURE
<222> (2)..(2)
<223> Xaa can be an alanine analogue of formula (Ib), phenylalanine (F) or tryptophan (W)

<400> 22

Tyr Xaa Leu Leu
1

<210> 23
<211> 4
<212> PRT
<213> Artificial Sequence

<220>
<223> synthetic peptide

<400> 23

Tyr Glu Leu Leu
1

<210> 24
<211> 4
<212> PRT
<213> Artificial Sequence

<220>
<223> synthetic peptide

<220>
<221> MISC_FEATURE
<222> (3)..(3)
<223> Xaa can be an alanine analogue of formula (Ib), phenylalanine (F) or tryptophan (W)

<220>
<221> MISC_FEATURE

<222> (4)..(4)
<223> Xaa can be alanine analogue of formula (Ia) or (I'a), phenylalanine (F) or tryptophan (W)

<400> 24

Tyr Glu Xaa Xaa
1

<210> 25
<211> 4
<212> PRT
<213> Artificial Sequence

<220>
<223> synthetic peptide

<220>
<221> MISC_FEATURE
<222> (2)..(2)
<223> Xaa can be an alanine analogue of formula (Ib), phenylalanine (F) or tryptophan (W)

<220>
<221> MISC_FEATURE
<222> (3)..(3)
<223> Xaa can be an alanine analogue of formula (Ib), phenylalanine (F) or tryptophan (W)

<400> 25

Tyr Xaa Xaa Leu
1

<210> 26
<211> 4
<212> PRT
<213> Artificial Sequence

<220>
<223> synthetic peptide

<220>
<221> MISC_FEATURE
<222> (1)..(1)
<223> Xaa can be an alanine analogue of formula (Ib), phenylalanine (F) or tryptophan (W)

<220>
<221> MISC_FEATURE
<222> (2)..(2)
<223> Xaa can be an alanine analogue of formula (Ib), phenylalanine (F) or tryptophan (W)

<400> 26

Xaa Xaa Leu Leu
1

<210> 27
<211> 4
<212> PRT
<213> Artificial Sequence

<220>
<223> synthetic peptide

<220>
<221> MISC_FEATURE
<222> (1)..(1)
<223> Xaa can be an alanine analogue of formula (Ib), phenylalanine (F) or tryptophan (W)

<220>
<221> MISC_FEATURE
<222> (3)..(3)
<223> Xaa can be an alanine analogue of formula (Ib), phenylalanine (F) or tryptophan (W)

<400> 27

Xaa Glu Xaa Leu
1

<210> 28
<211> 4
<212> PRT
<213> Artificial Sequence

<220>
<223> synthetic peptide

<220>
<221> MISC_FEATURE
<222> (1)..(1)
<223> Xaa can be an alanine analogue of formula (Ib), phenylalanine (F) or tryptophan (W)

<220>
<221> MISC_FEATURE
<222> (4)..(4)
<223> Xaa can be alanine analogue of formula (Ia) or (I’a), phenylalanine (F) or tryptophan (W)

<400> 28

Xaa Glu Leu Xaa
1

<210> 29
<211> 4
<212> PRT
<213> Artificial Sequence

<220>
<223> synthetic peptide

<220>
<221> MISC_FEATURE
<222> (2)..(2)
<223> Xaa can be an alanine analogue of formula (Ib), phenylalanine (F) or tryptophan (W)

<220>
<221> MISC_FEATURE
<222> (4)..(4)
<223> Xaa can be alanine analogue of formula (Ia) or (I'a), phenylalanine (F) or tryptophan (W)

<400> 29

Tyr Xaa Leu Xaa
1

<210> 30
<211> 4
<212> PRT
<213> Artificial Sequence

<220>
<223> synthetic peptide

<220>
<221> MISC_FEATURE
<222> (3)..(3)
<223> wherein Xaa is cyclohexylalanine

<400> 30

Tyr Glu Xaa Leu
1

<210> 31
<211> 4
<212> PRT
<213> Artificial Sequence

<220>
<223> synthetic peptide

<400> 31

Tyr Glu Phe Leu
1

<210> 32
<211> 4
<212> PRT
<213> Artificial Sequence

<220>
<223> synthetic peptide

<400> 32

Tyr Glu Trp Leu
1

<210> 33
<211> 4
<212> PRT
<213> Artificial Sequence

<220>
<223> synthetic peptide

<220>
<221> MISC_FEATURE
<222> (4)..(4)
<223> wherein Xaa is cyclohexylalanine

<400> 33

Tyr Glu Leu Xaa
1

<210> 34
<211> 4
<212> PRT
<213> Artificial Sequence

<220>
<223> synthetic peptide

<400> 34

Tyr Glu Leu Phe
1

<210> 35
<211> 4
<212> PRT
<213> Artificial Sequence

<220>
<223> synthetic peptide

<400> 35

Tyr Glu Leu Trp
1

<210> 36
<211> 9
<212> PRT
<213> Artificial Sequence

<220>
<223> synthetic peptide

<220>
<221> MISC_FEATURE

<222> (1)..(1)
<223> Xaa can be aspartic acid (D), cysteine (C), moiety R5, moiety S5, moiety R8, glutamic acid (E), lysine (K), moiety Pra or moiety Azk

<220>
<221> MISC_FEATURE
<222> (2)..(2)
<223> Xaa can be cysteine (C) or alanine (A)

<220>
<221> MISC_FEATURE
<222> (5)..(5)
<223> Xaa can be isoleucine (I), cysteine (C), moiety R5, moiety S5, moiety R8, glutamic acid (E), lysine (K), moiety Pra or moiety Azk

<220>
<221> MISC_FEATURE
<222> (9)..(9)
<223> Xaa can be methionine (M), cysteine (C), moiety R5, moiety S5, moiety R8, glutamic acid (E), lysine (K), moiety Pra or moiety Azk

<400> 36

Xaa Xaa Arg Asp Xaa Ile Gln Arg Xaa
1               5

<210> 37
<211> 9
<212> PRT
<213> Artificial Sequence

<220>
<223> synthetic peptide

<220>
<221> MISC_FEATURE
<222> (1)..(1)
<223> Xaa is moiety Pra

<220>
<221> MISC_FEATURE
<222> (2)..(2)
<223> Xaa can be cysteine (C) or alanine (A)

<220>
<221> MISC_FEATURE
<222> (5)..(5)
<223> Xaa is moiety Azk

<400> 37

Xaa Xaa Arg Asp Xaa Ile Gln Arg Met
1               5

<210> 38
<211> 9
<212> PRT

<213> Artificial Sequence

<220>
<223> synthetic peptide

<220>
<221> MISC_FEATURE
<222> (1)..(1)
<223> Xaa is moiety Azk

<220>
<221> MISC_FEATURE
<222> (2)..(2)
<223> Xaa can be cysteine (C) or alanine (A)

<220>
<221> MISC_FEATURE
<222> (5)..(5)
<223> Xaa is moiety Pra

<400> 38

Xaa Xaa Arg Asp Xaa Ile Gln Arg Met
1               5

<210> 39
<211> 9
<212> PRT
<213> Artificial Sequence

<220>
<223> synthetic peptide

<220>
<221> MISC_FEATURE
<222> (2)..(2)
<223> Xaa can be cysteine (C) or alanine (A)

<400> 39

Glu Xaa Arg Asp Lys Ile Gln Arg Met
1               5

<210> 40
<211> 9
<212> PRT
<213> Artificial Sequence

<220>
<223> synthetic peptide

<220>
<221> MISC_FEATURE
<222> (2)..(2)
<223> Xaa can be cysteine (C) or alanine (A)

<400> 40

Lys Xaa Arg Asp Glu Ile Gln Arg Met
1               5

<210>  41
<211>  9
<212>  PRT
<213>  Artificial Sequence

<220>
<223>  synthetic peptide

<220>
<221>  MISC_FEATURE
<222>  (2)..(2)
<223>  Xaa can be cysteine (C) or alanine (A)

<220>
<221>  MISC_FEATURE
<222>  (5)..(5)
<223>  Xaa is moiety Pra

<220>
<221>  MISC_FEATURE
<222>  (9)..(9)
<223>  Xaa is moiety Azk

<400>  41

Asp Xaa Arg Asp Xaa Ile Gln Arg Xaa
1               5

<210>  42
<211>  9
<212>  PRT
<213>  Artificial Sequence

<220>
<223>  synthetic peptide

<220>
<221>  MISC_FEATURE
<222>  (2)..(2)
<223>  Xaa can be cysteine (C) or alanine (A

<220>
<221>  MISC_FEATURE
<222>  (5)..(5)
<223>  Xaa is moiety Azk

<220>
<221>  MISC_FEATURE
<222>  (9)..(9)
<223>  Xaa is moiety Pra

<400>  42

Asp Xaa Arg Asp Xaa Ile Gln Arg Xaa
1               5

<210> 43
<211> 19
<212> PRT
<213> Artificial Sequence

<220>
<223> synthetic peptide

<220>
<221> MISC_FEATURE
<222> (3)..(3)
<223> Xaa is moiety Pra

<220>
<221> MISC_FEATURE
<222> (4)..(4)
<223> Xaa can be cysteine (C) or alanine (A)

<220>
<221> MISC_FEATURE
<222> (7)..(7)
<223> Xaa is moiety Azk

<220>
<221> MISC_FEATURE
<222> (16)..(16)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (17)..(17)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (18)..(18)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (19)..(19)
<223> Xaa can be leucine (L), L-NH2, isoleucine (I), alanine analogue of formula (Ia) or (I'a), phenylalanine (F) or tryptophan (W)

<400> 43

```
Phe Asn Xaa Xaa Arg Asp Xaa Ile Gln Arg Met His Leu Arg Gln Xaa
1               5                   10                  15

Xaa Xaa Xaa
```

<210> 44

<211> 19
<212> PRT
<213> Artificial Sequence

<220>
<223> synthetic peptide

<220>
<221> MISC_FEATURE
<222> (3)..(3)
<223> Xaa is moiety Azk

<220>
<221> MISC_FEATURE
<222> (4)..(4)
<223> Xaa can be cysteine (C) or alanine (A)

<220>
<221> MISC_FEATURE
<222> (7)..(7)
<223> Xaa is moiety Pra

<220>
<221> MISC_FEATURE
<222> (16)..(16)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (17)..(17)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (18)..(18)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (19)..(19)
<223> Xaa can be leucine (L), L-NH2, isoleucine (I), alanine analogue of formula (Ia) or (I’a), phenylalanine (F) or tryptophan (W)

<400> 44

Phe Asn Xaa Xaa Arg Asp Xaa Ile Gln Arg Met His Leu Arg Gln Xaa
1 5 10 15

Xaa Xaa Xaa

<210> 45
<211> 19
<212> PRT

<213> Artificial Sequence

<220>
<223> synthetic peptide

<220>
<221> MISC_FEATURE
<222> (4)..(4)
<223> Xaa can be cysteine (C) or alanine (A)

<220>
<221> MISC_FEATURE
<222> (16)..(16)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (17)..(17)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (18)..(18)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (19)..(19)
<223> Xaa can be leucine (L), L-NH2, isoleucine (I), alanine analogue of formula (Ia) or (I'a), phenylalanine (F) or tryptophan (W)

<400> 45

Phe Asn Glu Xaa Arg Asp Lys Ile Gln Arg Met His Leu Arg Gln Xaa
1               5                   10                  15

Xaa Xaa Xaa

<210> 46
<211> 19
<212> PRT
<213> Artificial Sequence

<220>
<223> synthetic peptide

<220>
<221> MISC_FEATURE
<222> (4)..(4)
<223> Xaa can be cysteine (C) or alanine (A)

<220>

<221> MISC_FEATURE
<222> (16)..(16)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (17)..(17)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (18)..(18)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (19)..(19)
<223> Xaa can be leucine (L), L-NH2, isoleucine (I), alanine analogue of formula (Ia) or (I'a), phenylalanine (F) or tryptophan (W)

<400> 46

Phe Asn Lys Xaa Arg Asp Glu Ile Gln Arg Met His Leu Arg Gln Xaa
1 5 10 15

Xaa Xaa Xaa

<210> 47
<211> 19
<212> PRT
<213> Artificial Sequence

<220>
<223> synthetic peptide

<220>
<221> MISC_FEATURE
<222> (4)..(4)
<223> Xaa can be cysteine (C) or alanine (A)

<220>
<221> MISC_FEATURE
<222> (7)..(7)
<223> Xaa is moiety Pra

<220>
<221> MISC_FEATURE
<222> (11)..(11)
<223> Xaa is moiety Azk

<220>
<221> MISC_FEATURE
<222> (16)..(16)

<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (17)..(17)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (18)..(18)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (19)..(19)
<223> Xaa can be leucine (L), L-NH2, isoleucine (I), alanine analogue of formula (Ia) or (I'a), phenylalanine (F) or tryptophan (W)

<400> 47

```
Phe Asn Asp Xaa Arg Asp Xaa Ile Gln Arg Xaa His Leu Arg Gln Xaa
1               5                   10                  15


Xaa Xaa Xaa
```

<210> 48
<211> 19
<212> PRT
<213> Artificial Sequence

<220>
<223> synthetic peptide

<220>
<221> MISC_FEATURE
<222> (4)..(4)
<223> Xaa can be cysteine (C) or alanine (A)

<220>
<221> MISC_FEATURE
<222> (7)..(7)
<223> Xaa is moiety Azk

<220>
<221> MISC_FEATURE
<222> (11)..(11)
<223> Xaa is moiety Pra

<220>
<221> MISC_FEATURE
<222> (16)..(16)
<223> Xaa can be tyrosine (Y), 2-naphthalanine (2-Nal), 1-naphthalanine (1-Nal), alanine analogue of formula (Ib), phenylalanine (F) or

tryptophan (W)

<220>
<221> MISC_FEATURE
<222> (17)..(17)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (18)..(18)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (19)..(19)
<223> Xaa can be leucine (L), L-NH2, isoleucine (I), alanine analogue of formula (Ia) or (I'a), phenylalanine (F) or tryptophan (W)

<400> 48

Phe Asn Asp Xaa Arg Asp Xaa Ile Gln Arg Xaa His Leu Arg Gln Xaa
1               5                   10                  15

Xaa Xaa Xaa

<210> 49
<211> 9
<212> PRT
<213> Artificial Sequence

<220>
<223> synthetic peptide

<220>
<221> MISC_FEATURE
<222> (2)..(2)
<223> Xaa can be cysteine (C) or alanine (A)

<400> 49

Asp Xaa Arg Asp Ile Ile Gln Arg Met
1               5

<210> 50
<211> 17
<212> PRT
<213> Artificial Sequence

<220>
<223> synthetic peptide

<220>
<221> MISC_FEATURE

<222> (2)..(2)
<223> Xaa can be cysteine (C) or alanine (A)

<220>
<221> MISC_FEATURE
<222> (14)..(14)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (15)..(15)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (16)..(16)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (17)..(17)
<223> Xaa can be leucine (L), L-NH2, isoleucine (I), alanine analogue of formula (Ia) or (I'a), phenylalanine (F) or tryptophan (W)

<400> 50

```
Asp Xaa Arg Asp Ile Ile Gln Arg Met His Leu Arg Gln Xaa Xaa Xaa
1               5                   10                  15


Xaa
```

<210> 51
<211> 19
<212> PRT
<213> Artificial Sequence

<220>
<223> synthetic peptide

<220>
<221> MISC_FEATURE
<222> (4)..(4)
<223> Xaa can be cysteine (C) or alanine (A)

<220>
<221> MISC_FEATURE
<222> (16)..(16)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE

<222> (17)..(17)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (18)..(18)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (19)..(19)
<223> Xaa can be leucine (L), L-NH2, isoleucine (I), alanine analogue of formula (Ia) or (I'a), phenylalanine (F) or tryptophan (W)

<400> 51

Phe Asn Asp Xaa Arg Asp Ile Ile Gln Arg Met His Leu Arg Gln Xaa
1 5 10 15

Xaa Xaa Xaa

<210> 52
<211> 24
<212> PRT
<213> Artificial Sequence

<220>
<223> synthetic peptide

<220>
<221> MISC_FEATURE
<222> (9)..(9)
<223> Xaa can be cysteine (C) or alanine (A)

<220>
<221> MISC_FEATURE
<222> (21)..(21)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (22)..(22)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (23)..(23)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (24)..(24)
<223> Xaa can be leucine (L), L-NH2, isoleucine (I), alanine analogue of formula (Ia) or (I'a), phenylalanine (F) or tryptophan (W)

<400> 52

Asn Ala Arg Arg Ile Phe Asn Asp Xaa Arg Asp Ile Ile Gln Arg Met
1               5                   10                  15

His Leu Arg Gln Xaa Xaa Xaa Xaa
            20

<210> 53
<211> 19
<212> PRT
<213> Artificial Sequence

<220>
<223> synthetic peptide

<220>
<221> MOD_RES
<222> (1)..(1)
<223> ACETYLATION

<400> 53

Phe Asn Asp Cys Arg Asp Ile Ile Gln Arg Met His Leu Arg Gln Tyr
1               5                   10                  15

Glu Leu Leu

<210> 54
<211> 19
<212> PRT
<213> Artificial Sequence

<220>
<223> synthetic peptide

<220>
<221> MOD_RES
<222> (1)..(1)
<223> ACETYLATION

<400> 54

Phe Asn Asp Cys Arg Asp Glu Ile Gln Arg Lys His Leu Arg Gln Tyr
1               5                   10                  15

Glu Leu Leu

<210> 55
<211> 19
<212> PRT
<213> Artificial Sequence

<220>
<223> synthetic peptide

<400> 55

Phe Asn Asp Cys Arg Asp Glu Ile Gln Arg Lys His Leu Arg Gln Tyr
1 5 10 15

Glu Leu Leu

<210> 56
<211> 19
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (7)..(7)
<223> Xaa is moiety S5

<220>
<221> MISC_FEATURE
<222> (11)..(11)
<223> Xaa is moiety S5

<400> 56

Phe Asn Asp Cys Arg Asp Xaa Ile Gln Arg Xaa His Leu Arg Gln Tyr
1 5 10 15

Glu Leu Leu

<210> 57
<211> 19
<212> PRT
<213> Artificial Sequence

<220>
<223> synthetic peptide

<220>

<221> MISC_FEATURE
<222> (7)..(7)
<223> Xaa is moiety S5

<220>
<221> MISC_FEATURE
<222> (11)..(11)
<223> Xaa is moiety S5

<400> 57

Phe Asn Asp Cys Arg Asp Xaa Ile Gln Arg Xaa His Leu Arg Gln Tyr
1 5 10 15

Glu Leu Leu

<210> 58
<211> 19
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (7)..(7)
<223> Xaa is moiety Pra

<220>
<221> MISC_FEATURE
<222> (11)..(11)
<223> Xaa is moiety Azk

<400> 58

Phe Asn Asp Cys Arg Asp Xaa Ile Gln Arg Xaa His Leu Arg Gln Tyr
1 5 10 15

Glu Leu Leu

<210> 59
<211> 19
<212> PRT
<213> Artificial Sequence

<220>
<223> synthetic peptide

<220>

<221> MISC_FEATURE
<222> (7)..(7)
<223> Xaa is moiety Pra

<220>
<221> MISC_FEATURE
<222> (11)..(11)
<223> Xaa is moiety Azk

<400> 59

Phe Asn Asp Cys Arg Asp Xaa Ile Gln Arg Xaa His Leu Arg Gln Tyr
1 5 10 15

Glu Leu Leu

<210> 60
<211> 19
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (3)..(3)
<223> Xaa is moiety Pra

<220>
<221> MISC_FEATURE
<222> (7)..(7)
<223> Xaa is moiety Azk

<400> 60

Phe Asn Xaa Cys Arg Asp Xaa Ile Gln Arg Met His Leu Arg Gln Tyr
1 5 10 15

Glu Leu Leu

<210> 61
<211> 19
<212> PRT
<213> Artificial Sequence

<220>
<223> synthetic peptide

<220>

<221> MOD_RES
<222> (1)..(1)
<223> ACETYLATION

<400> 61

Phe Asn Glu Cys Arg Asp Lys Ile Gln Arg Met His Leu Arg Gln Tyr
1 5 10 15

Glu Leu Leu

<210> 62
<211> 19
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (3)..(3)
<223> Xaa is moiety S5

<220>
<221> MISC_FEATURE
<222> (7)..(7)
<223> Xaa is moiety S5

<400> 62

Phe Asn Xaa Cys Arg Asp Xaa Ile Gln Arg Met His Leu Arg Gln Tyr
1 5 10 15

Glu Leu Leu

<210> 63
<211> 19
<212> PRT
<213> Artificial Sequence

<220>
<223> synthetic peptide

<220>
<221> MOD_RES
<222> (1)..(1)
<223> ACETYLATION

<220>

<221> MISC_FEATURE
<222> (11)..(11)
<223> Xaa is moiety R5

<220>
<221> MISC_FEATURE
<222> (14)..(14)
<223> Xaa is moiety S5

<400> 63

Phe Asn Asp Cys Arg Asp Ile Ile Gln Arg Xaa His Leu Xaa Gln Tyr
1 5 10 15

Glu Leu Leu

<210> 64
<211> 19
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (7)..(7)
<223> Xaa is moiety R8

<220>
<221> MISC_FEATURE
<222> (14)..(14)
<223> Xaa is moiety S5

<400> 64

Phe Asn Asp Cys Arg Asp Xaa Ile Gln Arg Met His Leu Xaa Gln Tyr
1 5 10 15

Glu Leu Leu

<210> 65
<211> 15
<212> PRT
<213> Artificial Sequence

<220>
<223> synthetic peptide

<220>

<221> MOD_RES
<222> (1)..(1)
<223> ACETYLATION

<400> 65

Arg Asp Ile Ile Gln Arg Met His Leu Arg Gln Tyr Glu Leu Leu
1 5 10 15

<210> 66
<211> 15
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (7)..(7)
<223> Xaa is moiety Azk

<220>
<221> MISC_FEATURE
<222> (11)..(11)
<223> Xaa is moiety Pra

<400> 66

Arg Asp Ile Ile Gln Arg Xaa His Leu Arg Xaa Tyr Glu Leu Leu
1 5 10 15

<210> 67
<211> 11
<212> PRT
<213> Artificial Sequence

<220>
<223> synthetic peptide

<220>
<221> MOD_RES
<222> (1)..(1)
<223> ACETYLATION

<400> 67

Gln Arg Met His Leu Arg Gln Tyr Glu Leu Leu
1 5 10

<210> 68
<211> 19
<212> PRT

<213> Artificial Sequence

<220>
<223> synthetic peptide

<220>
<221> MOD_RES
<222> (1)..(1)
<223> ACETYLATION

<220>
<221> MISC_FEATURE
<222> (3)..(3)
<223> Xaa is moiety Pra

<220>
<221> MISC_FEATURE
<222> (7)..(7)
<223> Xaa is moiety Azk

<400> 68

Phe Asn Xaa Cys Arg Asp Xaa Ile Gln Arg Met His Leu Arg Gln Tyr
1 5 10 15

Gln Leu Leu

<210> 69
<211> 19
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (3)..(3)
<223> Xaa is moiety Pra

<220>
<221> MISC_FEATURE
<222> (7)..(7)
<223> Xaa is moiety Azk

<400> 69

Phe Asn Xaa Cys Arg Asp Xaa Ile Gln Arg Met His Leu Arg Gln Trp
1 5 10 15

Glu Leu Leu

<210> 70
<211> 19
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (3)..(3)
<223> Xaa is moiety Pra

<220>
<221> MISC_FEATURE
<222> (7)..(7)
<223> Xaa is moiety Azk

<400> 70

Phe Asn Xaa Cys Arg Asp Xaa Ile Gln Arg Met His Leu Arg Gln Tyr
1 5 10 15

Glu Ile Leu

<210> 71
<211> 19
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (3)..(3)
<223> Xaa is moiety Pra

<220>
<221> MISC_FEATURE
<222> (7)..(7)
<223> Xaa is moiety Azk

<400> 71

Phe Asn Xaa Cys Arg Asp Xaa Ile Gln Arg Met His Leu Arg Gln Tyr

1 5 10 15

Glu Leu Ile

<210> 72
<211> 19
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (3)..(3)
<223> Xaa is moiety Azk

<220>
<221> MISC_FEATURE
<222> (7)..(7)
<223> Xaa is moiety Pra

<400> 72

Phe Asn Xaa Cys Arg Asp Xaa Ile Gln Arg Met His Leu Arg Gln Tyr
1 5 10 15

Glu Leu Leu

<210> 73
<211> 19
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (3)..(3)
<223> Xaa is moiety Pra

<220>
<221> MISC_FEATURE
<222> (7)..(7)

<223>  Xaa is moiety Azk

<220>
<221>  MOD_RES
<222>  (19)..(19)
<223>  AMIDATION

<400>  73

Phe Asn Xaa Cys Arg Asp Xaa Ile Gln Arg Met His Leu Arg Gln Tyr
1               5                   10                  15

Glu Leu Leu

<210>  74
<211>  19
<212>  PRT
<213>  Artificial Sequence

<220>
<223>  synthetic peptide

<220>
<221>  MOD_RES
<222>  (1)..(1)
<223>  ACETYLATION

<220>
<221>  MISC_FEATURE
<222>  (3)..(3)
<223>  Xaa is moiety Pra

<220>
<221>  MISC_FEATURE
<222>  (7)..(7)
<223>  Xaa is moiety Azk

<220>
<221>  MISC_FEATURE
<222>  (17)..(17)
<223>  Xaa is homoglutamic acid (hGlu)

<400>  74

Phe Asn Xaa Cys Arg Asp Xaa Ile Gln Arg Met His Leu Arg Gln Tyr
1               5                   10                  15

Xaa Leu Leu

<210>  75
<211>  19
<212>  PRT
<213>  Artificial Sequence

<220>
<223>  synthetic peptide

<220>
<221> MOD_RES
<222> (1)..(1)
<223> ACETYLATION

<220>
<221> MISC_FEATURE
<222> (3)..(3)
<223> Xaa is moiety Pra

<220>
<221> MISC_FEATURE
<222> (7)..(7)
<223> Xaa is moiety Azk

<400> 75

Phe Asn Xaa Cys Arg Asn Xaa Ile Gln Arg Met His Leu Arg Gln Tyr
1 5 10 15

Glu Leu Leu

<210> 76
<211> 19
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (3)..(3)
<223> Xaa is moiety Pra

<220>
<221> MISC_FEATURE
<222> (7)..(7)
<223> Xaa is moiety Azk

<400> 76

Phe Asn Xaa Cys Arg Asp Xaa Ile Gln Arg Met His Leu Arg Gln Tyr
1 5 10 15

Asp Leu Leu

<210> 77
<211> 19

<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (3)..(3)
<223> Xaa is moiety Pra

<220>
<221> MISC_FEATURE
<222> (7)..(7)
<223> Xaa is moiety Azk

<220>
<221> MISC_FEATURE
<222> (16)..(16)
<223> Xaa is 1-naphthalanine (1-Nal)

<400> 77

Phe Asn Xaa Cys Arg Asp Xaa Ile Gln Arg Met His Leu Arg Gln Xaa
1               5                   10                  15

Glu Leu Leu

<210> 78
<211> 20
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (4)..(4)
<223> Xaa is moiety Pra

<220>
<221> MISC_FEATURE
<222> (8)..(8)
<223> Xaa is moiety Azk

<400> 78

Arg Phe Asn Xaa Cys Arg Asp Xaa Ile Gln Arg Met His Leu Arg Gln
1               5                   10                  15

Tyr Glu Leu Leu
            20

<210> 79
<211> 20
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (4)..(4)
<223> Xaa is moiety Pra

<220>
<221> MISC_FEATURE
<222> (8)..(8)
<223> Xaa is moiety Azk

<400> 79

Lys Phe Asn Xaa Cys Arg Asp Xaa Ile Gln Arg Met His Leu Arg Gln
1               5                   10                  15

Tyr Glu Leu Leu
            20

<210> 80
<211> 22
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (6)..(6)
<223> Xaa is moiety Pra

<220>
<221> MISC_FEATURE

<222> (10)..(10)
<223> Xaa is moiety Azk

<400> 80

Lys Lys Lys Phe Asn Xaa Cys Arg Asp Xaa Ile Gln Arg Met His Leu
1 5 10 15

Arg Gln Tyr Glu Leu Leu
20

<210> 81
<211> 20
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (4)..(4)
<223> Xaa is moiety Pra

<220>
<221> MISC_FEATURE
<222> (8)..(8)
<223> Xaa is moiety Azk

<400> 81

Lys Phe Asn Xaa Cys Arg Asp Xaa Ile Gln Arg Met His Leu Arg Gln
1 5 10 15

Tyr Glu Leu Ile
20

<210> 82
<211> 22
<212> PRT
<213> Artificial Sequence

<220>
<223> synthetic peptide

<220>
<221> MOD_RES
<222> (1)..(1)
<223> ACETYLATION

<220>
<221> MISC_FEATURE

<222> (6)..(6)
<223> Xaa is moiety Pra

<220>
<221> MISC_FEATURE
<222> (10)..(10)
<223> Xaa is moiety Azk

<400> 82

Lys Lys Lys Phe Asn Xaa Cys Arg Asp Xaa Ile Gln Arg Met His Leu
1               5                   10                  15

Arg Gln Tyr Glu Leu Ile
            20

<210> 83
<211> 20
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (4)..(4)
<223> Xaa is moiety Pra

<220>
<221> MISC_FEATURE
<222> (8)..(8)
<223> Xaa is moiety Azk

<400> 83

Lys Phe Asn Xaa Cys Arg Asp Xaa Ile Gln Arg Met His Leu Arg Gln
1               5                   10                  15

Tyr Glu Ile Leu
            20

<210> 84
<211> 22
<212> PRT
<213> Artificial Sequence

<220>
<223> synthetic peptide

<220>
<221> MOD_RES

<222> (1)..(1)
<223> ACETYLATION

<220>
<221> MISC_FEATURE
<222> (6)..(6)
<223> Xaa is moiety Pra

<220>
<221> MISC_FEATURE
<222> (10)..(10)
<223> Xaa is moiety Azk

<400> 84

```
Lys Lys Lys Phe Asn Xaa Cys Arg Asp Xaa Ile Gln Arg Met His Leu
1               5                   10                  15


Arg Gln Tyr Glu Ile Leu
            20
```

<210> 85
<211> 20
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (4)..(4)
<223> Xaa is moiety Pra

<220>
<221> MISC_FEATURE
<222> (8)..(8)
<223> Xaa is moiety Azk

<220>
<221> MISC_FEATURE
<222> (19)..(19)
<223> Xaa is cyclohexylalanine (Cha)

<400> 85

```
Lys Phe Asn Xaa Cys Arg Asp Xaa Ile Gln Arg Met His Leu Arg Gln
1               5                   10                  15


Tyr Glu Xaa Leu
            20
```

<210> 86

<211> 22
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (6)..(6)
<223> Xaa is moiety Pra

<220>
<221> MISC_FEATURE
<222> (10)..(10)
<223> Xaa is moiety Azk

<220>
<221> MISC_FEATURE
<222> (21)..(21)
<223> Xaa is cyclohexylalanine (Cha)

<400> 86

```
Lys Lys Lys Phe Asn Xaa Cys Arg Asp Xaa Ile Gln Arg Met His Leu
1               5                   10                  15

Arg Gln Tyr Glu Xaa Leu
            20
```

<210> 87
<211> 20
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (4)..(4)
<223> Xaa is moiety Pra

<220>
<221> MISC_FEATURE
<222> (8)..(8)
<223> Xaa is moiety Azk

<220>

<221> MISC_FEATURE
<222> (18)..(18)
<223> Xaa is homoglutamic acid (hGlu)

<400> 87

Lys Phe Asn Xaa Cys Arg Asp Xaa Ile Gln Arg Met His Leu Arg Gln
1 5 10 15

Tyr Xaa Leu Leu
20

<210> 88
<211> 22
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (6)..(6)
<223> Xaa is moiety Pra

<220>
<221> MISC_FEATURE
<222> (10)..(10)
<223> Xaa is moiety Azk

<220>
<221> MISC_FEATURE
<222> (20)..(20)
<223> Xaa is homoglutamic acid (hGlu)

<400> 88

Lys Lys Lys Phe Asn Xaa Cys Arg Asp Xaa Ile Gln Arg Met His Leu
1 5 10 15

Arg Gln Tyr Xaa Leu Leu
20

<210> 89
<211> 20
<212> PRT
<213> Artificial Sequence

<220>
<223> synthetic peptide

<220>

<221> MOD_RES
<222> (1)..(1)
<223> ACETYLATION

<220>
<221> MISC_FEATURE
<222> (4)..(4)
<223> Xaa is moiety Pra

<220>
<221> MISC_FEATURE
<222> (8)..(8)
<223> Xaa is moiety Azk

<400> 89

```
Lys Phe Asn Xaa Cys Arg Asp Xaa Ile Gln Arg Met His Leu Arg Gln
1               5                   10                  15


Tyr Asp Leu Leu
            20
```

<210> 90
<211> 22
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (6)..(6)
<223> Xaa is moiety Pra

<220>
<221> MISC_FEATURE
<222> (10)..(10)
<223> Xaa is moiety Azk

<400> 90

```
Lys Lys Lys Phe Asn Xaa Cys Arg Asp Xaa Ile Gln Arg Met His Leu
1               5                   10                  15


Arg Gln Tyr Asp Leu Leu
            20
```

<210> 91
<211> 22
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (6)..(6)
<223> Xaa is moiety Pra

<220>
<221> MISC_FEATURE
<222> (10)..(10)
<223> Xaa is moiety Azk

<400> 91

Lys Lys Lys Phe Asn Xaa Cys Arg Asp Xaa Ile Gln Arg Met His Leu
1 5 10 15

Arg Gln Trp Glu Leu Leu
20

<210> 92
<211> 22
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (6)..(6)
<223> Xaa is moiety Pra

<220>
<221> MISC_FEATURE
<222> (10)..(10)
<223> Xaa is moiety Azk

<220>
<221> MISC_FEATURE
<222> (19)..(19)
<223> Xaa is 1-naphthalanine (1-Nal)

<400> 92

Lys Lys Lys Phe Asn Xaa Cys Arg Asp Xaa Ile Gln Arg Met His Leu
1 5 10 15

Arg Gln Xaa Glu Leu Leu
20

<210> 93
<211> 22
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (6)..(6)
<223> Xaa is moiety Pra

<220>
<221> MISC_FEATURE
<222> (10)..(10)
<223> Xaa is moiety Azk

<220>
<221> MISC_FEATURE
<222> (19)..(19)
<223> Xaa is 2-naphthalanine (2-Nal)

<400> 93

Lys Lys Lys Phe Asn Xaa Cys Arg Asp Xaa Ile Gln Arg Met His Leu
1 5 10 15

Arg Gln Xaa Glu Leu Leu
20

<210> 94
<211> 22
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (6)..(6)
<223> Xaa is moiety Pra

<220>
<221> MISC_FEATURE
<222> (10)..(10)
<223> Xaa is moiety Azk

<400> 94

Lys Lys Lys Phe Asn Xaa Cys Arg Asp Xaa Ile Gln Arg Met His Leu
1 5 10 15

Arg Gln Tyr Glu Phe Leu
20

<210> 95
<211> 19
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (18)..(18)
<223> Xaa is cyclohexylalanine (Cha)

<400> 95

Phe Asn Asp Cys Arg Asp Ile Ile Gln Arg Met His Leu Arg Gln Tyr
1 5 10 15

Glu Xaa Leu

<210> 96
<211> 15
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (7)..(7)
<223> Xaa is moiety Azk

<220>
<221> MISC_FEATURE
<222> (11)..(11)
<223> Xaa is moiety Pra

<220>
<221> MISC_FEATURE
<222> (14)..(14)
<223> Xaa is cyclohexylalanine (Cha)

<400> 96

Arg Asp Ile Ile Gln Arg Xaa His Leu Arg Xaa Tyr Glu Xaa Leu
1 5 10 15

<210> 97
<211> 18
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (10)..(10)
<223> Xaa is moiety Azk

<220>
<221> MISC_FEATURE
<222> (14)..(14)
<223> Xaa is moiety Pra

<220>
<221> MISC_FEATURE
<222> (17)..(17)
<223> Xaa is cyclohexylalanine (Cha)

<400> 97

Lys Lys Lys Arg Asp Ile Ile Gln Arg Xaa His Leu Arg Xaa Tyr Glu
1 5 10 15

Xaa Leu

<210> 98
<211> 11
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (10)..(10)
<223> Xaa is cyclohexylalanine (Cha)

<400> 98

Gln Arg Met His Leu Arg Gln Tyr Glu Xaa Leu
1 5 10

<210> 99
<211> 15
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (14)..(14)
<223> Xaa is cyclohexylalanine (Cha)

<400> 99

Arg Asp Ile Ile Gln Arg Met His Leu Arg Gln Tyr Glu Xaa Leu
1 5 10 15

<210> 100
<211> 19
<212> PRT
<213> Artificial Sequence

<220>
<223> synthetic peptide

<220>
<221> MOD_RES
<222> (1)..(1)
<223> ACETYLATION

<400> 100

Phe Asn Asp Cys Arg Asp Ile Ile Gln Arg Met His Leu Arg Gln Tyr
1 5 10 15

Glu Phe Leu

<210> 101
<211> 19
<212> PRT
<213> Artificial Sequence

<220>
<223> synthetic peptide

<220>
<221> MOD_RES
<222> (1)..(1)
<223> ACETYLATION

<400> 101

Phe Asn Asp Cys Arg Asp Ile Ile Gln Arg Met His Leu Arg Gln Tyr
1 5 10 15

Glu Trp Leu

<210> 102
<211> 19
<212> PRT
<213> Artificial Sequence

<220>
<223> synthetic peptide

<220>
<221> MOD_RES
<222> (1)..(1)
<223> ACETYLATION

<400> 102

Phe Asn Asp Cys Arg Asp Ile Ile Gln Arg Met His Leu Arg Gln Tyr
1 5 10 15

Glu Tyr Leu

<210> 103
<211> 19
<212> PRT
<213> Artificial Sequence

<220>
<223> synthetic peptide

<220>
<221> MOD_RES
<222> (1)..(1)

<223> ACETYLATION

<400> 103

Phe Asn Asp Cys Arg Asp Ile Ile Gln Arg Met His Leu Arg Gln Tyr
1 5 10 15

Glu His Leu

<210> 104
<211> 24
<212> PRT
<213> Artificial Sequence

<220>
<223> synthetic peptide

<220>
<221> MOD_RES
<222> (1)..(1)
<223> ACETYLATION

<400> 104

Asn Ala Arg Arg Ile Phe Asn Asp Cys Arg Asp Ile Ile Gln Arg Met
1 5 10 15

His Leu Arg Gln Tyr Glu Leu Leu
20

<210> 105
<211> 22
<212> PRT
<213> Artificial Sequence

<220>
<223> synthetic peptide

<220>
<221> MOD_RES
<222> (1)..(1)
<223> ACETYLATION

<400> 105

Arg Arg Ile Phe Asn Asp Cys Arg Asp Ile Ile Gln Arg Met His Leu
1 5 10 15

Arg Gln Tyr Glu Leu Leu
20

<210> 106
<211> 21
<212> PRT

<213> Artificial Sequence

<220>
<223> synthetic peptide

<220>
<221> MOD_RES
<222> (1)..(1)
<223> ACETYLATION

<220>
<221> MISC_FEATURE
<222> (5)..(5)
<223> Xaa is moiety Pra

<220>
<221> MISC_FEATURE
<222> (9)..(9)
<223> Xaa is moiety Azk

<220>
<221> MISC_FEATURE
<222> (20)..(20)
<223> Xaa is cyclohexylalanine (Cha)

<400> 106

```
Lys Lys Phe Asn Xaa Cys Arg Asp Xaa Ile Gln Arg Met His Leu Arg
1               5                   10                  15

Gln Tyr Glu Xaa Leu
            20
```

<210> 107
<211> 19
<212> PRT
<213> Artificial Sequence

<220>
<223> synthetic peptide

<220>
<221> MOD_RES
<222> (1)..(1)
<223> ACETYLATION

<400> 107

```
Phe Asn Asp Ala Arg Asp Ile Ile Gln Arg Met His Leu Arg Gln Tyr
1               5                   10                  15

Glu Leu Leu
```

<210> 108
<211> 19
<212> PRT

<213> Artificial Sequence

<220>
<223> synthetic peptide

<220>
<221> MOD_RES
<222> (1)..(1)
<223> ACETYLATION

<400> 108

Phe Asn Asp Cys Arg Asp Ile Ile Gln Arg Met His Leu Arg Gln Tyr
1 5 10 15

Glu Ala Leu

<210> 109
<211> 19
<212> PRT
<213> Artificial Sequence

<220>
<223> synthetic peptide

<220>
<221> MOD_RES
<222> (1)..(1)
<223> ACETYLATION

<400> 109

Phe Asn Asp Cys Arg Asp Ile Ile Gln Arg Met His Leu Arg Gln Tyr
1 5 10 15

Ala Leu Leu

<210> 110
<211> 19
<212> PRT
<213> Artificial Sequence

<220>
<223> synthetic peptide

<220>
<221> MOD_RES
<222> (1)..(1)
<223> ACETYLATION

<400> 110

Phe Asn Asp Cys Arg Asp Ile Ile Gln Arg Met His Leu Arg Gln Ala
1 5 10 15

Glu Leu Leu

<210> 111
<211> 19
<212> PRT
<213> Artificial Sequence

<220>
<223> synthetic peptide

<220>
<221> MOD_RES
<222> (1)..(1)
<223> ACETYLATION

<400> 111

```
Phe Asn Asp Cys Arg Asp Ile Ile Gln Arg Met His Leu Arg Gln Tyr
1               5                   10                  15

Glu Leu Ala
```

<210> 112
<211> 13
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (12)..(12)
<223> Xaa is cyclohexylalanine (Cha)

<400> 112

```
Ile Ile Gln Arg Met His Leu Arg Gln Tyr Glu Xaa Leu
1               5                   10
```

<210> 113
<211> 24
<212> PRT
<213> Artificial Sequence

<220>
<223> synthetic peptide

<220>
<221> MOD_RES
<222> (1)..(1)
<223> ACETYLATION

<400> 113

Asn Ala Arg Arg Ile Phe Asn Asp Ala Arg Asp Ile Ile Gln Arg Met
1               5                   10                  15

His Leu Arg Gln Tyr Glu Leu Leu
            20

<210> 114
<211> 19
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (16)..(16)
<223> Xaa is cyclohexylalanine (Cha)

<400> 114

Phe Asn Asp Cys Arg Asp Ile Ile Gln Arg Met His Leu Arg Gln Xaa
1               5                   10                  15

Glu Leu Leu

<210> 115
<211> 24
<212> PRT
<213> Artificial Sequence

<220>
<223> synthetic peptide

<220>
<221> MOD_RES
<222> (1)..(1)
<223> ACETYLATION

<400> 115

Asn Ala Arg Arg Ile Phe Asn Asp Ala Arg Asp Ile Ile Gln Arg Met
1               5                   10                  15

His Leu Arg Gln Tyr Glu Trp Leu
20

<210> 116
<211> 19
<212> PRT
<213> Artificial Sequence

<220>
<223> synthetic peptide

<220>
<221> MOD_RES
<222> (1)..(1)
<223> ACETYLATION

<220>
<221> MISC_FEATURE
<222> (19)..(19)
<223> Xaa is cyclohexylalanine (Cha)

<400> 116

Phe Asn Asp Cys Arg Asp Ile Ile Gln Arg Met His Leu Arg Gln Tyr
1 5 10 15

Glu Leu Xaa

<210> 117
<211> 19
<212> PRT
<213> Artificial Sequence

<220>
<223> synthetic peptide

<400> 117

Phe Asn Asp Cys Arg Asp Ile Ile Gln Arg Met His Leu Arg Gln Tyr
1 5 10 15

Glu Leu Leu

<210> 118
<211> 15
<212> PRT
<213> Artificial Sequence

<220>
<223> synthetic peptide

<400> 118

```
Arg Asp Ile Ile Gln Arg Met His Leu Arg Gln Tyr Glu Leu Leu
1               5                   10                  15


<210>  119
<211>  15
<212>  PRT
<213>  Artificial Sequence

<220>
<223>  synthetic peptide


<220>
<221>  MISC_FEATURE
<222>  (7)..(7)
<223>  Xaa is moiety Azk

<220>
<221>  MISC_FEATURE
<222>  (11)..(11)
<223>  Xaa is moiety Pra

<400>  119

Arg Asp Ile Ile Gln Arg Xaa His Leu Arg Xaa Tyr Glu Leu Leu
1               5                   10                  15


<210>  120
<211>  11
<212>  PRT
<213>  Artificial Sequence

<220>
<223>  synthetic peptide

<400>  120

Gln Arg Met His Leu Arg Gln Tyr Glu Leu Leu
1               5                   10


<210>  121
<211>  19
<212>  PRT
<213>  Artificial Sequence

<220>
<223>  synthetic peptide


<220>
<221>  MOD_RES
<222>  (1)..(1)
<223>  ACETYLATION

<220>
<221>  MISC_FEATURE
<222>  (3)..(3)
<223>  Xaa is moiety Pra

<220>
```

<221> MISC_FEATURE
<222> (7)..(7)
<223> Xaa is moiety Azk

<220>
<221> MISC_FEATURE
<222> (16)..(16)
<223> Xaa is 2-naphthalanine (2-Nal)

<400> 121

Phe Asn Xaa Cys Arg Asp Xaa Ile Gln Arg Met His Leu Arg Gln Xaa
1 5 10 15

Glu Leu Leu

<210> 122
<211> 19
<212> PRT
<213> Artificial Sequence

<220>
<223> synthetic peptide

<220>
<221> MISC_FEATURE
<222> (3)..(3)
<223> Xaa is moiety Pra

<220>
<221> MISC_FEATURE
<222> (7)..(7)
<223> Xaa is moiety Azk

<220>
<221> MISC_FEATURE
<222> (18)..(18)
<223> Xaa is cyclohexylalanine (Cha)

<400> 122

Phe Asn Xaa Cys Arg Asp Xaa Ile Gln Arg Met His Leu Arg Gln Tyr
1 5 10 15

Glu Xaa Leu

<210> 123
<211> 22
<212> PRT
<213> Artificial Sequence

<220>
<223> synthetic peptide

<220>

<221> MISC_FEATURE
<222> (6)..(6)
<223> Xaa is moiety Pra

<220>
<221> MISC_FEATURE
<222> (10)..(10)
<223> Xaa is moiety Azk

<220>
<221> MISC_FEATURE
<222> (21)..(21)
<223> Xaa is cyclohexylalanine (Cha)

<400> 123

```
Lys Lys Lys Phe Asn Xaa Cys Arg Asp Xaa Ile Gln Arg Met His Leu
1               5                   10                  15


Arg Gln Tyr Glu Xaa Leu
            20
```

<210> 124
<211> 15
<212> PRT
<213> Artificial Sequence

<220>
<223> synthetic peptide

<220>
<221> MISC_FEATURE
<222> (14)..(14)
<223> Xaa is cyclohexylalanine (Cha)

<400> 124

```
Arg Asp Ile Ile Gln Arg Met His Leu Arg Gln Tyr Glu Xaa Leu
1               5                   10                  15
```

<210> 125
<211> 19
<212> PRT
<213> Artificial Sequence

<220>
<223> synthetic peptide

<220>
<221> MISC_FEATURE
<222> (3)..(3)
<223> Xaa is moiety Pra

<220>
<221> MISC_FEATURE
<222> (7)..(7)
<223> Xaa is moiety Azk

<400> 125

Phe Asn Xaa Cys Arg Asp Xaa Ile Gln Arg Met His Leu Arg Gln Tyr
1 5 10 15

Glu Phe Leu

<210> 126
<211> 22
<212> PRT
<213> Artificial Sequence

<220>
<223> synthetic peptide

<220>
<221> MISC_FEATURE
<222> (6)..(6)
<223> Xaa is moiety Pra

<220>
<221> MISC_FEATURE
<222> (10)..(10)
<223> Xaa is moiety Azk

<400> 126

Lys Lys Lys Phe Asn Xaa Cys Arg Asp Xaa Ile Gln Arg Met His Leu
1 5 10 15

Arg Gln Tyr Glu Phe Leu
20

<210> 127
<211> 19
<212> PRT
<213> Artificial Sequence

<220>
<223> synthetic peptide

<220>
<221> MISC_FEATURE
<222> (18)..(18)
<223> Xaa is cyclohexylalanine (Cha)

<400> 127

Phe Asn Asp Cys Arg Asp Ile Ile Gln Arg Met His Leu Arg Gln Tyr
1 5 10 15

Glu Xaa Leu

<210> 128
<211> 19
<212> PRT
<213> Artificial Sequence

<220>
<223> synthetic peptide

<400> 128

Phe Asn Asp Cys Arg Asp Ile Ile Gln Arg Met His Leu Arg Gln Tyr
1 5 10 15

Glu Phe Leu

<210> 129
<211> 19
<212> PRT
<213> Artificial Sequence

<220>
<223> synthetic peptide

<400> 129

Phe Asn Asp Cys Arg Asp Ile Ile Gln Arg Met His Leu Arg Gln Tyr
1 5 10 15

Glu Trp Leu

<210> 130
<211> 21
<212> PRT
<213> Artificial Sequence

<220>
<223> synthetic peptide

<220>
<221> MISC_FEATURE
<222> (5)..(5)
<223> Xaa is moiety Pra

<220>
<221> MISC_FEATURE
<222> (9)..(9)
<223> Xaa is moiety Azk

<220>
<221> MISC_FEATURE
<222> (20)..(20)
<223> Xaa is cyclohexylalanine (Cha)

<400> 130

Lys Lys Phe Asn Xaa Cys Arg Asp Xaa Ile Gln Arg Met His Leu Arg

1 5 10 15

Gln Tyr Glu Xaa Leu
20

<210> 131
<211> 20
<212> PRT
<213> Artificial Sequence

<220>
<223> synthetic peptide

<220>
<221> MISC_FEATURE
<222> (4)..(4)
<223> Xaa is moiety Pra

<220>
<221> MISC_FEATURE
<222> (8)..(8)
<223> Xaa is moiety Azk

<220>
<221> MISC_FEATURE
<222> (19)..(19)
<223> Xaa is cyclohexylalanine (Cha)

<400> 131

Lys Phe Asn Xaa Cys Arg Asp Xaa Ile Gln Arg Met His Leu Arg Gln
1 5 10 15

Tyr Glu Xaa Leu
20

<210> 132
<211> 24
<212> PRT
<213> Artificial Sequence

<220>
<223> synthetic peptide

<400> 132

Asn Ala Arg Arg Ile Phe Asn Asp Cys Arg Asp Ile Ile Gln Arg Met
1 5 10 15

His Leu Arg Gln Tyr Glu Leu Leu
20

<210> 133
<211> 4
<212> PRT
<213> Artificial Sequence

<220>
<223> synthetic peptide

<220>
<221> MISC_FEATURE
<222> (1)..(1)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (2)..(2)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (3)..(3)
<223> Xaa can be alanine analogue of formula (Ib), phenylalanine (F) or tryptophan (W)

<220>
<221> MISC_FEATURE
<222> (4)..(4)
<223> Xaa can be leucine (L), L-NH2, isoleucine (I), alanine analogue of formula (Ia) or (I'a), phenylalanine (F) or tryptophan (W)

<400> 133

Xaa Xaa Xaa Xaa
1

<210> 134
<211> 4
<212> PRT
<213> Artificial Sequence

<220>
<223> synthetic peptide

<220>
<221> MISC_FEATURE
<222> (1)..(1)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (2)..(2)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (3)..(3)

<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (4)..(4)
<223> Xaa can be alanine analogue of formula (Ia) or (I'a), phenylalanine (F) or tryptophan (W)

<400> 134

Xaa Xaa Xaa Xaa
1

<210> 135
<211> 4
<212> PRT
<213> Artificial Sequence

<220>
<223> synthetic peptide

<220>
<221> MISC_FEATURE
<222> (1)..(1)
<223> Xaa can be alanine analogue of formula (Ib), phenylalanine (F) or tryptophan (W)

<220>
<221> MISC_FEATURE
<222> (2)..(2)
<223> Xaa can be glutamic acid (E), glutamine (Q), homoglutamic acid (hGlu), aspartic Acid (D), alanine (A), alanine analogue of formula (Ib), phenylalanine (F) or tryptophan (W)

<220>
<221> MISC_FEATURE
<222> (3)..(3)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (4)..(4)
<223> Xaa can be leucine (L), L-NH2, isoleucine (I), alanine analogue of formula (Ia) or (I'a), phenylalanine (F) or tryptophan (W)

<400> 135

Xaa Xaa Xaa Xaa
1

<210> 136
<211> 4
<212> PRT
<213> Artificial Sequence

<220>

<223> synthetic peptide

<220>
<221> MISC_FEATURE
<222> (1)..(1)
<223> Xaa can be tyrosine (Y), 2-naphthalanine (2-Nal), 1-naphthalanine (1-Nal), alanine analogue of formula (Ib), phenylalanine (F) or tryptophan (W)

<220>
<221> MISC_FEATURE
<222> (2)..(2)
<223> Xaa can be alanine analogue of formula (Ib), phenylalanine (F) or tryptophan (W)

<220>
<221> MISC_FEATURE
<222> (3)..(3)
<223> Xaa can be leucine (L), isoleucine (I), tyrosine (Y), histidine (H), alanine analogue of formula (Ib), phenylalanine (F) or tryptophan (W)

<220>
<221> MISC_FEATURE
<222> (4)..(4)
<223> Xaa can be leucine (L), L-NH2, isoleucine (I), alanine analogue of formula (Ia) or (I'a), phenylalanine (F) or tryptophan (W)

<400> 136

Xaa Xaa Xaa Xaa
1

<210> 137
<211> 19
<212> PRT
<213> Artificial Sequence

<220>
<223> synthetic peptide

<220>
<221> MOD_RES
<222> (7)..(7)
<223> modified glutamic acid (E) which is linked to modified lysine (K) at position (11) via the reaction of the carboxylic acid group side-chain of a glutamic acid (E) with the amine side-chain of a lysine (K)

<220>
<221> MOD_RES
<222> (11)..(11)
<223> modified lysine (K) which is linked to modified glutamic acid (E) at position (7) via the reaction of the amine side-chain of a lysine (K) with the carboxylic acid group side-chain of a glutamic acid (E)

<400> 137

Phe Asn Asp Cys Arg Asp Glu Ile Gln Arg Lys His Leu Arg Gln Tyr

1 5 10 15

Glu Leu Leu

<210> 138
<211> 19
<212> PRT
<213> Artificial Sequence

<220>
<223> synthetic peptide

<220>
<221> MOD_RES
<222> (1)..(1)
<223> ACETYLATION

<220>
<221> MOD_RES
<222> (7)..(7)
<223> modified glutamic acid (E) which is linked to modified lysine (K) at position (11) via the reaction of the carboxylic acid group side-chain of a glutamic acid (E) with the amine side-chain of a lysine (K)

<220>
<221> MOD_RES
<222> (11)..(11)
<223> modified lysine (K) which is linked to modified glutamic acid (E) at position (7) via the reaction of the amine side-chain of a lysine (K) with the carboxylic acid group side-chain of a glutamic acid (E)

<400> 138

Phe Asn Asp Cys Arg Asp Glu Ile Gln Arg Lys His Leu Arg Gln Tyr
1 5 10 15

Glu Leu Leu

<210> 139
<211> 19
<212> PRT
<213> Artificial Sequence

<220>
<223> synthetic peptide

<220>
<221> MOD_RES
<222> (7)..(7)
<223> modified "S5" which is linked to modified "S5" at position (11) via the reaction of the olefinic side-chain of an "S5" with the olefinic side-chain of an "S5"

<220>
<221> MOD_RES
<222> (11)..(11)
<223> modified "S5" which is linked to modified "S5" at position (7) via the reaction of the olefinic side-chain of an "S5" with the olefinic side-chain of an "S5"

<400> 139

Phe Asn Asp Cys Arg Asp Xaa Ile Gln Arg Xaa His Leu Arg Gln Tyr
1 5 10 15

Glu Leu Leu

<210> 140
<211> 19
<212> PRT
<213> Artificial Sequence

<220>
<223> synthetic peptide

<220>
<221> MOD_RES
<222> (1)..(1)
<223> ACETYLATION

<220>
<221> MOD_RES
<222> (7)..(7)
<223> modified "S5" which is linked to modified "S5" at position (11) via the reaction of the olefinic side-chain of an "S5" with the olefinic side-chain of an "S5"

<220>
<221> MOD_RES
<222> (11)..(11)
<223> modified "S5" which is linked to modified "S5" at position (7) via the reaction of the olefinic side-chain of an "S5" with the olefinic side-chain of an "S5"

<400> 140

Phe Asn Asp Cys Arg Asp Xaa Ile Gln Arg Xaa His Leu Arg Gln Tyr
1 5 10 15

Glu Leu Leu

<210> 141
<211> 19
<212> PRT
<213> Artificial Sequence

<220>
<223> synthetic peptide

<220>
<221> MOD_RES
<222> (7)..(7)
<223> modified "Pra" which is linked to modified "Azk" at position (11) via the reaction of the alkynyl side chain of a "Pra" with the azidated side-chain of an "Azk"

<220>
<221> MOD_RES
<222> (11)..(11)
<223> modified "Azk" which is linked to modified "Pra" at position (7) via the reaction of the azidated side-chain of an "Azk" with the alkynyl side-chain of a "Pra"

<400> 141

Phe Asn Asp Cys Arg Asp Xaa Ile Gln Arg Xaa His Leu Arg Gln Tyr
1 5 10 15

Glu Leu Leu

<210> 142
<211> 19
<212> PRT
<213> Artificial Sequence

<220>
<223> synthetic peptide

<220>
<221> MOD_RES
<222> (1)..(1)
<223> ACETYLATION

<220>
<221> MOD_RES
<222> (7)..(7)
<223> modified "Pra" which is linked to modified "Azk" at position (11) via the reaction of the alkynyl side chain of a "Pra" with the azidated side-chain of an "Azk"

<220>
<221> MOD_RES
<222> (11)..(11)
<223> modified "Azk" which is linked to modified "Pra" at position (7) via the reaction of the azidated side-chain of an "Azk" with the alkynyl side-chain of a "Pra"

<400> 142

Phe Asn Asp Cys Arg Asp Xaa Ile Gln Arg Xaa His Leu Arg Gln Tyr
1 5 10 15

Glu Leu Leu

<210> 143
<211> 19
<212> PRT
<213> Artificial Sequence

<220>
<223> synthetic peptide

<220>
<221> MOD_RES
<222> (1)..(1)
<223> ACETYLATION

<220>
<221> MOD_RES
<222> (3)..(3)
<223> modified "Pra" which is linked to modified "Azk" at position (7) via the reaction of the alkynyl side chain of a "Pra" with the azidated side-chain of an "Azk"

<220>
<221> MOD_RES
<222> (7)..(7)
<223> modified "Azk" which is linked to modified "Pra" at position (3) via the reaction of the azidated side-chain of an "Azk" with the alkynyl side-chain of a "Pra"

<400> 143

```
Phe Asn Xaa Cys Arg Asp Xaa Ile Gln Arg Met His Leu Arg Gln Tyr
1               5                   10                  15

Glu Leu Leu
```

<210> 144
<211> 19
<212> PRT
<213> Artificial Sequence

<220>
<223> synthetic peptide

<220>
<221> MOD_RES
<222> (1)..(1)
<223> ACETYLATION

<220>
<221> MOD_RES
<222> (3)..(3)
<223> modified glutamic acid (E) which is linked to modified lysine (K) at position (11) via the reaction of the carboxylic acid group side-chain of a glutamic acid (E) with the amine side-chain of a lysine (K)

<220>
<221> MOD_RES
<222> (7)..(7)

<223> modified lysine (K) which is linked to modified glutamic acid (E) at position (7) via the reaction of the amine side-chain of a lysine (K) with the carboxylic acid group side-chain of a glutamic acid (E)

<400> 144

Phe Asn Glu Cys Arg Asp Lys Ile Gln Arg Met His Leu Arg Gln Tyr
1               5                   10                  15

Glu Leu Leu

<210> 145
<211> 19
<212> PRT
<213> Artificial Sequence

<220>
<223> synthetic peptide

<220>
<221> MOD_RES
<222> (1)..(1)
<223> ACETYLATION

<220>
<221> MOD_RES
<222> (3)..(3)
<223> modified "S5" which is linked to modified "S5" at position (7) via the reaction of the olefinic side-chain of an "S5" with the olefinic side-chain of an "S5"

<220>
<221> MOD_RES
<222> (7)..(7)
<223> modified "S5" which is linked to modified "S5" at position (3) via the reaction of the olefinic side-chain of an "S5" with the olefinic side-chain of an "S5"

<400> 145

Phe Asn Xaa Cys Arg Asp Xaa Ile Gln Arg Met His Leu Arg Gln Tyr
1               5                   10                  15

Glu Leu Leu

<210> 146
<211> 19
<212> PRT
<213> Artificial Sequence

<220>
<223> synthetic peptide

<220>

<221> MOD_RES
<222> (1)..(1)
<223> ACETYLATION

<220>
<221> MOD_RES
<222> (11)..(11)
<223> modified "R5" which is linked to modified "S5" at position (14) via the reaction of the olefinic side-chain of an "R5" with the olefinic side-chain of an "S5"

<220>
<221> MOD_RES
<222> (14)..(14)
<223> modified "S5" which is linked to modified "R5" at position (11) via the reaction of the olefinic side-chain of an "S5" with the olefinic side-chain of an "R5"

<400> 146

```
Phe Asn Asp Cys Arg Asp Ile Ile Gln Arg Xaa His Leu Xaa Gln Tyr
1               5                   10                  15

Glu Leu Leu
```

<210> 147
<211> 19
<212> PRT
<213> Artificial Sequence

<220>
<223> synthetic peptide

<220>
<221> MOD_RES
<222> (1)..(1)
<223> ACETYLATION

<220>
<221> MOD_RES
<222> (7)..(7)
<223> modified "R8" which is linked to modified "S5" at position (14) via the reaction of the olefinic side-chain of an "R8" with the olefinic side-chain of an "S5"

<220>
<221> MOD_RES
<222> (14)..(14)
<223> modified "S5" which is linked to modified "R8" at position (7) via the reaction of the olefinic side-chain of an "S5" with the olefinic side-chain of an "R8"

<400> 147

```
Phe Asn Asp Cys Arg Asp Xaa Ile Gln Arg Met His Leu Xaa Gln Tyr
1               5                   10                  15

Glu Leu Leu
```

<210> 148
<211> 15
<212> PRT
<213> Artificial Sequence

<220>
<223> synthetic peptide

<220>
<221> MOD_RES
<222> (1)..(1)
<223> ACETYLATION

<220>
<221> MOD_RES
<222> (7)..(7)
<223> modified "Azk" which is linked to modified "Pra" at position (11) via the reaction of the azidated side-chain of an "Azk" with the alkynyl side-chain of a "Pra"

<220>
<221> MOD_RES
<222> (11)..(11)
<223> modified "Pra" which is linked to modified "Azk" at position (7) via the reaction of the alkynyl side chain of a "Pra" with the azidated side-chain of an "Azk"

<400> 148

```
Arg Asp Ile Ile Gln Arg Xaa His Leu Arg Xaa Tyr Glu Leu Leu
1               5                   10                  15
```

<210> 149
<211> 19
<212> PRT
<213> Artificial Sequence

<220>
<223> synthetic peptide

<220>
<221> MOD_RES
<222> (1)..(1)
<223> ACETYLATION

<220>
<221> MOD_RES
<222> (3)..(3)
<223> modified "Pra" which is linked to modified "Azk" at position (7) via the reaction of the alkynyl side chain of a "Pra" with the azidated side-chain of an "Azk"

<220>
<221> MOD_RES
<222> (7)..(7)
<223> modified "Azk" which is linked to modified "Pra" at position (3) via the reaction of the azidated side-chain of an "Azk" with the

alkynyl side-chain of a "Pra"

<400> 149

Phe Asn Xaa Cys Arg Asp Xaa Ile Gln Arg Met His Leu Arg Gln Tyr
1 5 10 15

Gln Leu Leu

<210> 150
<211> 19
<212> PRT
<213> Artificial Sequence

<220>
<223> synthetic peptide

<220>
<221> MOD_RES
<222> (1)..(1)
<223> ACETYLATION

<220>
<221> MOD_RES
<222> (3)..(3)
<223> modified "Pra" which is linked to modified "Azk" at position (7) via the reaction of the alkynyl side chain of a "Pra" with the azidated side-chain of an "Azk"

<220>
<221> MOD_RES
<222> (7)..(7)
<223> modified "Azk" which is linked to modified "Pra" at position (3) via the reaction of the azidated side-chain of an "Azk" with the alkynyl side-chain of a "Pra"

<400> 150

Phe Asn Xaa Cys Arg Asp Xaa Ile Gln Arg Met His Leu Arg Gln Trp
1 5 10 15

Glu Leu Leu

<210> 151
<211> 19
<212> PRT
<213> Artificial Sequence

<220>
<223> synthetic peptide

<220>
<221> MOD_RES
<222> (1)..(1)
<223> ACETYLATION

<220>
<221> MOD_RES
<222> (3)..(3)
<223> modified "Pra" which is linked to modified "Azk" at position (7) via the reaction of the alkynyl side chain of a "Pra" with the azidated side-chain of an "Azk"

<220>
<221> MOD_RES
<222> (7)..(7)
<223> modified "Azk" which is linked to modified "Pra" at position (3) via the reaction of the azidated side-chain of an "Azk" with the alkynyl side-chain of a "Pra"

<400> 151

Phe Asn Xaa Cys Arg Asp Xaa Ile Gln Arg Met His Leu Arg Gln Tyr
1 5 10 15

Glu Ile Leu

<210> 152
<211> 19
<212> PRT
<213> Artificial Sequence

<220>
<223> synthetic peptide

<220>
<221> MOD_RES
<222> (1)..(1)
<223> ACETYLATION

<220>
<221> MOD_RES
<222> (3)..(3)
<223> modified "Pra" which is linked to modified "Azk" at position (7) via the reaction of the alkynyl side chain of a "Pra" with the azidated side-chain of an "Azk"

<220>
<221> MOD_RES
<222> (7)..(7)
<223> modified "Azk" which is linked to modified "Pra" at position (3) via the reaction of the azidated side-chain of an "Azk" with the alkynyl side-chain of a "Pra"

<400> 152

Phe Asn Xaa Cys Arg Asp Xaa Ile Gln Arg Met His Leu Arg Gln Tyr
1 5 10 15

Glu Leu Ile

<210> 153
<211> 19
<212> PRT
<213> Artificial Sequence

<220>
<223> synthetic peptide

<220>
<221> MOD_RES
<222> (1)..(1)
<223> ACETYLATION

<220>
<221> MOD_RES
<222> (3)..(3)
<223> modified "Azk" which is linked to modified "Pra" at position (7) via the reaction of the azidated side-chain of an "Azk" with the alkynyl side-chain of a "Pra"

<220>
<221> MOD_RES
<222> (7)..(7)
<223> modified "Pra" which is linked to modified "Azk" at position (3) via the reaction of the alkynyl side chain of a "Pra" with the azidated side-chain of an "Azk"

<400> 153

```
Phe Asn Xaa Cys Arg Asp Xaa Ile Gln Arg Met His Leu Arg Gln Tyr
1               5                   10                  15

Glu Leu Leu
```

<210> 154
<211> 19
<212> PRT
<213> Artificial Sequence

<220>
<223> synthetic peptide

<220>
<221> MOD_RES
<222> (1)..(1)
<223> ACETYLATION

<220>
<221> MOD_RES
<222> (3)..(3)
<223> modified "Pra" which is linked to modified "Azk" at position (7) via the reaction of the alkynyl side chain of a "Pra" with the azidated side-chain of an "Azk"

<220>
<221> MOD_RES
<222> (7)..(7)
<223> modified "Azk" which is linked to modified "Pra" at position (3)

via the reaction of the azidated side-chain of an "Azk" with the alkynyl side-chain of a "Pra"

<220>
<221> MOD_RES
<222> (19)..(19)
<223> AMIDATION

<400> 154

Phe Asn Xaa Cys Arg Asp Xaa Ile Gln Arg Met His Leu Arg Gln Tyr
1 5 10 15

Glu Leu Leu

<210> 155
<211> 19
<212> PRT
<213> Artificial Sequence

<220>
<223> synthetic peptide

<220>
<221> MOD_RES
<222> (1)..(1)
<223> ACETYLATION

<220>
<221> MOD_RES
<222> (3)..(3)
<223> modified "Pra" which is linked to modified "Azk" at position (7) via the reaction of the alkynyl side chain of a "Pra" with the azidated side-chain of an "Azk"

<220>
<221> MOD_RES
<222> (7)..(7)
<223> modified "Azk" which is linked to modified "Pra" at position (3) via the reaction of the azidated side-chain of an "Azk" with the alkynyl side-chain of a "Pra"

<220>
<221> MISC_FEATURE
<222> (17)..(17)
<223> Xaa is homoglutamic acid (hGlu)

<400> 155

Phe Asn Xaa Cys Arg Asp Xaa Ile Gln Arg Met His Leu Arg Gln Tyr
1 5 10 15

Xaa Leu Leu

<210> 156
<211> 19

<212> PRT
<213> Artificial Sequence

<220>
<223> synthetic peptide

<220>
<221> MOD_RES
<222> (1)..(1)
<223> ACETYLATION

<220>
<221> MOD_RES
<222> (3)..(3)
<223> modified "Pra" which is linked to modified "Azk" at position (7) via the reaction of the alkynyl side chain of a "Pra" with the azidated side-chain of an "Azk"

<220>
<221> MOD_RES
<222> (7)..(7)
<223> modified "Azk" which is linked to modified "Pra" at position (3) via the reaction of the azidated side-chain of an "Azk" with the alkynyl side-chain of a "Pra"

<400> 156

Phe Asn Xaa Cys Arg Asn Xaa Ile Gln Arg Met His Leu Arg Gln Tyr
1               5                   10                  15

Glu Leu Leu

<210> 157
<211> 19
<212> PRT
<213> Artificial Sequence

<220>
<223> synthetic peptide

<220>
<221> MOD_RES
<222> (1)..(1)
<223> ACETYLATION

<220>
<221> MOD_RES
<222> (3)..(3)
<223> modified "Pra" which is linked to modified "Azk" at position (7) via the reaction of the alkynyl side chain of a "Pra" with the azidated side-chain of an "Azk"

<220>
<221> MOD_RES
<222> (7)..(7)
<223> modified "Azk" which is linked to modified "Pra" at position (3) via the reaction of the azidated side-chain of an "Azk" with the alkynyl side-chain of a "Pra"

<400> 157

Phe Asn Xaa Cys Arg Asp Xaa Ile Gln Arg Met His Leu Arg Gln Tyr
1 5 10 15

Asp Leu Leu

<210> 158
<211> 19
<212> PRT
<213> Artificial Sequence

<220>
<223> synthetic peptide

<220>
<221> MOD_RES
<222> (1)..(1)
<223> ACETYLATION

<220>
<221> MOD_RES
<222> (3)..(3)
<223> modified "Pra" which is linked to modified "Azk" at position (7) via the reaction of the alkynyl side chain of a "Pra" with the azidated side-chain of an "Azk"

<220>
<221> MOD_RES
<222> (7)..(7)
<223> modified "Azk" which is linked to modified "Pra" at position (3) via the reaction of the azidated side-chain of an "Azk" with the alkynyl side-chain of a "Pra"

<220>
<221> MISC_FEATURE
<222> (16)..(16)
<223> Xaa is 1-naphthalanine (1-Nal)

<400> 158

Phe Asn Xaa Cys Arg Asp Xaa Ile Gln Arg Met His Leu Arg Gln Xaa
1 5 10 15

Glu Leu Leu

<210> 159
<211> 20
<212> PRT
<213> Artificial Sequence

<220>
<223> synthetic peptide

<220>
<221> MOD_RES
<222> (1)..(1)
<223> ACETYLATION

<220>
<221> MOD_RES
<222> (4)..(4)
<223> modified "Pra" which is linked to modified "Azk" at position (8) via the reaction of the alkynyl side chain of a "Pra" with the azidated side-chain of an "Azk"

<220>
<221> MOD_RES
<222> (8)..(8)
<223> modified "Azk" which is linked to modified "Pra" at position (4) via the reaction of the azidated side-chain of an "Azk" with the alkynyl side-chain of a "Pra"

<400> 159

Arg Phe Asn Xaa Cys Arg Asp Xaa Ile Gln Arg Met His Leu Arg Gln
1               5                   10                  15

Tyr Glu Leu Leu
            20

<210> 160
<211> 20
<212> PRT
<213> Artificial Sequence

<220>
<223> synthetic peptide

<220>
<221> MOD_RES
<222> (1)..(1)
<223> ACETYLATION

<220>
<221> MOD_RES
<222> (4)..(4)
<223> modified "Pra" which is linked to modified "Azk" at position (8) via the reaction of the alkynyl side chain of a "Pra" with the azidated side-chain of an "Azk"

<220>
<221> MOD_RES
<222> (8)..(8)
<223> modified "Azk" which is linked to modified "Pra" at position (4) via the reaction of the azidated side-chain of an "Azk" with the alkynyl side-chain of a "Pra"

<400> 160

Lys Phe Asn Xaa Cys Arg Asp Xaa Ile Gln Arg Met His Leu Arg Gln
1               5                   10                  15

Tyr Glu Leu Leu
20

<210> 161
<211> 22
<212> PRT
<213> Artificial Sequence

<220>
<223> synthetic peptide

<220>
<221> MOD_RES
<222> (1)..(1)
<223> ACETYLATION

<220>
<221> MOD_RES
<222> (6)..(6)
<223> modified "Pra" which is linked to modified "Azk" at position (10) via the reaction of the alkynyl side chain of a "Pra" with the azidated side-chain of an "Azk"

<220>
<221> MOD_RES
<222> (10)..(10)
<223> modified "Azk" which is linked to modified "Pra" at position (6) via the reaction of the azidated side-chain of an "Azk" with the alkynyl side-chain of a "Pra"

<400> 161

Lys Lys Lys Phe Asn Xaa Cys Arg Asp Xaa Ile Gln Arg Met His Leu
1 5 10 15

Arg Gln Tyr Glu Leu Leu
20

<210> 162
<211> 20
<212> PRT
<213> Artificial Sequence

<220>
<223> synthetic peptide

<220>
<221> MOD_RES
<222> (1)..(1)
<223> ACETYLATION

<220>
<221> MOD_RES
<222> (4)..(4)
<223> modified "Pra" which is linked to modified "Azk" at position (8) via the reaction of the alkynyl side chain of a "Pra" with the azidated side-chain of an "Azk"

<220>
<221> MOD_RES
<222> (8)..(8)
<223> modified "Azk" which is linked to modified "Pra" at position (4) via the reaction of the azidated side-chain of an "Azk" with the alkynyl side-chain of a "Pra"

<400> 162

Lys Phe Asn Xaa Cys Arg Asp Xaa Ile Gln Arg Met His Leu Arg Gln
1 5 10 15

Tyr Glu Leu Ile
20

<210> 163
<211> 22
<212> PRT
<213> Artificial Sequence

<220>
<223> synthetic peptide

<220>
<221> MOD_RES
<222> (1)..(1)
<223> ACETYLATION

<220>
<221> MOD_RES
<222> (6)..(6)
<223> modified "Pra" which is linked to modified "Azk" at position (10) via the reaction of the alkynyl side chain of a "Pra" with the azidated side-chain of an "Azk"

<220>
<221> MOD_RES
<222> (10)..(10)
<223> modified "Azk" which is linked to modified "Pra" at position (6) via the reaction of the azidated side-chain of an "Azk" with the alkynyl side-chain of a "Pra"

<400> 163

Lys Lys Lys Phe Asn Xaa Cys Arg Asp Xaa Ile Gln Arg Met His Leu
1 5 10 15

Arg Gln Tyr Glu Leu Ile
20

<210> 164
<211> 20
<212> PRT
<213> Artificial Sequence

<220>
<223> synthetic peptide

<220>
<221> MOD_RES
<222> (1)..(1)
<223> ACETYLATION

<220>
<221> MOD_RES
<222> (4)..(4)
<223> modified "Pra" which is linked to modified "Azk" at position (8) via the reaction of the alkynyl side chain of a "Pra" with the azidated side-chain of an "Azk"

<220>
<221> MOD_RES
<222> (8)..(8)
<223> modified "Azk" which is linked to modified "Pra" at position (4) via the reaction of the azidated side-chain of an "Azk" with the alkynyl side-chain of a "Pra"

<400> 164

Lys Phe Asn Xaa Cys Arg Asp Xaa Ile Gln Arg Met His Leu Arg Gln
1               5                   10                  15

Tyr Glu Ile Leu
            20

<210> 165
<211> 22
<212> PRT
<213> Artificial Sequence

<220>
<223> synthetic peptide

<220>
<221> MOD_RES
<222> (1)..(1)
<223> ACETYLATION

<220>
<221> MOD_RES
<222> (6)..(6)
<223> modified "Pra" which is linked to modified "Azk" at position (10) via the reaction of the alkynyl side chain of a "Pra" with the azidated side-chain of an "Azk"

<220>
<221> MOD_RES
<222> (10)..(10)
<223> modified "Azk" which is linked to modified "Pra" at position (6) via the reaction of the azidated side-chain of an "Azk" with the alkynyl side-chain of a "Pra"

<400> 165

Lys Lys Lys Phe Asn Xaa Cys Arg Asp Xaa Ile Gln Arg Met His Leu
1               5                   10                  15

Arg Gln Tyr Glu Ile Leu
20

<210> 166
<211> 20
<212> PRT
<213> Artificial Sequence

<220>
<223> synthetic peptide

<220>
<221> MOD_RES
<222> (1)..(1)
<223> ACETYLATION

<220>
<221> MOD_RES
<222> (4)..(4)
<223> modified "Pra" which is linked to modified "Azk" at position (8) via the reaction of the alkynyl side chain of a "Pra" with the azidated side-chain of an "Azk"

<220>
<221> MOD_RES
<222> (8)..(8)
<223> modified "Azk" which is linked to modified "Pra" at position (4) via the reaction of the azidated side-chain of an "Azk" with the alkynyl side-chain of a "Pra"

<220>
<221> MISC_FEATURE
<222> (19)..(19)
<223> Xaa is cyclohexylalanine (Cha)

<400> 166

Lys Phe Asn Xaa Cys Arg Asp Xaa Ile Gln Arg Met His Leu Arg Gln
1 5 10 15

Tyr Glu Xaa Leu
20

<210> 167
<211> 22
<212> PRT
<213> Artificial Sequence

<220>
<223> synthetic peptide

<220>
<221> MOD_RES
<222> (1)..(1)
<223> ACETYLATION

<220>

<221> MOD_RES
<222> (6)..(6)
<223> modified "Pra" which is linked to modified "Azk" at position (10) via the reaction of the alkynyl side chain of a "Pra" with the azidated side-chain of an "Azk"

<220>
<221> MOD_RES
<222> (10)..(10)
<223> modified "Azk" which is linked to modified "Pra" at position (6) via the reaction of the azidated side-chain of an "Azk" with the alkynyl side-chain of a "Pra"

<220>
<221> MISC_FEATURE
<222> (21)..(21)
<223> Xaa is cyclohexylalanine (Cha)

<400> 167

```
Lys Lys Lys Phe Asn Xaa Cys Arg Asp Xaa Ile Gln Arg Met His Leu
1               5                   10                  15


Arg Gln Tyr Glu Xaa Leu
            20
```

<210> 168
<211> 20
<212> PRT
<213> Artificial Sequence

<220>
<223> synthetic peptide

<220>
<221> MOD_RES
<222> (1)..(1)
<223> ACETYLATION

<220>
<221> MOD_RES
<222> (4)..(4)
<223> modified "Pra" which is linked to modified "Azk" at position (8) via the reaction of the alkynyl side chain of a "Pra" with the azidated side-chain of an "Azk"

<220>
<221> MOD_RES
<222> (8)..(8)
<223> modified "Azk" which is linked to modified "Pra" at position (4) via the reaction of the azidated side-chain of an "Azk" with the alkynyl side-chain of a "Pra"

<220>
<221> MISC_FEATURE
<222> (18)..(18)
<223> Xaa is homoglutamic acid (hGlu)

<400> 168

Lys Phe Asn Xaa Cys Arg Asp Xaa Ile Gln Arg Met His Leu Arg Gln
1               5                   10                  15

Tyr Xaa Leu Leu
            20

<210> 169
<211> 22
<212> PRT
<213> Artificial Sequence

<220>
<223> synthetic peptide

<220>
<221> MOD_RES
<222> (1)..(1)
<223> ACETYLATION

<220>
<221> MOD_RES
<222> (6)..(6)
<223> modified "Pra" which is linked to modified "Azk" at position (10) via the reaction of the alkynyl side chain of a "Pra" with the azidated side-chain of an "Azk"

<220>
<221> MOD_RES
<222> (10)..(10)
<223> modified "Azk" which is linked to modified "Pra" at position (6) via the reaction of the azidated side-chain of an "Azk" with the alkynyl side-chain of a "Pra"

<220>
<221> MISC_FEATURE
<222> (20)..(20)
<223> Xaa is homoglutamic acid (hGlu)

<400> 169

Lys Lys Lys Phe Asn Xaa Cys Arg Asp Xaa Ile Gln Arg Met His Leu
1               5                   10                  15

Arg Gln Tyr Xaa Leu Leu
            20

<210> 170
<211> 20
<212> PRT
<213> Artificial Sequence

<220>
<223> synthetic peptide

<220>
<221> MOD_RES
<222> (1)..(1)

<223> ACETYLATION

<220>
<221> MOD_RES
<222> (4)..(4)
<223> modified "Pra" which is linked to modified "Azk" at position (8) via the reaction of the alkynyl side chain of a "Pra" with the azidated side-chain of an "Azk"

<220>
<221> MOD_RES
<222> (8)..(8)
<223> modified "Azk" which is linked to modified "Pra" at position (4) via the reaction of the azidated side-chain of an "Azk" with the alkynyl side-chain of a "Pra"

<400> 170

Lys Phe Asn Xaa Cys Arg Asp Xaa Ile Gln Arg Met His Leu Arg Gln
1 5 10 15

Tyr Asp Leu Leu
20

<210> 171
<211> 22
<212> PRT
<213> Artificial Sequence

<220>
<223> synthetic peptide

<220>
<221> MOD_RES
<222> (1)..(1)
<223> ACETYLATION

<220>
<221> MOD_RES
<222> (6)..(6)
<223> modified "Pra" which is linked to modified "Azk" at position (10) via the reaction of the alkynyl side chain of a "Pra" with the azidated side-chain of an "Azk"

<220>
<221> MOD_RES
<222> (10)..(10)
<223> modified "Azk" which is linked to modified "Pra" at position (6) via the reaction of the azidated side-chain of an "Azk" with the alkynyl side-chain of a "Pra"

<400> 171

Lys Lys Lys Phe Asn Xaa Cys Arg Asp Xaa Ile Gln Arg Met His Leu
1 5 10 15

Arg Gln Tyr Asp Leu Leu
20

<210> 172
<211> 22
<212> PRT
<213> Artificial Sequence

<220>
<223> synthetic peptide

<220>
<221> MOD_RES
<222> (1)..(1)
<223> ACETYLATION

<220>
<221> MOD_RES
<222> (6)..(6)
<223> modified "Pra" which is linked to modified "Azk" at position (10) via the reaction of the alkynyl side chain of a "Pra" with the azidated side-chain of an "Azk"

<220>
<221> MOD_RES
<222> (10)..(10)
<223> modified "Azk" which is linked to modified "Pra" at position (6) via the reaction of the azidated side-chain of an "Azk" with the alkynyl side-chain of a "Pra"

<400> 172

```
Lys Lys Lys Phe Asn Xaa Cys Arg Asp Xaa Ile Gln Arg Met His Leu
1               5                   10                  15

Arg Gln Trp Glu Leu Leu
            20
```

<210> 173
<211> 22
<212> PRT
<213> Artificial Sequence

<220>
<223> synthetic peptide

<220>
<221> MOD_RES
<222> (1)..(1)
<223> ACETYLATION

<220>
<221> MOD_RES
<222> (6)..(6)
<223> modified "Pra" which is linked to modified "Azk" at position (10) via the reaction of the alkynyl side chain of a "Pra" with the azidated side-chain of an "Azk"

<220>
<221> MOD_RES
<222> (10)..(10)

<223> modified "Azk" which is linked to modified "Pra" at position (6) via the reaction of the azidated side-chain of an "Azk" with the alkynyl side-chain of a "Pra"

<220>
<221> MISC_FEATURE
<222> (19)..(19)
<223> Xaa is 1-naphthalanine (1-Nal)

<400> 173

```
Lys Lys Lys Phe Asn Xaa Cys Arg Asp Xaa Ile Gln Arg Met His Leu
1               5                   10                  15


Arg Gln Xaa Glu Leu Leu
            20
```

<210> 174
<211> 22
<212> PRT
<213> Artificial Sequence

<220>
<223> synthetic peptide

<220>
<221> MOD_RES
<222> (1)..(1)
<223> ACETYLATION

<220>
<221> MOD_RES
<222> (6)..(6)
<223> modified "Pra" which is linked to modified "Azk" at position (10) via the reaction of the alkynyl side chain of a "Pra" with the azidated side-chain of an "Azk"

<220>
<221> MOD_RES
<222> (10)..(10)
<223> modified "Azk" which is linked to modified "Pra" at position (6) via the reaction of the azidated side-chain of an "Azk" with the alkynyl side-chain of a "Pra"

<220>
<221> MISC_FEATURE
<222> (19)..(19)
<223> Xaa is 2-naphthalanine (2-Nal)

<400> 174

```
Lys Lys Lys Phe Asn Xaa Cys Arg Asp Xaa Ile Gln Arg Met His Leu
1               5                   10                  15


Arg Gln Xaa Glu Leu Leu
            20
```

<210> 175

<211> 22
<212> PRT
<213> Artificial Sequence

<220>
<223> synthetic peptide

<220>
<221> MOD_RES
<222> (1)..(1)
<223> ACETYLATION

<220>
<221> MOD_RES
<222> (6)..(6)
<223> modified "Pra" which is linked to modified "Azk" at position (10) via the reaction of the alkynyl side chain of a "Pra" with the azidated side-chain of an "Azk"

<220>
<221> MOD_RES
<222> (10)..(10)
<223> modified "Azk" which is linked to modified "Pra" at position (6) via the reaction of the azidated side-chain of an "Azk" with the alkynyl side-chain of a "Pra"

<400> 175

Lys Lys Lys Phe Asn Xaa Cys Arg Asp Xaa Ile Gln Arg Met His Leu
1               5                   10                  15

Arg Gln Tyr Glu Phe Leu
            20

<210> 176
<211> 15
<212> PRT
<213> Artificial Sequence

<220>
<223> synthetic peptide

<220>
<221> MOD_RES
<222> (1)..(1)
<223> ACETYLATION

<220>
<221> MOD_RES
<222> (7)..(7)
<223> modified "Azk" which is linked to modified "Pra" at position (11) via the reaction of the azidated side-chain of an "Azk" with the alkynyl side-chain of a "Pra"

<220>
<221> MOD_RES
<222> (11)..(11)
<223> modified "Pra" which is linked to modified "Azk" at position (7) via the reaction of the alkynyl side chain of a "Pra" with the

azidated side-chain of an "Azk"

<220>
<221> MISC_FEATURE
<222> (14)..(14)
<223> Xaa is cyclohexylalanine (Cha)

<400> 176

Arg Asp Ile Ile Gln Arg Xaa His Leu Arg Xaa Tyr Glu Xaa Leu
1               5                   10                  15

<210> 177
<211> 18
<212> PRT
<213> Artificial Sequence

<220>
<223> synthetic peptide

<220>
<221> MOD_RES
<222> (1)..(1)
<223> ACETYLATION

<220>
<221> MOD_RES
<222> (10)..(10)
<223> modified "Azk" which is linked to modified "Pra" at position (14) via the reaction of the azidated side-chain of an "Azk" with the alkynyl side-chain of a "Pra"

<220>
<221> MOD_RES
<222> (14)..(14)
<223> modified "Pra" which is linked to modified "Azk" at position (10) via the reaction of the alkynyl side chain of a "Pra" with the azidated side-chain of an "Azk"

<220>
<221> MISC_FEATURE
<222> (17)..(17)
<223> Xaa is cyclohexylalanine (Cha)

<400> 177

Lys Lys Lys Arg Asp Ile Ile Gln Arg Xaa His Leu Arg Xaa Tyr Glu
1               5                   10                  15

Xaa Leu

<210> 178
<211> 21
<212> PRT
<213> Artificial Sequence

<220>
<223> synthetic peptide

<220>
<221> MOD_RES
<222> (1)..(1)
<223> ACETYLATION

<220>
<221> MOD_RES
<222> (5)..(5)
<223> modified "Pra" which is linked to modified "Azk" at position (9) via the reaction of the alkynyl side chain of a "Pra" with the azidated side-chain of an "Azk"

<220>
<221> MOD_RES
<222> (9)..(9)
<223> modified "Azk" which is linked to modified "Pra" at position (5) via the reaction of the azidated side-chain of an "Azk" with the alkynyl side-chain of a "Pra"

<220>
<221> MISC_FEATURE
<222> (20)..(20)
<223> Xaa is cyclohexylalanine (Cha)

<400> 178

```
Lys Lys Phe Asn Xaa Cys Arg Asp Xaa Ile Gln Arg Met His Leu Arg
1               5                   10                  15


Gln Tyr Glu Xaa Leu
            20
```

<210> 179
<211> 15
<212> PRT
<213> Artificial Sequence

<220>
<223> synthetic peptide

<220>
<221> MOD_RES
<222> (7)..(7)
<223> modified "Azk" which is linked to modified "Pra" at position (11) via the reaction of the azidated side-chain of an "Azk" with the alkynyl side-chain of a "Pra"

<220>
<221> MOD_RES
<222> (11)..(11)
<223> modified "Pra" which is linked to modified "Azk" at position (7) via the reaction of the alkynyl side chain of a "Pra" with the azidated side-chain of an "Azk"

<400> 179

```
Arg Asp Ile Ile Gln Arg Xaa His Leu Arg Xaa Tyr Glu Leu Leu
1               5                   10                  15
```

```
<210>  180
<211>  19
<212>  PRT
<213>  Artificial Sequence

<220>
<223>  synthetic peptide


<220>
<221>  MOD_RES
<222>  (1)..(1)
<223>  ACETYLATION

<220>
<221>  MOD_RES
<222>  (3)..(3)
<223>  modified "Pra" which is linked to modified "Azk" at position (7)
       via the reaction of the alkynyl side chain of a "Pra" with the
       azidated side-chain of an "Azk"

<220>
<221>  MOD_RES
<222>  (7)..(7)
<223>  modified "Azk" which is linked to modified "Pra" at position (3)
       via the reaction of the azidated side-chain of an "Azk" with the
       alkynyl side-chain of a "Pra"

<220>
<221>  MISC_FEATURE
<222>  (16)..(16)
<223>  Xaa is 2-naphthalanine (2-Nal)

<400>  180

Phe Asn Xaa Cys Arg Asp Xaa Ile Gln Arg Met His Leu Arg Gln Xaa
1               5                   10                  15


Glu Leu Leu
```